# EUROPEAN PATENT SPECIFICATION

(11) **EP 3 209 773 B1**
(45) Date of publication and mention of the grant of the patent: **18.03.2020**
(21) Application number: 15793724.4
(22) Date of filing: 23.10.2015
(51) Int. Cl.: C12N 9/48, C12P 21/06, A23J 3/34, A23K 10/14

(54) **PROLINE TOLERANT TRIPEPTIDYL PEPTIDASES AND USES THEREOF**
PROLINTOLERANTE TRIPEPTIDYLPEPTIDASEN UND VERWENDUNGEN DAVON
TRIPEPTIDYL-PEPTIDASES TOLÉRANT LA PROLINE ET LEURS UTILISATIONS

(30) Priority: 24.10.2014 US 201462068282 P; 24.10.2014 US 201462068264 P; 24.10.2014 US 201462068243 P; 17.12.2014 US 201462093301 P
(43) Date of publication of application: 30.08.2017
(73) Proprietor: DuPont Nutrition Biosciences ApS, 1411 Copenhagen K (DK)
(72) Inventor: KRAGH, Karsten Matthias, DK-8270 Hoejbjerg (DK); MEINJOHANNS, Ernst, 1801 Frederiksberg C (DK); MIASNIKOV, Andrei, Union City, California 94587 (US); MA, Maria, Fremont, California 94539 (US); EISELE, Thomas, 8362 Hørning (DK); HAANING, Svend, 8464 Galten (DK); DEGN, Peter Edvard, 8250 Egå (DK); BAK, Steffen Yde, 8200 Aarhus N (DK)
(74) Representative: D Young & Co LLP
(86) International application number: PCT/EP2015/074600
(87) International publication number: WO 2016/062855

(56) References cited:
- WO-A2-02/068623
- WO-A2-2011/077359
- WO-A2-2013/102674
- US-A- 5 821 104
- US-B2- 7 972 808
- U. REICHARD ET AL: "Sedolisins, a New Class of Secreted Proteases from Aspergillus fumigatus with Endoprotease or Tripeptidyl-Peptidase Activity at Acidic pHs", APPLIED AND ENVIRONMENTAL MICROBIOLOGY, vol. 72, no. 3, 1 March 2006 (2006-03-01), pages 1739-1748, XP055000173, ISSN: 0099-2240, DOI: 10.1128/AEM.72.3.1739-1748.2006 cited in the application
- DATABASE UniProt [Online] 26 June 2013 (2013-06-26), "SubName: Full=Tripeptidyl-peptidase sed4 {ECO:0000313|EMBL:EMT69593.1};", XP002753006, retrieved from EBI accession no. UNIPROT:N1RSG2 Database accession no. N1RSG2
- TIMO STRESSLER ET AL: "Characterization of the Recombinant Exopeptidases PepX and PepN from Lactobacillus helveticus ATCC 12046 Important for Food Protein Hydrolysis", PLOS ONE, vol. 8, no. 7, 19 July 2013 (2013-07-19), page e70055, XP55256260, DOI: 10.1371/journal.pone.0070055
- BYUN T ET AL: "SYNERGISTIC ACTION OF AN X-PROLYL DIPEPTIDYL AMINOPEPTIDASE AND A NON-SPECIFIC AMINOPEPTIDASE IN PROTEIN HYDROLYSIS", JOURNAL OF AGRICULTURAL AND FOOD CHEMISTRY, AMERICAN CHEMICAL SOCIETY, US, vol. 49, no. 4, 1 January 2001 (2001-01-01), pages 2061-2063, XP001109774, ISSN: 0021-8561, DOI: 10.1021/JF001091M

### SEQUENCE LISTING

<110> DuPont Nutrition Biosciences ApS
<120> Method
<130> P104228PCT
<150> US 62/068264
   <151> 2014-10-24
<150> US 62/093301
   <151> 2014-12-17
<150> US 62/068282
   <151> 2014-10-24
<150> US 62/068243
   <151> 2014-10-24
<160> 123
<170> PatentIn version 3.5
<210> 1
   <211> 612
   <212> PRT
   <213> Trichoderma reesei
<400> 1
   Met Ala Lys Leu Ser Thr Leu Arg Leu Ala Ser Leu Leu Ser Leu Val
   1               5                   10                  15
   Ser Val Gln Val Ser Ala Ser Val His Leu Leu Glu Ser Leu Glu Lys
               20                  25                  30
   Leu Pro His Gly Trp Lys Ala Ala Glu Thr Pro Ser Pro Ser Ser Gln
           35                  40                  45
   Ile Val Leu Gln Val Ala Leu Thr Gln Gln Asn Ile Asp Gln Leu Glu
       50                  55                  60
   Ser Arg Leu Ala Ala Val Ser Thr Pro Thr Ser Ser Thr Tyr Gly Lys
   65                  70                  75                  80
   Tyr Leu Asp Val Asp Glu Ile Asn Ser Ile Phe Ala Pro Ser Asp Ala
                   85                  90                  95
   Ser Ser Ser Ala Val Glu Ser Trp Leu Gln Ser His Gly Val Thr Ser
               100                 105                 110
   Tyr Thr Lys Gln Gly Ser Ser Ile Trp Phe Gln Thr Asn Ile Ser Thr
           115                 120                 125
   Ala Asn Ala Met Leu Ser Thr Asn Phe His Thr Tyr Ser Asp Leu Thr
       130                 135                 140
   Gly Ala Lys Lys Val Arg Thr Leu Lys Tyr Ser Ile Pro Glu Ser Leu
   145                 150                 155                 160
   Ile Gly His Val Asp Leu Ile Ser Pro Thr Thr Tyr Phe Gly Thr Thr
                   165                 170                 175
   Lys Ala Met Arg Lys Leu Lys Ser Ser Gly Val Ser Pro Ala Ala Asp
               180                 185                 190
   Ala Leu Ala Ala Arg Gln Glu Pro Ser Ser Cys Lys Gly Thr Leu Val
           195                 200                 205
   Phe Glu Gly Glu Thr Phe Asn Val Phe Gln Pro Asp Cys Leu Arg Thr
       210                 215                 220
   Glu Tyr Ser Val Asp Gly Tyr Thr Pro Ser Val Lys Ser Gly Ser Arg
   225                 230                 235                 240
   Ile Gly Phe Gly Ser Phe Leu Asn Glu Ser Ala Ser Phe Ala Asp Gln
                   245                 250                 255
   Ala Leu Phe Glu Lys His Phe Asn Ile Pro Ser Gln Asn Phe Ser Val
               260                 265                 270
   Val Leu Ile Asn Gly Gly Thr Asp Leu Pro Gln Pro Pro Ser Asp Ala
           275                 280                 285
   Asn Asp Gly Glu Ala Asn Leu Asp Ala Gln Thr Ile Leu Thr Ile Ala
       290                 295                 300
   His Pro Leu Pro Ile Thr Glu Phe Ile Thr Ala Gly Ser Pro Pro Tyr
   305                 310                 315                 320
   Phe Pro Asp Pro Val Glu Pro Ala Gly Thr Pro Asn Glu Asn Glu Pro
                   325                 330                 335
   Tyr Leu Gln Tyr Tyr Glu Phe Leu Leu Ser Lys Ser Asn Ala Glu Ile
               340                 345                 350
   Pro Gln Val Ile Thr Asn Ser Tyr Gly Asp Glu Glu Gln Thr Val Pro
           355                 360                 365
   Arg Ser Tyr Ala Val Arg Val Cys Asn Leu Ile Gly Leu Leu Gly Leu
       370                 375                 380
   Arg Gly Ile Ser Val Leu His Ser Ser Gly Asp Glu Gly Val Gly Ala
   385                 390                 395                 400
   Ser Cys Val Ala Thr Asn Ser Thr Thr Pro Gln Phe Asn Pro Ile Phe
                   405                 410                 415
   Pro Ala Thr Cys Pro Tyr Val Thr Ser Val Gly Gly Thr Val Ser Phe
               420                 425                 430
   Asn Pro Glu Val Ala Trp Ala Gly Ser Ser Gly Gly Phe Ser Tyr Tyr
           435                 440                 445
   Phe Ser Arg Pro Trp Tyr Gln Gln Glu Ala Val Gly Thr Tyr Leu Glu
       450                 455                 460
   Lys Tyr Val Ser Ala Glu Thr Lys Lys Tyr Tyr Gly Pro Tyr Val Asp
   465                 470                 475                 480
   Phe Ser Gly Arg Gly Phe Pro Asp Val Ala Ala His Ser Val Ser Pro
                   485                 490                 495
   Asp Tyr Pro Val Phe Gln Gly Gly Glu Leu Thr Pro Ser Gly Gly Thr
               500                 505                 510
   Ser Ala Ala Ser Pro Val Val Ala Ala Ile Val Ala Leu Leu Asn Asp
           515                 520                 525
   Ala Arg Leu Arg Glu Gly Lys Pro Thr Leu Gly Phe Leu Asn Pro Leu
       530                 535                 540
   Ile Tyr Leu His Ala Ser Lys Gly Phe Thr Asp Ile Thr Ser Gly Gln
   545                 550                 555                 560
   Ser Glu Gly Cys Asn Gly Asn Asn Thr Gln Thr Gly Ser Pro Leu Pro
                   565                 570                 575
   Gly Ala Gly Phe Ile Ala Gly Ala His Trp Asn Ala Thr Lys Gly Trp
               580                 585                 590
   Asp Pro Thr Thr Gly Phe Gly Val Pro Asn Leu Lys Lys Leu Leu Ala
           595                 600                 605
   Leu Val Arg Phe
       610
<210> 2
   <211> 590
   <212> PRT
   <213> Trichoderma reesei
<400> 2
   Ser Val His Leu Leu Glu Ser Leu Glu Lys Leu Pro His Gly Trp Lys
   1               5                   10                  15
   Ala Ala Glu Thr Pro Ser Pro Ser Ser Gln Ile Val Leu Gln Val Ala
               20                  25                  30
   Leu Thr Gln Gln Asn Ile Asp Gln Leu Glu Ser Arg Leu Ala Ala Val
           35                  40                  45
   Ser Thr Pro Thr Ser Ser Thr Tyr Gly Lys Tyr Leu Asp Val Asp Glu
       50                  55                  60
   Ile Asn Ser Ile Phe Ala Pro Ser Asp Ala Ser Ser Ser Ala Val Glu
   65                  70                  75                  80
   Ser Trp Leu Gln Ser His Gly Val Thr Ser Tyr Thr Lys Gln Gly Ser
                   85                  90                  95
   Ser Ile Trp Phe Gln Thr Asn Ile Ser Thr Ala Asn Ala Met Leu Ser
               100                 105                 110
   Thr Asn Phe His Thr Tyr Ser Asp Leu Thr Gly Ala Lys Lys Val Arg
           115                 120                 125
   Thr Leu Lys Tyr Ser Ile Pro Glu Ser Leu Ile Gly His Val Asp Leu
       130                 135                 140
   Ile Ser Pro Thr Thr Tyr Phe Gly Thr Thr Lys Ala Met Arg Lys Leu
   145                 150                 155                 160
   Lys Ser Ser Gly Val Ser Pro Ala Ala Asp Ala Leu Ala Ala Arg Gln
                   165                 170                 175
   Glu Pro Ser Ser Cys Lys Gly Thr Leu Val Phe Glu Gly Glu Thr Phe
               180                 185                 190
   Asn Val Phe Gln Pro Asp Cys Leu Arg Thr Glu Tyr Ser Val Asp Gly
           195                 200                 205
   Tyr Thr Pro Ser Val Lys Ser Gly Ser Arg Ile Gly Phe Gly Ser Phe
       210                 215                 220
   Leu Asn Glu Ser Ala Ser Phe Ala Asp Gln Ala Leu Phe Glu Lys His
   225                 230                 235                 240
   Phe Asn Ile Pro Ser Gln Asn Phe Ser Val Val Leu Ile Asn Gly Gly
   245 250 255
   Thr Asp Leu Pro Gln Pro Pro Ser Asp Ala Asn Asp Gly Glu Ala Asn
   260 265 270
   Leu Asp Ala Gln Thr Ile Leu Thr Ile Ala His Pro Leu Pro Ile Thr
   275 280 285
   Glu Phe Ile Thr Ala Gly Ser Pro Pro Tyr Phe Pro Asp Pro Val Glu
   290 295 300
   Pro Ala Gly Thr Pro Asn Glu Asn Glu Pro Tyr Leu Gln Tyr Tyr Glu
   305 310 315 320
   Phe Leu Leu Ser Lys Ser Asn Ala Glu Ile Pro Gln Val Ile Thr Asn
   325 330 335
   Ser Tyr Gly Asp Glu Glu Gln Thr Val Pro Arg Ser Tyr Ala Val Arg
   340 345 350
   Val Cys Asn Leu Ile Gly Leu Leu Gly Leu Arg Gly Ile Ser Val Leu
   355 360 365
   His Ser Ser Gly Asp Glu Gly Val Gly Ala Ser Cys Val Ala Thr Asn
   370 375 380
   Ser Thr Thr Pro Gln Phe Asn Pro Ile Phe Pro Ala Thr Cys Pro Tyr
   385 390 395 400
   Val Thr Ser Val Gly Gly Thr Val Ser Phe Asn Pro Glu Val Ala Trp
   405 410 415
   Ala Gly Ser Ser Gly Gly Phe Ser Tyr Tyr Phe Ser Arg Pro Trp Tyr
   420 425 430
   Gln Gln Glu Ala Val Gly Thr Tyr Leu Glu Lys Tyr Val Ser Ala Glu
   435 440 445
   Thr Lys Lys Tyr Tyr Gly Pro Tyr Val Asp Phe Ser Gly Arg Gly Phe
   450 455 460
   Pro Asp Val Ala Ala His Ser Val Ser Pro Asp Tyr Pro Val Phe Gln
   465 470 475 480
   Gly Gly Glu Leu Thr Pro Ser Gly Gly Thr Ser Ala Ala Ser Pro Val
   485 490 495
   Val Ala Ala Ile Val Ala Leu Leu Asn Asp Ala Arg Leu Arg Glu Gly
   500 505 510
   Lys Pro Thr Leu Gly Phe Leu Asn Pro Leu Ile Tyr Leu His Ala Ser
   515 520 525
   Lys Gly Phe Thr Asp Ile Thr Ser Gly Gln Ser Glu Gly Cys Asn Gly
   530 535 540
   Asn Asn Thr Gln Thr Gly Ser Pro Leu Pro Gly Ala Gly Phe Ile Ala
   545 550 555 560
   Gly Ala His Trp Asn Ala Thr Lys Gly Trp Asp Pro Thr Thr Gly Phe
   565 570 575
   Gly Val Pro Asn Leu Lys Lys Leu Leu Ala Leu Val Arg Phe
   580 585 590
<210> 3
   <211> 578
   <212> PRT
   <213> Aspergillus oryzae
<400> 3
   Glu Ala Phe Glu Lys Leu Ser Ala Val Pro Lys Gly Trp His Tyr Ser
   1               5                   10                  15
   Ser Thr Pro Lys Gly Asn Thr Glu Val Cys Leu Lys Ile Ala Leu Ala
               20                  25                  30
   Gln Lys Asp Ala Ala Gly Phe Glu Lys Thr Val Leu Glu Met Ser Asp
           35                  40                  45
   Pro Asp His Pro Ser Tyr Gly Gln His Phe Thr Thr His Asp Glu Met
       50                  55                  60
   Lys Arg Met Leu Leu Pro Arg Asp Asp Thr Val Asp Ala Val Arg Gln
   65                  70                  75                  80
   Trp Leu Glu Asn Gly Gly Val Thr Asp Phe Thr Gln Asp Ala Asp Trp
                   85                  90                  95
   Ile Asn Phe Cys Thr Thr Val Asp Thr Ala Asn Lys Leu Leu Asn Ala
               100                 105                 110
   Gln Phe Lys Trp Tyr Val Ser Asp Val Lys His Ile Arg Arg Leu Arg
   115 120 125
   Thr Leu Gln Tyr Asp Val Pro Glu Ser Val Thr Pro His Ile Asn Thr
   130 135 140
   Ile Gln Pro Thr Thr Arg Phe Gly Lys Ile Ser Pro Lys Lys Ala Val
   145 150 155 160
   Thr His Ser Lys Pro Ser Gln Leu Asp Val Thr Ala Leu Ala Ala Ala
   165 170 175
   Val Val Ala Lys Asn Ile Ser His Cys Asp Ser Ile Ile Thr Pro Thr
   180 185 190
   Cys Leu Lys Glu Leu Tyr Asn Ile Gly Asp Tyr Gln Ala Asp Ala Asn
   195 200 205
   Ser Gly Ser Lys Ile Ala Phe Ala Ser Tyr Leu Glu Glu Tyr Ala Arg
   210 215 220
   Tyr Ala Asp Leu Glu Asn Phe Glu Asn Tyr Leu Ala Pro Trp Ala Lys
   225 230 235 240
   Gly Gln Asn Phe Ser Val Thr Thr Phe Asn Gly Gly Leu Asn Asp Gln
   245 250 255
   Asn Ser Ser Ser Asp Ser Gly Glu Ala Asn Leu Asp Leu Gln Tyr Ile
   260 265 270
   Leu Gly Val Ser Ala Pro Leu Pro Val Thr Glu Phe Ser Thr Gly Gly
   275 280 285
   Arg Gly Pro Leu Val Pro Asp Leu Thr Gln Pro Asp Pro Asn Ser Asn
   290 295 300
   Ser Asn Glu Pro Tyr Leu Glu Phe Phe Gln Asn Val Leu Lys Leu Asp
   305 310 315 320
   Gln Lys Asp Leu Pro Gln Val Ile Ser Thr Ser Tyr Gly Glu Asn Glu
   325 330 335
   Gln Glu Ile Pro Glu Lys Tyr Ala Arg Thr Val Cys Asn Leu Ile Ala
   340 345 350
   Gln Leu Gly Ser Arg Gly Val Ser Val Leu Phe Ser Ser Gly Asp Ser
   355 360 365
   Gly Val Gly Glu Gly Cys Met Thr Asn Asp Gly Thr Asn Arg Thr His
       370                 375                 380
   Phe Pro Pro Gln Phe Pro Ala Ala Cys Pro Trp Val Thr Ser Val Gly
   385                 390                 395                 400
   Ala Thr Phe Lys Thr Thr Pro Glu Arg Gly Thr Tyr Phe Ser Ser Gly
                   405                 410                 415
   Gly Phe Ser Asp Tyr Trp Pro Arg Pro Glu Trp Gln Asp Glu Ala Val
               420                 425                 430
   Ser Ser Tyr Leu Glu Thr Ile Gly Asp Thr Phe Lys Gly Leu Tyr Asn
           435                 440                 445
   Ser Ser Gly Arg Ala Phe Pro Asp Val Ala Ala Gln Gly Met Asn Phe
       450                 455                 460
   Ala Val Tyr Asp Lys Gly Thr Leu Gly Glu Phe Asp Gly Thr Ser Ala
   465                 470                 475                 480
   Ser Ala Pro Ala Phe Ser Ala Val Ile Ala Leu Leu Asn Asp Ala Arg
                   485                 490                 495
   Leu Arg Ala Gly Lys Pro Thr Leu Gly Phe Leu Asn Pro Trp Leu Tyr
               500                 505                 510
   Lys Thr Gly Arg Gln Gly Leu Gln Asp Ile Thr Leu Gly Ala Ser Ile
           515                 520                 525
   Gly Cys Thr Gly Arg Ala Arg Phe Gly Gly Ala Pro Asp Gly Gly Pro
       530                 535                 540
   Val Val Pro Tyr Ala Ser Trp Asn Ala Thr Gln Gly Trp Asp Pro Val
   545                 550                 555                 560
   Thr Gly Leu Gly Thr Pro Asp Phe Ala Glu Leu Lys Lys Leu Ala Leu
                   565                 570                 575
   Gly Asn
<210> 4
   <211> 574
   <212> PRT
   <213> Phaeosphaeria nodorum
<400> 4
   Glu Pro Phe Glu Lys Leu Phe Ser Thr Pro Glu Gly Trp Lys Met Gln
   1               5                   10                  15
   Gly Leu Ala Thr Asn Glu Gln Ile Val Lys Leu Gln Ile Ala Leu Gln
               20                  25                  30
   Gln Gly Asp Val Ala Gly Phe Glu Gln His Val Ile Asp Ile Ser Thr
           35                  40                  45
   Pro Ser His Pro Ser Tyr Gly Ala His Tyr Gly Ser His Glu Glu Met
       50                  55                  60
   Lys Arg Met Ile Gln Pro Ser Ser Glu Thr Val Ala Ser Val Ser Ala
   65                  70                  75                  80
   Trp Leu Lys Ala Ala Gly Ile Asn Asp Ala Glu Ile Asp Ser Asp Trp
                   85                  90                  95
   Val Thr Phe Lys Thr Thr Val Gly Val Ala Asn Lys Met Leu Asp Thr
               100                 105                 110
   Lys Phe Ala Trp Tyr Val Ser Glu Glu Ala Lys Pro Arg Lys Val Leu
           115                 120                 125
   Arg Thr Leu Glu Tyr Ser Val Pro Asp Asp Val Ala Glu His Ile Asn
       130                 135                 140
   Leu Ile Gln Pro Thr Thr Arg Phe Ala Ala Ile Arg Gln Asn His Glu
   145                 150                 155                 160
   Val Ala His Glu Ile Val Gly Leu Gln Phe Ala Ala Leu Ala Asn Asn
                   165                 170                 175
   Thr Val Asn Cys Asp Ala Thr Ile Thr Pro Gln Cys Leu Lys Thr Leu
               180                 185                 190
   Tyr Lys Ile Asp Tyr Lys Ala Asp Pro Lys Ser Gly Ser Lys Val Ala
           195                 200                 205
   Phe Ala Ser Tyr Leu Glu Gln Tyr Ala Arg Tyr Asn Asp Leu Ala Leu
       210                 215                 220
   Phe Glu Lys Ala Phe Leu Pro Glu Ala Val Gly Gln Asn Phe Ser Val
   225                 230                 235                 240
   Val Gln Phe Ser Gly Gly Leu Asn Asp Gln Asn Thr Thr Gln Asp Ser
                   245                 250                 255
   Gly Glu Ala Asn Leu Asp Leu Gln Tyr Ile Val Gly Val Ser Ala Pro
               260                 265                 270
   Leu Pro Val Thr Glu Phe Ser Thr Gly Gly Arg Gly Pro Trp Val Ala
           275                 280                 285
   Asp Leu Asp Gln Pro Asp Glu Ala Asp Ser Ala Asn Glu Pro Tyr Leu
       290                 295                 300
   Glu Phe Leu Gln Gly Val Leu Lys Leu Pro Gln Ser Glu Leu Pro Gln
   305                 310                 315                 320
   Val Ile Ser Thr Ser Tyr Gly Glu Asn Glu Gln Ser Val Pro Lys Ser
                   325                 330                 335
   Tyr Ala Leu Ser Val Cys Asn Leu Phe Ala Gln Leu Gly Ser Arg Gly
               340                 345                 350
   Val Ser Val Ile Phe Ser Ser Gly Asp Ser Gly Pro Gly Ser Ala Cys
           355                 360                 365
   Gln Ser Asn Asp Gly Lys Asn Thr Thr Lys Phe Gln Pro Gln Tyr Pro
       370                 375                 380
   Ala Ala Cys Pro Phe Val Thr Ser Val Gly Ser Thr Arg Tyr Leu Asn
   385                 390                 395                 400
   Glu Thr Ala Thr Gly Phe Ser Ser Gly Gly Phe Ser Asp Tyr Trp Lys
                   405                 410                 415
   Arg Pro Ser Tyr Gln Asp Asp Ala Val Lys Ala Tyr Phe His His Leu
               420                 425                 430
   Gly Glu Lys Phe Lys Pro Tyr Phe Asn Arg His Gly Arg Gly Phe Pro
           435                 440                 445
   Asp Val Ala Thr Gln Gly Tyr Gly Phe Arg Val Tyr Asp Gln Gly Lys
       450                 455                 460
   Leu Lys Gly Leu Gln Gly Thr Ser Ala Ser Ala Pro Ala Phe Ala Gly
   465                 470                 475                 480
   Val Ile Gly Leu Leu Asn Asp Ala Arg Leu Lys Ala Lys Lys Pro Thr
                   485                 490                 495
   Leu Gly Phe Leu Asn Pro Leu Leu Tyr Ser Asn Ser Asp Ala Leu Asn
               500                 505                 510
   Asp Ile Val Leu Gly Gly Ser Lys Gly Cys Asp Gly His Ala Arg Phe
           515                 520                 525
   Asn Gly Pro Pro Asn Gly Ser Pro Val Ile Pro Tyr Ala Gly Trp Asn
       530                 535                 540
   Ala Thr Ala Gly Trp Asp Pro Val Thr Gly Leu Gly Thr Pro Asn Phe
   545                 550                 555                 560
   Pro Lys Leu Leu Lys Ala Ala Val Pro Ser Arg Tyr Arg Ala
                   565                 570
<210> 5
   <211> 590
   <212> PRT
   <213> Trichoderma atroviride
<400> 5
   Asn Ala Ala Val Leu Leu Asp Ser Leu Asp Lys Val Pro Val Gly Trp
   1               5                   10                  15
   Gln Ala Ala Ser Ala Pro Ala Pro Ser Ser Lys Ile Thr Leu Gln Val
               20                  25                  30
   Ala Leu Thr Gln Gln Asn Ile Asp Gln Leu Glu Ser Lys Leu Ala Ala
           35                  40                  45
   Val Ser Thr Pro Asn Ser Ser Asn Tyr Gly Lys Tyr Leu Asp Val Asp
       50                  55                  60
   Glu Ile Asn Gln Ile Phe Ala Pro Ser Ser Ala Ser Thr Ala Ala Val
   65                  70                  75                  80
   Glu Ser Trp Leu Lys Ser Tyr Gly Val Asp Tyr Lys Val Gln Gly Ser
                   85                  90                  95
   Ser Ile Trp Phe Gln Thr Asp Val Ser Thr Ala Asn Lys Met Leu Ser
               100                 105                 110
   Thr Asn Phe His Thr Tyr Thr Asp Ser Val Gly Ala Lys Lys Val Arg
           115                 120                 125
   Thr Leu Gln Tyr Ser Val Pro Glu Thr Leu Ala Asp His Ile Asp Leu
       130                 135                 140
   Ile Ser Pro Thr Thr Tyr Phe Gly Thr Ser Lys Ala Met Arg Ala Leu
   145                 150                 155                 160
   Lys Ile Gln Asn Ala Ala Ser Ala Val Ser Pro Leu Ala Ala Arg Gln
                   165                 170                 175
   Glu Pro Ser Ser Cys Lys Gly Thr Ile Glu Phe Glu Asn Arg Thr Phe
               180                 185                 190
   Asn Val Phe Gln Pro Asp Cys Leu Arg Thr Glu Tyr Ser Val Asn Gly
           195                 200                 205
   Tyr Lys Pro Ser Ala Lys Ser Gly Ser Arg Ile Gly Phe Gly Ser Phe
       210                 215                 220
   Leu Asn Gln Ser Ala Ser Ser Ser Asp Leu Ala Leu Phe Glu Lys His
   225                 230                 235                 240
   Phe Gly Phe Ala Ser Gln Gly Phe Ser Val Glu Leu Ile Asn Gly Gly
                   245                 250                 255
   Ser Asn Pro Gln Pro Pro Thr Asp Ala Asn Asp Gly Glu Ala Asn Leu
               260                 265                 270
   Asp Ala Gln Asn Ile Val Ser Phe Val Gln Pro Leu Pro Ile Thr Glu
           275                 280                 285
   Phe Ile Ala Gly Gly Thr Ala Pro Tyr Phe Pro Asp Pro Val Glu Pro
       290                 295                 300
   Ala Gly Thr Pro Asp Glu Asn Glu Pro Tyr Leu Glu Tyr Tyr Glu Tyr
   305                 310                 315                 320
   Leu Leu Ser Lys Ser Asn Lys Glu Leu Pro Gln Val Ile Thr Asn Ser
                   325                 330                 335
   Tyr Gly Asp Glu Glu Gln Thr Val Pro Gln Ala Tyr Ala Val Arg Val
               340                 345                 350
   Cys Asn Leu Ile Gly Leu Met Gly Leu Arg Gly Ile Ser Ile Leu Glu
           355                 360                 365
   Ser Ser Gly Asp Glu Gly Val Gly Ala Ser Cys Leu Ala Thr Asn Ser
       370                 375                 380
   Thr Thr Thr Pro Gln Phe Asn Pro Ile Phe Pro Ala Thr Cys Pro Tyr
   385                 390                 395                 400
   Val Thr Ser Val Gly Gly Thr Val Ser Phe Asn Pro Glu Val Ala Trp
   405 410 415
   Asp Gly Ser Ser Gly Gly Phe Ser Tyr Tyr Phe Ser Arg Pro Trp Tyr
   420 425 430
   Gln Glu Ala Ala Val Gly Thr Tyr Leu Asn Lys Tyr Val Ser Glu Glu
   435 440 445
   Thr Lys Glu Tyr Tyr Lys Ser Tyr Val Asp Phe Ser Gly Arg Gly Phe
   450 455 460
   Pro Asp Val Ala Ala His Ser Val Ser Pro Asp Tyr Pro Val Phe Gln
   465 470 475 480
   Gly Gly Glu Leu Thr Pro Ser Gly Gly Thr Ser Ala Ala Ser Pro Ile
   485 490 495
   Val Ala Ser Val Ile Ala Leu Leu Asn Asp Ala Arg Leu Arg Ala Gly
   500 505 510
   Lys Pro Ala Leu Gly Phe Leu Asn Pro Leu Ile Tyr Gly Tyr Ala Tyr
   515 520 525
   Lys Gly Phe Thr Asp Ile Thr Ser Gly Gln Ala Val Gly Cys Asn Gly
   530 535 540
   Asn Asn Thr Gln Thr Gly Gly Pro Leu Pro Gly Ala Gly Val Ile Pro
   545 550 555 560
   Gly Ala Phe Trp Asn Ala Thr Lys Gly Trp Asp Pro Thr Thr Gly Phe
   565 570 575
   Gly Val Pro Asn Phe Lys Lys Leu Leu Glu Leu Val Arg Tyr
   580 585 590
<210> 6
   <211> 580
   <212> PRT
   <213> Arthroderma benhamiae
<400> 6
   Lys Pro Thr Pro Gly Ala Ser His Lys Val Ile Glu His Leu Asp Phe
   1               5                   10                  15
   Val Pro Glu Gly Trp Gln Met Val Gly Ala Ala Asp Pro Ala Ala Ile
               20                  25                  30
   Ile Asp Phe Trp Leu Ala Ile Glu Arg Glu Asn Pro Glu Lys Leu Tyr
           35                  40                  45
   Asp Thr Ile Tyr Asp Val Ser Thr Pro Gly Arg Ala Gln Tyr Gly Lys
       50                  55                  60
   His Leu Lys Arg Glu Glu Leu Asp Asp Leu Leu Arg Pro Arg Ala Glu
   65                  70                  75                  80
   Thr Ser Glu Ser Ile Ile Asn Trp Leu Thr Asn Gly Gly Val Asn Pro
                   85                  90                  95
   Gln His Ile Arg Asp Glu Gly Asp Trp Val Arg Phe Ser Thr Asn Val
               100                 105                 110
   Lys Thr Ala Glu Thr Leu Met Asn Thr Arg Phe Asn Val Phe Lys Asp
           115                 120                 125
   Asn Leu Asn Ser Val Ser Lys Ile Arg Thr Leu Glu Tyr Ser Val Pro
       130                 135                 140
   Val Ala Ile Ser Ala His Val Gln Met Ile Gln Pro Thr Thr Leu Phe
   145                 150                 155                 160
   Gly Arg Gln Lys Pro Gln Asn Ser Leu Ile Leu Asn Pro Leu Thr Lys
                   165                 170                 175
   Asp Leu Glu Ser Met Ser Val Glu Glu Phe Ala Ala Ser Gln Cys Arg
               180                 185                 190
   Ser Leu Val Thr Thr Ala Cys Leu Arg Glu Leu Tyr Gly Leu Gly Asp
           195                 200                 205
   Arg Val Thr Gln Ala Arg Asp Asp Asn Arg Ile Gly Val Ser Gly Phe
       210                 215                 220
   Leu Glu Glu Tyr Ala Gln Tyr Arg Asp Leu Glu Leu Phe Leu Ser Arg
   225                 230                 235                 240
   Phe Glu Pro Ser Ala Lys Gly Phe Asn Phe Ser Glu Gly Leu Ile Ala
                   245                 250                 255
   Gly Gly Lys Asn Thr Gln Gly Gly Pro Gly Ser Ser Thr Glu Ala Asn
               260                 265                 270
   Leu Asp Met Gln Tyr Val Val Gly Leu Ser His Lys Ala Lys Val Thr
           275                 280                 285
   Tyr Tyr Ser Thr Ala Gly Arg Gly Pro Leu Ile Pro Asp Leu Ser Gln
       290                 295                 300
   Pro Ser Gln Ala Ser Asn Asn Asn Glu Pro Tyr Leu Glu Gln Leu Arg
   305                 310                 315                 320
   Tyr Leu Val Lys Leu Pro Lys Asn Gln Leu Pro Ser Val Leu Thr Thr
                   325                 330                 335
   Ser Tyr Gly Asp Thr Glu Gln Ser Leu Pro Ala Ser Tyr Thr Lys Ala
               340                 345                 350
   Thr Cys Asp Leu Phe Ala Gln Leu Gly Thr Met Gly Val Ser Val Ile
           355                 360                 365
   Phe Ser Ser Gly Asp Thr Gly Pro Gly Ser Ser Cys Gln Thr Asn Asp
       370                 375                 380
   Gly Lys Asn Ala Thr Arg Phe Asn Pro Ile Tyr Pro Ala Ser Cys Pro
   385                 390                 395                 400
   Phe Val Thr Ser Ile Gly Gly Thr Val Gly Thr Gly Pro Glu Arg Ala
                   405                 410                 415
   Val Ser Phe Ser Ser Gly Gly Phe Ser Asp Arg Phe Pro Arg Pro Gln
               420                 425                 430
   Tyr Gln Asp Asn Ala Val Lys Asp Tyr Leu Lys Ile Leu Gly Asn Gln
           435                 440                 445
   Trp Ser Gly Leu Phe Asp Pro Asn Gly Arg Ala Phe Pro Asp Ile Ala
       450                 455                 460
   Ala Gln Gly Ser Asn Tyr Ala Val Tyr Asp Lys Gly Arg Met Thr Gly
   465                 470                 475                 480
   Val Ser Gly Thr Ser Ala Ser Ala Pro Ala Met Ala Ala Ile Ile Ala
                   485                 490                 495
   Gln Leu Asn Asp Phe Arg Leu Ala Lys Gly Ser Pro Val Leu Gly Phe
               500                 505                 510
   Leu Asn Pro Trp Ile Tyr Ser Lys Gly Phe Ser Gly Phe Thr Asp Ile
           515                 520                 525
   Val Asp Gly Gly Ser Arg Gly Cys Thr Gly Tyr Asp Ile Tyr Ser Gly
       530                 535                 540
   Leu Lys Ala Lys Lys Val Pro Tyr Ala Ser Trp Asn Ala Thr Lys Gly
   545                 550                 555                 560
   Trp Asp Pro Val Thr Gly Phe Gly Thr Pro Asn Phe Gln Ala Leu Thr
                   565                 570                 575
   Lys Val Leu Pro
               580
<210> 7
   <211> 580
   <212> PRT
   <213> Fusarium graminearum
<400> 7
   Lys Ser Tyr Ser His His Ala Glu Ala Pro Lys Gly Trp Lys Val Asp
   1               5                   10                  15
   Asp Thr Ala Arg Val Ala Ser Thr Gly Lys Gln Gln Val Phe Ser Ile
               20                  25                  30
   Ala Leu Thr Met Gln Asn Val Asp Gln Leu Glu Ser Lys Leu Leu Asp
           35                  40                  45
   Leu Ser Ser Pro Asp Ser Lys Asn Tyr Gly Gln Trp Met Ser Gln Lys
       50                  55                  60
   Asp Val Thr Thr Ala Phe Tyr Pro Ser Lys Glu Ala Val Ser Ser Val
   65                  70                  75                  80
   Thr Lys Trp Leu Lys Ser Lys Gly Val Lys His Tyr Asn Val Asn Gly
                   85                  90                  95
   Gly Phe Ile Asp Phe Ala Leu Asp Val Lys Gly Ala Asn Ala Leu Leu
               100                 105                 110
   Asp Ser Asp Tyr Gln Tyr Tyr Thr Lys Glu Gly Gln Thr Lys Leu Arg
           115                 120                 125
   Thr Leu Ser Tyr Ser Ile Pro Asp Asp Val Ala Glu His Val Gln Phe
       130                 135                 140
   Val Asp Pro Ser Thr Asn Phe Gly Gly Thr Leu Ala Phe Ala Pro Val
   145                 150                 155                 160
   Thr His Pro Ser Arg Thr Leu Thr Glu Arg Lys Asn Lys Pro Thr Lys
                   165                 170                 175
   Ser Thr Val Asp Ala Ser Cys Gln Thr Ser Ile Thr Pro Ser Cys Leu
               180                 185                 190
   Lys Gln Met Tyr Asn Ile Gly Asp Tyr Thr Pro Lys Val Glu Ser Gly
           195                 200                 205
   Ser Thr Ile Gly Phe Ser Ser Phe Leu Gly Glu Ser Ala Ile Tyr Ser
       210                 215                 220
   Asp Val Phe Leu Phe Glu Glu Lys Phe Gly Ile Pro Thr Gln Asn Phe
   225                 230                 235                 240
   Thr Thr Val Leu Ile Asn Asn Gly Thr Asp Asp Gln Asn Thr Ala His
                   245                 250                 255
   Lys Asn Phe Gly Glu Ala Asp Leu Asp Ala Glu Asn Ile Val Gly Ile
               260                 265                 270
   Ala His Pro Leu Pro Phe Thr Gln Tyr Ile Thr Gly Gly Ser Pro Pro
           275                 280                 285
   Phe Leu Pro Asn Ile Asp Gln Pro Thr Ala Ala Asp Asn Gln Asn Glu
       290                 295                 300
   Pro Tyr Val Pro Phe Phe Arg Tyr Leu Leu Ser Gln Lys Glu Val Pro
   305                 310                 315                 320
   Ala Val Val Ser Thr Ser Tyr Gly Asp Glu Glu Asp Ser Val Pro Arg
                   325                 330                 335
   Glu Tyr Ala Thr Met Thr Cys Asn Leu Ile Gly Leu Leu Gly Leu Arg
               340                 345                 350
   Gly Ile Ser Val Ile Phe Ser Ser Gly Asp Ile Gly Val Gly Ala Gly
           355                 360                 365
   Cys Leu Gly Pro Asp His Lys Thr Val Glu Phe Asn Ala Ile Phe Pro
       370                 375                 380
   Ala Thr Cys Pro Tyr Leu Thr Ser Val Gly Gly Thr Val Asp Val Thr
   385                 390                 395                 400
   Pro Glu Ile Ala Trp Glu Gly Ser Ser Gly Gly Phe Ser Lys Tyr Phe
                   405                 410                 415
   Pro Arg Pro Ser Tyr Gln Asp Lys Ala Val Lys Thr Tyr Met Lys Thr
               420                 425                 430
   Val Ser Lys Gln Thr Lys Lys Tyr Tyr Gly Pro Tyr Thr Asn Trp Glu
   435 440 445
   Gly Arg Gly Phe Pro Asp Val Ala Gly His Ser Val Ser Pro Asn Tyr
   450 455 460
   Glu Val Ile Tyr Ala Gly Lys Gln Ser Ala Ser Gly Gly Thr Ser Ala
   465 470 475 480
   Ala Ala Pro Val Trp Ala Ala Ile Val Gly Leu Leu Asn Asp Ala Arg
   485 490 495
   Phe Arg Ala Gly Lys Pro Ser Leu Gly Trp Leu Asn Pro Leu Val Tyr
   500 505 510
   Lys Tyr Gly Pro Lys Val Leu Thr Asp Ile Thr Gly Gly Tyr Ala Ile
   515 520 525
   Gly Cys Asp Gly Asn Asn Thr Gln Ser Gly Lys Pro Glu Pro Ala Gly
   530 535 540
   Ser Gly Ile Val Pro Gly Ala Arg Trp Asn Ala Thr Ala Gly Trp Asp
   545 550 555 560
   Pro Val Thr Gly Tyr Gly Thr Pro Asp Phe Gly Lys Leu Lys Asp Leu
   565 570 575
   Val Leu Ser Phe
   580
<210> 8
   <211> 603
   <212> PRT
   <213> Acremonium alcalophilum
<400> 8
   Ala Val Val Ile Arg Ala Ala Val Leu Pro Asp Ala Val Lys Leu Met
   1 5 10 15
   Gly Lys Ala Met Pro Asp Asp Ile Ile Ser Leu Gln Phe Ser Leu Lys
   20 25 30
   Gln Gln Asn Ile Asp Gln Leu Glu Thr Arg Leu Arg Ala Val Ser Asp
   35 40 45
   Pro Ser Ser Pro Glu Tyr Gly Gln Tyr Met Ser Glu Ser Glu Val Asn
   50 55 60
   Glu Phe Phe Lys Pro Arg Asp Asp Ser Phe Ala Glu Val Ile Asp Trp
   65 70 75 80
   Val Ala Ala Ser Gly Phe Gln Asp Ile His Leu Thr Pro Gln Ala Ala
   85 90 95
   Ala Ile Asn Leu Ala Ala Thr Val Glu Thr Ala Asp Gln Leu Leu Gly
   100 105 110
   Ala Asn Phe Ser Trp Phe Asp Val Asp Gly Thr Arg Lys Leu Arg Thr
   115 120 125
   Leu Glu Tyr Thr Ile Pro Asp Arg Leu Ala Asp His Val Asp Leu Ile
   130 135 140
   Ser Pro Thr Thr Tyr Phe Gly Arg Ala Arg Leu Asp Gly Pro Arg Glu
   145 150 155 160
   Thr Pro Thr Arg Leu Asp Lys Arg Gln Arg Asp Pro Val Ala Asp Lys
   165 170 175
   Ala Tyr Phe His Leu Lys Trp Asp Arg Gly Thr Ser Asn Cys Asp Leu
   180 185 190
   Val Ile Thr Pro Pro Cys Leu Glu Ala Ala Tyr Asn Tyr Lys Asn Tyr
   195 200 205
   Met Pro Asp Pro Asn Ser Gly Ser Arg Val Ser Phe Thr Ser Phe Leu
   210 215 220
   Glu Gln Ala Ala Gln Gln Ser Asp Leu Thr Lys Phe Leu Ser Leu Thr
   225 230 235 240
   Gly Leu Asp Arg Leu Arg Pro Pro Ser Ser Lys Pro Ala Ser Phe Asp
   245 250 255
   Thr Val Leu Ile Asn Gly Gly Glu Thr His Gln Gly Thr Pro Pro Asn
   260 265 270
   Lys Thr Ser Glu Ala Asn Leu Asp Val Gln Trp Leu Ala Ala Val Ile
   275 280 285
   Lys Ala Arg Leu Pro Ile Thr Gln Trp Ile Thr Gly Gly Arg Pro Pro
   290 295 300
   Phe Val Pro Asn Leu Arg Leu Arg His Glu Lys Asp Asn Thr Asn Glu
   305 310 315 320
   Pro Tyr Leu Glu Phe Phe Glu Tyr Leu Val Arg Leu Pro Ala Arg Asp
   325 330 335
   Leu Pro Gln Val Ile Ser Asn Ser Tyr Ala Glu Asp Glu Gln Thr Val
   340 345 350
   Pro Glu Ala Tyr Ala Arg Arg Val Cys Asn Leu Ile Gly Ile Met Gly
   355 360 365
   Leu Arg Gly Val Thr Val Leu Thr Ala Ser Gly Asp Ser Gly Val Gly
   370 375 380
   Ala Pro Cys Arg Ala Asn Asp Gly Ser Asp Arg Leu Glu Phe Ser Pro
   385 390 395 400
   Gln Phe Pro Thr Ser Cys Pro Tyr Ile Thr Ala Val Gly Gly Thr Glu
   405 410 415
   Gly Trp Asp Pro Glu Val Ala Trp Glu Ala Ser Ser Gly Gly Phe Ser
   420 425 430
   His Tyr Phe Leu Arg Pro Trp Tyr Gln Ala Asn Ala Val Glu Lys Tyr
   435 440 445
   Leu Asp Glu Glu Leu Asp Pro Ala Thr Arg Ala Tyr Tyr Asp Gly Asn
   450 455 460
   Gly Phe Val Gln Phe Ala Gly Arg Ala Tyr Pro Asp Leu Ser Ala His
   465 470 475 480
   Ser Ser Ser Pro Arg Tyr Ala Tyr Ile Asp Lys Leu Ala Pro Gly Leu
   485 490 495
   Thr Gly Gly Thr Ser Ala Ser Cys Pro Val Val Ala Gly Ile Val Gly
   500 505 510
   Leu Leu Asn Asp Ala Arg Leu Arg Arg Gly Leu Pro Thr Met Gly Phe
   515 520 525
   Ile Asn Pro Trp Leu Tyr Thr Arg Gly Phe Glu Ala Leu Gln Asp Val
   530 535 540
   Thr Gly Gly Arg Ala Ser Gly Cys Gln Gly Ile Asp Leu Gln Arg Gly
   545 550 555 560
   Thr Arg Val Pro Gly Ala Gly Ile Ile Pro Trp Ala Ser Trp Asn Ala
   565 570 575
   Thr Pro Gly Trp Asp Pro Ala Thr Gly Leu Gly Leu Pro Asp Phe Trp
   580 585 590
   Ala Met Arg Gly Leu Ala Leu Gly Arg Gly Thr
   595 600
<210> 9
   <211> 614
   <212> PRT
   <213> Sodiomyces alkalinus
<400> 9
   Ala Val Val Ile Arg Ala Ala Pro Leu Pro Glu Ser Val Lys Leu Val
   1 5 10 15
   Arg Lys Ala Ala Ala Glu Asp Gly Ile Asn Leu Gln Leu Ser Leu Lys
   20 25 30
   Arg Gln Asn Met Asp Gln Leu Glu Lys Phe Leu Arg Ala Val Ser Asp
   35 40 45
   Pro Phe Ser Pro Lys Tyr Gly Gln Tyr Met Ser Asp Ala Glu Val His
   50 55 60
   Glu Ile Phe Arg Pro Thr Glu Asp Ser Phe Asp Gln Val Ile Asp Trp
   65 70 75 80
   Leu Thr Lys Ser Gly Phe Gly Asn Leu His Ile Thr Pro Gln Ala Ala
   85 90 95
   Ala Ile Asn Val Ala Thr Thr Val Glu Thr Ala Asp Gln Leu Phe Gly
   100 105 110
   Ala Asn Phe Ser Trp Phe Asp Val Asp Gly Thr Pro Lys Leu Arg Thr
   115 120 125
   Gly Glu Tyr Thr Ile Pro Asp Arg Leu Val Glu His Val Asp Leu Val
   130 135 140
   Ser Pro Thr Thr Tyr Phe Gly Arg Met Arg Pro Pro Pro Arg Gly Asp
   145 150 155 160
   Gly Val Asn Asp Trp Ile Thr Glu Asn Ser Pro Glu Gln Pro Ala Pro
   165 170 175
   Leu Asn Lys Arg Asp Thr Lys Thr Glu Ser Asp Gln Ala Arg Asp His
               180                 185                 190
   Pro Ser Trp Asp Ser Arg Thr Pro Asp Cys Ala Thr Ile Ile Thr Pro
           195                 200                 205
   Pro Cys Leu Glu Thr Ala Tyr Asn Tyr Lys Gly Tyr Ile Pro Asp Pro
       210                 215                 220
   Lys Ser Gly Ser Arg Val Ser Phe Thr Ser Phe Leu Glu Gln Ala Ala
   225                 230                 235                 240
   Gln Gln Ala Asp Leu Thr Lys Phe Leu Ser Leu Thr Arg Leu Glu Gly
                   245                 250                 255
   Phe Arg Thr Pro Ala Ser Lys Lys Lys Thr Phe Lys Thr Val Leu Ile
               260                 265                 270
   Asn Gly Gly Glu Ser His Glu Gly Val His Lys Lys Ser Lys Thr Ser
           275                 280                 285
   Glu Ala Asn Leu Asp Val Gln Trp Leu Ala Ala Val Thr Gln Thr Lys
       290                 295                 300
   Leu Pro Ile Thr Gln Trp Ile Thr Gly Gly Arg Pro Pro Phe Val Pro
   305                 310                 315                 320
   Asn Leu Arg Ile Pro Thr Pro Glu Ala Asn Thr Asn Glu Pro Tyr Leu
                   325                 330                 335
   Glu Phe Leu Glu Tyr Leu Phe Arg Leu Pro Asp Lys Asp Leu Pro Gln
               340                 345                 350
   Val Ile Ser Asn Ser Tyr Ala Glu Asp Glu Gln Ser Val Pro Glu Ala
           355                 360                 365
   Tyr Ala Arg Arg Val Cys Gly Leu Leu Gly Ile Met Gly Leu Arg Gly
       370                 375                 380
   Val Thr Val Leu Thr Ala Ser Gly Asp Ser Gly Val Gly Ala Pro Cys
   385                 390                 395                 400
   Arg Ala Asn Asp Gly Ser Gly Arg Glu Glu Phe Ser Pro Gln Phe Pro
                   405                 410                 415
   Ser Ser Cys Pro Tyr Ile Thr Thr Val Gly Gly Thr Gln Ala Trp Asp
               420                 425                 430
   Pro Glu Val Ala Trp Lys Gly Ser Ser Gly Gly Phe Ser Asn Tyr Phe
   435 440 445
   Pro Arg Pro Trp Tyr Gln Val Ala Ala Val Glu Lys Tyr Leu Glu Glu
   450 455 460
   Gln Leu Asp Pro Ala Ala Arg Glu Tyr Tyr Glu Glu Asn Gly Phe Val
   465 470 475 480
   Arg Phe Ala Gly Arg Ala Phe Pro Asp Leu Ser Ala His Ser Ser Ser
   485 490 495
   Pro Lys Tyr Ala Tyr Val Asp Lys Arg Val Pro Gly Leu Thr Gly Gly
   500 505 510
   Thr Ser Ala Ser Cys Pro Val Val Ala Gly Ile Val Gly Leu Leu Asn
   515 520 525
   Asp Ala Arg Leu Arg Arg Gly Leu Pro Thr Met Gly Phe Ile Asn Pro
   530 535 540
   Trp Leu Tyr Ala Lys Gly Tyr Gln Ala Leu Glu Asp Val Thr Gly Gly
   545 550 555 560
   Ala Ala Val Gly Cys Gln Gly Ile Asp Ile Gln Thr Gly Lys Arg Val
   565 570 575
   Pro Gly Ala Gly Ile Ile Pro Gly Ala Ser Trp Asn Ala Thr Pro Asp
   580 585 590
   Trp Asp Pro Ala Thr Gly Leu Gly Leu Pro Asn Phe Trp Ala Met Arg
   595 600 605
   Glu Leu Ala Leu Glu Asp
   610
<210> 10
   <211> 575
   <212> PRT
   <213> Aspergillus kawachii
<400> 10
   Val Val His Glu Lys Leu Ala Ala Val Pro Ser Gly Trp His His Leu
   1 5 10 15
   Glu Asp Ala Gly Ser Asp His Gln Ile Ser Leu Ser Ile Ala Leu Ala
   20 25 30
   Arg Lys Asn Leu Asp Gln Leu Glu Ser Lys Leu Lys Asp Leu Ser Thr
           35                  40                  45
   Pro Gly Glu Ser Gln Tyr Gly Gln Trp Leu Asp Gln Glu Glu Val Asp
       50                  55                  60
   Thr Leu Phe Pro Val Ala Ser Asp Lys Ala Val Ile Ser Trp Leu Arg
   65                  70                  75                  80
   Ser Ala Asn Ile Thr His Ile Ala Arg Gln Gly Ser Leu Val Asn Phe
                   85                  90                  95
   Ala Thr Thr Val Asp Lys Val Asn Lys Leu Leu Asn Thr Thr Phe Ala
               100                 105                 110
   Tyr Tyr Gln Arg Gly Ser Ser Gln Arg Leu Arg Thr Thr Glu Tyr Ser
           115                 120                 125
   Ile Pro Asp Asp Leu Val Asp Ser Ile Asp Leu Ile Ser Pro Thr Thr
       130                 135                 140
   Phe Phe Gly Lys Glu Lys Thr Ser Ala Gly Leu Thr Gln Arg Ser Gln
   145                 150                 155                 160
   Lys Val Asp Asn His Val Ala Lys Arg Ser Asn Ser Ser Ser Cys Ala
                   165                 170                 175
   Asp Thr Ile Thr Leu Ser Cys Leu Lys Glu Met Tyr Asn Phe Gly Asn
               180                 185                 190
   Tyr Thr Pro Ser Ala Ser Ser Gly Ser Lys Leu Gly Phe Ala Ser Phe
           195                 200                 205
   Leu Asn Glu Ser Ala Ser Tyr Ser Asp Leu Ala Lys Phe Glu Arg Leu
       210                 215                 220
   Phe Asn Leu Pro Ser Gln Asn Phe Ser Val Glu Leu Ile Asn Gly Gly
   225                 230                 235                 240
   Val Asn Asp Gln Asn Gln Ser Thr Ala Ser Leu Thr Glu Ala Asp Leu
                   245                 250                 255
   Asp Val Glu Leu Leu Val Gly Val Gly His Pro Leu Pro Val Thr Glu
               260                 265                 270
   Phe Ile Thr Ser Gly Glu Pro Pro Phe Ile Pro Asp Pro Asp Glu Pro
           275                 280                 285
   Ser Ala Ala Asp Asn Glu Asn Glu Pro Tyr Leu Gln Tyr Tyr Glu Tyr
   290 295 300
   Leu Leu Ser Lys Pro Asn Ser Ala Leu Pro Gln Val Ile Ser Asn Ser
   305 310 315 320
   Tyr Gly Asp Asp Glu Gln Thr Val Pro Glu Tyr Tyr Ala Lys Arg Val
   325 330 335
   Cys Asn Leu Ile Gly Leu Val Gly Leu Arg Gly Ile Ser Val Leu Glu
   340 345 350
   Ser Ser Gly Asp Glu Gly Ile Gly Ser Gly Cys Arg Thr Thr Asp Gly
   355 360 365
   Thr Asn Ser Thr Gln Phe Asn Pro Ile Phe Pro Ala Thr Cys Pro Tyr
   370 375 380
   Val Thr Ala Val Gly Gly Thr Met Ser Tyr Ala Pro Glu Ile Ala Trp
   385 390 395 400
   Glu Ala Ser Ser Gly Gly Phe Ser Asn Tyr Phe Glu Arg Ala Trp Phe
   405 410 415
   Gln Lys Glu Ala Val Gln Asn Tyr Leu Ala Asn His Ile Thr Asn Glu
   420 425 430
   Thr Lys Gln Tyr Tyr Ser Gln Phe Ala Asn Phe Ser Gly Arg Gly Phe
   435 440 445
   Pro Asp Val Ser Ala His Ser Phe Glu Pro Ser Tyr Glu Val Ile Phe
   450 455 460
   Tyr Gly Ala Arg Tyr Gly Ser Gly Gly Thr Ser Ala Ala Cys Pro Leu
   465 470 475 480
   Phe Ser Ala Leu Val Gly Met Leu Asn Asp Ala Arg Leu Arg Ala Gly
   485 490 495
   Lys Ser Thr Leu Gly Phe Leu Asn Pro Leu Leu Tyr Ser Lys Gly Tyr
   500 505 510
   Lys Ala Leu Thr Asp Val Thr Ala Gly Gln Ser Ile Gly Cys Asn Gly
   515 520 525
   Ile Asp Pro Gln Ser Asp Glu Ala Val Ala Gly Ala Gly Ile Ile Pro
   530 535 540
   Trp Ala His Trp Asn Ala Thr Val Gly Trp Asp Pro Val Thr Gly Leu
   545 550 555 560
   Gly Leu Pro Asp Phe Glu Lys Leu Arg Gln Leu Val Leu Ser Leu
   565 570 575
<210> 11
   <211> 582
   <212> PRT
   <213> Talaromyces stipitatus
<400> 11
   Ala Ala Ala Leu Val Gly His Glu Ser Leu Ala Ala Leu Pro Val Gly
   1               5                   10                  15
   Trp Asp Lys Val Ser Thr Pro Ala Ala Gly Thr Asn Ile Gln Leu Ser
               20                  25                  30
   Val Ala Leu Ala Leu Gln Asn Ile Glu Gln Leu Glu Asp His Leu Lys
           35                  40                  45
   Ser Val Ser Thr Pro Gly Ser Ala Ser Tyr Gly Gln Tyr Leu Asp Ser
       50                  55                  60
   Asp Gly Ile Ala Ala Gln Tyr Gly Pro Ser Asp Ala Ser Val Glu Ala
   65                  70                  75                  80
   Val Thr Asn Trp Leu Lys Glu Ala Gly Val Thr Asp Ile Tyr Asn Asn
                   85                  90                  95
   Gly Gln Ser Ile His Phe Ala Thr Ser Val Ser Lys Ala Asn Ser Leu
               100                 105                 110
   Leu Gly Ala Asp Phe Asn Tyr Tyr Ser Asp Gly Ser Ala Thr Lys Leu
           115                 120                 125
   Arg Thr Leu Ala Tyr Ser Val Pro Ser Asp Leu Lys Glu Ala Ile Asp
       130                 135                 140
   Leu Val Ser Pro Thr Thr Tyr Phe Gly Lys Thr Thr Ala Ser Arg Ser
   145                 150                 155                 160
   Ile Gln Ala Tyr Lys Asn Lys Arg Ala Ser Thr Thr Ser Lys Ser Gly
                   165                 170                 175
   Ser Ser Ser Val Gln Val Ser Ala Ser Cys Gln Thr Ser Ile Thr Pro
   180 185 190
   Ala Cys Leu Lys Gln Met Tyr Asn Val Gly Asn Tyr Thr Pro Ser Val
   195 200 205
   Ala His Gly Ser Arg Val Gly Phe Gly Ser Phe Leu Asn Gln Ser Ala
   210 215 220
   Ile Phe Asp Asp Leu Phe Thr Tyr Glu Lys Val Asn Asp Ile Pro Ser
   225 230 235 240
   Gln Asn Phe Thr Lys Val Ile Ile Ala Asn Ala Ser Asn Ser Gln Asp
   245 250 255
   Ala Ser Asp Gly Asn Tyr Gly Glu Ala Asn Leu Asp Val Gln Asn Ile
   260 265 270
   Val Gly Ile Ser His Pro Leu Pro Val Thr Glu Phe Leu Thr Gly Gly
   275 280 285
   Ser Pro Pro Phe Val Ala Ser Leu Asp Thr Pro Thr Asn Gln Asn Glu
   290 295 300
   Pro Tyr Ile Pro Tyr Tyr Glu Tyr Leu Leu Ser Gln Lys Asn Glu Asp
   305 310 315 320
   Leu Pro Gln Val Ile Ser Asn Ser Tyr Gly Asp Asp Glu Gln Ser Val
   325 330 335
   Pro Tyr Lys Tyr Ala Ile Arg Ala Cys Asn Leu Ile Gly Leu Thr Gly
   340 345 350
   Leu Arg Gly Ile Ser Val Leu Glu Ser Ser Gly Asp Leu Gly Val Gly
   355 360 365
   Ala Gly Cys Arg Ser Asn Asp Gly Lys Asn Lys Thr Gln Phe Asp Pro
   370 375 380
   Ile Phe Pro Ala Thr Cys Pro Tyr Val Thr Ser Val Gly Gly Thr Gln
   385 390 395 400
   Ser Val Thr Pro Glu Ile Ala Trp Val Ala Ser Ser Gly Gly Phe Ser
   405 410 415
   Asn Tyr Phe Pro Arg Thr Trp Tyr Gln Glu Pro Ala Ile Gln Thr Tyr
   420 425 430
   Leu Gly Leu Leu Asp Asp Glu Thr Lys Thr Tyr Tyr Ser Gln Tyr Thr
   435 440 445
   Asn Phe Glu Gly Arg Gly Phe Pro Asp Val Ser Ala His Ser Leu Thr
   450 455 460
   Pro Asp Tyr Gln Val Val Gly Gly Gly Tyr Leu Gln Pro Ser Gly Gly
   465 470 475 480
   Thr Ser Ala Ala Ser Pro Val Phe Ala Gly Ile Ile Ala Leu Leu Asn
   485 490 495
   Asp Ala Arg Leu Ala Ala Gly Lys Pro Thr Leu Gly Phe Leu Asn Pro
   500 505 510
   Phe Phe Tyr Leu Tyr Gly Tyr Lys Gly Leu Asn Asp Ile Thr Gly Gly
   515 520 525
   Gln Ser Val Gly Cys Asn Gly Ile Asn Gly Gln Thr Gly Ala Pro Val
   530 535 540
   Pro Gly Gly Gly Ile Val Pro Gly Ala Ala Trp Asn Ser Thr Thr Gly
   545 550 555 560
   Trp Asp Pro Ala Thr Gly Leu Gly Thr Pro Asp Phe Gln Lys Leu Lys
   565 570 575
   Glu Leu Val Leu Ser Phe
   580
<210> 12
   <211> 579
   <212> PRT
   <213> Fusarium oxysporum
<400> 12
   Lys Ser Phe Ser His His Ala Glu Ala Pro Gln Gly Trp Gln Val Gln
   1 5 10 15
   Lys Thr Ala Lys Val Ala Ser Asn Thr Gln His Val Phe Ser Leu Ala
   20 25 30
   Leu Thr Met Gln Asn Val Asp Gln Leu Glu Ser Lys Leu Leu Asp Leu
   35 40 45
   Ser Ser Pro Asp Ser Ala Asn Tyr Gly Asn Trp Leu Ser His Asp Glu
   50 55 60
   Leu Thr Ser Thr Phe Ser Pro Ser Lys Glu Ala Val Ala Ser Val Thr
   65                  70                  75                  80
   Lys Trp Leu Lys Ser Lys Gly Ile Lys His Tyr Lys Val Asn Gly Ala
                   85                  90                  95
   Phe Ile Asp Phe Ala Ala Asp Val Glu Lys Ala Asn Thr Leu Leu Gly
               100                 105                 110
   Gly Asp Tyr Gln Tyr Tyr Thr Lys Asp Gly Gln Thr Lys Leu Arg Thr
           115                 120                 125
   Leu Ser Tyr Ser Ile Pro Asp Asp Val Ala Gly His Val Gln Phe Val
       130                 135                 140
   Asp Pro Ser Thr Asn Phe Gly Gly Thr Val Ala Phe Asn Pro Val Pro
   145                 150                 155                 160
   His Pro Ser Arg Thr Leu Gln Glu Arg Lys Val Ser Pro Ser Lys Ser
                   165                 170                 175
   Thr Val Asp Ala Ser Cys Gln Thr Ser Ile Thr Pro Ser Cys Leu Lys
               180                 185                 190
   Gln Met Tyr Asn Ile Gly Asp Tyr Thr Pro Asp Ala Lys Ser Gly Ser
           195                 200                 205
   Glu Ile Gly Phe Ser Ser Phe Leu Gly Gln Ala Ala Ile Tyr Ser Asp
       210                 215                 220
   Val Phe Lys Phe Glu Glu Leu Phe Gly Ile Pro Lys Gln Asn Tyr Thr
   225                 230                 235                 240
   Thr Ile Leu Ile Asn Asn Gly Thr Asp Asp Gln Asn Thr Ala His Gly
                   245                 250                 255
   Asn Phe Gly Glu Ala Asn Leu Asp Ala Glu Asn Ile Val Gly Ile Ala
               260                 265                 270
   His Pro Leu Pro Phe Lys Gln Tyr Ile Thr Gly Gly Ser Pro Pro Phe
           275                 280                 285
   Val Pro Asn Ile Asp Gln Pro Thr Glu Lys Asp Asn Gln Asn Glu Pro
       290                 295                 300
   Tyr Val Pro Phe Phe Arg Tyr Leu Leu Gly Gln Lys Asp Leu Pro Ala
   305                 310                 315                 320
   Val Ile Ser Thr Ser Tyr Gly Asp Glu Glu Asp Ser Val Pro Arg Glu
   325 330 335
   Tyr Ala Thr Leu Thr Cys Asn Met Ile Gly Leu Leu Gly Leu Arg Gly
   340 345 350
   Ile Ser Val Ile Phe Ser Ser Gly Asp Ile Gly Val Gly Ser Gly Cys
   355 360 365
   Leu Ala Pro Asp Tyr Lys Thr Val Glu Phe Asn Ala Ile Phe Pro Ala
   370 375 380
   Thr Cys Pro Tyr Leu Thr Ser Val Gly Gly Thr Val Asp Val Thr Pro
   385 390 395 400
   Glu Ile Ala Trp Glu Gly Ser Ser Gly Gly Phe Ser Lys Tyr Phe Pro
   405 410 415
   Arg Pro Ser Tyr Gln Asp Lys Ala Ile Lys Lys Tyr Met Lys Thr Val
   420 425 430
   Ser Lys Glu Thr Lys Lys Tyr Tyr Gly Pro Tyr Thr Asn Trp Glu Gly
   435 440 445
   Arg Gly Phe Pro Asp Val Ala Gly His Ser Val Ala Pro Asp Tyr Glu
   450 455 460
   Val Ile Tyr Asn Gly Lys Gln Ala Arg Ser Gly Gly Thr Ser Ala Ala
   465 470 475 480
   Ala Pro Val Trp Ala Ala Ile Val Gly Leu Leu Asn Asp Ala Arg Phe
   485 490 495
   Lys Ala Gly Lys Lys Ser Leu Gly Trp Leu Asn Pro Leu Ile Tyr Lys
   500 505 510
   His Gly Pro Lys Val Leu Thr Asp Ile Thr Gly Gly Tyr Ala Ile Gly
   515 520 525
   Cys Asp Gly Asn Asn Thr Gln Ser Gly Lys Pro Glu Pro Ala Gly Ser
   530 535 540
   Gly Leu Val Pro Gly Ala Arg Trp Asn Ala Thr Ala Gly Trp Asp Pro
   545 550 555 560
   Thr Thr Gly Tyr Gly Thr Pro Asn Phe Gln Lys Leu Lys Asp Leu Val
   565 570 575
   Leu Ser Leu
<210> 13
   <211> 590
   <212> PRT
   <213> Trichoderma virens
<400> 13
   Ser Val Leu Val Glu Ser Leu Glu Lys Leu Pro His Gly Trp Lys Ala
   1               5                   10                  15
   Ala Ser Ala Pro Ser Pro Ser Ser Gln Ile Thr Leu Gln Val Ala Leu
               20                  25                  30
   Thr Gln Gln Asn Ile Asp Gln Leu Glu Ser Arg Leu Ala Ala Val Ser
           35                  40                  45
   Thr Pro Asn Ser Lys Thr Tyr Gly Asn Tyr Leu Asp Leu Asp Glu Ile
       50                  55                  60
   Asn Glu Ile Phe Ala Pro Ser Asp Ala Ser Ser Ala Ala Val Glu Ser
   65                  70                  75                  80
   Trp Leu His Ser His Gly Val Thr Lys Tyr Thr Lys Gln Gly Ser Ser
                   85                  90                  95
   Ile Trp Phe Gln Thr Glu Val Ser Thr Ala Asn Ala Met Leu Ser Thr
               100                 105                 110
   Asn Phe His Thr Tyr Ser Asp Ala Ala Gly Val Lys Lys Leu Arg Thr
           115                 120                 125
   Leu Gln Tyr Ser Ile Pro Glu Ser Leu Val Gly His Val Asp Leu Ile
       130                 135                 140
   Ser Pro Thr Thr Tyr Phe Gly Thr Ser Asn Ala Met Arg Ala Leu Arg
   145                 150                 155                 160
   Ser Lys Ser Val Ala Ser Val Ala Gln Ser Val Ala Ala Arg Gln Glu
                   165                 170                 175
   Pro Ser Ser Cys Lys Gly Thr Leu Val Phe Glu Gly Arg Thr Phe Asn
               180                 185                 190
   Val Phe Gln Pro Asp Cys Leu Arg Thr Glu Tyr Asn Val Asn Gly Tyr
           195                 200                 205
   Thr Pro Ser Ala Lys Ser Gly Ser Arg Ile Gly Phe Gly Ser Phe Leu
   210 215 220
   Asn Gln Ser Ala Ser Phe Ser Asp Leu Ala Leu Phe Glu Lys His Phe
   225 230 235 240
   Gly Phe Ser Ser Gln Asn Phe Ser Val Val Leu Ile Asn Gly Gly Thr
   245 250 255
   Asp Leu Pro Gln Pro Pro Ser Asp Asp Asn Asp Gly Glu Ala Asn Leu
   260 265 270
   Asp Val Gln Asn Ile Leu Thr Ile Ala His Pro Leu Pro Ile Thr Glu
   275 280 285
   Phe Ile Thr Ala Gly Ser Pro Pro Tyr Phe Pro Asp Pro Val Glu Pro
   290 295 300
   Ala Gly Thr Pro Asp Glu Asn Glu Pro Tyr Leu Gln Tyr Phe Glu Tyr
   305 310 315 320
   Leu Leu Ser Lys Pro Asn Arg Asp Leu Pro Gln Val Ile Thr Asn Ser
   325 330 335
   Tyr Gly Asp Glu Glu Gln Thr Val Pro Gln Ala Tyr Ala Val Arg Val
   340 345 350
   Cys Asn Leu Ile Gly Leu Met Gly Leu Arg Gly Ile Ser Ile Leu Glu
   355 360 365
   Ser Ser Gly Asp Glu Gly Val Gly Ala Ser Cys Val Ala Thr Asn Ser
   370 375 380
   Thr Thr Pro Gln Phe Asn Pro Ile Phe Pro Ala Thr Cys Pro Tyr Val
   385 390 395 400
   Thr Ser Val Gly Gly Thr Val Asn Phe Asn Pro Glu Val Ala Trp Asp
   405 410 415
   Gly Ser Ser Gly Gly Phe Ser Tyr Tyr Phe Ser Arg Pro Trp Tyr Gln
   420 425 430
   Glu Glu Ala Val Gly Asn Tyr Leu Glu Lys His Val Ser Ala Glu Thr
   435 440 445
   Lys Lys Tyr Tyr Gly Pro Tyr Val Asp Phe Ser Gly Arg Gly Phe Pro
   450 455 460
   Asp Val Ala Ala His Ser Val Ser Pro Asp Tyr Pro Val Phe Gln Gly
   465 470 475 480
   Gly Gln Leu Thr Pro Ser Gly Gly Thr Ser Ala Ala Ser Pro Val Val
   485 490 495
   Ala Ser Ile Ile Ala Leu Leu Asn Asp Ala Arg Leu Arg Glu Gly Lys
   500 505 510
   Pro Thr Leu Gly Phe Leu Asn Pro Leu Ile Tyr Gln Tyr Ala Tyr Lys
   515 520 525
   Gly Phe Thr Asp Ile Thr Ser Gly Gln Ser Asp Gly Cys Asn Gly Asn
   530 535 540
   Asn Thr Gln Thr Asp Ala Pro Leu Pro Gly Ala Gly Val Val Leu Gly
   545 550 555 560
   Ala His Trp Asn Ala Thr Lys Gly Trp Asp Pro Thr Thr Gly Phe Gly
   565 570 575
   Val Pro Asn Phe Lys Lys Leu Leu Glu Leu Ile Arg Tyr Ile
   580 585 590
<210> 14
   <211> 569
   <212> PRT
   <213> Trichoderma atroviride
<400> 14
   Ala Val Leu Val Glu Ser Leu Lys Gln Val Pro Asn Gly Trp Asn Ala
   1               5                   10                  15
   Val Ser Thr Pro Asp Pro Ser Thr Ser Ile Val Leu Gln Ile Ala Leu
               20                  25                  30
   Ala Gln Gln Asn Ile Asp Glu Leu Glu Trp Arg Leu Ala Ala Val Ser
           35                  40                  45
   Thr Pro Asn Ser Gly Asn Tyr Gly Lys Tyr Leu Asp Ile Gly Glu Ile
       50                  55                  60
   Glu Gly Ile Phe Ala Pro Ser Asn Ala Ser Tyr Lys Ala Val Ala Ser
   65                  70                  75                  80
   Trp Leu Gln Ser His Gly Val Lys Asn Phe Val Lys Gln Ala Gly Ser
   85 90 95
   Ile Trp Phe Tyr Thr Thr Val Ser Thr Ala Asn Lys Met Leu Ser Thr
   100 105 110
   Asp Phe Lys His Tyr Ser Asp Pro Val Gly Ile Glu Lys Leu Arg Thr
   115 120 125
   Leu Gln Tyr Ser Ile Pro Glu Glu Leu Val Gly His Val Asp Leu Ile
   130 135 140
   Ser Pro Thr Thr Tyr Phe Gly Asn Asn His Pro Ala Thr Ala Arg Thr
   145 150 155 160
   Pro Asn Met Lys Ala Ile Asn Val Thr Tyr Gln Ile Phe His Pro Asp
   165 170 175
   Cys Leu Lys Thr Lys Tyr Gly Val Asp Gly Tyr Ala Pro Ser Pro Arg
   180 185 190
   Cys Gly Ser Arg Ile Gly Phe Gly Ser Phe Leu Asn Glu Thr Ala Ser
   195 200 205
   Tyr Ser Asp Leu Ala Gln Phe Glu Lys Tyr Phe Asp Leu Pro Asn Gln
   210 215 220
   Asn Leu Ser Thr Leu Leu Ile Asn Gly Ala Ile Asp Val Gln Pro Pro
   225 230 235 240
   Ser Asn Lys Asn Asp Ser Glu Ala Asn Met Asp Val Gln Thr Ile Leu
   245 250 255
   Thr Phe Val Gln Pro Leu Pro Ile Thr Glu Phe Val Val Ala Gly Ile
   260 265 270
   Pro Pro Tyr Ile Pro Asp Ala Ala Leu Pro Ile Gly Asp Pro Val Gln
   275 280 285
   Asn Glu Pro Trp Leu Glu Tyr Phe Glu Phe Leu Met Ser Arg Thr Asn
   290 295 300
   Ala Glu Leu Pro Gln Val Ile Ala Asn Ser Tyr Gly Asp Glu Glu Gln
   305 310 315 320
   Thr Val Pro Gln Ala Tyr Ala Val Arg Val Cys Asn Gln Ile Gly Leu
   325 330 335
   Leu Gly Leu Arg Gly Ile Ser Val Ile Ala Ser Ser Gly Asp Thr Gly
   340 345 350
   Val Gly Met Ser Cys Met Ala Ser Asn Ser Thr Thr Pro Gln Phe Asn
   355 360 365
   Pro Met Phe Pro Ala Ser Cys Pro Tyr Ile Thr Thr Val Gly Gly Thr
   370 375 380
   Gln His Leu Asp Asn Glu Ile Ala Trp Glu Leu Ser Ser Gly Gly Phe
   385 390 395 400
   Ser Asn Tyr Phe Thr Arg Pro Trp Tyr Gln Glu Asp Ala Ala Lys Thr
   405 410 415
   Tyr Leu Glu Arg His Val Ser Thr Glu Thr Lys Ala Tyr Tyr Glu Arg
   420 425 430
   Tyr Ala Asn Phe Leu Gly Arg Gly Phe Pro Asp Val Ala Ala Leu Ser
   435 440 445
   Leu Asn Pro Asp Tyr Pro Val Ile Ile Gly Gly Glu Leu Gly Pro Asn
   450 455 460
   Gly Gly Thr Ser Ala Ala Ala Pro Val Val Ala Ser Ile Ile Ala Leu
   465 470 475 480
   Leu Asn Asp Ala Arg Leu Cys Leu Gly Lys Pro Ala Leu Gly Phe Leu
   485 490 495
   Asn Pro Leu Ile Tyr Gln Tyr Ala Asp Lys Gly Gly Phe Thr Asp Ile
   500 505 510
   Thr Ser Gly Gln Ser Trp Gly Cys Ala Gly Asn Thr Thr Gln Thr Gly
   515 520 525
   Pro Pro Pro Pro Gly Ala Gly Val Ile Pro Gly Ala His Trp Asn Ala
   530 535 540
   Thr Lys Gly Trp Asp Pro Val Thr Gly Phe Gly Thr Pro Asn Phe Lys
   545 550 555 560
   Lys Leu Leu Ser Leu Ala Leu Ser Val
   565
<210> 15
   <211> 565
   <212> PRT
   <213> Agaricus bisporus
<400> 15
   Ser Pro Leu Ala Arg Arg Trp Asp Asp Phe Ala Glu Lys His Ala Trp
   1               5                   10                  15
   Val Glu Val Pro Arg Gly Trp Glu Met Val Ser Glu Ala Pro Ser Asp
               20                  25                  30
   His Thr Phe Asp Leu Arg Ile Gly Val Lys Ser Ser Gly Met Glu Gln
           35                  40                  45
   Leu Ile Glu Asn Leu Met Gln Thr Ser Asp Pro Thr His Ser Arg Tyr
       50                  55                  60
   Gly Gln His Leu Ser Lys Glu Glu Leu His Asp Phe Val Gln Pro His
   65                  70                  75                  80
   Pro Asp Ser Thr Gly Ala Val Glu Ala Trp Leu Glu Asp Phe Gly Ile
                   85                  90                  95
   Ser Asp Asp Phe Ile Asp Arg Thr Gly Ser Gly Asn Trp Val Thr Val
               100                 105                 110
   Arg Val Ser Val Ala Gln Ala Glu Arg Met Leu Gly Thr Lys Tyr Asn
           115                 120                 125
   Val Tyr Arg His Ser Glu Ser Gly Glu Ser Val Val Arg Thr Met Ser
       130                 135                 140
   Tyr Ser Leu Pro Ser Glu Leu His Ser His Ile Asp Val Val Ala Pro
   145                 150                 155                 160
   Thr Thr Tyr Phe Gly Thr Met Lys Ser Met Arg Val Thr Ser Phe Leu
                   165                 170                 175
   Gln Pro Glu Ile Glu Pro Val Asp Pro Ser Ala Lys Pro Ser Ala Ala
               180                 185                 190
   Pro Ala Ser Cys Leu Ser Thr Thr Val Ile Thr Pro Asp Cys Leu Arg
           195                 200                 205
   Asp Leu Tyr Asn Thr Ala Asp Tyr Val Pro Ser Ala Thr Ser Arg Asn
       210                 215                 220
   Ala Ile Gly Ile Ala Gly Tyr Leu Asp Arg Ser Asn Arg Ala Asp Leu
   225                 230                 235                 240
   Gln Thr Phe Phe Arg Arg Phe Arg Pro Asp Ala Val Gly Phe Asn Tyr
                   245                 250                 255
   Thr Thr Val Gln Leu Asn Gly Gly Gly Asp Asp Gln Asn Asp Pro Gly
               260                 265                 270
   Val Glu Ala Asn Leu Asp Ile Gln Tyr Ala Ala Gly Ile Ala Phe Pro
           275                 280                 285
   Thr Pro Ala Thr Tyr Trp Ser Thr Gly Gly Ser Pro Pro Phe Ile Pro
       290                 295                 300
   Asp Thr Gln Thr Pro Thr Asn Thr Asn Glu Pro Tyr Leu Asp Trp Ile
   305                 310                 315                 320
   Asn Phe Val Leu Gly Gln Asp Glu Ile Pro Gln Val Ile Ser Thr Ser
                   325                 330                 335
   Tyr Gly Asp Asp Glu Gln Thr Val Pro Glu Asp Tyr Ala Thr Ser Val
               340                 345                 350
   Cys Asn Leu Phe Ala Gln Leu Gly Ser Arg Gly Val Thr Val Phe Phe
           355                 360                 365
   Ser Ser Gly Asp Phe Gly Val Gly Gly Gly Asp Cys Leu Thr Asn Asp
       370                 375                 380
   Gly Ser Asn Gln Val Leu Phe Gln Pro Ala Phe Pro Ala Ser Cys Pro
   385                 390                 395                 400
   Phe Val Thr Ala Val Gly Gly Thr Val Arg Leu Asp Pro Glu Ile Ala
                   405                 410                 415
   Val Ser Phe Ser Gly Gly Gly Phe Ser Arg Tyr Phe Ser Arg Pro Ser
               420                 425                 430
   Tyr Gln Asn Gln Thr Val Ala Gln Phe Val Ser Asn Leu Gly Asn Thr
           435                 440                 445
   Phe Asn Gly Leu Tyr Asn Lys Asn Gly Arg Ala Tyr Pro Asp Leu Ala
       450                 455                 460
   Ala Gln Gly Asn Gly Phe Gln Val Val Ile Asp Gly Ile Val Arg Ser
   465                 470                 475                 480
   Val Gly Gly Thr Ser Ala Ser Ser Pro Thr Val Ala Gly Ile Phe Ala
                   485                 490                 495
   Leu Leu Asn Asp Phe Lys Leu Ser Arg Gly Gln Ser Thr Leu Gly Phe
               500                 505                 510
   Ile Asn Pro Leu Ile Tyr Ser Ser Ala Thr Ser Gly Phe Asn Asp Ile
           515                 520                 525
   Arg Ala Gly Thr Asn Pro Gly Cys Gly Thr Arg Gly Phe Thr Ala Gly
       530                 535                 540
   Thr Gly Trp Asp Pro Val Thr Gly Leu Gly Thr Pro Asp Phe Leu Arg
   545                 550                 555                 560
   Leu Gln Gly Leu Ile
                   565
<210> 16
   <211> 583
   <212> PRT
   <213> Magnaporthe oryzae
<400> 16
   Arg Val Phe Asp Ser Leu Pro His Pro Pro Arg Gly Trp Ser Tyr Ser
   1               5                   10                  15
   His Ala Ala Glu Ser Thr Glu Pro Leu Thr Leu Arg Ile Ala Leu Arg
               20                  25                  30
   Gln Gln Asn Ala Ala Ala Leu Glu Gln Val Val Leu Gln Val Ser Asn
           35                  40                  45
   Pro Arg His Ala Asn Tyr Gly Gln His Leu Thr Arg Asp Glu Leu Arg
       50                  55                  60
   Ser Tyr Thr Ala Pro Thr Pro Arg Ala Val Arg Ser Val Thr Ser Trp
   65                  70                  75                  80
   Leu Val Asp Asn Gly Val Asp Asp Tyr Thr Val Glu His Asp Trp Val
                   85                  90                  95
   Thr Leu Arg Thr Thr Val Gly Ala Ala Asp Arg Leu Leu Gly Ala Asp
               100                 105                 110
   Phe Ala Trp Tyr Ala Gly Pro Gly Glu Thr Leu Gln Leu Arg Thr Leu
           115                 120                 125
   Ser Tyr Gly Val Asp Asp Ser Val Ala Pro His Val Asp Leu Val Gln
       130                 135                 140
   Pro Thr Thr Arg Phe Gly Gly Pro Val Gly Gln Ala Ser His Ile Phe
   145                 150                 155                 160
   Lys Gln Asp Asp Phe Asp Glu Gln Gln Leu Lys Thr Leu Ser Val Gly
                   165                 170                 175
   Phe Gln Val Met Ala Asp Leu Pro Ala Asn Gly Pro Gly Ser Ile Lys
               180                 185                 190
   Ala Ala Cys Asn Glu Ser Gly Val Thr Pro Leu Cys Leu Arg Thr Leu
           195                 200                 205
   Tyr Arg Val Asn Tyr Lys Pro Ala Thr Thr Gly Asn Leu Val Ala Phe
       210                 215                 220
   Ala Ser Phe Leu Glu Gln Tyr Ala Arg Tyr Ser Asp Gln Gln Ala Phe
   225                 230                 235                 240
   Thr Gln Arg Val Leu Gly Pro Gly Val Pro Leu Gln Asn Phe Ser Val
                   245                 250                 255
   Glu Thr Val Asn Gly Gly Ala Asn Asp Gln Gln Ser Lys Leu Asp Ser
               260                 265                 270
   Gly Glu Ala Asn Leu Asp Leu Gln Tyr Val Met Ala Met Ser His Pro
           275                 280                 285
   Ile Pro Ile Leu Glu Tyr Ser Thr Gly Gly Arg Gly Pro Leu Val Pro
       290                 295                 300
   Thr Leu Asp Gln Pro Asn Ala Asn Asn Ser Ser Asn Glu Pro Tyr Leu
   305                 310                 315                 320
   Glu Phe Leu Thr Tyr Leu Leu Ala Gln Pro Asp Ser Ala Ile Pro Gln
                   325                 330                 335
   Thr Leu Ser Val Ser Tyr Gly Glu Glu Glu Gln Ser Val Pro Arg Asp
               340                 345                 350
   Tyr Ala Ile Lys Val Cys Asn Met Phe Met Gln Leu Gly Ala Arg Gly
           355                 360                 365
   Val Ser Val Met Phe Ser Ser Gly Asp Ser Gly Pro Gly Asn Asp Cys
       370                 375                 380
   Val Arg Ala Ser Asp Asn Ala Thr Phe Phe Gly Ser Thr Phe Pro Ala
   385 390 395 400
   Gly Cys Pro Tyr Val Thr Ser Val Gly Ser Thr Val Gly Phe Glu Pro
   405 410 415
   Glu Arg Ala Val Ser Phe Ser Ser Gly Gly Phe Ser Ile Tyr His Ala
   420 425 430
   Arg Pro Asp Tyr Gln Asn Glu Val Val Pro Lys Tyr Ile Glu Ser Ile
   435 440 445
   Lys Ala Ser Gly Tyr Glu Lys Phe Phe Asp Gly Asn Gly Arg Gly Ile
   450 455 460
   Pro Asp Val Ala Ala Gln Gly Ala Arg Phe Val Val Ile Asp Lys Gly
   465 470 475 480
   Arg Val Ser Leu Ile Ser Gly Thr Ser Ala Ser Ser Pro Ala Phe Ala
   485 490 495
   Gly Met Val Ala Leu Val Asn Ala Ala Arg Lys Ser Lys Asp Met Pro
   500 505 510
   Ala Leu Gly Phe Leu Asn Pro Met Leu Tyr Gln Asn Ala Ala Ala Met
   515 520 525
   Thr Asp Ile Val Asn Gly Ala Gly Ile Gly Cys Arg Lys Gln Arg Thr
   530 535 540
   Glu Phe Pro Asn Gly Ala Arg Phe Asn Ala Thr Ala Gly Trp Asp Pro
   545 550 555 560
   Val Thr Gly Leu Gly Thr Pro Leu Phe Asp Lys Leu Leu Ala Val Gly
   565 570 575
   Ala Pro Gly Val Pro Asn Ala
   580
<210> 17
   <211> 594
   <212> PRT
   <213> Togninia minima
<400> 17
   Ser Asp Val Val Leu Glu Ser Leu Arg Glu Val Pro Gln Gly Trp Lys
   1               5                   10                  15
   Arg Leu Arg Asp Ala Asp Pro Glu Gln Ser Ile Lys Leu Arg Ile Ala
               20                  25                  30
   Leu Glu Gln Pro Asn Leu Asp Leu Phe Glu Gln Thr Leu Tyr Asp Ile
           35                  40                  45
   Ser Ser Pro Asp His Pro Lys Tyr Gly Gln His Leu Lys Ser His Glu
       50                  55                  60
   Leu Arg Asp Ile Met Ala Pro Arg Glu Glu Ser Thr Ala Ala Val Ile
   65                  70                  75                  80
   Ala Trp Leu Gln Asp Ala Gly Leu Ser Gly Ser Gln Ile Glu Asp Asp
                   85                  90                  95
   Ser Asp Trp Ile Asn Ile Gln Thr Thr Val Ala Gln Ala Asn Asp Met
               100                 105                 110
   Leu Asn Thr Thr Phe Gly Leu Phe Ala Gln Glu Gly Thr Glu Val Asn
           115                 120                 125
   Arg Ile Arg Ala Leu Ala Tyr Ser Val Pro Glu Glu Ile Val Pro His
       130                 135                 140
   Val Lys Met Ile Ala Pro Ile Ile Arg Phe Gly Gln Leu Arg Pro Gln
   145                 150                 155                 160
   Met Ser His Ile Phe Ser His Glu Lys Val Glu Glu Thr Pro Ser Ile
                   165                 170                 175
   Gly Thr Ile Lys Ala Ala Ala Ile Pro Ser Val Asp Leu Asn Val Thr
               180                 185                 190
   Ala Cys Asn Ala Ser Ile Thr Pro Glu Cys Leu Arg Ala Leu Tyr Asn
           195                 200                 205
   Val Gly Asp Tyr Glu Ala Asp Pro Ser Lys Lys Ser Leu Phe Gly Val
       210                 215                 220
   Cys Gly Tyr Leu Glu Gln Tyr Ala Lys His Asp Gln Leu Ala Lys Phe
   225                 230                 235                 240
   Glu Gln Thr Tyr Ala Pro Tyr Ala Ile Gly Ala Asp Phe Ser Val Val
                   245                 250                 255
   Thr Ile Asn Gly Gly Gly Asp Asn Gln Thr Ser Thr Ile Asp Asp Gly
               260                 265                 270
   Glu Ala Asn Leu Asp Met Gln Tyr Ala Val Ser Met Ala Tyr Lys Thr
   275 280 285
   Pro Ile Thr Tyr Tyr Ser Thr Gly Gly Arg Gly Pro Leu Val Pro Asp
   290 295 300
   Leu Asp Gln Pro Asp Pro Asn Asp Val Ser Asn Glu Pro Tyr Leu Asp
   305 310 315 320
   Phe Val Ser Tyr Leu Leu Lys Leu Pro Asp Ser Lys Leu Pro Gln Thr
   325 330 335
   Ile Thr Thr Ser Tyr Gly Glu Asp Glu Gln Ser Val Pro Arg Ser Tyr
   340 345 350
   Val Glu Lys Val Cys Thr Met Phe Gly Ala Leu Gly Ala Arg Gly Val
   355 360 365
   Ser Val Ile Phe Ser Ser Gly Asp Thr Gly Val Gly Ser Ala Cys Gln
   370 375 380
   Thr Asn Asp Gly Lys Asn Thr Thr Arg Phe Leu Pro Ile Phe Pro Ala
   385 390 395 400
   Ala Cys Pro Tyr Val Thr Ser Val Gly Gly Thr Arg Tyr Val Asp Pro
   405 410 415
   Glu Val Ala Val Ser Phe Ser Ser Gly Gly Phe Ser Asp Ile Phe Pro
   420 425 430
   Thr Pro Leu Tyr Gln Lys Gly Ala Val Ser Gly Tyr Leu Lys Ile Leu
   435 440 445
   Gly Asp Arg Trp Lys Gly Leu Tyr Asn Pro His Gly Arg Gly Phe Pro
   450 455 460
   Asp Val Ser Gly Gln Ser Val Arg Tyr His Val Phe Asp Tyr Gly Lys
   465 470 475 480
   Asp Val Met Tyr Ser Gly Thr Ser Ala Ser Ala Pro Met Phe Ala Ala
   485 490 495
   Leu Val Ser Leu Leu Asn Asn Ala Arg Leu Ala Lys Lys Leu Pro Pro
   500 505 510
   Met Gly Phe Leu Asn Pro Trp Leu Tyr Thr Val Gly Phe Asn Gly Leu
   515 520 525
   Thr Asp Ile Val His Gly Gly Ser Thr Gly Cys Thr Gly Thr Asp Val
   530 535 540
   Tyr Ser Gly Leu Pro Thr Pro Phe Val Pro Tyr Ala Ser Trp Asn Ala
   545 550 555 560
   Thr Val Gly Trp Asp Pro Val Thr Gly Leu Gly Thr Pro Leu Phe Asp
   565 570 575
   Lys Leu Leu Asn Leu Ser Thr Pro Asn Phe His Leu Pro His Ile Gly
   580 585 590
   Gly His
<210> 18
   <211> 595
   <212> PRT
   <213> Bipolaris maydis
<400> 18
   Ser Thr Thr Ser His Val Glu Gly Glu Val Val Glu Arg Leu His Gly
   1 5 10 15
   Val Pro Glu Gly Trp Ser Gln Val Gly Ala Pro Asn Pro Asp Gln Lys
   20 25 30
   Leu Arg Phe Arg Ile Ala Val Arg Ser Ala Asp Ser Glu Leu Phe Glu
   35 40 45
   Arg Thr Leu Met Glu Val Ser Ser Pro Ser His Pro Arg Tyr Gly Gln
   50 55 60
   His Leu Lys Arg His Glu Leu Lys Asp Leu Ile Lys Pro Arg Ala Lys
   65 70 75 80
   Ser Thr Ser Asn Ile Leu Asn Trp Leu Gln Glu Ser Gly Ile Glu Ala
   85 90 95
   Arg Asp Ile Gln Asn Asp Gly Glu Trp Ile Ser Phe Tyr Ala Pro Val
   100 105 110
   Lys Arg Ala Glu Gln Met Met Ser Thr Thr Phe Lys Thr Tyr Gln Asn
   115 120 125
   Glu Ala Arg Ala Asn Ile Lys Lys Ile Arg Ser Leu Asp Tyr Ser Val
   130 135 140
   Pro Lys His Ile Arg Asp Asp Ile Asp Ile Ile Gln Pro Thr Thr Arg
   145 150 155 160
   Phe Gly Gln Ile Gln Pro Glu Arg Ser Gln Val Phe Ser Gln Glu Glu
   165 170 175
   Val Pro Phe Ser Ala Leu Val Val Asn Ala Thr Cys Asn Lys Lys Ile
   180 185 190
   Thr Pro Asp Cys Leu Ala Asn Leu Tyr Asn Phe Lys Asp Tyr Asp Ala
   195 200 205
   Ser Asp Ala Asn Val Thr Ile Gly Val Ser Gly Phe Leu Glu Gln Tyr
   210 215 220
   Ala Arg Phe Asp Asp Leu Lys Gln Phe Ile Ser Thr Phe Gln Pro Lys
   225 230 235 240
   Ala Ala Gly Ser Thr Phe Gln Val Thr Ser Val Asn Ala Gly Pro Phe
   245 250 255
   Asp Gln Asn Ser Thr Ala Ser Ser Val Glu Ala Asn Leu Asp Ile Gln
   260 265 270
   Tyr Thr Thr Gly Leu Val Ala Pro Asp Ile Glu Thr Arg Tyr Phe Thr
   275 280 285
   Val Pro Gly Arg Gly Ile Leu Ile Pro Asp Leu Asp Gln Pro Thr Glu
   290 295 300
   Ser Asp Asn Ala Asn Glu Pro Tyr Leu Asp Tyr Phe Thr Tyr Leu Asn
   305 310 315 320
   Asn Leu Glu Asp Glu Glu Leu Pro Asp Val Leu Thr Thr Ser Tyr Gly
   325 330 335
   Glu Ser Glu Gln Ser Val Pro Ala Glu Tyr Ala Lys Lys Val Cys Asn
   340 345 350
   Leu Ile Gly Gln Leu Gly Ala Arg Gly Val Ser Val Ile Phe Ser Ser
   355 360 365
   Gly Asp Thr Gly Pro Gly Ser Ala Cys Gln Thr Asn Asp Gly Lys Asn
   370 375 380
   Thr Thr Arg Phe Leu Pro Ile Phe Pro Ala Ser Cys Pro Tyr Val Thr
   385 390 395 400
   Ser Val Gly Gly Thr Val Gly Val Glu Pro Glu Lys Ala Val Ser Phe
   405 410 415
   Ser Ser Gly Gly Phe Ser Asp Leu Trp Pro Arg Pro Ala Tyr Gln Glu
   420 425 430
   Lys Ala Val Ser Glu Tyr Leu Glu Lys Leu Gly Asp Arg Trp Asn Gly
   435 440 445
   Leu Tyr Asn Pro Gln Gly Arg Gly Phe Pro Asp Val Ala Ala Gln Gly
   450 455 460
   Gln Gly Phe Gln Val Phe Asp Lys Gly Arg Leu Ile Ser Val Gly Gly
   465 470 475 480
   Thr Ser Ala Ser Ala Pro Val Phe Ala Ser Val Val Ala Leu Leu Asn
   485 490 495
   Asn Ala Arg Lys Ala Ala Gly Met Ser Ser Leu Gly Phe Leu Asn Pro
   500 505 510
   Trp Ile Tyr Glu Gln Gly Tyr Lys Gly Leu Thr Asp Ile Val Ala Gly
   515 520 525
   Gly Ser Thr Gly Cys Thr Gly Arg Ser Ile Tyr Ser Gly Leu Pro Ala
   530 535 540
   Pro Leu Val Pro Tyr Ala Ser Trp Asn Ala Thr Glu Gly Trp Asp Pro
   545 550 555 560
   Val Thr Gly Tyr Gly Thr Pro Asp Phe Lys Gln Leu Leu Thr Leu Ala
   565 570 575
   Thr Ala Pro Lys Ser Gly Glu Arg Arg Val Arg Arg Gly Gly Leu Gly
   580 585 590
   Gly Gln Ala
   595
<210> 19
   <211> 613
   <212> PRT
   <213> Aspergillus kawachii
<400> 19
   Met Leu Ser Ser Phe Leu Ser Gln Gly Ala Ala Val Ser Leu Ala Leu
   1 5 10 15
   Leu Ser Leu Leu Pro Ser Pro Val Ala Ala Glu Ile Phe Glu Lys Leu
   20 25 30
   Ser Gly Val Pro Asn Gly Trp Arg Tyr Ala Asn Asn Pro His Gly Asn
   35 40 45
   Glu Val Ile Arg Leu Gln Ile Ala Leu Gln Gln His Asp Val Ala Gly
   50 55 60
   Phe Glu Gln Ala Val Met Asp Met Ser Thr Pro Gly His Ala Asp Tyr
   65 70 75 80
   Gly Lys His Phe Arg Thr His Asp Glu Met Lys Arg Met Leu Leu Pro
   85 90 95
   Ser Asp Thr Ala Val Asp Ser Val Arg Asp Trp Leu Glu Ser Ala Gly
   100 105 110
   Val His Asn Ile Gln Val Asp Ala Asp Trp Val Lys Phe His Thr Thr
   115 120 125
   Val Asn Lys Ala Asn Ala Leu Leu Asp Ala Asp Phe Lys Trp Tyr Val
   130 135 140
   Ser Glu Ala Lys His Ile Arg Arg Leu Arg Thr Leu Gln Tyr Ser Ile
   145 150 155 160
   Pro Asp Ala Leu Val Ser His Ile Asn Met Ile Gln Pro Thr Thr Arg
   165 170 175
   Phe Gly Gln Ile Gln Pro Asn Arg Ala Thr Met Arg Ser Lys Pro Lys
   180 185 190
   His Ala Asp Glu Thr Phe Leu Thr Ala Ala Thr Leu Ala Gln Asn Thr
   195 200 205
   Ser His Cys Asp Ser Ile Ile Thr Pro His Cys Leu Lys Gln Leu Tyr
   210 215 220
   Asn Ile Gly Asp Tyr Gln Ala Asp Pro Lys Ser Gly Ser Lys Val Gly
   225 230 235 240
   Phe Ala Ser Tyr Leu Glu Glu Tyr Ala Arg Tyr Ala Asp Leu Glu Arg
   245 250 255
   Phe Glu Gln His Leu Ala Pro Asn Ala Ile Gly Gln Asn Phe Ser Val
   260 265 270
   Val Gln Phe Asn Gly Gly Leu Asn Asp Gln Leu Ser Leu Ser Asp Ser
   275 280 285
   Gly Glu Ala Asn Leu Asp Leu Gln Tyr Ile Leu Gly Val Ser Ala Pro
   290 295 300
   Val Pro Val Thr Glu Tyr Ser Thr Gly Gly Arg Gly Glu Leu Val Pro
   305 310 315 320
   Asp Leu Ser Ser Pro Asp Pro Asn Asp Asn Ser Asn Glu Pro Tyr Leu
   325 330 335
   Asp Phe Leu Gln Gly Ile Leu Lys Leu Asp Asn Ser Asp Leu Pro Gln
   340 345 350
   Val Ile Ser Thr Ser Tyr Gly Glu Asp Glu Gln Thr Ile Pro Val Pro
   355 360 365
   Tyr Ala Arg Thr Val Cys Asn Leu Tyr Ala Gln Leu Gly Ser Arg Gly
   370 375 380
   Val Ser Val Ile Phe Ser Ser Gly Asp Ser Gly Val Gly Ala Ala Cys
   385 390 395 400
   Leu Thr Asn Asp Gly Thr Asn Arg Thr His Phe Pro Pro Gln Phe Pro
   405 410 415
   Ala Ser Cys Pro Trp Val Thr Ser Val Gly Ala Thr Ser Lys Thr Ser
   420 425 430
   Pro Glu Gln Ala Val Ser Phe Ser Ser Gly Gly Phe Ser Asp Leu Trp
   435 440 445
   Pro Arg Pro Ser Tyr Gln Gln Ala Ala Val Gln Thr Tyr Leu Thr Gln
   450 455 460
   His Leu Gly Asn Lys Phe Ser Gly Leu Phe Asn Ala Ser Gly Arg Ala
   465 470 475 480
   Phe Pro Asp Val Ala Ala Gln Gly Val Asn Tyr Ala Val Tyr Asp Lys
   485 490 495
   Gly Met Leu Gly Gln Phe Asp Gly Thr Ser Cys Ser Ala Pro Thr Phe
   500 505 510
   Ser Gly Val Ile Ala Leu Leu Asn Asp Ala Arg Leu Arg Ala Gly Leu
   515 520 525
   Pro Val Met Gly Phe Leu Asn Pro Phe Leu Tyr Gly Val Gly Ser Glu
   530 535 540
   Ser Gly Ala Leu Asn Asp Ile Val Asn Gly Gly Ser Leu Gly Cys Asp
   545 550 555 560
   Gly Arg Asn Arg Phe Gly Gly Thr Pro Asn Gly Ser Pro Val Val Pro
   565 570 575
   Phe Ala Ser Trp Asn Ala Thr Thr Gly Trp Asp Pro Val Ser Gly Leu
   580 585 590
   Gly Thr Pro Asp Phe Ala Lys Leu Arg Gly Val Ala Leu Gly Glu Ala
   595 600 605
   Lys Ala Tyr Gly Asn
   610
<210> 20
   <211> 658
   <212> PRT
   <213> Aspergillus nidulans
<400> 20
   Met Ala Ala Thr Gly Arg Phe Thr Ala Phe Trp Asn Val Ala Ser Val
   1 5 10 15
   Pro Ala Leu Ile Gly Ile Leu Pro Leu Ala Gly Ser His Leu Arg Ala
   20 25 30
   Val Leu Cys Pro Val Cys Ile Trp Arg His Ser Lys Ala Val Cys Ala
   35 40 45
   Pro Asp Thr Leu Gln Ala Met Arg Ala Phe Thr Arg Val Thr Ala Ile
   50 55 60
   Ser Leu Ala Gly Phe Ser Cys Phe Ala Ala Ala Ala Ala Ala Ala Phe
   65 70 75 80
   Glu Ser Leu Arg Ala Val Pro Asp Gly Trp Ile Tyr Glu Ser Thr Pro
   85 90 95
   Asp Pro Asn Gln Pro Leu Arg Leu Arg Ile Ala Leu Lys Gln His Asn
   100 105 110
   Val Ala Gly Phe Glu Gln Ala Leu Leu Asp Met Ser Thr Pro Gly His
   115 120 125
   Ser Ser Tyr Gly Gln His Phe Gly Ser Tyr His Glu Met Lys Gln Leu
   130 135 140
   Leu Leu Pro Thr Glu Glu Ala Ser Ser Ser Val Arg Asp Trp Leu Ser
   145 150 155 160
   Ala Ala Gly Val Glu Phe Glu Gln Asp Ala Asp Trp Ile Asn Phe Arg
   165 170 175
   Thr Thr Val Asp Gln Ala Asn Ala Leu Leu Asp Ala Asp Phe Leu Trp
   180 185 190
   Tyr Thr Thr Thr Gly Ser Thr Gly Asn Pro Thr Arg Ile Leu Arg Thr
   195 200 205
   Leu Ser Tyr Ser Val Pro Ser Glu Leu Ala Gly Tyr Val Asn Met Ile
   210 215 220
   Gln Pro Thr Thr Arg Phe Gly Gly Thr His Ala Asn Arg Ala Thr Val
   225 230 235 240
   Arg Ala Lys Pro Ile Phe Leu Glu Thr Asn Arg Gln Leu Ile Asn Ala
   245 250 255
   Ile Ser Ser Gly Ser Leu Glu His Cys Glu Lys Ala Ile Thr Pro Ser
   260 265 270
   Cys Leu Ala Asp Leu Tyr Asn Thr Glu Gly Tyr Lys Ala Ser Asn Arg
   275 280 285
   Ser Gly Ser Lys Val Ala Phe Ala Ser Phe Leu Glu Glu Tyr Ala Arg
   290 295 300
   Tyr Asp Asp Leu Ala Glu Phe Glu Glu Thr Tyr Ala Pro Tyr Ala Ile
   305 310 315 320
   Gly Gln Asn Phe Ser Val Ile Ser Ile Asn Gly Gly Leu Asn Asp Gln
   325 330 335
   Asp Ser Thr Ala Asp Ser Gly Glu Ala Asn Leu Asp Leu Gln Tyr Ile
   340 345 350
   Ile Gly Val Ser Ser Pro Leu Pro Val Thr Glu Phe Thr Thr Gly Gly
   355 360 365
   Arg Gly Lys Leu Ile Pro Asp Leu Ser Ser Pro Asp Pro Asn Asp Asn
       370                 375                 380
   Thr Asn Glu Pro Phe Leu Asp Phe Leu Glu Ala Val Leu Lys Leu Asp
   385                 390                 395                 400
   Gln Lys Asp Leu Pro Gln Val Ile Ser Thr Ser Tyr Gly Glu Asp Glu
                   405                 410                 415
   Gln Thr Ile Pro Glu Pro Tyr Ala Arg Ser Val Cys Asn Leu Tyr Ala
               420                 425                 430
   Gln Leu Gly Ser Arg Gly Val Ser Val Leu Phe Ser Ser Gly Asp Ser
           435                 440                 445
   Gly Val Gly Ala Ala Cys Gln Thr Asn Asp Gly Lys Asn Thr Thr His
       450                 455                 460
   Phe Pro Pro Gln Phe Pro Ala Ser Cys Pro Trp Val Thr Ala Val Gly
   465                 470                 475                 480
   Gly Thr Asn Gly Thr Ala Pro Glu Ser Gly Val Tyr Phe Ser Ser Gly
                   485                 490                 495
   Gly Phe Ser Asp Tyr Trp Ala Arg Pro Ala Tyr Gln Asn Ala Ala Val
               500                 505                 510
   Glu Ser Tyr Leu Arg Lys Leu Gly Ser Thr Gln Ala Gln Tyr Phe Asn
           515                 520                 525
   Arg Ser Gly Arg Ala Phe Pro Asp Val Ala Ala Gln Ala Gln Asn Phe
       530                 535                 540
   Ala Val Val Asp Lys Gly Arg Val Gly Leu Phe Asp Gly Thr Ser Cys
   545                 550                 555                 560
   Ser Ser Pro Val Phe Ala Gly Ile Val Ala Leu Leu Asn Asp Val Arg
                   565                 570                 575
   Leu Lys Ala Gly Leu Pro Val Leu Gly Phe Leu Asn Pro Trp Leu Tyr
               580                 585                 590
   Gln Asp Gly Leu Asn Gly Leu Asn Asp Ile Val Asp Gly Gly Ser Thr
           595                 600                 605
   Gly Cys Asp Gly Asn Asn Arg Phe Asn Gly Ser Pro Asn Gly Ser Pro
       610                 615                 620
   Val Ile Pro Tyr Ala Gly Trp Asn Ala Thr Glu Gly Trp Asp Pro Val
   625 630 635 640
   Thr Gly Leu Gly Thr Pro Asp Phe Ala Lys Leu Lys Ala Leu Val Leu
   645 650 655
   Asp Ala
<210> 21
   <211> 604
   <212> PRT
   <213> Aspergillus ruber
<400> 21
   Met Leu Ser Phe Val Arg Arg Gly Ala Leu Ser Leu Ala Leu Val Ser
   1 5 10 15
   Leu Leu Thr Ser Ser Val Ala Ala Glu Val Phe Glu Lys Leu His Val
   20 25 30
   Val Pro Glu Gly Trp Arg Tyr Ala Ser Thr Pro Asn Pro Lys Gln Pro
   35 40 45
   Ile Arg Leu Gln Ile Ala Leu Gln Gln His Asp Val Thr Gly Phe Glu
   50 55 60
   Gln Ser Leu Leu Glu Met Ser Thr Pro Asp His Pro Asn Tyr Gly Lys
   65 70 75 80
   His Phe Arg Thr His Asp Glu Met Lys Arg Met Leu Leu Pro Asn Glu
   85 90 95
   Asn Ala Val His Ala Val Arg Glu Trp Leu Gln Asp Ala Gly Ile Ser
   100 105 110
   Asp Ile Glu Glu Asp Ala Asp Trp Val Arg Phe His Thr Thr Val Asp
   115 120 125
   Gln Ala Asn Asp Leu Leu Asp Ala Asn Phe Leu Trp Tyr Ala His Lys
   130 135 140
   Ser His Arg Asn Thr Ala Arg Leu Arg Thr Leu Glu Tyr Ser Ile Pro
   145 150 155 160
   Asp Ser Ile Ala Pro Gln Val Asn Val Ile Gln Pro Thr Thr Arg Phe
   165 170 175
   Gly Gln Ile Arg Ala Asn Arg Ala Thr His Ser Ser Lys Pro Lys Gly
   180 185 190
   Gly Leu Asp Glu Leu Ala Ile Ser Gln Ala Ala Thr Ala Asp Asp Asp
   195 200 205
   Ser Ile Cys Asp Gln Ile Thr Thr Pro His Cys Leu Arg Lys Leu Tyr
   210 215 220
   Asn Val Asn Gly Tyr Lys Ala Asp Pro Ala Ser Gly Ser Lys Ile Gly
   225 230 235 240
   Phe Ala Ser Phe Leu Glu Glu Tyr Ala Arg Tyr Ser Asp Leu Val Leu
   245 250 255
   Phe Glu Glu Asn Leu Ala Pro Phe Ala Glu Gly Glu Asn Phe Thr Val
   260 265 270
   Val Met Tyr Asn Gly Gly Lys Asn Asp Gln Asn Ser Lys Ser Asp Ser
   275 280 285
   Gly Glu Ala Asn Leu Asp Leu Gln Tyr Ile Val Gly Met Ser Ala Gly
   290 295 300
   Ala Pro Val Thr Glu Phe Ser Thr Ala Gly Arg Ala Pro Val Ile Pro
   305 310 315 320
   Asp Leu Asp Gln Pro Asp Pro Ser Ala Gly Thr Asn Glu Pro Tyr Leu
   325 330 335
   Glu Phe Leu Gln Asn Val Leu His Met Asp Gln Glu His Leu Pro Gln
   340 345 350
   Val Ile Ser Thr Ser Tyr Gly Glu Asn Glu Gln Thr Ile Pro Glu Lys
   355 360 365
   Tyr Ala Arg Thr Val Cys Asn Met Tyr Ala Gln Leu Gly Ser Arg Gly
   370 375 380
   Val Ser Val Ile Phe Ser Ser Gly Asp Ser Gly Val Gly Ser Ala Cys
   385 390 395 400
   Met Thr Asn Asp Gly Thr Asn Arg Thr His Phe Pro Pro Gln Phe Pro
   405 410 415
   Ala Ser Cys Pro Trp Val Thr Ser Val Gly Ala Thr Glu Lys Met Ala
                   420                 425                 430
   Pro Glu Gln Ala Thr Tyr Phe Ser Ser Gly Gly Phe Ser Asp Leu Phe
               435                 440                 445
   Pro Arg Pro Lys Tyr Gln Asp Ala Ala Val Ser Ser Tyr Leu Gln Thr
       450                 455                 460
   Leu Gly Ser Arg Tyr Gln Gly Leu Tyr Asn Gly Ser Asn Arg Ala Phe
   465                 470                 475                 480
   Pro Asp Val Ser Ala Gln Gly Thr Asn Phe Ala Val Tyr Asp Lys Gly
                   485                 490                 495
   Arg Leu Gly Gln Phe Asp Gly Thr Ser Cys Ser Ala Pro Ala Phe Ser
               500                 505                 510
   Gly Ile Ile Ala Leu Leu Asn Asp Val Arg Leu Gln Asn Asn Lys Pro
           515                 520                 525
   Val Leu Gly Phe Leu Asn Pro Trp Leu Tyr Gly Ala Gly Ser Lys Gly
       530                 535                 540
   Leu Asn Asp Val Val His Gly Gly Ser Thr Gly Cys Asp Gly Gln Glu
   545                 550                 555                 560
   Arg Phe Ala Gly Lys Ala Asn Gly Ser Pro Val Val Pro Tyr Ala Ser
                   565                 570                 575
   Trp Asn Ala Thr Gln Gly Trp Asp Pro Val Thr Gly Leu Gly Thr Pro
               580                 585                 590
   Asp Phe Gly Lys Leu Lys Asp Leu Ala Leu Ser Ala
           595                 600
<210> 22
   <211> 600
   <212> PRT
   <213> Aspergillus terreus
<400> 22
   Met Leu Pro Ser Leu Val Asn Asn Gly Ala Leu Ser Leu Ala Val Leu
   1               5                   10                  15
   Ser Leu Leu Thr Ser Ser Val Ala Gly Glu Val Phe Glu Lys Leu Ser
               20                  25                  30
   Ala Val Pro Lys Gly Trp His Phe Ser His Ala Ala Gln Ala Asp Ala
   35 40 45
   Pro Ile Asn Leu Lys Ile Ala Leu Lys Gln His Asp Val Glu Gly Phe
   50 55 60
   Glu Gln Ala Leu Leu Asp Met Ser Thr Pro Gly His Glu Asn Tyr Gly
   65 70 75 80
   Lys His Phe His Glu His Asp Glu Met Lys Arg Met Leu Leu Pro Ser
   85 90 95
   Asp Ser Ala Val Asp Ala Val Gln Thr Trp Leu Thr Ser Ala Gly Ile
   100 105 110
   Thr Asp Tyr Asp Leu Asp Ala Asp Trp Ile Asn Leu Arg Thr Thr Val
   115 120 125
   Glu His Ala Asn Ala Leu Leu Asp Thr Gln Phe Gly Trp Tyr Glu Asn
   130 135 140
   Glu Val Arg His Ile Thr Arg Leu Arg Thr Leu Gln Tyr Ser Ile Pro
   145 150 155 160
   Glu Thr Val Ala Ala His Ile Asn Met Val Gln Pro Thr Thr Arg Phe
   165 170 175
   Gly Gln Ile Arg Pro Asp Arg Ala Thr Phe His Ala His His Thr Ser
   180 185 190
   Asp Ala Arg Ile Leu Ser Ala Leu Ala Ala Ala Ser Asn Ser Thr Ser
   195 200 205
   Cys Asp Ser Val Ile Thr Pro Lys Cys Leu Lys Asp Leu Tyr Lys Val
   210 215 220
   Gly Asp Tyr Glu Ala Asp Pro Asp Ser Gly Ser Gln Val Ala Phe Ala
   225 230 235 240
   Ser Tyr Leu Glu Glu Tyr Ala Arg Tyr Ala Asp Met Val Lys Phe Gln
   245 250 255
   Asn Ser Leu Ala Pro Tyr Ala Lys Gly Gln Asn Phe Ser Val Val Leu
   260 265 270
   Tyr Asn Gly Gly Val Asn Asp Gln Ser Ser Ser Ala Asp Ser Gly Glu
   275 280 285
   Ala Asn Leu Asp Leu Gln Thr Ile Met Gly Leu Ser Ala Pro Leu Pro
       290                 295                 300
   Ile Thr Glu Tyr Ile Thr Gly Gly Arg Gly Lys Leu Ile Pro Asp Leu
   305                 310                 315                 320
   Ser Gln Pro Asn Pro Asn Asp Asn Ser Asn Glu Pro Tyr Leu Glu Phe
                   325                 330                 335
   Leu Gln Asn Ile Leu Lys Leu Asp Gln Asp Glu Leu Pro Gln Val Ile
               340                 345                 350
   Ser Thr Ser Tyr Gly Glu Asp Glu Gln Thr Ile Pro Arg Gly Tyr Ala
           355                 360                 365
   Glu Ser Val Cys Asn Met Leu Ala Gln Leu Gly Ser Arg Gly Val Ser
       370                 375                 380
   Val Val Phe Ser Ser Gly Asp Ser Gly Val Gly Ala Ala Cys Gln Thr
   385                 390                 395                 400
   Asn Asp Gly Arg Asn Gln Thr His Phe Asn Pro Gln Phe Pro Ala Ser
                   405                 410                 415
   Cys Pro Trp Val Thr Ser Val Gly Ala Thr Thr Lys Thr Asn Pro Glu
               420                 425                 430
   Gln Ala Val Tyr Phe Ser Ser Gly Gly Phe Ser Asp Phe Trp Lys Arg
           435                 440                 445
   Pro Lys Tyr Gln Asp Glu Ala Val Ala Ala Tyr Leu Asp Thr Leu Gly
       450                 455                 460
   Asp Lys Phe Ala Gly Leu Phe Asn Lys Gly Gly Arg Ala Phe Pro Asp
   465                 470                 475                 480
   Val Ala Ala Gln Gly Met Asn Tyr Ala Ile Tyr Asp Lys Gly Thr Leu
                   485                 490                 495
   Gly Arg Leu Asp Gly Thr Ser Cys Ser Ala Pro Ala Phe Ser Ala Ile
               500                 505                 510
   Ile Ser Leu Leu Asn Asp Ala Arg Leu Arg Glu Gly Lys Pro Thr Met
           515                 520                 525
   Gly Phe Leu Asn Pro Trp Leu Tyr Gly Glu Gly Arg Glu Ala Leu Asn
       530                 535                 540
   Asp Val Val Val Gly Gly Ser Lys Gly Cys Asp Gly Arg Asp Arg Phe
   545                 550                 555                 560
   Gly Gly Lys Pro Asn Gly Ser Pro Val Val Pro Phe Ala Ser Trp Asn
                   565                 570                 575
   Ala Thr Gln Gly Trp Asp Pro Val Thr Gly Leu Gly Thr Pro Asn Phe
               580                 585                 590
   Ala Lys Met Leu Glu Leu Ala Pro
           595                 600
<210> 23
   <211> 601
   <212> PRT
   <213> Penicillium digitatum
<400> 23
   Met Ile Ala Ser Leu Phe Asn Arg Arg Ala Leu Thr Leu Ala Leu Leu
   1               5                   10                  15
   Ser Leu Phe Ala Ser Ser Ala Thr Ala Asp Val Phe Glu Ser Leu Ser
               20                  25                  30
   Ala Val Pro Gln Gly Trp Arg Tyr Ser Arg Thr Pro Ser Ala Asn Gln
           35                  40                  45
   Pro Leu Lys Leu Gln Ile Ala Leu Ala Gln Gly Asp Val Ala Gly Phe
       50                  55                  60
   Glu Ala Ala Val Ile Asp Met Ser Thr Pro Asp His Pro Ser Tyr Gly
   65                  70                  75                  80
   Asn His Phe Asn Thr His Glu Glu Met Lys Arg Met Leu Gln Pro Ser
                   85                  90                  95
   Ala Glu Ser Val Asp Ser Ile Arg Asn Trp Leu Glu Ser Ala Gly Ile
               100                 105                 110
   Ser Lys Ile Glu Gln Asp Ala Asp Trp Met Thr Phe Tyr Thr Thr Val
           115                 120                 125
   Lys Thr Ala Asn Glu Leu Leu Ala Ala Asn Phe Gln Phe Tyr Ile Asn
       130                 135                 140
   Gly Val Lys Lys Ile Glu Arg Leu Arg Thr Leu Lys Tyr Ser Val Pro
   145                 150                 155                 160
   Asp Ala Leu Val Ser His Ile Asn Met Ile Gln Pro Thr Thr Arg Phe
   165 170 175
   Gly Gln Leu Arg Ala Gln Arg Ala Ile Leu His Thr Glu Val Lys Asp
   180 185 190
   Asn Asp Glu Ala Phe Arg Ser Asn Ala Met Ser Ala Asn Pro Asp Cys
   195 200 205
   Asn Ser Ile Ile Thr Pro Gln Cys Leu Lys Asp Leu Tyr Ser Ile Gly
   210 215 220
   Asp Tyr Glu Ala Asp Pro Thr Asn Gly Asn Lys Val Ala Phe Ala Ser
   225 230 235 240
   Tyr Leu Glu Glu Tyr Ala Arg Tyr Ser Asp Leu Ala Leu Phe Glu Lys
   245 250 255
   Asn Ile Ala Pro Phe Ala Lys Gly Gln Asn Phe Ser Val Val Gln Tyr
   260 265 270
   Asn Gly Gly Gly Asn Asp Gln Gln Ser Ser Ser Gly Ser Ser Glu Ala
   275 280 285
   Asn Leu Asp Leu Gln Tyr Ile Val Gly Val Ser Ser Pro Val Pro Val
   290 295 300
   Thr Glu Phe Ser Thr Gly Gly Arg Gly Glu Leu Val Pro Asp Leu Asp
   305 310 315 320
   Gln Pro Asn Pro Asn Asp Asn Asn Asn Glu Pro Tyr Leu Glu Phe Leu
   325 330 335
   Gln Asn Val Leu Lys Leu His Lys Lys Asp Leu Pro Gln Val Ile Ser
   340 345 350
   Thr Ser Tyr Gly Glu Asp Glu Gln Ser Val Pro Glu Lys Tyr Ala Arg
   355 360 365
   Ala Val Cys Asn Leu Tyr Ser Gln Leu Gly Ser Arg Gly Val Ser Val
   370 375 380
   Ile Phe Ser Ser Gly Asp Ser Gly Val Gly Ala Ala Cys Gln Thr Asn
   385 390 395 400
   Asp Gly Arg Asn Ala Thr His Phe Pro Pro Gln Phe Pro Ala Ala Cys
   405 410 415
   Pro Trp Val Thr Ser Val Gly Ala Thr Thr His Thr Ala Pro Glu Arg
   420 425 430
   Ala Val Tyr Phe Ser Ser Gly Gly Phe Ser Asp Leu Trp Asp Arg Pro
   435 440 445
   Thr Trp Gln Glu Asp Ala Val Ser Glu Tyr Leu Glu Asn Leu Gly Asp
   450 455 460
   Arg Trp Ser Gly Leu Phe Asn Pro Lys Gly Arg Ala Phe Pro Asp Val
   465 470 475 480
   Ala Ala Gln Gly Glu Asn Tyr Ala Ile Tyr Asp Lys Gly Ser Leu Ile
   485 490 495
   Ser Val Asp Gly Thr Ser Cys Ser Ala Pro Ala Phe Ala Gly Val Ile
   500 505 510
   Ala Leu Leu Asn Asp Ala Arg Ile Lys Ala Asn Arg Pro Pro Met Gly
   515 520 525
   Phe Leu Asn Pro Trp Leu Tyr Ser Glu Gly Arg Ser Gly Leu Asn Asp
   530 535 540
   Ile Val Asn Gly Gly Ser Thr Gly Cys Asp Gly His Gly Arg Phe Ser
   545 550 555 560
   Gly Pro Thr Asn Gly Gly Thr Ser Ile Pro Gly Ala Ser Trp Asn Ala
   565 570 575
   Thr Lys Gly Trp Asp Pro Val Ser Gly Leu Gly Ser Pro Asn Phe Ala
   580 585 590
   Ala Met Arg Lys Leu Ala Asn Ala Glu
   595 600
<210> 24
   <211> 601
   <212> PRT
   <213> Penicillium oxalicum
<400> 24
   Met His Val Pro Leu Leu Asn Gln Gly Ala Leu Ser Leu Ala Val Val
   1 5 10 15
   Ser Leu Leu Ala Ser Thr Val Ser Ala Glu Val Phe Asp Lys Leu Val
   20 25 30
   Ala Val Pro Glu Gly Trp Arg Phe Ser Arg Thr Pro Ser Gly Asp Gln
           35                  40                  45
   Pro Ile Arg Leu Gln Val Ala Leu Thr Gln Gly Asp Val Glu Gly Phe
       50                  55                  60
   Glu Lys Ala Val Leu Asp Met Ser Thr Pro Asp His Pro Asn Tyr Gly
   65                  70                  75                  80
   Lys His Phe Lys Ser His Glu Glu Val Lys Arg Met Leu Gln Pro Ala
                   85                  90                  95
   Gly Glu Ser Val Glu Ala Ile His Gln Trp Leu Glu Lys Ala Gly Ile
               100                 105                 110
   Thr His Ile Gln Gln Asp Ala Asp Trp Met Thr Phe Tyr Thr Thr Val
           115                 120                 125
   Glu Lys Ala Asn Asn Leu Leu Asp Ala Asn Phe Gln Tyr Tyr Leu Asn
       130                 135                 140
   Glu Asn Lys Gln Val Glu Arg Leu Arg Thr Leu Glu Tyr Ser Val Pro
   145                 150                 155                 160
   Asp Glu Leu Val Ser His Ile Asn Leu Val Thr Pro Thr Thr Arg Phe
                   165                 170                 175
   Gly Gln Leu His Ala Glu Gly Val Thr Leu His Gly Lys Ser Lys Asp
               180                 185                 190
   Val Asp Glu Gln Phe Arg Gln Ala Ala Thr Ser Pro Ser Ser Asp Cys
           195                 200                 205
   Asn Ser Ala Ile Thr Pro Gln Cys Leu Lys Asp Leu Tyr Lys Val Gly
       210                 215                 220
   Asp Tyr Lys Ala Ser Ala Ser Asn Gly Asn Lys Val Ala Phe Thr Ser
   225                 230                 235                 240
   Tyr Leu Glu Gln Tyr Ala Arg Tyr Ser Asp Leu Ala Leu Phe Glu Gln
                   245                 250                 255
   Asn Ile Ala Pro Tyr Ala Gln Gly Gln Asn Phe Thr Val Ile Gln Tyr
               260                 265                 270
   Asn Gly Gly Leu Asn Asp Gln Ser Ser Pro Ala Asp Ser Ser Glu Ala
               275                 280                 285
   Asn Leu Asp Leu Gln Tyr Ile Ile Gly Thr Ser Ser Pro Val Pro Val
           290                 295                 300
   Thr Glu Phe Ser Thr Gly Gly Arg Gly Pro Leu Val Pro Asp Leu Asp
   305                 310                 315                 320
   Gln Pro Asp Ile Asn Asp Asn Asn Asn Glu Pro Tyr Leu Asp Phe Leu
                   325                 330                 335
   Gln Asn Val Ile Lys Met Ser Asp Lys Asp Leu Pro Gln Val Ile Ser
               340                 345                 350
   Thr Ser Tyr Gly Glu Asp Glu Gln Ser Val Pro Ala Ser Tyr Ala Arg
           355                 360                 365
   Ser Val Cys Asn Leu Ile Ala Gln Leu Gly Gly Arg Gly Val Ser Val
       370                 375                 380
   Ile Phe Ser Ser Gly Asp Ser Gly Val Gly Ser Ala Cys Gln Thr Asn
   385                 390                 395                 400
   Asp Gly Lys Asn Thr Thr Arg Phe Pro Ala Gln Phe Pro Ala Ala Cys
                   405                 410                 415
   Pro Trp Val Thr Ser Val Gly Ala Thr Thr Gly Ile Ser Pro Glu Arg
               420                 425                 430
   Gly Val Phe Phe Ser Ser Gly Gly Phe Ser Asp Leu Trp Ser Arg Pro
           435                 440                 445
   Ser Trp Gln Ser His Ala Val Lys Ala Tyr Leu His Lys Leu Gly Lys
       450                 455                 460
   Arg Gln Asp Gly Leu Phe Asn Arg Glu Gly Arg Ala Phe Pro Asp Val
   465                 470                 475                 480
   Ser Ala Gln Gly Glu Asn Tyr Ala Ile Tyr Ala Lys Gly Arg Leu Gly
                   485                 490                 495
   Lys Val Asp Gly Thr Ser Cys Ser Ala Pro Ala Phe Ala Gly Leu Val
               500                 505                 510
   Ser Leu Leu Asn Asp Ala Arg Ile Lys Ala Gly Lys Ser Ser Leu Gly
           515                 520                 525
   Phe Leu Asn Pro Trp Leu Tyr Ser His Pro Asp Ala Leu Asn Asp Ile
       530                 535                 540
   Thr Val Gly Gly Ser Thr Gly Cys Asp Gly Asn Ala Arg Phe Gly Gly
   545                 550                 555                 560
   Arg Pro Asn Gly Ser Pro Val Val Pro Tyr Ala Ser Trp Asn Ala Thr
                   565                 570                 575
   Glu Gly Trp Asp Pro Val Thr Gly Leu Gly Thr Pro Asn Phe Gln Lys
               580                 585                 590
   Leu Leu Lys Ser Ala Val Lys Gln Lys
           595                 600
<210> 25
   <211> 601
   <212> PRT
   <213> Penicillium roqueforti
<400> 25
   Met Ile Ala Ser Leu Phe Ser Arg Gly Ala Leu Ser Leu Ala Val Leu
   1               5                   10                  15
   Ser Leu Leu Ala Ser Ser Ala Ala Ala Asp Val Phe Glu Ser Leu Ser
               20                  25                  30
   Ala Val Pro Gln Gly Trp Arg Tyr Ser Arg Arg Pro Arg Ala Asp Gln
           35                  40                  45
   Pro Leu Lys Leu Gln Ile Ala Leu Thr Gln Gly Asp Thr Ala Gly Phe
       50                  55                  60
   Glu Glu Ala Val Met Glu Met Ser Thr Pro Asp His Pro Ser Tyr Gly
   65                  70                  75                  80
   His His Phe Thr Thr His Glu Glu Met Lys Arg Met Leu Gln Pro Ser
                   85                  90                  95
   Ala Glu Ser Ala Glu Ser Ile Arg Asp Trp Leu Glu Gly Ala Gly Ile
               100                 105                 110
   Thr Arg Ile Glu Gln Asp Ala Asp Trp Met Thr Phe Tyr Thr Thr Val
           115                 120                 125
   Glu Thr Ala Asn Glu Leu Leu Ala Ala Asn Phe Gln Phe Tyr Val Ser
       130                 135                 140
   Asn Val Arg His Ile Glu Arg Leu Arg Thr Leu Lys Tyr Ser Val Pro
   145                 150                 155                 160
   Lys Ala Leu Val Pro His Ile Asn Met Ile Gln Pro Thr Thr Arg Phe
                   165                 170                 175
   Gly Gln Leu Arg Ala His Arg Gly Ile Leu His Gly Gln Val Lys Glu
               180                 185                 190
   Ser Asp Glu Ala Phe Arg Ser Asn Ala Val Ser Ala Gln Pro Asp Cys
           195                 200                 205
   Asn Ser Ile Ile Thr Pro Gln Cys Leu Lys Asp Ile Tyr Asn Ile Gly
       210                 215                 220
   Asp Tyr Gln Ala Asn Asp Thr Asn Gly Asn Lys Val Gly Phe Ala Ser
   225                 230                 235                 240
   Tyr Leu Glu Glu Tyr Ala Arg Tyr Ser Asp Leu Ala Leu Phe Glu Lys
                   245                 250                 255
   Asn Ile Ala Pro Ser Ala Lys Gly Gln Asn Phe Ser Val Thr Arg Tyr
               260                 265                 270
   Asn Gly Gly Leu Asn Asp Gln Ser Ser Ser Gly Ser Ser Ser Glu Ala
           275                 280                 285
   Asn Leu Asp Leu Gln Tyr Ile Val Gly Val Ser Ser Pro Val Pro Val
       290                 295                 300
   Thr Glu Phe Ser Val Gly Gly Arg Gly Glu Leu Val Pro Asp Leu Asp
   305                 310                 315                 320
   Gln Pro Asp Pro Asn Asp Asn Asn Asn Glu Pro Tyr Leu Glu Phe Leu
                   325                 330                 335
   Gln Asn Val Leu Lys Leu Asp Lys Lys Asp Leu Pro Gln Val Ile Ser
               340                 345                 350
   Thr Ser Tyr Gly Glu Asp Glu Gln Ser Ile Pro Glu Lys Tyr Ala Arg
           355                 360                 365
   Ser Val Cys Asn Leu Tyr Ser Gln Leu Gly Ser Arg Gly Val Ser Val
       370                 375                 380
   Ile Phe Ser Ser Gly Asp Ser Gly Val Gly Ser Ala Cys Leu Thr Asn
   385                 390                 395                 400
   Asp Gly Arg Asn Ala Thr Arg Phe Pro Pro Gln Phe Pro Ala Ala Cys
                   405                 410                 415
   Pro Trp Val Thr Ser Val Gly Ala Thr Thr His Thr Ala Pro Glu Gln
               420                 425                 430
   Ala Val Tyr Phe Ser Ser Gly Gly Phe Ser Asp Leu Trp Ala Arg Pro
           435                 440                 445
   Lys Trp Gln Glu Glu Ala Val Ser Glu Tyr Leu Glu Ile Leu Gly Asn
       450                 455                 460
   Arg Trp Ser Gly Leu Phe Asn Pro Lys Gly Arg Ala Phe Pro Asp Val
   465                 470                 475                 480
   Thr Ala Gln Gly Arg Asn Tyr Ala Ile Tyr Asp Lys Gly Ser Leu Thr
                   485                 490                 495
   Ser Val Asp Gly Thr Ser Cys Ser Ala Pro Ala Phe Ala Gly Val Val
               500                 505                 510
   Ala Leu Leu Asn Asp Ala Arg Leu Lys Val Asn Lys Pro Pro Met Gly
           515                 520                 525
   Phe Leu Asn Pro Trp Leu Tyr Ser Thr Gly Arg Ala Gly Leu Lys Asp
       530                 535                 540
   Ile Val Asp Gly Gly Ser Thr Gly Cys Asp Gly Lys Ser Arg Phe Gly
   545                 550                 555                 560
   Gly Ala Asn Asn Gly Gly Pro Ser Ile Pro Gly Ala Ser Trp Asn Ala
                   565                 570                 575
   Thr Lys Gly Trp Asp Pro Val Ser Gly Leu Gly Ser Pro Asn Phe Ala
               580                 585                 590
   Thr Met Arg Lys Leu Ala Asn Ala Glu
           595                 600
<210> 26
   <211> 601
   <212> PRT
   <213> Penicillium rubens Wisconsin
<400> 26
   Met Ile Ala Ser Leu Phe Asn Arg Gly Ala Leu Ser Leu Ala Val Leu
   1               5                   10                  15
   Ser Leu Leu Ala Ser Ser Ala Ser Ala Asp Val Phe Glu Ser Leu Ser
   20 25 30
   Ala Val Pro Gln Gly Trp Arg Tyr Ser Arg Arg Pro Arg Ala Asp Gln
   35 40 45
   Pro Leu Lys Leu Gln Ile Ala Leu Ala Gln Gly Asp Thr Ala Gly Phe
   50 55 60
   Glu Glu Ala Val Met Asp Met Ser Thr Pro Asp His Pro Ser Tyr Gly
   65 70 75 80
   Asn His Phe His Thr His Glu Glu Met Lys Arg Met Leu Gln Pro Ser
   85 90 95
   Ala Glu Ser Ala Asp Ser Ile Arg Asp Trp Leu Glu Ser Ala Gly Ile
   100 105 110
   Asn Arg Ile Glu Gln Asp Ala Asp Trp Met Thr Phe Tyr Thr Thr Val
   115 120 125
   Glu Thr Ala Asn Glu Leu Leu Ala Ala Asn Phe Gln Phe Tyr Ala Asn
   130 135 140
   Ser Ala Lys His Ile Glu Arg Leu Arg Thr Leu Gln Tyr Ser Val Pro
   145 150 155 160
   Glu Ala Leu Met Pro His Ile Asn Met Ile Gln Pro Thr Thr Arg Phe
   165 170 175
   Gly Gln Leu Arg Val Gln Gly Ala Ile Leu His Thr Gln Val Lys Glu
   180 185 190
   Thr Asp Glu Ala Phe Arg Ser Asn Ala Val Ser Thr Ser Pro Asp Cys
   195 200 205
   Asn Ser Ile Ile Thr Pro Gln Cys Leu Lys Asn Met Tyr Asn Val Gly
   210 215 220
   Asp Tyr Gln Ala Asp Asp Asp Asn Gly Asn Lys Val Gly Phe Ala Ser
   225 230 235 240
   Tyr Leu Glu Glu Tyr Ala Arg Tyr Ser Asp Leu Glu Leu Phe Glu Lys
   245 250 255
   Asn Val Ala Pro Phe Ala Lys Gly Gln Asn Phe Ser Val Ile Gln Tyr
   260 265 270
   Asn Gly Gly Leu Asn Asp Gln His Ser Ser Ala Ser Ser Ser Glu Ala
   275 280 285
   Asn Leu Asp Leu Gln Tyr Ile Val Gly Val Ser Ser Pro Val Pro Val
   290 295 300
   Thr Glu Phe Ser Val Gly Gly Arg Gly Glu Leu Val Pro Asp Leu Asp
   305 310 315 320
   Gln Pro Asp Pro Asn Asp Asn Asn Asn Glu Pro Tyr Leu Glu Phe Leu
   325 330 335
   Gln Asn Val Leu Lys Met Glu Gln Gln Asp Leu Pro Gln Val Ile Ser
   340 345 350
   Thr Ser Tyr Gly Glu Asn Glu Gln Ser Val Pro Glu Lys Tyr Ala Arg
   355 360 365
   Thr Val Cys Asn Leu Phe Ser Gln Leu Gly Ser Arg Gly Val Ser Val
   370 375 380
   Ile Phe Ala Ser Gly Asp Ser Gly Val Gly Ala Ala Cys Gln Thr Asn
   385 390 395 400
   Asp Gly Arg Asn Ala Thr Arg Phe Pro Ala Gln Phe Pro Ala Ala Cys
   405 410 415
   Pro Trp Val Thr Ser Val Gly Ala Thr Thr His Thr Ala Pro Glu Lys
   420 425 430
   Ala Val Tyr Phe Ser Ser Gly Gly Phe Ser Asp Leu Trp Asp Arg Pro
   435 440 445
   Lys Trp Gln Glu Asp Ala Val Ser Asp Tyr Leu Asp Thr Leu Gly Asp
   450 455 460
   Arg Trp Ser Gly Leu Phe Asn Pro Lys Gly Arg Ala Phe Pro Asp Val
   465 470 475 480
   Ser Ala Gln Gly Gln Asn Tyr Ala Ile Tyr Asp Lys Gly Ser Leu Thr
   485 490 495
   Ser Val Asp Gly Thr Ser Cys Ser Ala Pro Ala Phe Ala Gly Val Ile
   500 505 510
   Ala Leu Leu Asn Asp Ala Arg Leu Lys Ala Asn Lys Pro Pro Met Gly
   515 520 525
   Phe Leu Asn Pro Trp Leu Tyr Ser Thr Gly Arg Asp Gly Leu Asn Asp
   530 535 540
   Ile Val His Gly Gly Ser Thr Gly Cys Asp Gly Asn Ala Arg Phe Gly
   545 550 555 560
   Gly Pro Gly Asn Gly Ser Pro Arg Val Pro Gly Ala Ser Trp Asn Ala
   565 570 575
   Thr Lys Gly Trp Asp Pro Val Ser Gly Leu Gly Ser Pro Asn Phe Ala
   580 585 590
   Thr Met Arg Lys Leu Ala Asn Gly Glu
   595 600
<210> 27
   <211> 594
   <212> PRT
   <213> Neosartorya fischeri
<400> 27
   Met Leu Ser Ser Thr Leu Tyr Ala Gly Leu Leu Cys Ser Leu Ala Ala
   1               5                   10                  15
   Pro Ala Leu Gly Val Val His Glu Lys Leu Ser Ala Val Pro Ser Gly
               20                  25                  30
   Trp Thr Leu Val Glu Asp Ala Ser Glu Ser Asp Thr Thr Thr Leu Ser
           35                  40                  45
   Ile Ala Leu Ala Arg Gln Asn Leu Asp Gln Leu Glu Ser Lys Leu Thr
       50                  55                  60
   Thr Leu Ala Thr Pro Gly Asn Ala Glu Tyr Gly Lys Trp Leu Asp Gln
   65                  70                  75                  80
   Ser Asp Ile Glu Ser Leu Phe Pro Thr Ala Ser Asp Asp Ala Val Ile
                   85                  90                  95
   Gln Trp Leu Lys Asp Ala Gly Val Thr Gln Val Ser Arg Gln Gly Ser
               100                 105                 110
   Leu Val Asn Phe Ala Thr Thr Val Gly Thr Ala Asn Lys Leu Phe Asp
           115                 120                 125
   Thr Lys Phe Ser Tyr Tyr Arg Asn Gly Ala Ser Gln Lys Leu Arg Thr
       130                 135                 140
   Thr Gln Tyr Ser Ile Pro Asp Ser Leu Thr Glu Ser Ile Asp Leu Ile
   145                 150                 155                 160
   Ala Pro Thr Val Phe Phe Gly Lys Glu Gln Asp Ser Ala Leu Pro Pro
                   165                 170                 175
   His Ala Val Lys Leu Pro Ala Leu Pro Arg Arg Ala Ala Thr Asn Ser
               180                 185                 190
   Ser Cys Ala Asn Leu Ile Thr Pro Asp Cys Leu Val Glu Met Tyr Asn
           195                 200                 205
   Leu Gly Asp Tyr Lys Pro Asp Ala Ser Ser Gly Ser Arg Val Gly Phe
       210                 215                 220
   Gly Ser Phe Leu Asn Gln Ser Ala Asn Tyr Ala Asp Leu Ala Ala Tyr
   225                 230                 235                 240
   Glu Gln Leu Phe Asn Ile Pro Pro Gln Asn Phe Ser Val Glu Leu Ile
                   245                 250                 255
   Asn Gly Gly Ala Asn Asp Gln Asn Trp Ala Thr Ala Ser Leu Gly Glu
               260                 265                 270
   Ala Asn Leu Asp Val Glu Leu Ile Val Ala Val Ser His Ala Leu Pro
           275                 280                 285
   Val Val Glu Phe Ile Thr Gly Gly Ser Pro Pro Phe Val Pro Asn Val
       290                 295                 300
   Asp Glu Pro Thr Ala Ala Asp Asn Gln Asn Glu Pro Tyr Leu Gln Tyr
   305                 310                 315                 320
   Tyr Glu Tyr Leu Leu Ser Lys Pro Asn Ser His Leu Pro Gln Val Ile
                   325                 330                 335
   Ser Asn Ser Tyr Gly Asp Asp Glu Gln Thr Val Pro Glu Tyr Tyr Ala
               340                 345                 350
   Arg Arg Val Cys Asn Leu Ile Gly Leu Met Gly Leu Arg Gly Ile Thr
           355                 360                 365
   Val Leu Glu Ser Ser Gly Asp Thr Gly Ile Gly Ser Ala Cys Met Ser
       370                 375                 380
   Asn Asp Gly Thr Asn Thr Pro Gln Phe Thr Pro Thr Phe Pro Gly Thr
   385 390 395 400
   Cys Pro Phe Ile Thr Ala Val Gly Gly Thr Gln Ser Tyr Ala Pro Glu
   405 410 415
   Val Ala Trp Asp Ala Ser Ser Gly Gly Phe Ser Asn Tyr Phe Ser Arg
   420 425 430
   Pro Trp Tyr Gln Tyr Phe Ala Val Glu Asn Tyr Leu Asn Asn His Ile
   435 440 445
   Thr Lys Asp Thr Lys Lys Tyr Tyr Ser Gln Tyr Thr Asn Phe Lys Gly
   450 455 460
   Arg Gly Phe Pro Asp Val Ser Ala His Ser Leu Thr Pro Asp Tyr Glu
   465 470 475 480
   Val Val Leu Thr Gly Lys His Tyr Lys Ser Gly Gly Thr Ser Ala Ala
   485 490 495
   Cys Pro Val Phe Ala Gly Ile Val Gly Leu Leu Asn Asp Ala Arg Leu
   500 505 510
   Arg Ala Gly Lys Ser Thr Leu Gly Phe Leu Asn Pro Leu Leu Tyr Ser
   515 520 525
   Ile Leu Ala Glu Gly Phe Thr Asp Ile Thr Ala Gly Ser Ser Ile Gly
   530 535 540
   Cys Asn Gly Ile Asn Pro Gln Thr Gly Lys Pro Val Pro Gly Gly Gly
   545 550 555 560
   Ile Ile Pro Tyr Ala His Trp Asn Ala Thr Ala Gly Trp Asp Pro Val
   565 570 575
   Thr Gly Leu Gly Val Pro Asp Phe Met Lys Leu Lys Glu Leu Val Leu
   580 585 590
   Ser Leu
<210> 28
   <211> 568
   <212> PRT
   <213> Aspergillus fumigatus
<400> 28
   Met Leu Ser Ser Thr Leu Tyr Ala Gly Trp Leu Leu Ser Leu Ala Ala
   1               5                   10                  15
   Pro Ala Leu Cys Val Val Gln Glu Lys Leu Ser Ala Val Pro Ser Gly
               20                  25                  30
   Trp Thr Leu Ile Glu Asp Ala Ser Glu Ser Asp Thr Ile Thr Leu Ser
           35                  40                  45
   Ile Ala Leu Ala Arg Gln Asn Leu Asp Gln Leu Glu Ser Lys Leu Thr
       50                  55                  60
   Thr Leu Ala Thr Pro Gly Asn Pro Glu Tyr Gly Lys Trp Leu Asp Gln
   65                  70                  75                  80
   Ser Asp Ile Glu Ser Leu Phe Pro Thr Ala Ser Asp Asp Ala Val Leu
                   85                  90                  95
   Gln Trp Leu Lys Ala Ala Gly Ile Thr Gln Val Ser Arg Gln Gly Ser
               100                 105                 110
   Leu Val Asn Phe Ala Thr Thr Val Gly Thr Ala Asn Lys Leu Phe Asp
           115                 120                 125
   Thr Lys Phe Ser Tyr Tyr Arg Asn Gly Ala Ser Gln Lys Leu Arg Thr
       130                 135                 140
   Thr Gln Tyr Ser Ile Pro Asp His Leu Thr Glu Ser Ile Asp Leu Ile
   145                 150                 155                 160
   Ala Pro Thr Val Phe Phe Gly Lys Glu Gln Asn Ser Ala Leu Ser Ser
                   165                 170                 175
   His Ala Val Lys Leu Pro Ala Leu Pro Arg Arg Ala Ala Thr Asn Ser
               180                 185                 190
   Ser Cys Ala Asn Leu Ile Thr Pro Asp Cys Leu Val Glu Met Tyr Asn
           195                 200                 205
   Leu Gly Asp Tyr Lys Pro Asp Ala Ser Ser Gly Ser Arg Val Gly Phe
       210                 215                 220
   Gly Ser Phe Leu Asn Glu Ser Ala Asn Tyr Ala Asp Leu Ala Ala Tyr
   225                 230                 235                 240
   Glu Gln Leu Phe Asn Ile Pro Pro Gln Asn Phe Ser Val Glu Leu Ile
                   245                 250                 255
   Asn Arg Gly Val Asn Asp Gln Asn Trp Ala Thr Ala Ser Leu Gly Glu
               260                 265                 270
   Ala Asn Leu Asp Val Glu Leu Ile Val Ala Val Ser His Pro Leu Pro
           275                 280                 285
   Val Val Glu Phe Ile Thr Gly Ala Leu Pro Pro Val Leu Arg Val Leu
       290                 295                 300
   Ala Leu Gln Thr Gln Leu Pro Ser Ser Ser Gly Asp Phe Gln Leu Thr
   305                 310                 315                 320
   Val Pro Glu Tyr Tyr Ala Arg Arg Val Cys Asn Leu Ile Gly Leu Met
                   325                 330                 335
   Gly Leu Arg Gly Ile Thr Val Leu Glu Ser Ser Gly Asp Thr Gly Ile
               340                 345                 350
   Gly Ser Ala Cys Met Ser Asn Asp Gly Thr Asn Lys Pro Gln Phe Thr
           355                 360                 365
   Pro Thr Phe Pro Gly Thr Cys Pro Phe Ile Thr Ala Val Gly Gly Thr
       370                 375                 380
   Gln Ser Tyr Ala Pro Glu Val Ala Trp Asp Gly Ser Ser Gly Gly Phe
   385                 390                 395                 400
   Ser Asn Tyr Phe Ser Arg Pro Trp Tyr Gln Ser Phe Ala Val Asp Asn
                   405                 410                 415
   Tyr Leu Asn Asn His Ile Thr Lys Asp Thr Lys Lys Tyr Tyr Ser Gln
               420                 425                 430
   Tyr Thr Asn Phe Lys Gly Arg Gly Phe Pro Asp Val Ser Ala His Ser
           435                 440                 445
   Leu Thr Pro Tyr Tyr Glu Val Val Leu Thr Gly Lys His Tyr Lys Ser
       450                 455                 460
   Gly Gly Thr Ser Ala Ala Ser Pro Val Phe Ala Gly Ile Val Gly Leu
   465                 470                 475                 480
   Leu Asn Asp Ala Arg Leu Arg Ala Gly Lys Ser Thr Leu Gly Phe Leu
                   485                 490                 495
   Asn Pro Leu Leu Tyr Ser Ile Leu Ala Glu Gly Phe Thr Asp Ile Thr
               500                 505                 510
   Ala Gly Ser Ser Ile Gly Cys Asn Gly Ile Asn Pro Gln Thr Gly Lys
           515                 520                 525
   Pro Val Pro Gly Gly Gly Ile Ile Pro Tyr Ala His Trp Asn Ala Thr
       530                 535                 540
   Ala Gly Trp Asp Pro Val Thr Gly Leu Gly Val Pro Asp Phe Met Lys
   545                 550                 555                 560
   Leu Lys Glu Leu Val Leu Ser Leu
                   565
<210> 29
   <211> 415
   <212> PRT
   <213> Trichoderma reesei
<400> 29
   Gln Glu Pro Ser Ser Cys Lys Gly Thr Leu Val Phe Glu Gly Glu Thr
   1               5                   10                  15
   Phe Asn Val Phe Gln Pro Asp Cys Leu Arg Thr Glu Tyr Ser Val Asp
               20                  25                  30
   Gly Tyr Thr Pro Ser Val Lys Ser Gly Ser Arg Ile Gly Phe Gly Ser
           35                  40                  45
   Phe Leu Asn Glu Ser Ala Ser Phe Ala Asp Gln Ala Leu Phe Glu Lys
       50                  55                  60
   His Phe Asn Ile Pro Ser Gln Asn Phe Ser Val Val Leu Ile Asn Gly
   65                  70                  75                  80
   Gly Thr Asp Leu Pro Gln Pro Pro Ser Asp Ala Asn Asp Gly Glu Ala
                   85                  90                  95
   Asn Leu Asp Ala Gln Thr Ile Leu Thr Ile Ala His Pro Leu Pro Ile
               100                 105                 110
   Thr Glu Phe Ile Thr Ala Gly Ser Pro Pro Tyr Phe Pro Asp Pro Val
           115                 120                 125
   Glu Pro Ala Gly Thr Pro Asn Glu Asn Glu Pro Tyr Leu Gln Tyr Tyr
       130                 135                 140
   Glu Phe Leu Leu Ser Lys Ser Asn Ala Glu Ile Pro Gln Val Ile Thr
   145                 150                 155                 160
   Asn Ser Tyr Gly Asp Glu Glu Gln Thr Val Pro Arg Ser Tyr Ala Val
   165 170 175
   Arg Val Cys Asn Leu Ile Gly Leu Leu Gly Leu Arg Gly Ile Ser Val
   180 185 190
   Leu His Ser Ser Gly Asp Glu Gly Val Gly Ala Ser Cys Val Ala Thr
   195 200 205
   Asn Ser Thr Thr Pro Gln Phe Asn Pro Ile Phe Pro Ala Thr Cys Pro
   210 215 220
   Tyr Val Thr Ser Val Gly Gly Thr Val Ser Phe Asn Pro Glu Val Ala
   225 230 235 240
   Trp Ala Gly Ser Ser Gly Gly Phe Ser Tyr Tyr Phe Ser Arg Pro Trp
   245 250 255
   Tyr Gln Gln Glu Ala Val Gly Thr Tyr Leu Glu Lys Tyr Val Ser Ala
   260 265 270
   Glu Thr Lys Lys Tyr Tyr Gly Pro Tyr Val Asp Phe Ser Gly Arg Gly
   275 280 285
   Phe Pro Asp Val Ala Ala His Ser Val Ser Pro Asp Tyr Pro Val Phe
   290 295 300
   Gln Gly Gly Glu Leu Thr Pro Ser Gly Gly Thr Ser Ala Ala Ser Pro
   305 310 315 320
   Val Val Ala Ala Ile Val Ala Leu Leu Asn Asp Ala Arg Leu Arg Glu
   325 330 335
   Gly Lys Pro Thr Leu Gly Phe Leu Asn Pro Leu Ile Tyr Leu His Ala
   340 345 350
   Ser Lys Gly Phe Thr Asp Ile Thr Ser Gly Gln Ser Glu Gly Cys Asn
   355 360 365
   Gly Asn Asn Thr Gln Thr Gly Ser Pro Leu Pro Gly Ala Gly Phe Ile
   370 375 380
   Ala Gly Ala His Trp Asn Ala Thr Lys Gly Trp Asp Pro Thr Thr Gly
   385 390 395 400
   Phe Gly Val Pro Asn Leu Lys Lys Leu Leu Ala Leu Val Arg Phe
   405 410 415
<210> 30
   <211> 391
   <212> PRT
   <213> Aspergillus oryzae
<400> 30
   Cys Asp Ser Ile Ile Thr Pro Thr Cys Leu Lys Glu Leu Tyr Asn Ile
   1               5                   10                  15
   Gly Asp Tyr Gln Ala Asp Ala Asn Ser Gly Ser Lys Ile Ala Phe Ala
               20                  25                  30
   Ser Tyr Leu Glu Glu Tyr Ala Arg Tyr Ala Asp Leu Glu Asn Phe Glu
           35                  40                  45
   Asn Tyr Leu Ala Pro Trp Ala Lys Gly Gln Asn Phe Ser Val Thr Thr
       50                  55                  60
   Phe Asn Gly Gly Leu Asn Asp Gln Asn Ser Ser Ser Asp Ser Gly Glu
   65                  70                  75                  80
   Ala Asn Leu Asp Leu Gln Tyr Ile Leu Gly Val Ser Ala Pro Leu Pro
                   85                  90                  95
   Val Thr Glu Phe Ser Thr Gly Gly Arg Gly Pro Leu Val Pro Asp Leu
               100                 105                 110
   Thr Gln Pro Asp Pro Asn Ser Asn Ser Asn Glu Pro Tyr Leu Glu Phe
           115                 120                 125
   Phe Gln Asn Val Leu Lys Leu Asp Gln Lys Asp Leu Pro Gln Val Ile
       130                 135                 140
   Ser Thr Ser Tyr Gly Glu Asn Glu Gln Glu Ile Pro Glu Lys Tyr Ala
   145                 150                 155                 160
   Arg Thr Val Cys Asn Leu Ile Ala Gln Leu Gly Ser Arg Gly Val Ser
                   165                 170                 175
   Val Leu Phe Ser Ser Gly Asp Ser Gly Val Gly Glu Gly Cys Met Thr
               180                 185                 190
   Asn Asp Gly Thr Asn Arg Thr His Phe Pro Pro Gln Phe Pro Ala Ala
           195                 200                 205
   Cys Pro Trp Val Thr Ser Val Gly Ala Thr Phe Lys Thr Thr Pro Glu
       210                 215                 220
   Arg Gly Thr Tyr Phe Ser Ser Gly Gly Phe Ser Asp Tyr Trp Pro Arg
   225                 230                 235                 240
   Pro Glu Trp Gln Asp Glu Ala Val Ser Ser Tyr Leu Glu Thr Ile Gly
                   245                 250                 255
   Asp Thr Phe Lys Gly Leu Tyr Asn Ser Ser Gly Arg Ala Phe Pro Asp
               260                 265                 270
   Val Ala Ala Gln Gly Met Asn Phe Ala Val Tyr Asp Lys Gly Thr Leu
           275                 280                 285
   Gly Glu Phe Asp Gly Thr Ser Ala Ser Ala Pro Ala Phe Ser Ala Val
       290                 295                 300
   Ile Ala Leu Leu Asn Asp Ala Arg Leu Arg Ala Gly Lys Pro Thr Leu
   305                 310                 315                 320
   Gly Phe Leu Asn Pro Trp Leu Tyr Lys Thr Gly Arg Gln Gly Leu Gln
                   325                 330                 335
   Asp Ile Thr Leu Gly Ala Ser Ile Gly Cys Thr Gly Arg Ala Arg Phe
               340                 345                 350
   Gly Gly Ala Pro Asp Gly Gly Pro Val Val Pro Tyr Ala Ser Trp Asn
           355                 360                 365
   Ala Thr Gln Gly Trp Asp Pro Val Thr Gly Leu Gly Thr Pro Asp Phe
       370                 375                 380
   Ala Glu Leu Lys Lys Leu Ala
   385                 390
<210> 31
   <211> 388
   <212> PRT
   <213> Phaeosphaeria nodorum
<400> 31
   Cys Asp Ala Thr Ile Thr Pro Gln Cys Leu Lys Thr Leu Tyr Lys Ile
   1               5                   10                  15
   Asp Tyr Lys Ala Asp Pro Lys Ser Gly Ser Lys Val Ala Phe Ala Ser
               20                  25                  30
   Tyr Leu Glu Gln Tyr Ala Arg Tyr Asn Asp Leu Ala Leu Phe Glu Lys
   35 40 45
   Ala Phe Leu Pro Glu Ala Val Gly Gln Asn Phe Ser Val Val Gln Phe
   50 55 60
   Ser Gly Gly Leu Asn Asp Gln Asn Thr Thr Gln Asp Ser Gly Glu Ala
   65 70 75 80
   Asn Leu Asp Leu Gln Tyr Ile Val Gly Val Ser Ala Pro Leu Pro Val
   85 90 95
   Thr Glu Phe Ser Thr Gly Gly Arg Gly Pro Trp Val Ala Asp Leu Asp
   100 105 110
   Gln Pro Asp Glu Ala Asp Ser Ala Asn Glu Pro Tyr Leu Glu Phe Leu
   115 120 125
   Gln Gly Val Leu Lys Leu Pro Gln Ser Glu Leu Pro Gln Val Ile Ser
   130 135 140
   Thr Ser Tyr Gly Glu Asn Glu Gln Ser Val Pro Lys Ser Tyr Ala Leu
   145 150 155 160
   Ser Val Cys Asn Leu Phe Ala Gln Leu Gly Ser Arg Gly Val Ser Val
   165 170 175
   Ile Phe Ser Ser Gly Asp Ser Gly Pro Gly Ser Ala Cys Gln Ser Asn
   180 185 190
   Asp Gly Lys Asn Thr Thr Lys Phe Gln Pro Gln Tyr Pro Ala Ala Cys
   195 200 205
   Pro Phe Val Thr Ser Val Gly Ser Thr Arg Tyr Leu Asn Glu Thr Ala
   210 215 220
   Thr Gly Phe Ser Ser Gly Gly Phe Ser Asp Tyr Trp Lys Arg Pro Ser
   225 230 235 240
   Tyr Gln Asp Asp Ala Val Lys Ala Tyr Phe His His Leu Gly Glu Lys
   245 250 255
   Phe Lys Pro Tyr Phe Asn Arg His Gly Arg Gly Phe Pro Asp Val Ala
   260 265 270
   Thr Gln Gly Tyr Gly Phe Arg Val Tyr Asp Gln Gly Lys Leu Lys Gly
   275 280 285
   Leu Gln Gly Thr Ser Ala Ser Ala Pro Ala Phe Ala Gly Val Ile Gly
       290                 295                 300
   Leu Leu Asn Asp Ala Arg Leu Lys Ala Lys Lys Pro Thr Leu Gly Phe
   305                 310                 315                 320
   Leu Asn Pro Leu Leu Tyr Ser Asn Ser Asp Ala Leu Asn Asp Ile Val
                   325                 330                 335
   Leu Gly Gly Ser Lys Gly Cys Asp Gly His Ala Arg Phe Asn Gly Pro
               340                 345                 350
   Pro Asn Gly Ser Pro Val Ile Pro Tyr Ala Gly Trp Asn Ala Thr Ala
           355                 360                 365
   Gly Trp Asp Pro Val Thr Gly Leu Gly Thr Pro Asn Phe Pro Lys Leu
       370                 375                 380
   Leu Lys Ala Ala
   385
<210> 32
   <211> 395
   <212> PRT
   <213> Trichoderma atroviride
<400> 32
   Val Phe Gln Pro Asp Cys Leu Arg Thr Glu Tyr Ser Val Asn Gly Tyr
   1               5                   10                  15
   Lys Pro Ser Ala Lys Ser Gly Ser Arg Ile Gly Phe Gly Ser Phe Leu
               20                  25                  30
   Asn Gln Ser Ala Ser Ser Ser Asp Leu Ala Leu Phe Glu Lys His Phe
           35                  40                  45
   Gly Phe Ala Ser Gln Gly Phe Ser Val Glu Leu Ile Asn Gly Gly Ser
       50                  55                  60
   Asn Pro Gln Pro Pro Thr Asp Ala Asn Asp Gly Glu Ala Asn Leu Asp
   65                  70                  75                  80
   Ala Gln Asn Ile Val Ser Phe Val Gln Pro Leu Pro Ile Thr Glu Phe
                   85                  90                  95
   Ile Ala Gly Gly Thr Ala Pro Tyr Phe Pro Asp Pro Val Glu Pro Ala
               100                 105                 110
   Gly Thr Pro Asp Glu Asn Glu Pro Tyr Leu Glu Tyr Tyr Glu Tyr Leu
   115 120 125
   Leu Ser Lys Ser Asn Lys Glu Leu Pro Gln Val Ile Thr Asn Ser Tyr
   130 135 140
   Gly Asp Glu Glu Gln Thr Val Pro Gln Ala Tyr Ala Val Arg Val Cys
   145 150 155 160
   Asn Leu Ile Gly Leu Met Gly Leu Arg Gly Ile Ser Ile Leu Glu Ser
   165 170 175
   Ser Gly Asp Glu Gly Val Gly Ala Ser Cys Leu Ala Thr Asn Ser Thr
   180 185 190
   Thr Thr Pro Gln Phe Asn Pro Ile Phe Pro Ala Thr Cys Pro Tyr Val
   195 200 205
   Thr Ser Val Gly Gly Thr Val Ser Phe Asn Pro Glu Val Ala Trp Asp
   210 215 220
   Gly Ser Ser Gly Gly Phe Ser Tyr Tyr Phe Ser Arg Pro Trp Tyr Gln
   225 230 235 240
   Glu Ala Ala Val Gly Thr Tyr Leu Asn Lys Tyr Val Ser Glu Glu Thr
   245 250 255
   Lys Glu Tyr Tyr Lys Ser Tyr Val Asp Phe Ser Gly Arg Gly Phe Pro
   260 265 270
   Asp Val Ala Ala His Ser Val Ser Pro Asp Tyr Pro Val Phe Gln Gly
   275 280 285
   Gly Glu Leu Thr Pro Ser Gly Gly Thr Ser Ala Ala Ser Pro Ile Val
   290 295 300
   Ala Ser Val Ile Ala Leu Leu Asn Asp Ala Arg Leu Arg Ala Gly Lys
   305 310 315 320
   Pro Ala Leu Gly Phe Leu Asn Pro Leu Ile Tyr Gly Tyr Ala Tyr Lys
   325 330 335
   Gly Phe Thr Asp Ile Thr Ser Gly Gln Ala Val Gly Cys Asn Gly Asn
   340 345 350
   Asn Thr Gln Thr Gly Gly Pro Leu Pro Gly Ala Gly Val Ile Pro Gly
   355 360 365
   Ala Phe Trp Asn Ala Thr Lys Gly Trp Asp Pro Thr Thr Gly Phe Gly
   370 375 380
   Val Pro Asn Phe Lys Lys Leu Leu Glu Leu Val
   385 390 395
<210> 33
   <211> 389
   <212> PRT
   <213> Arthroderma benhamiae
<400> 33
   Cys Arg Ser Leu Val Thr Thr Ala Cys Leu Arg Glu Leu Tyr Gly Leu
   1 5 10 15
   Gly Asp Arg Val Thr Gln Ala Arg Asp Asp Asn Arg Ile Gly Val Ser
   20 25 30
   Gly Phe Leu Glu Glu Tyr Ala Gln Tyr Arg Asp Leu Glu Leu Phe Leu
   35 40 45
   Ser Arg Phe Glu Pro Ser Ala Lys Gly Phe Asn Phe Ser Glu Gly Leu
   50 55 60
   Ile Ala Gly Gly Lys Asn Thr Gln Gly Gly Pro Gly Ser Ser Thr Glu
   65 70 75 80
   Ala Asn Leu Asp Met Gln Tyr Val Val Gly Leu Ser His Lys Ala Lys
   85 90 95
   Val Thr Tyr Tyr Ser Thr Ala Gly Arg Gly Pro Leu Ile Pro Asp Leu
   100 105 110
   Ser Gln Pro Ser Gln Ala Ser Asn Asn Asn Glu Pro Tyr Leu Glu Gln
   115 120 125
   Leu Arg Tyr Leu Val Lys Leu Pro Lys Asn Gln Leu Pro Ser Val Leu
   130 135 140
   Thr Thr Ser Tyr Gly Asp Thr Glu Gln Ser Leu Pro Ala Ser Tyr Thr
   145 150 155 160
   Lys Ala Thr Cys Asp Leu Phe Ala Gln Leu Gly Thr Met Gly Val Ser
   165 170 175
   Val Ile Phe Ser Ser Gly Asp Thr Gly Pro Gly Ser Ser Cys Gln Thr
   180 185 190
   Asn Asp Gly Lys Asn Ala Thr Arg Phe Asn Pro Ile Tyr Pro Ala Ser
   195 200 205
   Cys Pro Phe Val Thr Ser Ile Gly Gly Thr Val Gly Thr Gly Pro Glu
   210 215 220
   Arg Ala Val Ser Phe Ser Ser Gly Gly Phe Ser Asp Arg Phe Pro Arg
   225 230 235 240
   Pro Gln Tyr Gln Asp Asn Ala Val Lys Asp Tyr Leu Lys Ile Leu Gly
   245 250 255
   Asn Gln Trp Ser Gly Leu Phe Asp Pro Asn Gly Arg Ala Phe Pro Asp
   260 265 270
   Ile Ala Ala Gln Gly Ser Asn Tyr Ala Val Tyr Asp Lys Gly Arg Met
   275 280 285
   Thr Gly Val Ser Gly Thr Ser Ala Ser Ala Pro Ala Met Ala Ala Ile
   290 295 300
   Ile Ala Gln Leu Asn Asp Phe Arg Leu Ala Lys Gly Ser Pro Val Leu
   305 310 315 320
   Gly Phe Leu Asn Pro Trp Ile Tyr Ser Lys Gly Phe Ser Gly Phe Thr
   325 330 335
   Asp Ile Val Asp Gly Gly Ser Arg Gly Cys Thr Gly Tyr Asp Ile Tyr
   340 345 350
   Ser Gly Leu Lys Ala Lys Lys Val Pro Tyr Ala Ser Trp Asn Ala Thr
   355 360 365
   Lys Gly Trp Asp Pro Val Thr Gly Phe Gly Thr Pro Asn Phe Gln Ala
   370 375 380
   Leu Thr Lys Val Leu
   385
<210> 34
   <211> 397
   <212> PRT
   <213> Fusarium graminearum
<400> 34
   Cys Gln Thr Ser Ile Thr Pro Ser Cys Leu Lys Gln Met Tyr Asn Ile
   1 5 10 15
   Gly Asp Tyr Thr Pro Lys Val Glu Ser Gly Ser Thr Ile Gly Phe Ser
   20 25 30
   Ser Phe Leu Gly Glu Ser Ala Ile Tyr Ser Asp Val Phe Leu Phe Glu
   35 40 45
   Glu Lys Phe Gly Ile Pro Thr Gln Asn Phe Thr Thr Val Leu Ile Asn
   50 55 60
   Asn Gly Thr Asp Asp Gln Asn Thr Ala His Lys Asn Phe Gly Glu Ala
   65 70 75 80
   Asp Leu Asp Ala Glu Asn Ile Val Gly Ile Ala His Pro Leu Pro Phe
   85 90 95
   Thr Gln Tyr Ile Thr Gly Gly Ser Pro Pro Phe Leu Pro Asn Ile Asp
   100 105 110
   Gln Pro Thr Ala Ala Asp Asn Gln Asn Glu Pro Tyr Val Pro Phe Phe
   115 120 125
   Arg Tyr Leu Leu Ser Gln Lys Glu Val Pro Ala Val Val Ser Thr Ser
   130 135 140
   Tyr Gly Asp Glu Glu Asp Ser Val Pro Arg Glu Tyr Ala Thr Met Thr
   145 150 155 160
   Cys Asn Leu Ile Gly Leu Leu Gly Leu Arg Gly Ile Ser Val Ile Phe
   165 170 175
   Ser Ser Gly Asp Ile Gly Val Gly Ala Gly Cys Leu Gly Pro Asp His
   180 185 190
   Lys Thr Val Glu Phe Asn Ala Ile Phe Pro Ala Thr Cys Pro Tyr Leu
   195 200 205
   Thr Ser Val Gly Gly Thr Val Asp Val Thr Pro Glu Ile Ala Trp Glu
   210 215 220
   Gly Ser Ser Gly Gly Phe Ser Lys Tyr Phe Pro Arg Pro Ser Tyr Gln
   225 230 235 240
   Asp Lys Ala Val Lys Thr Tyr Met Lys Thr Val Ser Lys Gln Thr Lys
   245 250 255
   Lys Tyr Tyr Gly Pro Tyr Thr Asn Trp Glu Gly Arg Gly Phe Pro Asp
   260 265 270
   Val Ala Gly His Ser Val Ser Pro Asn Tyr Glu Val Ile Tyr Ala Gly
   275 280 285
   Lys Gln Ser Ala Ser Gly Gly Thr Ser Ala Ala Ala Pro Val Trp Ala
   290 295 300
   Ala Ile Val Gly Leu Leu Asn Asp Ala Arg Phe Arg Ala Gly Lys Pro
   305 310 315 320
   Ser Leu Gly Trp Leu Asn Pro Leu Val Tyr Lys Tyr Gly Pro Lys Val
   325 330 335
   Leu Thr Asp Ile Thr Gly Gly Tyr Ala Ile Gly Cys Asp Gly Asn Asn
   340 345 350
   Thr Gln Ser Gly Lys Pro Glu Pro Ala Gly Ser Gly Ile Val Pro Gly
   355 360 365
   Ala Arg Trp Asn Ala Thr Ala Gly Trp Asp Pro Val Thr Gly Tyr Gly
   370 375 380
   Thr Pro Asp Phe Gly Lys Leu Lys Asp Leu Val Leu Ser
   385 390 395
<210> 35
   <211> 408
   <212> PRT
   <213> Acremonium alcalophilum
<400> 35
   Cys Asp Leu Val Ile Thr Pro Pro Cys Leu Glu Ala Ala Tyr Asn Tyr
   1 5 10 15
   Lys Asn Tyr Met Pro Asp Pro Asn Ser Gly Ser Arg Val Ser Phe Thr
   20 25 30
   Ser Phe Leu Glu Gln Ala Ala Gln Gln Ser Asp Leu Thr Lys Phe Leu
   35 40 45
   Ser Leu Thr Gly Leu Asp Arg Leu Arg Pro Pro Ser Ser Lys Pro Ala
   50 55 60
   Ser Phe Asp Thr Val Leu Ile Asn Gly Gly Glu Thr His Gln Gly Thr
   65 70 75 80
   Pro Pro Asn Lys Thr Ser Glu Ala Asn Leu Asp Val Gln Trp Leu Ala
   85 90 95
   Ala Val Ile Lys Ala Arg Leu Pro Ile Thr Gln Trp Ile Thr Gly Gly
   100 105 110
   Arg Pro Pro Phe Val Pro Asn Leu Arg Leu Arg His Glu Lys Asp Asn
   115 120 125
   Thr Asn Glu Pro Tyr Leu Glu Phe Phe Glu Tyr Leu Val Arg Leu Pro
   130 135 140
   Ala Arg Asp Leu Pro Gln Val Ile Ser Asn Ser Tyr Ala Glu Asp Glu
   145 150 155 160
   Gln Thr Val Pro Glu Ala Tyr Ala Arg Arg Val Cys Asn Leu Ile Gly
   165 170 175
   Ile Met Gly Leu Arg Gly Val Thr Val Leu Thr Ala Ser Gly Asp Ser
   180 185 190
   Gly Val Gly Ala Pro Cys Arg Ala Asn Asp Gly Ser Asp Arg Leu Glu
   195 200 205
   Phe Ser Pro Gln Phe Pro Thr Ser Cys Pro Tyr Ile Thr Ala Val Gly
   210 215 220
   Gly Thr Glu Gly Trp Asp Pro Glu Val Ala Trp Glu Ala Ser Ser Gly
   225 230 235 240
   Gly Phe Ser His Tyr Phe Leu Arg Pro Trp Tyr Gln Ala Asn Ala Val
   245 250 255
   Glu Lys Tyr Leu Asp Glu Glu Leu Asp Pro Ala Thr Arg Ala Tyr Tyr
   260 265 270
   Asp Gly Asn Gly Phe Val Gln Phe Ala Gly Arg Ala Tyr Pro Asp Leu
   275 280 285
   Ser Ala His Ser Ser Ser Pro Arg Tyr Ala Tyr Ile Asp Lys Leu Ala
   290 295 300
   Pro Gly Leu Thr Gly Gly Thr Ser Ala Ser Cys Pro Val Val Ala Gly
   305 310 315 320
   Ile Val Gly Leu Leu Asn Asp Ala Arg Leu Arg Arg Gly Leu Pro Thr
   325 330 335
   Met Gly Phe Ile Asn Pro Trp Leu Tyr Thr Arg Gly Phe Glu Ala Leu
   340 345 350
   Gln Asp Val Thr Gly Gly Arg Ala Ser Gly Cys Gln Gly Ile Asp Leu
   355 360 365
   Gln Arg Gly Thr Arg Val Pro Gly Ala Gly Ile Ile Pro Trp Ala Ser
   370 375 380
   Trp Asn Ala Thr Pro Gly Trp Asp Pro Ala Thr Gly Leu Gly Leu Pro
   385 390 395 400
   Asp Phe Trp Ala Met Arg Gly Leu
   405
<210> 36
   <211> 410
   <212> PRT
   <213> Sodiomyces alkalinus
<400> 36
   Cys Ala Thr Ile Ile Thr Pro Pro Cys Leu Glu Thr Ala Tyr Asn Tyr
   1               5                   10                  15
   Lys Gly Tyr Ile Pro Asp Pro Lys Ser Gly Ser Arg Val Ser Phe Thr
               20                  25                  30
   Ser Phe Leu Glu Gln Ala Ala Gln Gln Ala Asp Leu Thr Lys Phe Leu
           35                  40                  45
   Ser Leu Thr Arg Leu Glu Gly Phe Arg Thr Pro Ala Ser Lys Lys Lys
       50                  55                  60
   Thr Phe Lys Thr Val Leu Ile Asn Gly Gly Glu Ser His Glu Gly Val
   65                  70                  75                  80
   His Lys Lys Ser Lys Thr Ser Glu Ala Asn Leu Asp Val Gln Trp Leu
                   85                  90                  95
   Ala Ala Val Thr Gln Thr Lys Leu Pro Ile Thr Gln Trp Ile Thr Gly
               100                 105                 110
   Gly Arg Pro Pro Phe Val Pro Asn Leu Arg Ile Pro Thr Pro Glu Ala
           115                 120                 125
   Asn Thr Asn Glu Pro Tyr Leu Glu Phe Leu Glu Tyr Leu Phe Arg Leu
       130                 135                 140
   Pro Asp Lys Asp Leu Pro Gln Val Ile Ser Asn Ser Tyr Ala Glu Asp
   145 150 155 160
   Glu Gln Ser Val Pro Glu Ala Tyr Ala Arg Arg Val Cys Gly Leu Leu
   165 170 175
   Gly Ile Met Gly Leu Arg Gly Val Thr Val Leu Thr Ala Ser Gly Asp
   180 185 190
   Ser Gly Val Gly Ala Pro Cys Arg Ala Asn Asp Gly Ser Gly Arg Glu
   195 200 205
   Glu Phe Ser Pro Gln Phe Pro Ser Ser Cys Pro Tyr Ile Thr Thr Val
   210 215 220
   Gly Gly Thr Gln Ala Trp Asp Pro Glu Val Ala Trp Lys Gly Ser Ser
   225 230 235 240
   Gly Gly Phe Ser Asn Tyr Phe Pro Arg Pro Trp Tyr Gln Val Ala Ala
   245 250 255
   Val Glu Lys Tyr Leu Glu Glu Gln Leu Asp Pro Ala Ala Arg Glu Tyr
   260 265 270
   Tyr Glu Glu Asn Gly Phe Val Arg Phe Ala Gly Arg Ala Phe Pro Asp
   275 280 285
   Leu Ser Ala His Ser Ser Ser Pro Lys Tyr Ala Tyr Val Asp Lys Arg
   290 295 300
   Val Pro Gly Leu Thr Gly Gly Thr Ser Ala Ser Cys Pro Val Val Ala
   305 310 315 320
   Gly Ile Val Gly Leu Leu Asn Asp Ala Arg Leu Arg Arg Gly Leu Pro
   325 330 335
   Thr Met Gly Phe Ile Asn Pro Trp Leu Tyr Ala Lys Gly Tyr Gln Ala
   340 345 350
   Leu Glu Asp Val Thr Gly Gly Ala Ala Val Gly Cys Gln Gly Ile Asp
   355 360 365
   Ile Gln Thr Gly Lys Arg Val Pro Gly Ala Gly Ile Ile Pro Gly Ala
   370 375 380
   Ser Trp Asn Ala Thr Pro Asp Trp Asp Pro Ala Thr Gly Leu Gly Leu
   385 390 395 400
   Pro Asn Phe Trp Ala Met Arg Glu Leu Ala
   405 410
<210> 37
   <211> 400
   <212> PRT
   <213> Aspergillus kawachii
<400> 37
   Cys Ala Asp Thr Ile Thr Leu Ser Cys Leu Lys Glu Met Tyr Asn Phe
   1 5 10 15
   Gly Asn Tyr Thr Pro Ser Ala Ser Ser Gly Ser Lys Leu Gly Phe Ala
   20 25 30
   Ser Phe Leu Asn Glu Ser Ala Ser Tyr Ser Asp Leu Ala Lys Phe Glu
   35 40 45
   Arg Leu Phe Asn Leu Pro Ser Gln Asn Phe Ser Val Glu Leu Ile Asn
   50 55 60
   Gly Gly Val Asn Asp Gln Asn Gln Ser Thr Ala Ser Leu Thr Glu Ala
   65 70 75 80
   Asp Leu Asp Val Glu Leu Leu Val Gly Val Gly His Pro Leu Pro Val
   85 90 95
   Thr Glu Phe Ile Thr Ser Gly Glu Pro Pro Phe Ile Pro Asp Pro Asp
   100 105 110
   Glu Pro Ser Ala Ala Asp Asn Glu Asn Glu Pro Tyr Leu Gln Tyr Tyr
   115 120 125
   Glu Tyr Leu Leu Ser Lys Pro Asn Ser Ala Leu Pro Gln Val Ile Ser
   130 135 140
   Asn Ser Tyr Gly Asp Asp Glu Gln Thr Val Pro Glu Tyr Tyr Ala Lys
   145 150 155 160
   Arg Val Cys Asn Leu Ile Gly Leu Val Gly Leu Arg Gly Ile Ser Val
   165 170 175
   Leu Glu Ser Ser Gly Asp Glu Gly Ile Gly Ser Gly Cys Arg Thr Thr
   180 185 190
   Asp Gly Thr Asn Ser Thr Gln Phe Asn Pro Ile Phe Pro Ala Thr Cys
   195 200 205
   Pro Tyr Val Thr Ala Val Gly Gly Thr Met Ser Tyr Ala Pro Glu Ile
   210 215 220
   Ala Trp Glu Ala Ser Ser Gly Gly Phe Ser Asn Tyr Phe Glu Arg Ala
   225 230 235 240
   Trp Phe Gln Lys Glu Ala Val Gln Asn Tyr Leu Ala Asn His Ile Thr
   245 250 255
   Asn Glu Thr Lys Gln Tyr Tyr Ser Gln Phe Ala Asn Phe Ser Gly Arg
   260 265 270
   Gly Phe Pro Asp Val Ser Ala His Ser Phe Glu Pro Ser Tyr Glu Val
   275 280 285
   Ile Phe Tyr Gly Ala Arg Tyr Gly Ser Gly Gly Thr Ser Ala Ala Cys
   290 295 300
   Pro Leu Phe Ser Ala Leu Val Gly Met Leu Asn Asp Ala Arg Leu Arg
   305 310 315 320
   Ala Gly Lys Ser Thr Leu Gly Phe Leu Asn Pro Leu Leu Tyr Ser Lys
   325 330 335
   Gly Tyr Lys Ala Leu Thr Asp Val Thr Ala Gly Gln Ser Ile Gly Cys
   340 345 350
   Asn Gly Ile Asp Pro Gln Ser Asp Glu Ala Val Ala Gly Ala Gly Ile
   355 360 365
   Ile Pro Trp Ala His Trp Asn Ala Thr Val Gly Trp Asp Pro Val Thr
   370 375 380
   Gly Leu Gly Leu Pro Asp Phe Glu Lys Leu Arg Gln Leu Val Leu Ser
   385 390 395 400
<210> 38
   <211> 396
   <212> PRT
   <213> Talaromyces stipitatus
<400> 38
   Cys Gln Thr Ser Ile Thr Pro Ala Cys Leu Lys Gln Met Tyr Asn Val
   1 5 10 15
   Gly Asn Tyr Thr Pro Ser Val Ala His Gly Ser Arg Val Gly Phe Gly
   20 25 30
   Ser Phe Leu Asn Gln Ser Ala Ile Phe Asp Asp Leu Phe Thr Tyr Glu
           35                  40                  45
   Lys Val Asn Asp Ile Pro Ser Gln Asn Phe Thr Lys Val Ile Ile Ala
       50                  55                  60
   Asn Ala Ser Asn Ser Gln Asp Ala Ser Asp Gly Asn Tyr Gly Glu Ala
   65                  70                  75                  80
   Asn Leu Asp Val Gln Asn Ile Val Gly Ile Ser His Pro Leu Pro Val
                   85                  90                  95
   Thr Glu Phe Leu Thr Gly Gly Ser Pro Pro Phe Val Ala Ser Leu Asp
               100                 105                 110
   Thr Pro Thr Asn Gln Asn Glu Pro Tyr Ile Pro Tyr Tyr Glu Tyr Leu
           115                 120                 125
   Leu Ser Gln Lys Asn Glu Asp Leu Pro Gln Val Ile Ser Asn Ser Tyr
       130                 135                 140
   Gly Asp Asp Glu Gln Ser Val Pro Tyr Lys Tyr Ala Ile Arg Ala Cys
   145                 150                 155                 160
   Asn Leu Ile Gly Leu Thr Gly Leu Arg Gly Ile Ser Val Leu Glu Ser
                   165                 170                 175
   Ser Gly Asp Leu Gly Val Gly Ala Gly Cys Arg Ser Asn Asp Gly Lys
               180                 185                 190
   Asn Lys Thr Gln Phe Asp Pro Ile Phe Pro Ala Thr Cys Pro Tyr Val
           195                 200                 205
   Thr Ser Val Gly Gly Thr Gln Ser Val Thr Pro Glu Ile Ala Trp Val
       210                 215                 220
   Ala Ser Ser Gly Gly Phe Ser Asn Tyr Phe Pro Arg Thr Trp Tyr Gln
   225                 230                 235                 240
   Glu Pro Ala Ile Gln Thr Tyr Leu Gly Leu Leu Asp Asp Glu Thr Lys
                   245                 250                 255
   Thr Tyr Tyr Ser Gln Tyr Thr Asn Phe Glu Gly Arg Gly Phe Pro Asp
               260                 265                 270
   Val Ser Ala His Ser Leu Thr Pro Asp Tyr Gln Val Val Gly Gly Gly
           275                 280                 285
   Tyr Leu Gln Pro Ser Gly Gly Thr Ser Ala Ala Ser Pro Val Phe Ala
   290 295 300
   Gly Ile Ile Ala Leu Leu Asn Asp Ala Arg Leu Ala Ala Gly Lys Pro
   305 310 315 320
   Thr Leu Gly Phe Leu Asn Pro Phe Phe Tyr Leu Tyr Gly Tyr Lys Gly
   325 330 335
   Leu Asn Asp Ile Thr Gly Gly Gln Ser Val Gly Cys Asn Gly Ile Asn
   340 345 350
   Gly Gln Thr Gly Ala Pro Val Pro Gly Gly Gly Ile Val Pro Gly Ala
   355 360 365
   Ala Trp Asn Ser Thr Thr Gly Trp Asp Pro Ala Thr Gly Leu Gly Thr
   370 375 380
   Pro Asp Phe Gln Lys Leu Lys Glu Leu Val Leu Ser
   385 390 395
<210> 39
   <211> 397
   <212> PRT
   <213> Fusarium oxysporum
<400> 39
   Cys Gln Thr Ser Ile Thr Pro Ser Cys Leu Lys Gln Met Tyr Asn Ile
   1 5 10 15
   Gly Asp Tyr Thr Pro Asp Ala Lys Ser Gly Ser Glu Ile Gly Phe Ser
   20 25 30
   Ser Phe Leu Gly Gln Ala Ala Ile Tyr Ser Asp Val Phe Lys Phe Glu
   35 40 45
   Glu Leu Phe Gly Ile Pro Lys Gln Asn Tyr Thr Thr Ile Leu Ile Asn
   50 55 60
   Asn Gly Thr Asp Asp Gln Asn Thr Ala His Gly Asn Phe Gly Glu Ala
   65 70 75 80
   Asn Leu Asp Ala Glu Asn Ile Val Gly Ile Ala His Pro Leu Pro Phe
   85 90 95
   Lys Gln Tyr Ile Thr Gly Gly Ser Pro Pro Phe Val Pro Asn Ile Asp
   100 105 110
   Gln Pro Thr Glu Lys Asp Asn Gln Asn Glu Pro Tyr Val Pro Phe Phe
   115 120 125
   Arg Tyr Leu Leu Gly Gln Lys Asp Leu Pro Ala Val Ile Ser Thr Ser
   130 135 140
   Tyr Gly Asp Glu Glu Asp Ser Val Pro Arg Glu Tyr Ala Thr Leu Thr
   145 150 155 160
   Cys Asn Met Ile Gly Leu Leu Gly Leu Arg Gly Ile Ser Val Ile Phe
   165 170 175
   Ser Ser Gly Asp Ile Gly Val Gly Ser Gly Cys Leu Ala Pro Asp Tyr
   180 185 190
   Lys Thr Val Glu Phe Asn Ala Ile Phe Pro Ala Thr Cys Pro Tyr Leu
   195 200 205
   Thr Ser Val Gly Gly Thr Val Asp Val Thr Pro Glu Ile Ala Trp Glu
   210 215 220
   Gly Ser Ser Gly Gly Phe Ser Lys Tyr Phe Pro Arg Pro Ser Tyr Gln
   225 230 235 240
   Asp Lys Ala Ile Lys Lys Tyr Met Lys Thr Val Ser Lys Glu Thr Lys
   245 250 255
   Lys Tyr Tyr Gly Pro Tyr Thr Asn Trp Glu Gly Arg Gly Phe Pro Asp
   260 265 270
   Val Ala Gly His Ser Val Ala Pro Asp Tyr Glu Val Ile Tyr Asn Gly
   275 280 285
   Lys Gln Ala Arg Ser Gly Gly Thr Ser Ala Ala Ala Pro Val Trp Ala
   290 295 300
   Ala Ile Val Gly Leu Leu Asn Asp Ala Arg Phe Lys Ala Gly Lys Lys
   305 310 315 320
   Ser Leu Gly Trp Leu Asn Pro Leu Ile Tyr Lys His Gly Pro Lys Val
   325 330 335
   Leu Thr Asp Ile Thr Gly Gly Tyr Ala Ile Gly Cys Asp Gly Asn Asn
   340 345 350
   Thr Gln Ser Gly Lys Pro Glu Pro Ala Gly Ser Gly Leu Val Pro Gly
   355 360 365
   Ala Arg Trp Asn Ala Thr Ala Gly Trp Asp Pro Thr Thr Gly Tyr Gly
       370                 375                 380
   Thr Pro Asn Phe Gln Lys Leu Lys Asp Leu Val Leu Ser
   385                 390                 395
<210> 40
   <211> 395
   <212> PRT
   <213> Trichoderma virens
<400> 40
   Val Phe Gln Pro Asp Cys Leu Arg Thr Glu Tyr Asn Val Asn Gly Tyr
   1               5                   10                  15
   Thr Pro Ser Ala Lys Ser Gly Ser Arg Ile Gly Phe Gly Ser Phe Leu
               20                  25                  30
   Asn Gln Ser Ala Ser Phe Ser Asp Leu Ala Leu Phe Glu Lys His Phe
           35                  40                  45
   Gly Phe Ser Ser Gln Asn Phe Ser Val Val Leu Ile Asn Gly Gly Thr
       50                  55                  60
   Asp Leu Pro Gln Pro Pro Ser Asp Asp Asn Asp Gly Glu Ala Asn Leu
   65                  70                  75                  80
   Asp Val Gln Asn Ile Leu Thr Ile Ala His Pro Leu Pro Ile Thr Glu
                   85                  90                  95
   Phe Ile Thr Ala Gly Ser Pro Pro Tyr Phe Pro Asp Pro Val Glu Pro
               100                 105                 110
   Ala Gly Thr Pro Asp Glu Asn Glu Pro Tyr Leu Gln Tyr Phe Glu Tyr
           115                 120                 125
   Leu Leu Ser Lys Pro Asn Arg Asp Leu Pro Gln Val Ile Thr Asn Ser
       130                 135                 140
   Tyr Gly Asp Glu Glu Gln Thr Val Pro Gln Ala Tyr Ala Val Arg Val
   145                 150                 155                 160
   Cys Asn Leu Ile Gly Leu Met Gly Leu Arg Gly Ile Ser Ile Leu Glu
                   165                 170                 175
   Ser Ser Gly Asp Glu Gly Val Gly Ala Ser Cys Val Ala Thr Asn Ser
               180                 185                 190
   Thr Thr Pro Gln Phe Asn Pro Ile Phe Pro Ala Thr Cys Pro Tyr Val
   195 200 205
   Thr Ser Val Gly Gly Thr Val Asn Phe Asn Pro Glu Val Ala Trp Asp
   210 215 220
   Gly Ser Ser Gly Gly Phe Ser Tyr Tyr Phe Ser Arg Pro Trp Tyr Gln
   225 230 235 240
   Glu Glu Ala Val Gly Asn Tyr Leu Glu Lys His Val Ser Ala Glu Thr
   245 250 255
   Lys Lys Tyr Tyr Gly Pro Tyr Val Asp Phe Ser Gly Arg Gly Phe Pro
   260 265 270
   Asp Val Ala Ala His Ser Val Ser Pro Asp Tyr Pro Val Phe Gln Gly
   275 280 285
   Gly Gln Leu Thr Pro Ser Gly Gly Thr Ser Ala Ala Ser Pro Val Val
   290 295 300
   Ala Ser Ile Ile Ala Leu Leu Asn Asp Ala Arg Leu Arg Glu Gly Lys
   305 310 315 320
   Pro Thr Leu Gly Phe Leu Asn Pro Leu Ile Tyr Gln Tyr Ala Tyr Lys
   325 330 335
   Gly Phe Thr Asp Ile Thr Ser Gly Gln Ser Asp Gly Cys Asn Gly Asn
   340 345 350
   Asn Thr Gln Thr Asp Ala Pro Leu Pro Gly Ala Gly Val Val Leu Gly
   355 360 365
   Ala His Trp Asn Ala Thr Lys Gly Trp Asp Pro Thr Thr Gly Phe Gly
   370 375 380
   Val Pro Asn Phe Lys Lys Leu Leu Glu Leu Ile
   385 390 395
<210> 41
   <211> 398
   <212> PRT
   <213> Trichoderma atroviride
<400> 41
   Gln Ile Phe His Pro Asp Cys Leu Lys Thr Lys Tyr Gly Val Asp Gly
   1 5 10 15
   Tyr Ala Pro Ser Pro Arg Cys Gly Ser Arg Ile Gly Phe Gly Ser Phe
               20                  25                  30
   Leu Asn Glu Thr Ala Ser Tyr Ser Asp Leu Ala Gln Phe Glu Lys Tyr
           35                  40                  45
   Phe Asp Leu Pro Asn Gln Asn Leu Ser Thr Leu Leu Ile Asn Gly Ala
       50                  55                  60
   Ile Asp Val Gln Pro Pro Ser Asn Lys Asn Asp Ser Glu Ala Asn Met
   65                  70                  75                  80
   Asp Val Gln Thr Ile Leu Thr Phe Val Gln Pro Leu Pro Ile Thr Glu
                   85                  90                  95
   Phe Val Val Ala Gly Ile Pro Pro Tyr Ile Pro Asp Ala Ala Leu Pro
               100                 105                 110
   Ile Gly Asp Pro Val Gln Asn Glu Pro Trp Leu Glu Tyr Phe Glu Phe
           115                 120                 125
   Leu Met Ser Arg Thr Asn Ala Glu Leu Pro Gln Val Ile Ala Asn Ser
       130                 135                 140
   Tyr Gly Asp Glu Glu Gln Thr Val Pro Gln Ala Tyr Ala Val Arg Val
   145                 150                 155                 160
   Cys Asn Gln Ile Gly Leu Leu Gly Leu Arg Gly Ile Ser Val Ile Ala
                   165                 170                 175
   Ser Ser Gly Asp Thr Gly Val Gly Met Ser Cys Met Ala Ser Asn Ser
               180                 185                 190
   Thr Thr Pro Gln Phe Asn Pro Met Phe Pro Ala Ser Cys Pro Tyr Ile
           195                 200                 205
   Thr Thr Val Gly Gly Thr Gln His Leu Asp Asn Glu Ile Ala Trp Glu
       210                 215                 220
   Leu Ser Ser Gly Gly Phe Ser Asn Tyr Phe Thr Arg Pro Trp Tyr Gln
   225                 230                 235                 240
   Glu Asp Ala Ala Lys Thr Tyr Leu Glu Arg His Val Ser Thr Glu Thr
                   245                 250                 255
   Lys Ala Tyr Tyr Glu Arg Tyr Ala Asn Phe Leu Gly Arg Gly Phe Pro
   260 265 270
   Asp Val Ala Ala Leu Ser Leu Asn Pro Asp Tyr Pro Val Ile Ile Gly
   275 280 285
   Gly Glu Leu Gly Pro Asn Gly Gly Thr Ser Ala Ala Ala Pro Val Val
   290 295 300
   Ala Ser Ile Ile Ala Leu Leu Asn Asp Ala Arg Leu Cys Leu Gly Lys
   305 310 315 320
   Pro Ala Leu Gly Phe Leu Asn Pro Leu Ile Tyr Gln Tyr Ala Asp Lys
   325 330 335
   Gly Gly Phe Thr Asp Ile Thr Ser Gly Gln Ser Trp Gly Cys Ala Gly
   340 345 350
   Asn Thr Thr Gln Thr Gly Pro Pro Pro Pro Gly Ala Gly Val Ile Pro
   355 360 365
   Gly Ala His Trp Asn Ala Thr Lys Gly Trp Asp Pro Val Thr Gly Phe
   370 375 380
   Gly Thr Pro Asn Phe Lys Lys Leu Leu Ser Leu Ala Leu Ser
   385 390 395
<210> 42
   <211> 363
   <212> PRT
   <213> Agaricus bisporus
<400> 42
   Thr Val Ile Thr Pro Asp Cys Leu Arg Asp Leu Tyr Asn Thr Ala Asp
   1               5                   10                  15
   Tyr Val Pro Ser Ala Thr Ser Arg Asn Ala Ile Gly Ile Ala Gly Tyr
               20                  25                  30
   Leu Asp Arg Ser Asn Arg Ala Asp Leu Gln Thr Phe Phe Arg Arg Phe
           35                  40                  45
   Arg Pro Asp Ala Val Gly Phe Asn Tyr Thr Thr Val Gln Leu Asn Gly
       50                  55                  60
   Gly Gly Asp Asp Gln Asn Asp Pro Gly Val Glu Ala Asn Leu Asp Ile
   65                  70                  75                  80
   Gln Tyr Ala Ala Gly Ile Ala Phe Pro Thr Pro Ala Thr Tyr Trp Ser
   85 90 95
   Thr Gly Gly Ser Pro Pro Phe Ile Pro Asp Thr Gln Thr Pro Thr Asn
   100 105 110
   Thr Asn Glu Pro Tyr Leu Asp Trp Ile Asn Phe Val Leu Gly Gln Asp
   115 120 125
   Glu Ile Pro Gln Val Ile Ser Thr Ser Tyr Gly Asp Asp Glu Gln Thr
   130 135 140
   Val Pro Glu Asp Tyr Ala Thr Ser Val Cys Asn Leu Phe Ala Gln Leu
   145 150 155 160
   Gly Ser Arg Gly Val Thr Val Phe Phe Ser Ser Gly Asp Phe Gly Val
   165 170 175
   Gly Gly Gly Asp Cys Leu Thr Asn Asp Gly Ser Asn Gln Val Leu Phe
   180 185 190
   Gln Pro Ala Phe Pro Ala Ser Cys Pro Phe Val Thr Ala Val Gly Gly
   195 200 205
   Thr Val Arg Leu Asp Pro Glu Ile Ala Val Ser Phe Ser Gly Gly Gly
   210 215 220
   Phe Ser Arg Tyr Phe Ser Arg Pro Ser Tyr Gln Asn Gln Thr Val Ala
   225 230 235 240
   Gln Phe Val Ser Asn Leu Gly Asn Thr Phe Asn Gly Leu Tyr Asn Lys
   245 250 255
   Asn Gly Arg Ala Tyr Pro Asp Leu Ala Ala Gln Gly Asn Gly Phe Gln
   260 265 270
   Val Val Ile Asp Gly Ile Val Arg Ser Val Gly Gly Thr Ser Ala Ser
   275 280 285
   Ser Pro Thr Val Ala Gly Ile Phe Ala Leu Leu Asn Asp Phe Lys Leu
   290 295 300
   Ser Arg Gly Gln Ser Thr Leu Gly Phe Ile Asn Pro Leu Ile Tyr Ser
   305 310 315 320
   Ser Ala Thr Ser Gly Phe Asn Asp Ile Arg Ala Gly Thr Asn Pro Gly
   325 330 335
   Cys Gly Thr Arg Gly Phe Thr Ala Gly Thr Gly Trp Asp Pro Val Thr
               340                 345                 350
   Gly Leu Gly Thr Pro Asp Phe Leu Arg Leu Gln
           355                 360
<210> 43
   <211> 376
   <212> PRT
   <213> Magnaporthe oryzae
<400> 43
   Gly Val Thr Pro Leu Cys Leu Arg Thr Leu Tyr Arg Val Asn Tyr Lys
   1               5                   10                  15
   Pro Ala Thr Thr Gly Asn Leu Val Ala Phe Ala Ser Phe Leu Glu Gln
               20                  25                  30
   Tyr Ala Arg Tyr Ser Asp Gln Gln Ala Phe Thr Gln Arg Val Leu Gly
           35                  40                  45
   Pro Gly Val Pro Leu Gln Asn Phe Ser Val Glu Thr Val Asn Gly Gly
       50                  55                  60
   Ala Asn Asp Gln Gln Ser Lys Leu Asp Ser Gly Glu Ala Asn Leu Asp
   65                  70                  75                  80
   Leu Gln Tyr Val Met Ala Met Ser His Pro Ile Pro Ile Leu Glu Tyr
                   85                  90                  95
   Ser Thr Gly Gly Arg Gly Pro Leu Val Pro Thr Leu Asp Gln Pro Asn
               100                 105                 110
   Ala Asn Asn Ser Ser Asn Glu Pro Tyr Leu Glu Phe Leu Thr Tyr Leu
           115                 120                 125
   Leu Ala Gln Pro Asp Ser Ala Ile Pro Gln Thr Leu Ser Val Ser Tyr
       130                 135                 140
   Gly Glu Glu Glu Gln Ser Val Pro Arg Asp Tyr Ala Ile Lys Val Cys
   145                 150                 155                 160
   Asn Met Phe Met Gln Leu Gly Ala Arg Gly Val Ser Val Met Phe Ser
                   165                 170                 175
   Ser Gly Asp Ser Gly Pro Gly Asn Asp Cys Val Arg Ala Ser Asp Asn
               180                 185                 190
   Ala Thr Phe Phe Gly Ser Thr Phe Pro Ala Gly Cys Pro Tyr Val Thr
           195                 200                 205
   Ser Val Gly Ser Thr Val Gly Phe Glu Pro Glu Arg Ala Val Ser Phe
       210                 215                 220
   Ser Ser Gly Gly Phe Ser Ile Tyr His Ala Arg Pro Asp Tyr Gln Asn
   225                 230                 235                 240
   Glu Val Val Pro Lys Tyr Ile Glu Ser Ile Lys Ala Ser Gly Tyr Glu
                   245                 250                 255
   Lys Phe Phe Asp Gly Asn Gly Arg Gly Ile Pro Asp Val Ala Ala Gln
               260                 265                 270
   Gly Ala Arg Phe Val Val Ile Asp Lys Gly Arg Val Ser Leu Ile Ser
           275                 280                 285
   Gly Thr Ser Ala Ser Ser Pro Ala Phe Ala Gly Met Val Ala Leu Val
       290                 295                 300
   Asn Ala Ala Arg Lys Ser Lys Asp Met Pro Ala Leu Gly Phe Leu Asn
   305                 310                 315                 320
   Pro Met Leu Tyr Gln Asn Ala Ala Ala Met Thr Asp Ile Val Asn Gly
                   325                 330                 335
   Ala Gly Ile Gly Cys Arg Lys Gln Arg Thr Glu Phe Pro Asn Gly Ala
               340                 345                 350
   Arg Phe Asn Ala Thr Ala Gly Trp Asp Pro Val Thr Gly Leu Gly Thr
           355                 360                 365
   Pro Leu Phe Asp Lys Leu Leu Ala
       370                 375
<210> 44
   <211> 388
   <212> PRT
   <213> Togninia minima
<400> 44
   Cys Asn Ala Ser Ile Thr Pro Glu Cys Leu Arg Ala Leu Tyr Asn Val
   1               5                   10                  15
   Gly Asp Tyr Glu Ala Asp Pro Ser Lys Lys Ser Leu Phe Gly Val Cys
               20                  25                  30
   Gly Tyr Leu Glu Gln Tyr Ala Lys His Asp Gln Leu Ala Lys Phe Glu
           35                  40                  45
   Gln Thr Tyr Ala Pro Tyr Ala Ile Gly Ala Asp Phe Ser Val Val Thr
       50                  55                  60
   Ile Asn Gly Gly Gly Asp Asn Gln Thr Ser Thr Ile Asp Asp Gly Glu
   65                  70                  75                  80
   Ala Asn Leu Asp Met Gln Tyr Ala Val Ser Met Ala Tyr Lys Thr Pro
                   85                  90                  95
   Ile Thr Tyr Tyr Ser Thr Gly Gly Arg Gly Pro Leu Val Pro Asp Leu
               100                 105                 110
   Asp Gln Pro Asp Pro Asn Asp Val Ser Asn Glu Pro Tyr Leu Asp Phe
           115                 120                 125
   Val Ser Tyr Leu Leu Lys Leu Pro Asp Ser Lys Leu Pro Gln Thr Ile
       130                 135                 140
   Thr Thr Ser Tyr Gly Glu Asp Glu Gln Ser Val Pro Arg Ser Tyr Val
   145                 150                 155                 160
   Glu Lys Val Cys Thr Met Phe Gly Ala Leu Gly Ala Arg Gly Val Ser
                   165                 170                 175
   Val Ile Phe Ser Ser Gly Asp Thr Gly Val Gly Ser Ala Cys Gln Thr
               180                 185                 190
   Asn Asp Gly Lys Asn Thr Thr Arg Phe Leu Pro Ile Phe Pro Ala Ala
           195                 200                 205
   Cys Pro Tyr Val Thr Ser Val Gly Gly Thr Arg Tyr Val Asp Pro Glu
       210                 215                 220
   Val Ala Val Ser Phe Ser Ser Gly Gly Phe Ser Asp Ile Phe Pro Thr
   225                 230                 235                 240
   Pro Leu Tyr Gln Lys Gly Ala Val Ser Gly Tyr Leu Lys Ile Leu Gly
                   245                 250                 255
   Asp Arg Trp Lys Gly Leu Tyr Asn Pro His Gly Arg Gly Phe Pro Asp
               260                 265                 270
   Val Ser Gly Gln Ser Val Arg Tyr His Val Phe Asp Tyr Gly Lys Asp
           275                 280                 285
   Val Met Tyr Ser Gly Thr Ser Ala Ser Ala Pro Met Phe Ala Ala Leu
   290 295 300
   Val Ser Leu Leu Asn Asn Ala Arg Leu Ala Lys Lys Leu Pro Pro Met
   305 310 315 320
   Gly Phe Leu Asn Pro Trp Leu Tyr Thr Val Gly Phe Asn Gly Leu Thr
   325 330 335
   Asp Ile Val His Gly Gly Ser Thr Gly Cys Thr Gly Thr Asp Val Tyr
   340 345 350
   Ser Gly Leu Pro Thr Pro Phe Val Pro Tyr Ala Ser Trp Asn Ala Thr
   355 360 365
   Val Gly Trp Asp Pro Val Thr Gly Leu Gly Thr Pro Leu Phe Asp Lys
   370 375 380
   Leu Leu Asn Leu
   385
<210> 45
   <211> 390
   <212> PRT
   <213> Bipolaris maydis
<400> 45
   Cys Asn Lys Lys Ile Thr Pro Asp Cys Leu Ala Asn Leu Tyr Asn Phe
   1 5 10 15
   Lys Asp Tyr Asp Ala Ser Asp Ala Asn Val Thr Ile Gly Val Ser Gly
   20 25 30
   Phe Leu Glu Gln Tyr Ala Arg Phe Asp Asp Leu Lys Gln Phe Ile Ser
   35 40 45
   Thr Phe Gln Pro Lys Ala Ala Gly Ser Thr Phe Gln Val Thr Ser Val
   50 55 60
   Asn Ala Gly Pro Phe Asp Gln Asn Ser Thr Ala Ser Ser Val Glu Ala
   65 70 75 80
   Asn Leu Asp Ile Gln Tyr Thr Thr Gly Leu Val Ala Pro Asp Ile Glu
   85 90 95
   Thr Arg Tyr Phe Thr Val Pro Gly Arg Gly Ile Leu Ile Pro Asp Leu
   100 105 110
   Asp Gln Pro Thr Glu Ser Asp Asn Ala Asn Glu Pro Tyr Leu Asp Tyr
           115                 120                 125
   Phe Thr Tyr Leu Asn Asn Leu Glu Asp Glu Glu Leu Pro Asp Val Leu
       130                 135                 140
   Thr Thr Ser Tyr Gly Glu Ser Glu Gln Ser Val Pro Ala Glu Tyr Ala
   145                 150                 155                 160
   Lys Lys Val Cys Asn Leu Ile Gly Gln Leu Gly Ala Arg Gly Val Ser
                   165                 170                 175
   Val Ile Phe Ser Ser Gly Asp Thr Gly Pro Gly Ser Ala Cys Gln Thr
               180                 185                 190
   Asn Asp Gly Lys Asn Thr Thr Arg Phe Leu Pro Ile Phe Pro Ala Ser
           195                 200                 205
   Cys Pro Tyr Val Thr Ser Val Gly Gly Thr Val Gly Val Glu Pro Glu
       210                 215                 220
   Lys Ala Val Ser Phe Ser Ser Gly Gly Phe Ser Asp Leu Trp Pro Arg
   225                 230                 235                 240
   Pro Ala Tyr Gln Glu Lys Ala Val Ser Glu Tyr Leu Glu Lys Leu Gly
                   245                 250                 255
   Asp Arg Trp Asn Gly Leu Tyr Asn Pro Gln Gly Arg Gly Phe Pro Asp
               260                 265                 270
   Val Ala Ala Gln Gly Gln Gly Phe Gln Val Phe Asp Lys Gly Arg Leu
           275                 280                 285
   Ile Ser Val Gly Gly Thr Ser Ala Ser Ala Pro Val Phe Ala Ser Val
       290                 295                 300
   Val Ala Leu Leu Asn Asn Ala Arg Lys Ala Ala Gly Met Ser Ser Leu
   305                 310                 315                 320
   Gly Phe Leu Asn Pro Trp Ile Tyr Glu Gln Gly Tyr Lys Gly Leu Thr
                   325                 330                 335
   Asp Ile Val Ala Gly Gly Ser Thr Gly Cys Thr Gly Arg Ser Ile Tyr
               340                 345                 350
   Ser Gly Leu Pro Ala Pro Leu Val Pro Tyr Ala Ser Trp Asn Ala Thr
           355                 360                 365
   Glu Gly Trp Asp Pro Val Thr Gly Tyr Gly Thr Pro Asp Phe Lys Gln
       370                 375                 380
   Leu Leu Thr Leu Ala Thr
   385                 390
<210> 46
   <211> 393
   <212> PRT
   <213> Aspergillus kawachii
<400> 46
   Cys Asp Ser Ile Ile Thr Pro His Cys Leu Lys Gln Leu Tyr Asn Ile
   1               5                   10                  15
   Gly Asp Tyr Gln Ala Asp Pro Lys Ser Gly Ser Lys Val Gly Phe Ala
               20                  25                  30
   Ser Tyr Leu Glu Glu Tyr Ala Arg Tyr Ala Asp Leu Glu Arg Phe Glu
           35                  40                  45
   Gln His Leu Ala Pro Asn Ala Ile Gly Gln Asn Phe Ser Val Val Gln
       50                  55                  60
   Phe Asn Gly Gly Leu Asn Asp Gln Leu Ser Leu Ser Asp Ser Gly Glu
   65                  70                  75                  80
   Ala Asn Leu Asp Leu Gln Tyr Ile Leu Gly Val Ser Ala Pro Val Pro
                   85                  90                  95
   Val Thr Glu Tyr Ser Thr Gly Gly Arg Gly Glu Leu Val Pro Asp Leu
               100                 105                 110
   Ser Ser Pro Asp Pro Asn Asp Asn Ser Asn Glu Pro Tyr Leu Asp Phe
           115                 120                 125
   Leu Gln Gly Ile Leu Lys Leu Asp Asn Ser Asp Leu Pro Gln Val Ile
       130                 135                 140
   Ser Thr Ser Tyr Gly Glu Asp Glu Gln Thr Ile Pro Val Pro Tyr Ala
   145                 150                 155                 160
   Arg Thr Val Cys Asn Leu Tyr Ala Gln Leu Gly Ser Arg Gly Val Ser
                   165                 170                 175
   Val Ile Phe Ser Ser Gly Asp Ser Gly Val Gly Ala Ala Cys Leu Thr
               180                 185                 190
   Asn Asp Gly Thr Asn Arg Thr His Phe Pro Pro Gln Phe Pro Ala Ser
   195 200 205
   Cys Pro Trp Val Thr Ser Val Gly Ala Thr Ser Lys Thr Ser Pro Glu
   210 215 220
   Gln Ala Val Ser Phe Ser Ser Gly Gly Phe Ser Asp Leu Trp Pro Arg
   225 230 235 240
   Pro Ser Tyr Gln Gln Ala Ala Val Gln Thr Tyr Leu Thr Gln His Leu
   245 250 255
   Gly Asn Lys Phe Ser Gly Leu Phe Asn Ala Ser Gly Arg Ala Phe Pro
   260 265 270
   Asp Val Ala Ala Gln Gly Val Asn Tyr Ala Val Tyr Asp Lys Gly Met
   275 280 285
   Leu Gly Gln Phe Asp Gly Thr Ser Cys Ser Ala Pro Thr Phe Ser Gly
   290 295 300
   Val Ile Ala Leu Leu Asn Asp Ala Arg Leu Arg Ala Gly Leu Pro Val
   305 310 315 320
   Met Gly Phe Leu Asn Pro Phe Leu Tyr Gly Val Gly Ser Glu Ser Gly
   325 330 335
   Ala Leu Asn Asp Ile Val Asn Gly Gly Ser Leu Gly Cys Asp Gly Arg
   340 345 350
   Asn Arg Phe Gly Gly Thr Pro Asn Gly Ser Pro Val Val Pro Phe Ala
   355 360 365
   Ser Trp Asn Ala Thr Thr Gly Trp Asp Pro Val Ser Gly Leu Gly Thr
   370 375 380
   Pro Asp Phe Ala Lys Leu Arg Gly Val
   385 390
<210> 47
   <211> 392
   <212> PRT
   <213> Aspergillus nidulans
<400> 47
   Cys Glu Lys Ala Ile Thr Pro Ser Cys Leu Ala Asp Leu Tyr Asn Thr
   1 5 10 15
   Glu Gly Tyr Lys Ala Ser Asn Arg Ser Gly Ser Lys Val Ala Phe Ala
               20                  25                  30
   Ser Phe Leu Glu Glu Tyr Ala Arg Tyr Asp Asp Leu Ala Glu Phe Glu
           35                  40                  45
   Glu Thr Tyr Ala Pro Tyr Ala Ile Gly Gln Asn Phe Ser Val Ile Ser
       50                  55                  60
   Ile Asn Gly Gly Leu Asn Asp Gln Asp Ser Thr Ala Asp Ser Gly Glu
   65                  70                  75                  80
   Ala Asn Leu Asp Leu Gln Tyr Ile Ile Gly Val Ser Ser Pro Leu Pro
                   85                  90                  95
   Val Thr Glu Phe Thr Thr Gly Gly Arg Gly Lys Leu Ile Pro Asp Leu
               100                 105                 110
   Ser Ser Pro Asp Pro Asn Asp Asn Thr Asn Glu Pro Phe Leu Asp Phe
           115                 120                 125
   Leu Glu Ala Val Leu Lys Leu Asp Gln Lys Asp Leu Pro Gln Val Ile
       130                 135                 140
   Ser Thr Ser Tyr Gly Glu Asp Glu Gln Thr Ile Pro Glu Pro Tyr Ala
   145                 150                 155                 160
   Arg Ser Val Cys Asn Leu Tyr Ala Gln Leu Gly Ser Arg Gly Val Ser
                   165                 170                 175
   Val Leu Phe Ser Ser Gly Asp Ser Gly Val Gly Ala Ala Cys Gln Thr
               180                 185                 190
   Asn Asp Gly Lys Asn Thr Thr His Phe Pro Pro Gln Phe Pro Ala Ser
           195                 200                 205
   Cys Pro Trp Val Thr Ala Val Gly Gly Thr Asn Gly Thr Ala Pro Glu
       210                 215                 220
   Ser Gly Val Tyr Phe Ser Ser Gly Gly Phe Ser Asp Tyr Trp Ala Arg
   225                 230                 235                 240
   Pro Ala Tyr Gln Asn Ala Ala Val Glu Ser Tyr Leu Arg Lys Leu Gly
                   245                 250                 255
   Ser Thr Gln Ala Gln Tyr Phe Asn Arg Ser Gly Arg Ala Phe Pro Asp
                   260                 265                 270
   Val Ala Ala Gln Ala Gln Asn Phe Ala Val Val Asp Lys Gly Arg Val
           275                 280                 285
   Gly Leu Phe Asp Gly Thr Ser Cys Ser Ser Pro Val Phe Ala Gly Ile
       290                 295                 300
   Val Ala Leu Leu Asn Asp Val Arg Leu Lys Ala Gly Leu Pro Val Leu
   305                 310                 315                 320
   Gly Phe Leu Asn Pro Trp Leu Tyr Gln Asp Gly Leu Asn Gly Leu Asn
                   325                 330                 335
   Asp Ile Val Asp Gly Gly Ser Thr Gly Cys Asp Gly Asn Asn Arg Phe
               340                 345                 350
   Asn Gly Ser Pro Asn Gly Ser Pro Val Ile Pro Tyr Ala Gly Trp Asn
           355                 360                 365
   Ala Thr Glu Gly Trp Asp Pro Val Thr Gly Leu Gly Thr Pro Asp Phe
       370                 375                 380
   Ala Lys Leu Lys Ala Leu Val Leu
   385                 390
<210> 48
   <211> 392
   <212> PRT
   <213> Aspergillus ruber
<400> 48
   Cys Asp Gln Ile Thr Thr Pro His Cys Leu Arg Lys Leu Tyr Asn Val
   1               5                   10                  15
   Asn Gly Tyr Lys Ala Asp Pro Ala Ser Gly Ser Lys Ile Gly Phe Ala
               20                  25                  30
   Ser Phe Leu Glu Glu Tyr Ala Arg Tyr Ser Asp Leu Val Leu Phe Glu
           35                  40                  45
   Glu Asn Leu Ala Pro Phe Ala Glu Gly Glu Asn Phe Thr Val Val Met
       50                  55                  60
   Tyr Asn Gly Gly Lys Asn Asp Gln Asn Ser Lys Ser Asp Ser Gly Glu
   65                  70                  75                  80
   Ala Asn Leu Asp Leu Gln Tyr Ile Val Gly Met Ser Ala Gly Ala Pro
   85 90 95
   Val Thr Glu Phe Ser Thr Ala Gly Arg Ala Pro Val Ile Pro Asp Leu
   100 105 110
   Asp Gln Pro Asp Pro Ser Ala Gly Thr Asn Glu Pro Tyr Leu Glu Phe
   115 120 125
   Leu Gln Asn Val Leu His Met Asp Gln Glu His Leu Pro Gln Val Ile
   130 135 140
   Ser Thr Ser Tyr Gly Glu Asn Glu Gln Thr Ile Pro Glu Lys Tyr Ala
   145 150 155 160
   Arg Thr Val Cys Asn Met Tyr Ala Gln Leu Gly Ser Arg Gly Val Ser
   165 170 175
   Val Ile Phe Ser Ser Gly Asp Ser Gly Val Gly Ser Ala Cys Met Thr
   180 185 190
   Asn Asp Gly Thr Asn Arg Thr His Phe Pro Pro Gln Phe Pro Ala Ser
   195 200 205
   Cys Pro Trp Val Thr Ser Val Gly Ala Thr Glu Lys Met Ala Pro Glu
   210 215 220
   Gln Ala Thr Tyr Phe Ser Ser Gly Gly Phe Ser Asp Leu Phe Pro Arg
   225 230 235 240
   Pro Lys Tyr Gln Asp Ala Ala Val Ser Ser Tyr Leu Gln Thr Leu Gly
   245 250 255
   Ser Arg Tyr Gln Gly Leu Tyr Asn Gly Ser Asn Arg Ala Phe Pro Asp
   260 265 270
   Val Ser Ala Gln Gly Thr Asn Phe Ala Val Tyr Asp Lys Gly Arg Leu
   275 280 285
   Gly Gln Phe Asp Gly Thr Ser Cys Ser Ala Pro Ala Phe Ser Gly Ile
   290 295 300
   Ile Ala Leu Leu Asn Asp Val Arg Leu Gln Asn Asn Lys Pro Val Leu
   305 310 315 320
   Gly Phe Leu Asn Pro Trp Leu Tyr Gly Ala Gly Ser Lys Gly Leu Asn
   325 330 335
   Asp Val Val His Gly Gly Ser Thr Gly Cys Asp Gly Gln Glu Arg Phe
   340 345 350
   Ala Gly Lys Ala Asn Gly Ser Pro Val Val Pro Tyr Ala Ser Trp Asn
   355 360 365
   Ala Thr Gln Gly Trp Asp Pro Val Thr Gly Leu Gly Thr Pro Asp Phe
   370 375 380
   Gly Lys Leu Lys Asp Leu Ala Leu
   385 390
<210> 49
   <211> 391
   <212> PRT
   <213> Aspergillus terreus
<400> 49
   Cys Asp Ser Val Ile Thr Pro Lys Cys Leu Lys Asp Leu Tyr Lys Val
   1 5 10 15
   Gly Asp Tyr Glu Ala Asp Pro Asp Ser Gly Ser Gln Val Ala Phe Ala
   20 25 30
   Ser Tyr Leu Glu Glu Tyr Ala Arg Tyr Ala Asp Met Val Lys Phe Gln
   35 40 45
   Asn Ser Leu Ala Pro Tyr Ala Lys Gly Gln Asn Phe Ser Val Val Leu
   50 55 60
   Tyr Asn Gly Gly Val Asn Asp Gln Ser Ser Ser Ala Asp Ser Gly Glu
   65 70 75 80
   Ala Asn Leu Asp Leu Gln Thr Ile Met Gly Leu Ser Ala Pro Leu Pro
   85 90 95
   Ile Thr Glu Tyr Ile Thr Gly Gly Arg Gly Lys Leu Ile Pro Asp Leu
   100 105 110
   Ser Gln Pro Asn Pro Asn Asp Asn Ser Asn Glu Pro Tyr Leu Glu Phe
   115 120 125
   Leu Gln Asn Ile Leu Lys Leu Asp Gln Asp Glu Leu Pro Gln Val Ile
   130 135 140
   Ser Thr Ser Tyr Gly Glu Asp Glu Gln Thr Ile Pro Arg Gly Tyr Ala
   145 150 155 160
   Glu Ser Val Cys Asn Met Leu Ala Gln Leu Gly Ser Arg Gly Val Ser
   165 170 175
   Val Val Phe Ser Ser Gly Asp Ser Gly Val Gly Ala Ala Cys Gln Thr
   180 185 190
   Asn Asp Gly Arg Asn Gln Thr His Phe Asn Pro Gln Phe Pro Ala Ser
   195 200 205
   Cys Pro Trp Val Thr Ser Val Gly Ala Thr Thr Lys Thr Asn Pro Glu
   210 215 220
   Gln Ala Val Tyr Phe Ser Ser Gly Gly Phe Ser Asp Phe Trp Lys Arg
   225 230 235 240
   Pro Lys Tyr Gln Asp Glu Ala Val Ala Ala Tyr Leu Asp Thr Leu Gly
   245 250 255
   Asp Lys Phe Ala Gly Leu Phe Asn Lys Gly Gly Arg Ala Phe Pro Asp
   260 265 270
   Val Ala Ala Gln Gly Met Asn Tyr Ala Ile Tyr Asp Lys Gly Thr Leu
   275 280 285
   Gly Arg Leu Asp Gly Thr Ser Cys Ser Ala Pro Ala Phe Ser Ala Ile
   290 295 300
   Ile Ser Leu Leu Asn Asp Ala Arg Leu Arg Glu Gly Lys Pro Thr Met
   305 310 315 320
   Gly Phe Leu Asn Pro Trp Leu Tyr Gly Glu Gly Arg Glu Ala Leu Asn
   325 330 335
   Asp Val Val Val Gly Gly Ser Lys Gly Cys Asp Gly Arg Asp Arg Phe
   340 345 350
   Gly Gly Lys Pro Asn Gly Ser Pro Val Val Pro Phe Ala Ser Trp Asn
   355 360 365
   Ala Thr Gln Gly Trp Asp Pro Val Thr Gly Leu Gly Thr Pro Asn Phe
   370 375 380
   Ala Lys Met Leu Glu Leu Ala
   385 390
<210> 50
   <211> 391
   <212> PRT
   <213> Penicillium digitatum
<400> 50
   Cys Asn Ser Ile Ile Thr Pro Gln Cys Leu Lys Asp Leu Tyr Ser Ile
   1 5 10 15
   Gly Asp Tyr Glu Ala Asp Pro Thr Asn Gly Asn Lys Val Ala Phe Ala
   20 25 30
   Ser Tyr Leu Glu Glu Tyr Ala Arg Tyr Ser Asp Leu Ala Leu Phe Glu
   35 40 45
   Lys Asn Ile Ala Pro Phe Ala Lys Gly Gln Asn Phe Ser Val Val Gln
   50 55 60
   Tyr Asn Gly Gly Gly Asn Asp Gln Gln Ser Ser Ser Gly Ser Ser Glu
   65 70 75 80
   Ala Asn Leu Asp Leu Gln Tyr Ile Val Gly Val Ser Ser Pro Val Pro
   85 90 95
   Val Thr Glu Phe Ser Thr Gly Gly Arg Gly Glu Leu Val Pro Asp Leu
   100 105 110
   Asp Gln Pro Asn Pro Asn Asp Asn Asn Asn Glu Pro Tyr Leu Glu Phe
   115 120 125
   Leu Gln Asn Val Leu Lys Leu His Lys Lys Asp Leu Pro Gln Val Ile
   130 135 140
   Ser Thr Ser Tyr Gly Glu Asp Glu Gln Ser Val Pro Glu Lys Tyr Ala
   145 150 155 160
   Arg Ala Val Cys Asn Leu Tyr Ser Gln Leu Gly Ser Arg Gly Val Ser
   165 170 175
   Val Ile Phe Ser Ser Gly Asp Ser Gly Val Gly Ala Ala Cys Gln Thr
   180 185 190
   Asn Asp Gly Arg Asn Ala Thr His Phe Pro Pro Gln Phe Pro Ala Ala
   195 200 205
   Cys Pro Trp Val Thr Ser Val Gly Ala Thr Thr His Thr Ala Pro Glu
   210 215 220
   Arg Ala Val Tyr Phe Ser Ser Gly Gly Phe Ser Asp Leu Trp Asp Arg
   225 230 235 240
   Pro Thr Trp Gln Glu Asp Ala Val Ser Glu Tyr Leu Glu Asn Leu Gly
   245 250 255
   Asp Arg Trp Ser Gly Leu Phe Asn Pro Lys Gly Arg Ala Phe Pro Asp
   260 265 270
   Val Ala Ala Gln Gly Glu Asn Tyr Ala Ile Tyr Asp Lys Gly Ser Leu
   275 280 285
   Ile Ser Val Asp Gly Thr Ser Cys Ser Ala Pro Ala Phe Ala Gly Val
   290 295 300
   Ile Ala Leu Leu Asn Asp Ala Arg Ile Lys Ala Asn Arg Pro Pro Met
   305 310 315 320
   Gly Phe Leu Asn Pro Trp Leu Tyr Ser Glu Gly Arg Ser Gly Leu Asn
   325 330 335
   Asp Ile Val Asn Gly Gly Ser Thr Gly Cys Asp Gly His Gly Arg Phe
   340 345 350
   Ser Gly Pro Thr Asn Gly Gly Thr Ser Ile Pro Gly Ala Ser Trp Asn
   355 360 365
   Ala Thr Lys Gly Trp Asp Pro Val Ser Gly Leu Gly Ser Pro Asn Phe
   370 375 380
   Ala Ala Met Arg Lys Leu Ala
   385 390
<210> 51
   <211> 391
   <212> PRT
   <213> Penicillium oxalicum
<400> 51
   Cys Asn Ser Ala Ile Thr Pro Gln Cys Leu Lys Asp Leu Tyr Lys Val
   1 5 10 15
   Gly Asp Tyr Lys Ala Ser Ala Ser Asn Gly Asn Lys Val Ala Phe Thr
   20 25 30
   Ser Tyr Leu Glu Gln Tyr Ala Arg Tyr Ser Asp Leu Ala Leu Phe Glu
   35 40 45
   Gln Asn Ile Ala Pro Tyr Ala Gln Gly Gln Asn Phe Thr Val Ile Gln
   50 55 60
   Tyr Asn Gly Gly Leu Asn Asp Gln Ser Ser Pro Ala Asp Ser Ser Glu
   65 70 75 80
   Ala Asn Leu Asp Leu Gln Tyr Ile Ile Gly Thr Ser Ser Pro Val Pro
   85 90 95
   Val Thr Glu Phe Ser Thr Gly Gly Arg Gly Pro Leu Val Pro Asp Leu
   100 105 110
   Asp Gln Pro Asp Ile Asn Asp Asn Asn Asn Glu Pro Tyr Leu Asp Phe
   115 120 125
   Leu Gln Asn Val Ile Lys Met Ser Asp Lys Asp Leu Pro Gln Val Ile
   130 135 140
   Ser Thr Ser Tyr Gly Glu Asp Glu Gln Ser Val Pro Ala Ser Tyr Ala
   145 150 155 160
   Arg Ser Val Cys Asn Leu Ile Ala Gln Leu Gly Gly Arg Gly Val Ser
   165 170 175
   Val Ile Phe Ser Ser Gly Asp Ser Gly Val Gly Ser Ala Cys Gln Thr
   180 185 190
   Asn Asp Gly Lys Asn Thr Thr Arg Phe Pro Ala Gln Phe Pro Ala Ala
   195 200 205
   Cys Pro Trp Val Thr Ser Val Gly Ala Thr Thr Gly Ile Ser Pro Glu
   210 215 220
   Arg Gly Val Phe Phe Ser Ser Gly Gly Phe Ser Asp Leu Trp Ser Arg
   225 230 235 240
   Pro Ser Trp Gln Ser His Ala Val Lys Ala Tyr Leu His Lys Leu Gly
   245 250 255
   Lys Arg Gln Asp Gly Leu Phe Asn Arg Glu Gly Arg Ala Phe Pro Asp
   260 265 270
   Val Ser Ala Gln Gly Glu Asn Tyr Ala Ile Tyr Ala Lys Gly Arg Leu
   275 280 285
   Gly Lys Val Asp Gly Thr Ser Cys Ser Ala Pro Ala Phe Ala Gly Leu
   290 295 300
   Val Ser Leu Leu Asn Asp Ala Arg Ile Lys Ala Gly Lys Ser Ser Leu
   305 310 315 320
   Gly Phe Leu Asn Pro Trp Leu Tyr Ser His Pro Asp Ala Leu Asn Asp
   325 330 335
   Ile Thr Val Gly Gly Ser Thr Gly Cys Asp Gly Asn Ala Arg Phe Gly
   340 345 350
   Gly Arg Pro Asn Gly Ser Pro Val Val Pro Tyr Ala Ser Trp Asn Ala
   355 360 365
   Thr Glu Gly Trp Asp Pro Val Thr Gly Leu Gly Thr Pro Asn Phe Gln
   370 375 380
   Lys Leu Leu Lys Ser Ala Val
   385 390
<210> 52
   <211> 391
   <212> PRT
   <213> Penicillium roqueforti
<400> 52
   Cys Asn Ser Ile Ile Thr Pro Gln Cys Leu Lys Asp Ile Tyr Asn Ile
   1 5 10 15
   Gly Asp Tyr Gln Ala Asn Asp Thr Asn Gly Asn Lys Val Gly Phe Ala
   20 25 30
   Ser Tyr Leu Glu Glu Tyr Ala Arg Tyr Ser Asp Leu Ala Leu Phe Glu
   35 40 45
   Lys Asn Ile Ala Pro Ser Ala Lys Gly Gln Asn Phe Ser Val Thr Arg
   50 55 60
   Tyr Asn Gly Gly Leu Asn Asp Gln Ser Ser Ser Gly Ser Ser Ser Glu
   65 70 75 80
   Ala Asn Leu Asp Leu Gln Tyr Ile Val Gly Val Ser Ser Pro Val Pro
   85 90 95
   Val Thr Glu Phe Ser Val Gly Gly Arg Gly Glu Leu Val Pro Asp Leu
   100 105 110
   Asp Gln Pro Asp Pro Asn Asp Asn Asn Asn Glu Pro Tyr Leu Glu Phe
   115 120 125
   Leu Gln Asn Val Leu Lys Leu Asp Lys Lys Asp Leu Pro Gln Val Ile
   130 135 140
   Ser Thr Ser Tyr Gly Glu Asp Glu Gln Ser Ile Pro Glu Lys Tyr Ala
   145 150 155 160
   Arg Ser Val Cys Asn Leu Tyr Ser Gln Leu Gly Ser Arg Gly Val Ser
   165 170 175
   Val Ile Phe Ser Ser Gly Asp Ser Gly Val Gly Ser Ala Cys Leu Thr
   180 185 190
   Asn Asp Gly Arg Asn Ala Thr Arg Phe Pro Pro Gln Phe Pro Ala Ala
   195 200 205
   Cys Pro Trp Val Thr Ser Val Gly Ala Thr Thr His Thr Ala Pro Glu
   210 215 220
   Gln Ala Val Tyr Phe Ser Ser Gly Gly Phe Ser Asp Leu Trp Ala Arg
   225 230 235 240
   Pro Lys Trp Gln Glu Glu Ala Val Ser Glu Tyr Leu Glu Ile Leu Gly
   245 250 255
   Asn Arg Trp Ser Gly Leu Phe Asn Pro Lys Gly Arg Ala Phe Pro Asp
   260 265 270
   Val Thr Ala Gln Gly Arg Asn Tyr Ala Ile Tyr Asp Lys Gly Ser Leu
   275 280 285
   Thr Ser Val Asp Gly Thr Ser Cys Ser Ala Pro Ala Phe Ala Gly Val
   290 295 300
   Val Ala Leu Leu Asn Asp Ala Arg Leu Lys Val Asn Lys Pro Pro Met
   305 310 315 320
   Gly Phe Leu Asn Pro Trp Leu Tyr Ser Thr Gly Arg Ala Gly Leu Lys
   325 330 335
   Asp Ile Val Asp Gly Gly Ser Thr Gly Cys Asp Gly Lys Ser Arg Phe
   340 345 350
   Gly Gly Ala Asn Asn Gly Gly Pro Ser Ile Pro Gly Ala Ser Trp Asn
   355 360 365
   Ala Thr Lys Gly Trp Asp Pro Val Ser Gly Leu Gly Ser Pro Asn Phe
   370 375 380
   Ala Thr Met Arg Lys Leu Ala
   385 390
<210> 53
   <211> 391
   <212> PRT
   <213> Penicillium rubens Wisconsin
<400> 53
   Cys Asn Ser Ile Ile Thr Pro Gln Cys Leu Lys Asn Met Tyr Asn Val
   1               5                   10                  15
   Gly Asp Tyr Gln Ala Asp Asp Asp Asn Gly Asn Lys Val Gly Phe Ala
               20                  25                  30
   Ser Tyr Leu Glu Glu Tyr Ala Arg Tyr Ser Asp Leu Glu Leu Phe Glu
           35                  40                  45
   Lys Asn Val Ala Pro Phe Ala Lys Gly Gln Asn Phe Ser Val Ile Gln
       50                  55                  60
   Tyr Asn Gly Gly Leu Asn Asp Gln His Ser Ser Ala Ser Ser Ser Glu
   65                  70                  75                  80
   Ala Asn Leu Asp Leu Gln Tyr Ile Val Gly Val Ser Ser Pro Val Pro
                   85                  90                  95
   Val Thr Glu Phe Ser Val Gly Gly Arg Gly Glu Leu Val Pro Asp Leu
               100                 105                 110
   Asp Gln Pro Asp Pro Asn Asp Asn Asn Asn Glu Pro Tyr Leu Glu Phe
           115                 120                 125
   Leu Gln Asn Val Leu Lys Met Glu Gln Gln Asp Leu Pro Gln Val Ile
       130                 135                 140
   Ser Thr Ser Tyr Gly Glu Asn Glu Gln Ser Val Pro Glu Lys Tyr Ala
   145                 150                 155                 160
   Arg Thr Val Cys Asn Leu Phe Ser Gln Leu Gly Ser Arg Gly Val Ser
                   165                 170                 175
   Val Ile Phe Ala Ser Gly Asp Ser Gly Val Gly Ala Ala Cys Gln Thr
               180                 185                 190
   Asn Asp Gly Arg Asn Ala Thr Arg Phe Pro Ala Gln Phe Pro Ala Ala
           195                 200                 205
   Cys Pro Trp Val Thr Ser Val Gly Ala Thr Thr His Thr Ala Pro Glu
   210 215 220
   Lys Ala Val Tyr Phe Ser Ser Gly Gly Phe Ser Asp Leu Trp Asp Arg
   225 230 235 240
   Pro Lys Trp Gln Glu Asp Ala Val Ser Asp Tyr Leu Asp Thr Leu Gly
   245 250 255
   Asp Arg Trp Ser Gly Leu Phe Asn Pro Lys Gly Arg Ala Phe Pro Asp
   260 265 270
   Val Ser Ala Gln Gly Gln Asn Tyr Ala Ile Tyr Asp Lys Gly Ser Leu
   275 280 285
   Thr Ser Val Asp Gly Thr Ser Cys Ser Ala Pro Ala Phe Ala Gly Val
   290 295 300
   Ile Ala Leu Leu Asn Asp Ala Arg Leu Lys Ala Asn Lys Pro Pro Met
   305 310 315 320
   Gly Phe Leu Asn Pro Trp Leu Tyr Ser Thr Gly Arg Asp Gly Leu Asn
   325 330 335
   Asp Ile Val His Gly Gly Ser Thr Gly Cys Asp Gly Asn Ala Arg Phe
   340 345 350
   Gly Gly Pro Gly Asn Gly Ser Pro Arg Val Pro Gly Ala Ser Trp Asn
   355 360 365
   Ala Thr Lys Gly Trp Asp Pro Val Ser Gly Leu Gly Ser Pro Asn Phe
   370 375 380
   Ala Thr Met Arg Lys Leu Ala
   385 390
<210> 54
   <211> 400
   <212> PRT
   <213> Neosartorya fischeri
<400> 54
   Cys Ala Asn Leu Ile Thr Pro Asp Cys Leu Val Glu Met Tyr Asn Leu
   1               5                   10                  15
   Gly Asp Tyr Lys Pro Asp Ala Ser Ser Gly Ser Arg Val Gly Phe Gly
               20                  25                  30
   Ser Phe Leu Asn Gln Ser Ala Asn Tyr Ala Asp Leu Ala Ala Tyr Glu
   35 40 45
   Gln Leu Phe Asn Ile Pro Pro Gln Asn Phe Ser Val Glu Leu Ile Asn
   50 55 60
   Gly Gly Ala Asn Asp Gln Asn Trp Ala Thr Ala Ser Leu Gly Glu Ala
   65 70 75 80
   Asn Leu Asp Val Glu Leu Ile Val Ala Val Ser His Ala Leu Pro Val
   85 90 95
   Val Glu Phe Ile Thr Gly Gly Ser Pro Pro Phe Val Pro Asn Val Asp
   100 105 110
   Glu Pro Thr Ala Ala Asp Asn Gln Asn Glu Pro Tyr Leu Gln Tyr Tyr
   115 120 125
   Glu Tyr Leu Leu Ser Lys Pro Asn Ser His Leu Pro Gln Val Ile Ser
   130 135 140
   Asn Ser Tyr Gly Asp Asp Glu Gln Thr Val Pro Glu Tyr Tyr Ala Arg
   145 150 155 160
   Arg Val Cys Asn Leu Ile Gly Leu Met Gly Leu Arg Gly Ile Thr Val
   165 170 175
   Leu Glu Ser Ser Gly Asp Thr Gly Ile Gly Ser Ala Cys Met Ser Asn
   180 185 190
   Asp Gly Thr Asn Thr Pro Gln Phe Thr Pro Thr Phe Pro Gly Thr Cys
   195 200 205
   Pro Phe Ile Thr Ala Val Gly Gly Thr Gln Ser Tyr Ala Pro Glu Val
   210 215 220
   Ala Trp Asp Ala Ser Ser Gly Gly Phe Ser Asn Tyr Phe Ser Arg Pro
   225 230 235 240
   Trp Tyr Gln Tyr Phe Ala Val Glu Asn Tyr Leu Asn Asn His Ile Thr
   245 250 255
   Lys Asp Thr Lys Lys Tyr Tyr Ser Gln Tyr Thr Asn Phe Lys Gly Arg
   260 265 270
   Gly Phe Pro Asp Val Ser Ala His Ser Leu Thr Pro Asp Tyr Glu Val
   275 280 285
   Val Leu Thr Gly Lys His Tyr Lys Ser Gly Gly Thr Ser Ala Ala Cys
   290 295 300
   Pro Val Phe Ala Gly Ile Val Gly Leu Leu Asn Asp Ala Arg Leu Arg
   305 310 315 320
   Ala Gly Lys Ser Thr Leu Gly Phe Leu Asn Pro Leu Leu Tyr Ser Ile
   325 330 335
   Leu Ala Glu Gly Phe Thr Asp Ile Thr Ala Gly Ser Ser Ile Gly Cys
   340 345 350
   Asn Gly Ile Asn Pro Gln Thr Gly Lys Pro Val Pro Gly Gly Gly Ile
   355 360 365
   Ile Pro Tyr Ala His Trp Asn Ala Thr Ala Gly Trp Asp Pro Val Thr
   370 375 380
   Gly Leu Gly Val Pro Asp Phe Met Lys Leu Lys Glu Leu Val Leu Ser
   385 390 395 400
<210> 55
   <211> 374
   <212> PRT
   <213> Aspergillus fumigatus
<400> 55
   Cys Ala Asn Leu Ile Thr Pro Asp Cys Leu Val Glu Met Tyr Asn Leu
   1 5 10 15
   Gly Asp Tyr Lys Pro Asp Ala Ser Ser Gly Ser Arg Val Gly Phe Gly
   20 25 30
   Ser Phe Leu Asn Glu Ser Ala Asn Tyr Ala Asp Leu Ala Ala Tyr Glu
   35 40 45
   Gln Leu Phe Asn Ile Pro Pro Gln Asn Phe Ser Val Glu Leu Ile Asn
   50 55 60
   Arg Gly Val Asn Asp Gln Asn Trp Ala Thr Ala Ser Leu Gly Glu Ala
   65 70 75 80
   Asn Leu Asp Val Glu Leu Ile Val Ala Val Ser His Pro Leu Pro Val
   85 90 95
   Val Glu Phe Ile Thr Gly Ala Leu Pro Pro Val Leu Arg Val Leu Ala
   100 105 110
   Leu Gln Thr Gln Leu Pro Ser Ser Ser Gly Asp Phe Gln Leu Thr Val
   115 120 125
   Pro Glu Tyr Tyr Ala Arg Arg Val Cys Asn Leu Ile Gly Leu Met Gly
   130 135 140
   Leu Arg Gly Ile Thr Val Leu Glu Ser Ser Gly Asp Thr Gly Ile Gly
   145 150 155 160
   Ser Ala Cys Met Ser Asn Asp Gly Thr Asn Lys Pro Gln Phe Thr Pro
   165 170 175
   Thr Phe Pro Gly Thr Cys Pro Phe Ile Thr Ala Val Gly Gly Thr Gln
   180 185 190
   Ser Tyr Ala Pro Glu Val Ala Trp Asp Gly Ser Ser Gly Gly Phe Ser
   195 200 205
   Asn Tyr Phe Ser Arg Pro Trp Tyr Gln Ser Phe Ala Val Asp Asn Tyr
   210 215 220
   Leu Asn Asn His Ile Thr Lys Asp Thr Lys Lys Tyr Tyr Ser Gln Tyr
   225 230 235 240
   Thr Asn Phe Lys Gly Arg Gly Phe Pro Asp Val Ser Ala His Ser Leu
   245 250 255
   Thr Pro Tyr Tyr Glu Val Val Leu Thr Gly Lys His Tyr Lys Ser Gly
   260 265 270
   Gly Thr Ser Ala Ala Ser Pro Val Phe Ala Gly Ile Val Gly Leu Leu
   275 280 285
   Asn Asp Ala Arg Leu Arg Ala Gly Lys Ser Thr Leu Gly Phe Leu Asn
   290 295 300
   Pro Leu Leu Tyr Ser Ile Leu Ala Glu Gly Phe Thr Asp Ile Thr Ala
   305 310 315 320
   Gly Ser Ser Ile Gly Cys Asn Gly Ile Asn Pro Gln Thr Gly Lys Pro
   325 330 335
   Val Pro Gly Gly Gly Ile Ile Pro Tyr Ala His Trp Asn Ala Thr Ala
   340 345 350
   Gly Trp Asp Pro Val Thr Gly Leu Gly Val Pro Asp Phe Met Lys Leu
   355 360 365
   Lys Glu Leu Val Leu Ser
   370
<210> 56
   <211> 2027
   <212> DNA
   <213> Trichoderma reesei
<400> 56
   atggcaaagt tgagcactct ccggcttgcg agccttcttt cccttgtcag tgtgcaggta      60
   tctgcctctg tccatctatt ggagagtctg gagaagctgc ctcatggatg gaaagcagct     120
   gaaaccccga gcccttcgtc tcaaatcgtc ttgcaggttg ctctgacgca gcagaacatt     180
   gaccagcttg aatcgaggct cgcagctgta tccacaccca cttctagcac ctacggcaaa     240
   tacttggatg tagacgagat caacagcatc ttcgctccaa gtgatgctag cagttctgcc     300
   gtcgagtctt ggcttcagtc ccacggagtg acgagttaca ccaagcaagg cagcagcatt     360
   tggtttcaaa caaacatctc cactgcaaat gcgatgctca gcaccaattt ccacacgtac     420
   agcgatctca ccggcgcgaa gaaggtgcgc actctcaagt actcgatccc ggagagcctc     480
   atcggccatg tcgatctcat ctctcccacg acctattttg gcacgacaaa ggccatgagg     540
   aagttgaaat ccagtggcgt gagcccagcc gctgatgctc tagccgctcg ccaagaacct     600
   tccagctgca aaggaactct agtctttgag ggagaaacgt tcaatgtctt tcagccagac     660
   tgtctcagga ccgagtatag tgttgatgga tacaccccgt ctgtcaagtc tggcagcaga     720
   attgggtttg gttcctttct caatgagagc gcaagcttcg cagatcaagc actctttgag     780
   aagcacttca acatccccag tcaaaacttc tccgttgtcc tgatcaacgg tggaacggat     840
   ctccctcagc cgccttctga cgccaacgat ggcgaagcca acctggacgc tcaaaccatt     900
   ttgaccatcg cacatcctct ccccatcacc gaattcatca ccgccggcag tccgccatac     960
   ttccccgatc cagttgaacc tgcgggaaca cccaacgaga acgagcctta tttacagtat    1020
   tacgaatttc tgttgtccaa gtccaacgct gaaattccgc aagtcattac caactcctac    1080
   ggcgacgagg agcaaactgt gccgcggtca tatgccgttc gagtttgcaa tctgattggt    1140
   ctgctaggac tacgcggtat ctctgtcctt cattcctcgg gcgacgaggg tgtgggcgcc    1200
   tcttgcgttg ctaccaacag caccacgcct cagtttaacc ccatctttcc tgtaggtctt    1260
   ctacgtcaac acttccagac aaccattttc tcctactaac cactctaccc tactctctgt    1320
   tcacataggc tacatgtcct tatgttacaa gtgttggcgg aaccgtgagc ttcaatcccg    1380
   aggttgcctg ggctggttca tctggaggtt tcagctacta cttctctaga ccctggtacc    1440
   agcaggaagc tgtgggtact taccttgaga aatatgtcag tgctgagaca aagaaatact    1500
   atggacctta tgtcgatttc tccggacgag gtttccccga tgttgcagcc cacagcgtca    1560
   gccccgagtg agttctattc ctacctatgc aaatcataga atgtatgcta actcgccatg 1620
   aagctatcct gtgtttcagg gcggtgaact caccccaagc ggaggcactt cagcagcctc 1680
   tcctgtcgta gcagccatcg tggcgctgtt gaacgatgcc cgtctccgcg aaggaaaacc 1740
   cacgcttgga tttctcaatc cgctgattta cctacacgcc tccaaagggt tcaccgacat 1800
   cacctcgggc caatctgaag ggtgcaacgg caataacacc cagacgggca gtcctctccc 1860
   aggagtatgc agaacatcaa gaagccttct atcagacgcc aatgctaact tgtggatagg 1920
   ccggcttcat tgcaggcgca cactggaacg cgaccaaggg atgggacccg acgactggat 1980
   ttggtgttcc aaacctcaaa aagctcctcg cacttgtccg gttctaa 2027
<210> 57
   <211> 1803
   <212> DNA
   <213> Aspergillus oryzae
<400> 57
   atgttcttca gtcgtggagc gctttcgctc gcagtgcttt cactgctcag ctcctccgcc 60
   gcaggggagg cttttgagaa gctgtctgcc gttccaaagg gatggcacta ttctagtacc 120
   cctaaaggca acactgaggt ttgtctgaag atcgccctcg cgcagaagga tgctgctggg 180
   ttcgaaaaga ccgtcttgga gatgtcggat cccgaccacc ccagctacgg ccagcacttc 240
   accacccacg acgagatgaa gcgcatgctt cttcccagag atgacaccgt tgatgccgtt 300
   cgacaatggc tcgaaaacgg cggcgtgacc gactttaccc aggatgccga ctggatcaac 360
   ttctgtacta ccgtcgatac cgcgaacaaa ctcttgaatg cccagttcaa atggtacgtc 420
   agcgatgtga agcacatccg ccgtctcaga acactgcagt acgacgtccc cgagtcggtc 480
   acccctcaca tcaacaccat ccaaccgacc acccgttttg gcaagattag ccccaagaag 540
   gccgttaccc acagcaagcc ctcccagttg gacgtgaccg cccttgctgc cgctgtcgtt 600
   gcaaagaaca tctcgcactg tgattctatc attaccccca cctgtctgaa ggagctttac 660
   aacattggtg attaccaggc cgatgcaaac tcgggcagca agatcgcctt cgccagctat 720
   ctggaggagt acgcgcgcta cgctgacctg gagaactttg agaactacct tgctccctgg 780
   gctaagggcc agaacttctc cgttaccacc ttcaacggcg gtctcaatga tcagaactcc 840
   tcgtccgata gcggtgaggc caacctggac ctgcagtaca ttcttggtgt cagcgctcca 900
   ctgcccgtta ctgaattcag caccggaggc cgtggtcccc tcgttcctga tctgacccag 960
   ccggatccca actctaacag caatgagccg taccttgagt tcttccagaa tgtgttgaag 1020
   ctcgaccaga aggacctccc ccaggtcatc tcgacctcct atggagagaa cgaacaggaa 1080
   atccccgaaa agtacgctcg caccgtctgc aacctgatcg ctcagcttgg cagccgcggt 1140
   gtctccgttc tcttctcctc cggtgactct ggtgttggcg agggctgcat gaccaacgac 1200
   ggcaccaacc ggactcactt cccaccccag ttccccgccg cttgcccgtg ggtcacctcc 1260
   gtcggcgcca ccttcaagac cactcccgag cgcggcacct acttctcctc gggcggtttc 1320
   tccgactact ggccccgtcc cgaatggcag gatgaggccg tgagcagcta cctcgagacg 1380
   atcggcgaca ctttcaaggg cctctacaac tcctccggcc gtgctttccc cgacgtcgca 1440
   gcccagggca tgaacttcgc cgtctacgac aagggcacct tgggcgagtt cgacggcacc 1500
   tccgcctccg ccccggcctt cagcgccgtc atcgctctcc tgaacgatgc ccgtctccgc 1560
   gccggcaagc ccactctcgg cttcctgaac ccctggttgt acaagaccgg ccgccagggt 1620
   ctgcaagata tcaccctcgg tgctagcatt ggctgcaccg gtcgcgctcg cttcggcggc 1680
   gcccctgacg gtggtcccgt cgtgccttac gctagctgga acgctaccca gggctgggat 1740
   cccgtcactg gtctcggaac tcccgatttc gccgagctca agaagcttgc ccttggcaac 1800
   taa 1803
<210> 58
   <211> 1782
   <212> DNA
   <213> Phaeosphaeria nodorum
<400> 58
   atggcgccca tcctctcgtt ccttgttggc tctctcctgg cggttcgcgc tcttgctgag      60
   ccatttgaga agctgttcag caccccggaa ggatggaaga tgcaaggtct tgctaccaat     120
   gagcagatcg tcaagctcca gattgctctt cagcaaggcg atgttgcagg tttcgagcaa     180
   catgtgattg acatctcaac gcctagccac ccgagctatg gtgctcacta tggctcgcat     240
   gaggagatga agaggatgat ccagccaagc agcgagacag tcgcttctgt gtctgcatgg     300
   ctgaaggccg ccggtatcaa cgacgctgag attgacagcg actgggtcac cttcaagacg     360
   accgttggcg ttgccaacaa gatgctcgac accaagttcg cttggtacgt gagcgaggag     420
   gccaagcccc gcaaggtcct tcgcacactc gagtactctg taccagatga tgttgcagaa     480
   cacatcaact tgatccagcc cactactcgg tttgctgcga tccgccaaaa ccacgaggtt     540
   gcgcacgaga ttgttggtct tcagttcgct gctcttgcca acaacaccgt taactgcgat     600
   gccaccatca ctccccagtg cttgaagact ctttacaaga ttgactacaa ggccgatccc     660
   aagagtggtt ccaaggtcgc ttttgcttcg tatttggagc agtacgcgcg ttacaatgac     720
   ctcgccctct tcgagaaggc cttcctcccc gaagcagttg gccagaactt ctctgtcgtc     780
   cagttcagcg gcggtctcaa cgaccagaac accacgcaag acagtggcga ggccaacttg     840
   gacttgcagt acattgtcgg tgtcagcgct cctcttcccg tcaccgagtt cagcaccggt     900
   ggtcgcggcc catgggtcgc tgacctagac caacctgacg aggcggacag cgccaacgag     960
   ccctaccttg aattccttca gggtgtgctc aaacttcccc agtctgagct acctcaggtc    1020
   atctccacat cctatggcga gaatgagcag agtgtaccta agtcatacgc tctctccgtc 1080
   tgcaacttgt tcgcccaact cggttcccgt ggcgtctccg tcatcttctc ttctggtgac 1140
   agcggccctg gatccgcatg ccagagcaac gacggcaaga acacgaccaa gttccagcct 1200
   cagtaccccg ctgcctgccc ctttgtcacc tcggttggat cgactcgcta cctcaacgag 1260
   accgcaaccg gcttctcatc tggtggtttc tccgactact ggaagcgccc atcgtaccag 1320
   gacgatgctg ttaaggcgta tttccaccac ctcggtgaga aattcaagcc atacttcaac 1380
   cgccacggcc gtggattccc cgacgttgca acccagggat atggcttccg cgtctacgac 1440
   cagggcaagc tcaagggtct ccaaggtact tctgcctccg cgcctgcatt cgccggtgtg 1500
   attggtctcc tcaacgacgc gcgattgaag gcgaagaagc ctaccttggg attcctaaac 1560
   ccactgcttt actctaactc agacgcgcta aatgacattg ttctcggtgg aagcaaggga 1620
   tgcgatggtc atgctcgctt taacgggccg ccaaatggca gcccagtaat cccatatgcg 1680
   ggatggaacg cgactgctgg gtgggatcca gtgactggtc ttggaacgcc gaacttcccc 1740
   aagcttctta aggctgcggt gcctagccgg tacagggcgt ga 1782
<210> 59
   <211> 1830
   <212> DNA
   <213> Trichoderma atroviride
<400> 59
   atggcgaaac tgacagctct tgccggtctc ctgacccttg ccagcgtgca ggcaaatgcc      60
   gccgtgctct tggacagcct cgacaaggtg cctgttggat ggcaggctgc ttcggccccg     120
   gccccgtcat ccaagatcac cctccaagtt gccctcacgc agcagaacat tgatcagttg     180
   gaatcaaagc tcgctgccgt ctccacgccc aactccagca actatggaaa gtacctggat     240
   gtcgatgaga ttaaccaaat cttcgctccc agcagcgcca gcaccgctgc tgttgagtcc     300
   tggctcaagt cgtacggcgt ggactacaag gtgcagggca gcagcatctg gttccagacg     360
   gatgtctcca cggccaacaa gatgctcagc acaaacttcc acacttacac cgactcggtt     420
   ggtgccaaga aagtgcgaac tctccagtac tcggtccccg agaccctggc cgaccacatc     480
   gatctgattt cgcccacaac ctactttggc acgtccaagg ccatgcgggc gttgaagatc     540
   cagaacgcgg cctctgccgt ctcgcccctg gctgctcgtc aggagccctc cagctgcaag     600
   ggcacaattg agtttgagaa ccgcacattc aacgtcttcc agcccgactg tctcaggacc     660
   gagtacagcg tcaacggata caagccctca gccaagtccg gtagcaggat tggcttcggc     720
   tctttcctga accagagcgc cagctcctca gatctcgctc tgttcgagaa gcactttggc     780
   tttgccagcc agggcttctc cgtcgagctc atcaatggcg gatcaaaccc ccagccgccc     840
   acagacgcca atgacggcga ggccaacctg gacgcccaga acattgtgtc gtttgtgcag     900
   cctctgccca tcaccgagtt tattgctgga ggaactgcgc cgtacttccc agaccccgtt      960
   gagccggctg gaactcccga tgagaacgag ccttacctcg agtactacga gtacctgctc     1020
   tccaagtcaa acaaggagct tccccaagtc atcaccaact cctacggtga tgaggagcag     1080
   actgttcccc aggcatatgc cgtccgcgtg tgcaacctca ttggattgat gggccttcgt     1140
   ggtatctcta tcctcgagtc atccggtgat gagggtgttg gtgcctcttg tctcgctacc     1200
   aacagcacca ccactcccca gttcaacccc atcttcccgg ctacatgccc ctatgtcacc     1260
   agtgttggtg gaaccgtcag cttcaacccc gaggttgcct gggacggctc atccggaggc     1320
   ttcagctact acttctcaag accttggtac caggaggccg cagtcggcac ataccttaac     1380
   aagtatgtca gcgaggagac caaggaatac tacaagtcgt atgtcgactt ttccggacgt     1440
   ggcttccccg atgttgcagc tcacagcgtg agccccgatt accccgtgtt ccaaggcggc     1500
   gagcttaccc ccagcggcgg tacttctgcg gcctctccca tcgtggccag tgttattgcc     1560
   ctcctgaacg atgctcgtct ccgtgcaggc aagcctgctc tcggattctt gaaccctctg     1620
   atctacggat atgcctacaa gggctttacc gatatcacga gtggccaagc tgtcggctgc     1680
   aacggcaaca acactcaaac tggaggccct cttcctggtg cgggtgttat tccaggtgct     1740
   ttctggaacg cgaccaaggg ctgggatcct acaactggat tcggtgtccc caacttcaag     1800
   aagctgcttg agcttgtccg atacatttag                                      1830
<210> 60
   <211> 1791
   <212> DNA
   <213> Arthroderma benhamiae
<400> 60
   atgcgtcttc tcaaatttgt gtgcctgttg gcatcagttg ccgccgcaaa gcctactcca 60
   ggggcgtcac acaaggtcat tgaacatctt gactttgttc cagaaggatg gcagatggtt 120
   ggtgccgcgg accctgctgc tatcattgat ttctggcttg ccatcgagcg cgaaaaccca 180
   gaaaagctct acgacaccat ctatgacgtc tccacccctg gacgcgcaca atatggcaaa 240
   catttgaagc gtgaggaatt ggatgactta ctacgcccaa gggcagagac gagtgagagc 300
   atcatcaact ggctcaccaa tggtggagtc aacccacaac atattcggga tgaaggggac 360
   tgggtcagat tctctaccaa tgtcaagact gccgaaacgt tgatgaatac ccgcttcaac 420
   gtcttcaagg acaacctaaa ttccgtttca aaaattcgaa ctttggagta ttccgtccct 480
   gtagctatat cagctcatgt ccaaatgatc cagccaacta ccttatttgg acgacagaag 540
   ccacagaaca gtttgatcct aaaccccttg accaaggatc tagaatccat gtccgttgaa 600
   gaatttgctg cttctcagtg caggtcctta gtgactactg cctgccttcg agaattgtac 660
   ggacttggtg accgtgtcac tcaggctagg gatgacaacc gtattggagt atccggcttt 720
   ttggaggagt acgcccaata ccgcgatctt gagctcttcc tctctcgctt tgagccatcc 780
   gccaaaggat ttaatttcag tgaaggcctt attgccggag gaaagaacac tcagggtggt 840
   cctggaagct ctactgaggc caaccttgat atgcaatatg tcgtcggtct gtcccacaag 900
   gcaaaggtca cctattactc caccgctggc cgtggcccat taattcccga tctatctcag 960
   ccaagccaag cttcaaacaa caacgaacca taccttgaac agctgcggta cctcgtaaag 1020
   ctccccaaga accagcttcc atctgtattg acaacttcct atggagacac agaacagagc 1080
   ttgcccgcca gctataccaa agccacttgc gacctctttg ctcagctagg aactatgggt 1140
   gtgtctgtta tcttcagcag tggtgatacc gggcccggaa gctcatgcca gaccaacgat 1200
   ggcaagaatg cgactcgctt caaccctatc tacccagctt cttgcccgtt tgtgacctcc 1260
   atcggtggaa ccgttggtac cggtcctgag cgtgcagttt cattctcctc tggtggcttc 1320
   tcagacaggt tcccccgccc acaatatcag gataacgctg ttaaagacta cctgaaaatt 1380
   ttgggcaacc agtggagcgg attgtttgac cccaacggcc gtgctttccc agatatcgca 1440
   gctcagggat caaattatgc tgtctatgac aagggaagga tgactggagt ctccggcacc 1500
   agtgcatccg cccctgccat ggctgccatc attgcccagc ttaacgattt ccgactggca 1560
   aagggctctc ctgtgctggg attcttgaac ccatggatat attccaaggg tttctctggc 1620
   tttacagata ttgttgatgg cggttccagg ggttgcactg gttacgatat atacagcggc 1680
   ttgaaagcga agaaggttcc ctacgcaagc tggaatgcaa ctaagggatg ggacccagta 1740
   acgggatttg gtactcccaa cttccaagct ctcactaaag tgctgcccta a 1791
<210> 61
   <211> 1803
   <212> DNA
   <213> Fusarium graminearum
<400> 61
   atgtatatca cctcatcccg cctcgtgctg gccttagcgg cacttccgac agcatttggt      60
   aaatcatact cccaccatgc cgaagcacca aagggatgga aggtcgacga caccgctcgt     120
   gttgcctcca ccggtaaaca acaggtcttc agcatcgcac tgaccatgca aaatgttgat     180
   cagctcgagt ccaagctcct tgacctctcc agccccgaca gcaagaacta tggccagtgg     240
   atgtctcaaa aggacgtaac aactgctttc tatccttcga aagaagctgt ttccagtgtg     300
   acaaagtggc tcaagtccaa gggtgtcaag cactacaacg tcaacggtgg tttcattgac     360
   tttgctctcg atgtcaaggg tgccaatgcg ctacttgata gtgactatca atactacacc     420
   aaagagggcc agaccaagtt gcgaactctg tcttactcta tccctgatga tgtagccgaa     480
   cacgttcagt tcgtcgaccc aagcaccaac tttggcggca cactggcttt cgcccctgtc     540
   actcacccat cgcgtactct aaccgagcgc aagaacaagc ccaccaagag cacagtcgat     600
   gcttcatgcc aaaccagcat cacaccctca tgcttgaagc agatgtacaa cattggtgac 660
   tacactccca aggtcgagtc tggaagcact attggtttca gcagcttcct tggcgagtcc 720
   gccatctact ccgatgtttt cctgtttgag gagaagtttg gaattcccac gcagaacttt 780
   accactgttc tcatcaacaa cggcactgat gaccagaaca ctgctcacaa gaactttggc 840
   gaggctgact tggatgccga gaacattgtt ggaattgccc accctcttcc cttcacccag 900
   tacatcactg gcggttcacc accttttctt cccaacatcg atcagccaac tgctgccgat 960
   aaccagaacg agccttatgt gcctttcttc cgctaccttc tatcgcagaa ggaagtccct 1020
   gcagttgtct ctacctcgta tggtgacgaa gaagatagcg tccctcgcga atatgctacc 1080
   atgacctgca acctgattgg tcttctcgga cttcgaggaa tcagtgtcat cttctcctct 1140
   ggcgatatcg gcgttggtgc tggatgtctc ggccctgacc acaagactgt cgagttcaac 1200
   gccatcttcc ctgccacctg cccttacctc acctccgtcg gcggtaccgt tgatgtcacc 1260
   cccgaaatcg cctgggaagg ttcttctggt ggtttcagca agtacttccc ccgacccagc 1320
   taccaggaca aggctgtcaa gacgtacatg aagactgtct ccaagcagac aaagaagtac 1380
   tacggccctt acaccaactg ggaaggccga ggcttccctg atgttgctgg ccacagtgtc 1440
   tctcccaact atgaggttat ctatgctggt aagcagagtg caagcggagg taccagtgct 1500
   gctgctcctg tttgggctgc cattgtcggt ctgctcaacg atgcccgttt cagagctggg 1560
   aagccaagct tgggatggtt gaaccctctt gtttacaagt atggaccaaa ggtgttgact 1620
   gacatcactg gtggttacgc cattggatgt gatggcaaca acacccagtc cggaaagcct 1680
   gagcctgcag gatccggtat tgtgcccggt gccagatgga atgccactgc cggatgggat 1740
   cctgtcactg gttatggtac acccgacttt ggaaagttga aggatttggt tcttagcttc 1800
   taa 1803
<210> 62
   <211> 1788
   <212> DNA
   <213> Aspergillus kawachii
<400> 62
   atgcgttcct ccggtcttta cgcagcactg ctgtgctctc tggccgcatc gaccaacgca      60
   gttgttcatg agaagctcgc cgcggtcccc tcgggctggc accatctcga agatgctggc     120
   tccgatcacc agattagcct gtcgatcgca ttggcacgca agaacctcga tcagcttgaa     180
   tccaagctga aagacttgtc cacaccaggt gaatcgcagt atggccagtg gctggatcaa     240
   gaggaagtcg acacactgtt cccagtggcc agcgacaagg ccgtgatcag ctggttgcgc     300
   agcgccaaca tcacccatat tgcccggcag ggcagcttgg tgaactttgc gaccaccgtc     360
   gacaaggtga acaagcttct caacaccact tttgcttact accaaagagg ttcttcccag     420
   agactgcgca cgacagagta ctccattccc gatgatctgg tcgactcgat cgacctcatc     480
   tccccgacaa cctttttcgg caaggaaaag accagtgctg gcctgaccca gcggtcgcag     540
   aaagtcgaca accatgtggc caaacgctcc aacagctcgt cctgcgccga taccatcacg     600
   ttatcctgcc tgaaggagat gtacaacttt ggcaactaca ctcccagcgc ctcgtcagga     660
   agcaagctgg gattcgccag cttcctgaac gagtccgcct cgtattccga tcttgccaag     720
   ttcgagagac tgttcaactt gccgtctcag aacttctccg tggagctgat caacggcggc     780
   gtcaatgacc agaaccaatc gacggcttct ctgaccgagg ctgacctcga tgtggaattg     840
   ctcgttggcg taggtcatcc tcttccggtg accgagttta tcacttctgg cgaacctcct     900
   ttcattcccg accccgatga gccgagtgcc gccgataatg agaatgagcc ttaccttcag     960
   tactacgagt acctcctctc caagcccaac tcggccctgc cccaagtgat ttccaactcc    1020
   tacggtgacg acgaacagac cgttccagaa tactacgcca agcgagtctg caacctgatc    1080
   ggactggtcg gcctgcgcgg catcagcgtc ctggaatcat ccggtgacga aggaattgga    1140
   tctggctgcc gcaccaccga cggcactaac agcacccaat tcaatcccat cttccccgcc    1200
   acctgtccct acgtgaccgc cgtaggaggc accatgtcct acgcgcccga aattgcctgg    1260
   gaagccagtt ccggtggttt cagcaactac ttcgagcgag cctggttcca gaaggaagcc    1320
   gtgcagaact acctggcgaa ccacatcacc aacgagacga agcagtatta ctcacaattc    1380
   gctaacttta gcggtcgcgg atttcccgat gtttcggccc atagctttga gccttcgtac    1440
   gaagttatct tctacggcgc ccgttacggc tccggcggta cttccgccgc atgtcctctg    1500
   ttctctgcgc tagtgggcat gttgaacgat gctcgtctgc gggcgggcaa gtccacgctt    1560
   ggtttcttga accccctgct gtacagtaag gggtacaagg cgctgacaga tgtcacggcg    1620
   ggacaatcga tcgggtgcaa tggcattgat ccgcagagtg atgaggctgt tgcgggcgcg    1680
   ggcattatcc cgtgggcgca ttggaatgcc acagtcggat gggatccggt gacgggattg    1740
   ggacttcctg attttgagaa gttgaggcag ttggtgctgt cgttgtag                 1788
<210> 63
   <211> 1818
   <212> DNA
   <213> Talaromyces stipitatus
<400> 63
   atgagtcgaa atctcctcgt tggtgctggc ctgttggccc tcgcccaatt gagcggtcaa        60
   gctctcgctg ccgctgccct cgtcggccat gaatccctag ctgcgctgcc agttggctgg       120
   gataaggtca gcacgccagc tgcagggacg aacattcaat tgtccgtcgc cctcgctctg       180
   caaaacatcg agcagctgga agaccacttg aagtctgtgt caacccccgg ttctgccagc       240
   tacggtcagt acctggattc cgacggtatt gccgctcaat acggtcccag cgacgcatcc       300
   gttgaggctg tcaccaactg gctgaaggag gccggtgtca ctgacatcta caacaacggc 360
   cagtcgattc acttcgcaac cagtgtcagc aaggccaaca gcttgctcgg ggccgatttc 420
   aactactatt ctgatggtag tgcgaccaag ttgcgtacct tagcttattc cgttcccagt 480
   gacctcaaag aggccatcga ccttgtctcg cccaccacct atttcggcaa gaccactgct 540
   tctcgtagca tccaggctta caagaacaag cgcgcctcta ctacttccaa gtctggatcg 600
   agctctgtgc aagtatctgc ttcctgccag accagcatca ctcctgcctg cttgaaacag 660
   atgtacaatg ttggcaacta cacacccagc gtcgctcacg gcagtcgtgt cggattcggt 720
   agcttcttga atcaatctgc catctttgac gacttgttca cctacgaaaa ggtcaatgat 780
   attccatcac agaatttcac taaggtgatt attgcaaatg catccaacag ccaagatgcc 840
   agcgatggca actacggcga agccaacctt gacgtgcaaa acattgtcgg catctctcat 900
   cctctccccg tgactgaatt cctcactggt ggctcacctc ccttcgttgc tagcctcgac 960
   acccctacca accagaacga gccatatatt ccttactacg aatatctttt gtctcagaag 1020
   aacgaggatc tcccccaggt catttccaac tcttacggag acgacgagca gtctgtgccg 1080
   tacaagtatg ccatccgtgc atgcaacctg atcggcctga caggtttacg aggtatctcg 1140
   gtcttggaat ccagcggtga tctcggcgtt ggagccggct gtcgcagcaa cgatggcaag 1200
   aacaagactc aatttgaccc catcttccct gccacttgcc cctacgttac ctctgttggt 1260
   ggtacccaat ccgttacccc tgaaattgcc tgggtcgcca gctccggtgg tttcagcaac 1320
   tacttccctc gtacctggta ccaggaaccc gcaattcaga cctatctcgg actccttgac 1380
   gatgagacca agacatacta ttctcaatac accaactttg aaggccgtgg tttccccgat 1440
   gtttccgccc acagcttgac ccctgattac caggtcgtcg gtggtggcta tctccagcca 1500
   agcggtggta cttccgctgc ttctcctgtc tttgccggca tcattgcgct tttgaacgac 1560
   gctcgtctcg ctgctggcaa gcccactctt ggcttcttga acccgttctt ctacctttat 1620
   ggatacaagg gtttaaacga tatcactgga ggacagtcag tgggttgcaa cggtatcaac 1680
   ggccaaactg gggctcctgt tcccggtggt ggcattgttc ctggagcggc ctggaactct 1740
   actactggct gggacccagc cactggtctc ggaacacccg acttccagaa gttgaaagaa 1800
   ctcgtactta gcttttaa 1818
<210> 64
   <211> 1800
   <212> DNA
   <213> Fusarium oxysporum
<400> 64
   atgtatatct cctcccaaaa tctggtactc gccttatcgg cgctgccttc agcatttggc 60
   aaatccttct ctcaccatgc tgaagctcct caaggctggc aagtccaaaa gactgccaaa 120
   gtcgcttcca acacgcagca tgtcttcagt cttgcactaa ccatgcaaaa cgtggatcag 180
   ctcgaatcca agcttcttga cctctccagc cccgacagcg ccaactacgg taactggctc 240
   tcccacgatg agctcacaag cactttctct ccttccaagg aggcggtggc tagtgtgaca 300
   aagtggctca agtcaaaggg catcaagcac tacaaggtca acggtgcttt cattgacttt 360
   gctgctgatg ttgagaaggc caatacgctt ctcggaggtg attaccagta ctacactaag 420
   gatggtcaga cgaagctgag aacgctgtct tactccattc ctgatgatgt cgccggtcac 480
   gttcaatttg ttgatcctag cacaaacttc ggtggcaccg ttgcgttcaa ccctgtgcct 540
   cacccctcgc gcaccctcca agagcgcaag gtctctccct ccaagagcac cgttgatgct 600
   tcatgccaga caagcatcac cccttcttgc ctcaagcaga tgtacaacat tggagactac 660
   actcccgatg ccaagtctgg aagtgagatt ggtttcagca gctttctcgg ccaggctgct 720
   atttactctg atgtcttcaa gtttgaggag ctgtttggta ttcctaagca gaactacacc 780
   actattctga tcaacaatgg caccgatgat cagaatactg cgcatggaaa ctttggagag 840
   gctaaccttg atgctgagaa cattgttgga atcgctcatc ctcttccttt caagcagtac 900
   attactggag gttcaccacc tttcgttccc aacatcgatc agcccaccga gaaggataac 960
   cagaacgagc cctacgtgcc tttcttccgt tacctcttgg gccagaagga tctcccagcc 1020
   gtcatctcca cttcctacgg cgatgaagaa gacagcgttc ctcgtgagta tgctacactc 1080
   acctgcaaca tgatcggtct tctcggtctc cgtggcatca gtgtcatctt ctcttccggt 1140
   gacatcggtg tcggttccgg ctgccttgct cccgactaca agaccgtcga gttcaatgcc 1200
   atcttccccg ccacatgccc ctacctcacc tccgtcggcg gtaccgtcga cgtcaccccc 1260
   gagatcgcct gggagggatc ctccggcgga ttcagcaagt acttcccccg acccagctac 1320
   caggacaagg ccatcaagaa gtacatgaag acagtctcca aggagaccaa gaagtactac 1380
   ggcccttaca ccaactggga gggccgaggt ttccctgatg tcgctggaca cagtgttgcg 1440
   cctgactacg aggttatcta caatggtaag caggctcgaa gtggaggtac cagcgctgct 1500
   gcccctgttt gggctgctat cgttggtctg ttgaacgatg cccgcttcaa ggctggtaag 1560
   aagagcttgg gatggttgaa ccctcttatc tacaagcatg gacccaaggt cttgactgac 1620
   atcaccggtg gctatgctat tggatgtgac ggtaacaaca ctcagtctgg aaagcccgag 1680
   cccgctggat ctggtcttgt tcccggtgct cgatggaacg ccacagctgg atgggatcct 1740
   accactggct atggaactcc caacttccag aagttgaagg acttggttct cagcttgtaa 1800
<210> 65
   <211> 1839
   <212> DNA
   <213> Trichoderma virens
<400> 65
   atgcctaagt ccacagcgct tcggcttgtt agcctccttt ccctggccag tgtgccgata      60
   tctgcctccg tccttgtgga aagtctcgaa aagctgcctc acggatggaa agctgcttcg     120
   gctcctagcc cttcctccca gataacccta caagtcgctc ttacgcagca gaacatcgat     180
   cagctggaat cgaggctcgc ggctgtatcc acaccaaatt ccaagacata cggaaattat     240
   ctggatcttg atgagatcaa tgagatcttc gcgccaagcg atgccagcag cgcagccgtg     300
   gagtcttggc tccattctca cggtgtgaca aaatacacga agcaaggcag cagtatctgg     360
   ttccaaaccg aagtttctac agcaaatgca atgttgagca caaacttcca cacttacagt     420
   gatgctgctg gcgttaagaa gttgcgaact cttcagtatt caattccgga gagtcttgtg     480
   ggccatgtcg atctcatctc acccacgacc tactttggca cctctaacgc tatgagagct     540
   ttgagatcta aaagcgtggc ttcagttgct caaagtgtgg cagcccgcca agaaccttct     600
   agctgcaagg gaactctggt tttcgaagga agaacgttca atgtcttcca accagattgt     660
   cttaggacag agtacaatgt caatggatac actccatcag ccaagtctgg tagtagaata     720
   ggatttggtt ccttcttaaa ccaaagtgca agcttttcag acctcgcact ctttgaaaaa     780
   cactttgggt tttccagcca aaatttctcc gtcgttctga tcaatggtgg aacggacctg     840
   ccccaaccac cctctgacga caacgatggc gaggccaatt tggatgtcca aaacattttg     900
   acaatcgcac accctctgcc catcactgaa ttcatcactg ccggaagccc gccgtacttc     960
   ccagatcccg ttgaacctgc aggaactccc gatgagaacg agccttactt gcagtacttt    1020
   gagtatctgt tgtcgaagcc caacagagat cttcctcagg tcattaccaa ctcttacggt    1080
   gatgaggagc aaacagtacc tcaggcttat gctgtccgag tgtgcaacct aattggattg    1140
   atgggactgc gtggtatcag tatcctcgag tcctccggcg atgagggagt gggtgcttcc    1200
   tgcgttgcta ccaacagcac cactcctcaa tttaacccca ttttcccggc aacatgcccc    1260
   tatgtcacta gcgtaggtgg aactgtgaac ttcaacccag aagttgcctg ggacggttca    1320
   tctggaggtt tcagctacta tttctccagg ccatggtacc aagaggaagc agttggaaac    1380
   tacctagaga agcatgtcag cgccgaaaca aagaagtact acgggcctta tgtcgatttc    1440
   tctggacgtg gcttccctga tgttgcagct cacagcgtga gccccgatta tcctgtgttc    1500
   caaggcggcc agctcactcc tagcggaggc acttctgcgg cttctcccgt cgtagccagt    1560
   atcattgccc ttctgaacga tgcacgcctc cgtgaaggca agcccacact tgggttcctg    1620
   aacccgctga tttaccaata tgcttacaag ggtttcacgg atatcacatc cggccagtct    1680
   gatggctgca atggcaacaa cacccaaacg gatgcccctc ttcctggagc tggcgttgtc    1740
   ctaggagcac actggaatgc gaccaaagga tgggatccta cgacaggatt tggtgtccct    1800
   aactttaaga agctactcga gctgatccga tatatatag                           1839
<210> 66
   <211> 1776
   <212> DNA
   <213> Trichoderma atroviride
<400> 66
   atggctaaac tgacggcact tcggctcgtc agccttcttt gccttgcggc tgcgcaggcc 60
   tctgctgctg tgctcgtgga aagcctcaaa caagtgccca acgggtggaa tgcagtctcg 120
   accccagacc cttcgacatc gattgtcttg caaatcgccc tcgcgcaaca gaatatcgat 180
   gaattggaat ggcgtctcgc ggctgtatcc acgcccaact ctggcaatta tggcaaatac 240
   ctggatattg gagagattga aggaattttc gccccaagca atgcctctta caaagccgtg 300
   gcatcgtggc tccagtctca tggggtgaag aacttcgtca aacaagccgg cagtatttgg 360
   ttctacacta ctgtctctac cgcaaacaag atgcttagca cagatttcaa acactatagc 420
   gatcctgttg gcattgagaa gctgcgtact cttcagtact cgatcccaga agaactagtc 480
   ggccatgttg atctcatctc gcctacaaca tattttggaa acaaccaccc cgcgacagcg 540
   agaacaccca acatgaaggc cattaacgta acctaccaaa tctttcaccc agactgcctt 600
   aaaacgaaat acggcgttga tggctatgcc ccatctccaa gatgtggcag caggattggt 660
   tttggctcat tcctcaacga aactgccagt tattcggatc ttgcgcagtt tgagaagtac 720
   tttgaccttc ccaaccaaaa cctttccacc ttattgatca atggcgcaat cgacgttcag 780
   ccaccttcca acaaaaacga cagcgaggcc aacatggacg ttcagaccat cttgaccttt 840
   gtccaacctc ttcctattac tgagtttgtt gttgccggaa tcccgccgta tattcctgat 900
   gcggctttgc cgatcggcga ccctgtccaa aacgagccgt ggctggaata ctttgagttt 960
   ttgatgtcca ggaccaacgc agagcttccc caggtcattg ccaactcata cggtgacgag 1020
   gaacaaacgg taccacaggc gtatgccgtc cgagtatgca accagattgg gctgttgggc 1080
   cttcgcggta tatccgttat cgcatcatct ggcgatacgg gtgttggaat gtcttgtatg 1140
   gcttcgaaca gcactactcc tcagtttaac cccatgttcc cggcttcgtg tccttatatc 1200
   accactgtcg gtggaactca gcaccttgat aatgagattg cttgggagct ttcatcggga 1260
   ggcttcagta actatttcac aaggccatgg tatcaagaag acgcagccaa aacatatctt 1320
   gaacgtcatg tcagcaccga gacaaaggca tattacgaac gttacgccaa tttcttggga 1380
   cgcggctttc ccgacgttgc agcacttagt ctcaaccccg attatccagt gattattggc 1440
   ggagaacttg gtcccaatgg aggcacttct gcggccgcac ccgtcgtcgc tagtattatt 1500
   gcactcttga acgatgcacg cctttgccta ggcaaacctg cccttgggtt cttgaacccc 1560
   ctgatctatc aatatgctga taagggtggc ttcacggata tcacgtccgg ccagtcttgg 1620
   ggctgtgccg gaaataccac tcagacgggg cctcctcccc ctggagctgg tgtcattccg 1680
   ggggcacact ggaatgcgac caagggatgg gatcctgtaa caggatttgg aaccccgaac 1740
   ttcaagaaat tactctcact ggccctgtcc gtctaa 1776
<210> 67
   <211> 1749
   <212> DNA
   <213> Agaricus bisporus
<400> 67
   atgttttggc gtccagcttt tgtccttctt ctcgctcagc ttgtcactgc tagtccttta 60
   gctcgacgct gggatgattt cgcagaaaaa catgcctggg ttgaagttcc tcgcgggtgg 120
   gaaatggtct ccgaggctcc cagtgaccat acctttgatc ttcgcattgg agtaaagtca 180
   agtggcatgg agcagctcat tgaaaacttg atgcaaacca gcgatcctac tcattccaga 240
   tatggtcaac atcttagtaa agaagagctc catgatttcg ttcagcctca tcctgattct 300
   accggagcgg tcgaagcatg gcttgaagat ttcggtatct ccgatgattt cattgatcgt 360
   actggaagtg gcaactgggt tactgttcga gtttcagtag cccaggctga acgtatgctt 420
   ggtaccaagt ataacgtcta ccgccattct gaatcagggg aatcggttgt acgaacaatg 480
   tcttattcgc ttcccagcga acttcactcc cacatagatg ttgtcgcacc caccacttat 540
   ttcggcacga tgaaaagcat gcgggtgacc agcttcttac agccggaaat agagcctgtt 600
   gacccaagcg ctaaaccatc ggctgctcca gcttcctgtt tgagtaccac tgtcataacc 660
   cccgattgcc tccgtgacct ttataatacg gctgactacg ttccttccgc cacttcacgg 720
   aatgccattg gtattgctgg gtacttggat cgttcaaatc gtgcagatct tcagactttc 780
   ttccgacgct tccggcccga tgccgttggc ttcaattaca cgactgtcca actaaatggc 840
   ggaggagacg accagaatga tcccggtgta gaggccaacc tcgatattca atacgccgct 900
   ggtattgctt tccccacacc agctacatac tggagtactg gcggctctcc acctttcatt 960
   ccagatactc aaaccccgac aaacaccaat gagccctacc tggattggat caattttgtc 1020
   ctaggccagg acgagattcc acaggtgatt tcaacgtcct atggtgacga cgagcaaaca 1080
   gttcctgaag attacgctac tagcgtgtgt aatctcttcg cgcaactcgg cagccgtggc 1140
   gttacagtat tcttctccag cggtgacttt ggtgttggtg gtggagattg cctcacgaat 1200
   gatggctcaa accaagtcct tttccagccg gctttccccg cttcctgccc attcgtaaca 1260
   gctgttggcg gaactgtcag gcttgatcct gagattgctg tcagtttctc tggaggaggc 1320
   ttttcccgtt acttctccag gccatcgtac cagaatcaaa ctgtggctca atttgtttct 1380
   aatcttggga atacattcaa cggactctac aataaaaatg gaagggccta cccagatctt 1440
   gcagcacagg gcaatggctt ccaagttgtt atagacggca tcgtccgttc ggttggaggg 1500
   accagcgcca gctctccgac ggttgccggt atctttgcgc ttttgaatga cttcaagctc 1560
   tcaagaggcc agtcgacact cggatttatc aacccactta tatactcctc cgctacatcc 1620
   ggcttcaatg acatcagggc gggtacaaac cctggttgtg gtactcgcgg atttaccgct      1680
   ggtactggtt gggatccggt cactggtctg ggcactcccg attttttgag gcttcaggga      1740
   cttatttaa                                                             1749
<210> 68
   <211> 1806
   <212> DNA
   <213> Magnaporthe oryzae
<400> 68
   atgctcgacc gtatccttct ccccctcggc ctcctggcct cccttgccac cgctcgtgtc 60
   tttgacagcc taccccaccc tccccgaggc tggtcatact cgcacgcggc ggaatcgacg 120
   gagccgctga ccctgcgcat cgccctccgc cagcaaaatg ccgccgccct ggagcaggtg 180
   gtgctgcagg tctcgaaccc caggcacgcc aattacggcc agcacctgac gcgcgacgag 240
   ctgcgcagct acacggcgcc cacgccgcgg gccgtccgca gcgtgacgtc gtggctggtc 300
   gacaacggcg tcgacgacta cacggtcgag cacgactggg tgacgctgcg cacgacggtc 360
   ggggccgcgg acaggctgct cggcgcagac tttgcctggt atgccggccc gggcgagacg 420
   ctgcagctgc ggacgctctc gtacggcgtc gacgactcgg tggcgccgca cgtcgacctc 480
   gtgcagccca cgacgcggtt tggcggtccc gtcgggcagg cgtcgcacat cttcaagcag 540
   gacgactttg acgagcagca gctcaagacc ttgtcggtgg ggttccaggt catggctgac 600
   ctgccggcca acgggcctgg gtcgatcaag gcggcatgta acgagtctgg cgtgacgccc 660
   ctgtgcctgc gaactctgta cagggtcaac tacaagccgg caaccacggg gaacctggtc 720
   gctttcgcgt cgttcctgga gcagtacgcc aggtacagtg atcagcaggc attcactcag 780
   cgggtccttg gccctggtgt tccgttgcag aacttttcgg tcgaaacggt caacggtgga 840
   gccaatgacc agcagagcaa acttgacagc ggcgaggcga acctcgatct gcagtacgtc 900
   atggcaatga gccaccctat tccaattttg gagtacagca ctggaggcag aggacccctc 960
   gtcccaactc tggaccagcc caacgccaac aacagcagca atgagcctta cctggagttc 1020
   ctgacgtacc tcctggccca acccgactca gccatccctc agaccctgtc ggtgtcgtat 1080
   ggcgaggagg aacagtcggt gccgcgcgac tacgccatca aggtttgcaa catgttcatg 1140
   cagctcggcg cccgcggcgt gtcggttatg ttttcgtcgg gcgactcggg cccgggtaat 1200
   gactgtgttc gagcctcgga caacgcaacc ttttttggct caacattccc cgcaggctgc 1260
   ccctacgtca cgtcggtggg ctccaccgtc ggcttcgagc cggagcgcgc cgtctccttt 1320
   tcctcgggcg gcttcagcat ttaccacgct cgccccgact accaaaacga agtggtcccc 1380
   aagtacattg aatcgatcaa ggcttcgggc tacgaaaagt tctttgacgg caacggccgc 1440
   ggaattcccg acgtggctgc ccagggcgcc cgcttcgtcg tcatcgacaa gggccgcgtt 1500
   tctctaatct cggggaccag cgccagctca cctgcgtttg ctggcatggt ggcgctcgtc 1560
   aacgccgccc gcaagtcaaa ggacatgccg gccttgggct tcctcaaccc catgctgtac 1620
   cagaacgccg cggccatgac ggacattgtc aacggcgctg gcatcggctg caggaagcaa 1680
   cgtacagaat tcccgaatgg cgccaggttc aacgccacgg ccggctggga tcccgtcaca 1740
   gggctgggga cgccgttgtt tgacaagctg ctggctgttg gcgcacctgg agttcccaac 1800
   gcgtga 1806
<210> 69
   <211> 1845
   <212> DNA
   <213> Togninia minima
<400> 69
   atgcgtagcc agttgctctt ctgcacagca tttgctgctc tccagtcgct tgtggagggc      60
   agcgatgtgg tgttggagtc attgcgagag gtccctcagg gctggaagag gcttcgagat     120
   gcggaccccg agcagtccat caagctgcgc attgcgcttg agcagcctaa cctggacctg     180
   ttcgagcaga ccctctacga catctcgtca ccggatcacc caaaatatgg ccagcatctc     240
   aagagccacg agttacggga tattatggca cctcgcgagg agtcaactgc tgctgtcatc     300
   gcttggctgc aagacgctgg gctttctggc tcgcagattg aggacgacag cgactggatc     360
   aacatccaga cgacagtcgc ccaagccaac gacatgctga acacgacttt cggtctcttc     420
   gcccaggaag gcaccgaggt caatcgaatt cgagctctgg catattccgt gcctgaggag     480
   atcgtccctc acgtcaagat gattgctccc atcatccgct tcggtcagtt gagacctcag     540
   atgagccaca tcttctcgca tgagaaagtc gaggagaccc cgtctattgg caccatcaag     600
   gccgccgcta tcccatctgt ggatcttaac gtcaccgctt gcaatgccag catcaccccc     660
   gagtgcctcc gagcgcttta caacgttggt gattacgagg cggacccatc gaagaagtct     720
   cttttcggag tctgtggcta cttggagcaa tatgccaagc acgatcagct ggccaagttt     780
   gagcagacct acgctccgta tgctatcggt gccgacttca gcgtcgtgac catcaatggc     840
   ggaggcgaca accagaccag tacgatcgat gatggagaag ccaacctgga tatgcagtat     900
   gctgtcagca tggcatacaa gacgccaatc acatactatt caactggggg tcgaggacct     960
   cttgttccag atctcgacca acctgatccc aacgacgtct caaacgagcc gtaccttgat    1020
   tttgtgagct accttctcaa gctgcccgac tccaaattgc cgcagaccat cacaacttcg    1080
   tacggagagg atgagcaatc cgttccacgc tcctacgtgg agaaggtctg caccatgttc    1140
   ggcgcgctcg gtgcccgagg cgtgtctgtg atcttctcct ctggtgatac cggtgtcggc    1200
   tcagcgtgcc agaccaacga cggcaagaac accacccgct tcctgcctat attccctgct    1260
   gcgtgccctt atgtgacctc ggttggaggc actcgctatg tcgacccgga agtcgctgtg    1320
   tccttctcgt ctggaggctt ctcggacatc ttccctacgc cactctacca gaagggcgct     1380
   gtctctggct acctgaagat cctcggcgat cgctggaagg gcctctataa ccctcacggc     1440
   cgcggtttcc ctgacgtctc cggacagagt gtcagatacc acgtcttcga ctacggcaag     1500
   gacgtcatgt actctggcac aagtgcctct gcaccgatgt tcgccgcgct tgtctcgctg     1560
   ctgaacaacg cccgtctcgc aaagaagttg ccgcccatgg gattcctgaa tccctggctg     1620
   tataccgttg gttttaacgg gctgacggat attgtgcacg gtggatctac tgggtgcact     1680
   ggcacagacg tgtacagcgg cctgcccaca cctttcgttc cgtatgcgtc ttggaacgca     1740
   accgtgggat gggaccccgt tactggactt ggcacgcctc tctttgataa gctgctcaat     1800
   ttgagcacgc caaacttcca cttgccgcac attggcggtc actag                     1845
<210> 70
   <211> 1914
   <212> DNA
   <213> Bipolaris maydis
<400> 70
   atgaagtaca acacactcct caccggcctg ctggctgttg cccatggcag tgccgtttcc 60
   gcttcaacta cttcacatgt cgagggtgaa gttgtcgagc gacttcatgg cgttcctgag 120
   ggttggagtc aagtgggcgc ccccaatcca gaccagaagc tgcgctttcg catcgcagta 180
   cgctcggtga gtaattgctt ttgtgaaccc atgtttgaat cttgcggtgc tttttactga 240
   acataacagg cggatagcga gctgtttgag aggacgctta tggaggtttc ttctcccagc 300
   catcctcgct acggacagca cctaaagcga cacgaactca aggacctcat caaaccgcgc 360
   gccaagtcaa cttcaaacat cctgaactgg ctgcaagagt ctggaattga ggccagagat 420
   atccagaacg atggcgagtg gatcagcttc tatgctccgg ttaaacgtgc cgagcaaatg 480
   atgagcacta cattcaagac ctatcagaac gaggcccgag cgaatatcaa gaagatccgc 540
   tctctagact actcggtgcc gaagcacatt cgagatgaca tcgacatcat ccagcctacg 600
   actcgcttcg gccagatcca accggagcgt agccaagtct ttagtcaaga agaggtccca 660
   ttctcagcgc ttgttgtcaa tgcgacgtgt aacaagaaaa tcactcccga ctgcctcgcc 720
   aacctctaca acttcaaaga ctatgatgcc agcgatgcca atgtcactat cggagtcagc 780
   ggcttcctgg agcaatatgc tcgctttgac gacttgaagc aattcatcag cactttccaa 840
   ccaaaagcag ctggttccac attccaagtt acatctgtca atgcagggcc ttttgaccag 900
   aactcgacag ccagcagtgt tgaagccaat cttgacattc agtacacaac aggtcttgtt 960
   gcgcccgaca ttgaaacccg ctacttcact gttcccggtc gcggtatcct gatccctgat 1020
   ctggaccagc ctacggagag cgacaacgct aatgagccgt atctggatta ctttacatat 1080
   cttaataacc tcgaagacga agaactcccc gacgtgctga ccacatctta cggcgagagc 1140
   gagcagagtg tacccgccga atatgcaaaa aaggtgtgca atttgatcgg ccagttgggt 1200
   gctcgtggtg tgtccgtcat cttctccagc ggtgatactg gccctggctc tgcatgccaa 1260
   accaatgatg gaaaaaacac gacacgtttc ttgcccatct tccctgcttc ttgcccctac 1320
   gtcacttcgg ttggcggcac tgttggtgtt gagcccgaaa aggctgtcag cttctcttcg 1380
   ggcggctttt ctgacctatg gcctcgaccc gcttatcaag agaaggccgt atcagaatat 1440
   cttgaaaagc tcggagaccg ctggaacggg ctttacaacc ctcaaggacg cggatttcct 1500
   gatgtagctg ctcagggcca aggcttccag gtgtttgaca agggcaggct gatttcggtc 1560
   ggaggaacga gcgcttcagc tcctgttttc gcatccgtag tcgcactcct gaacaatgct 1620
   cgcaaggctg ccggcatgtc ttcactcggc ttcttgaacc catggatcta cgagcaaggc 1680
   tacaagggct tgaccgatat cgttgctgga ggctcgacag gatgcacagg aagatccatc 1740
   tattcaggcc tcccagcacc actcgtgccg tatgcttctt ggaatgccac cgaaggatgg 1800
   gatccggtga cgggctatgg tacacctgat ttcaagcaat tgctcaccct cgcgacggca 1860
   cccaagtctg gcgagcgtcg cgttcgtcgt ggcggtctcg gtggccaggc ttag 1914
<210> 71
   <211> 1842
   <212> DNA
   <213> Aspergillus kawachii
<400> 71
   atgttatctt ccttccttag ccagggagca gccgtatccc tcgcgttatt gtcgctgctc      60
   ccttcgcctg tagccgcgga gatcttcgag aagctgtccg gcgtccccaa tggctggaga     120
   tacgccaaca atcctcacgg caacgaggtc attcgccttc aaatcgccct tcagcagcac     180
   gatgttgccg gtttcgaaca agccgtgatg gacatgtcca cccccggtca cgccgactat     240
   ggaaagcatt tccgcacaca tgatgagatg aagcgcatgc tgctccccag cgacactgcc     300
   gtcgactcag ttcgcgactg gctggaatcc gccggagtcc acaatatcca ggtcgacgcc     360
   gactgggtca agttccatac caccgtcaac aaggccaatg ccctgctgga tgccgacttc     420
   aagtggtatg tcagcgaggc caagcacatt cgtcgtctac gcaccctgca atactccatc     480
   cccgacgccc tggtctcgca catcaacatg atccagccca ccactcgctt tggccagatc     540
   cagccgaacc gtgccaccat gcgcagcaag cccaagcacg ccgacgagac attcctgacc     600
   gcagccacct tggcccagaa cacctcccac tgcgactcca tcatcacgcc gcactgtctg     660
   aagcagctct acaacatcgg tgactaccag gccgacccca agtccggtag caaggtcggc     720
   ttcgccagct acctcgaaga atacgcccgg tatgccgatc tcgaaaggtt cgagcagcac     780
   ctggctccca acgccatcgg ccagaacttc agcgtcgttc aattcaacgg cggcctcaac     840
   gaccagcttt cattgagcga cagcggcgaa gccaacctcg acctgcagta catcctgggc     900
   gtcagcgctc ccgtcccggt cactgaatac agcactggcg gacgcggcga actggtcccc 960
   gacctgagct ccccggaccc caacgacaac agcaacgagc cctacctcga cttcctccag 1020
   ggtattctca aactcgacaa ttccgacctc ccccaagtca tctctacctc ctacggcgaa 1080
   gacgaacaga ccatccccgt cccctacgcc cgcacagtct gcaatctcta cgcccaactc 1140
   ggcagccgcg gtgtctccgt gatcttctcg agcggcgact ccggcgtcgg cgccgcctgc 1200
   ctcaccaacg acggcaccaa ccgcacccac ttccctcctc aattcccggc ctcctgcccc 1260
   tgggtaacct ccgtcggtgc caccagcaaa acctccccgg agcaagccgt ctccttctcc 1320
   tcaggaggct tctccgacct ctggccccgc ccctcctacc aacaggctgc cgtccaaacc 1380
   tacctcaccc agcacctggg caacaagttc tcaggcctct tcaacgcctc cggccgcgcc 1440
   ttccccgacg tcgccgcgca gggcgtcaac tacgccgtct acgacaaggg catgcttggc 1500
   cagttcgatg gaaccagttg ctccgcgccg acgttcagtg gtgtcattgc cttgttgaat 1560
   gacgccagac tgagggcggg tttgcccgtt atgggattcc tgaacccgtt cctctatgga 1620
   gttggtagtg agagtggcgc gttgaatgat attgtcaacg gcgggagcct gggttgtgat 1680
   ggtaggaatc gatttggagg cacgcccaat ggaagtcccg ttgtgccgtt tgctagttgg 1740
   aatgcgacca ccgggtggga tccggtttct gggctgggaa cgccggattt tgcgaagttg 1800
   aggggtgtgg cgttgggtga agctaaggcg tatggtaatt aa 1842
<210> 72
   <211> 1977
   <212> DNA
   <213> Aspergillus nidulans
<400> 72
   auggcagcga cuggacgauu cacugccuuc uggaaugucg cgagcgugcc cgccuugauu 60
   ggcauucucc cccuugcugg aucucauuua agagcugucc uuugcccugu cuguaucugg 120
   cgucacucga aggccguuug ugcaccagac acuuugcaag ccaugcgcgc cuucacccgu 180
   guaacggcca ucucccuggc cgguuucucc ugcuucgcug cugcggcggc ugcggcuuuu 240
   gagagccugc gagcuguccc ugacggcugg aucuacgaga gcacccccga cccuaaccaa 300
   ccgcugcguc uacgcaucgc gcugaaacag cacaaugucg ccggcuucga gcaggcacug 360
   cuggauaugu ccacacccgg ucacuccagc uacgggcagc auuucggcuc cuaccacgag 420
   augaagcagc ugcuucuccc uaccgaggag gcguccuccu cggugcgaga cuggcucucg 480
   gcggcgggcg uugaguucga acaggacgcc gacuggauca acuuccgcac gaccgucgac 540
   caggcuaacg cccuccucga cgccgauuuc cucugguaca caacgaccgg cucgacgggc 600
   aacccgacgc ggauccuccg aacccucucc uacagcguuc ccagcgagcu cgcuggauac 660
   gucaacauga uccagccgac uacgcguuuc ggcggcacgc augccaaccg ggccaccguu 720
   cgcgcgaagc cgaucuuccu cgagaccaac cggcagcuca ucaacgccau cuccucuggc 780
   ucgcucgagc acugcgagaa ggccaucacc ccaucgugcc uggcggaucu guacaacacu 840
   gaaggguaca aggcguccaa ccgcagcggg agcaaggugg ccuuugccuc cuuccucgaa 900
   gaguacgcgc gcuacgacga ucucgccgag uucgaggaga ccuacgcucc cuaugcgauc 960
   gggcagaacu ucucgguuau cuccaucaac ggcggccuca acgaccagga cuccacggcc 1020
   gacagcggcg aggcgaaccu cgaccugcag uacaucaucg gcgucucguc gccgcuaccu 1080
   gugaccgagu ucacaaccgg uggccgcggc aagcucauuc cugaccucuc cucccccgac 1140
   ccgaaugaca acaccaacga gccuuuccuu gacuuccuug aggccguccu caagcucgau 1200
   cagaaagacc ugccccaggu caucucgacc uccuacggcg aggacgagca gacaaucccu 1260
   gagccguacg cccgcuccgu cugcaaccug uacgcucagc ucgguucccg cggcgugucu 1320
   gugcucuucu ccucgggugu cucuggcguc ggcgccgccu gccagaccaa cgauggcaaa 1380
   aacacgacgc acuucccgcc gcaguucccg gccucuugcc ccugggugac cgccgucggc 1440
   ggcacgaacg gcacagcgcc cgaauccggu guauacuucu ccagcggcgg guucuccgac 1500
   uacugggcgc gcccggcgua ccagaacgcc gcgguugagu cauaccugcg caaacucggu 1560
   agcacacagg cgcaguacuu caaccgcagc ggacgcgccu ucccggacgu cgcagcgcag 1620
   gcgcagaacu ucgcugucgu cgacaagggc cgugucgguc ucuucgacgg aacgagcugc 1680
   aguucgccug uauuugcggg caucguggcg uugcucaacg acgugcgucu gaaggcaggc 1740
   cugcccgugc ugggauuccu caacccuugg cucuaccagg auggccugaa cgggcucaac 1800
   gauaucgugg auggagggag caccggcugc gacgggaaca accgguuuaa cggaucgcca 1860
   aaugggagcc ccguaauccc guaugcgggu uggaacgcga cggaggggug ggauccugug 1920
   acggggcugg gaacgccgga uuucgcgaag cugaaagcgc ucgugcuuga ugcuuag 1977
<210> 73
   <211> 1815
   <212> DNA
   <213> Aspergillus ruber
<400> 73
   atgttgtcat ttgttcgtcg gggagctctc tccctcgctc tcgtttcgct gttgacctcg      60
   tctgtcgccg ccgaggtctt cgagaagctg catgttgtgc ccgaaggttg gagatatgcc     120
   tccactccta accccaaaca acccattcgt cttcagatcg ctctgcagca gcacgatgtc     180
   accggtttcg aacagtccct cttggagatg tcgactcccg accatcccaa ctacggaaaa     240
   cacttccgca cccacgatga gatgaagcgc atgcttctcc ccaatgaaaa tgccgttcac     300
   gccgtccgcg aatggctgca agacgccgga atcagcgaca tcgaagaaga cgccgattgg     360
   gtccgtttcc acaccaccgt ggaccaggcc aacgacctcc tcgacgccaa cttcctctgg     420
   tacgcgcaca agagccatcg taacacggcg cgtctccgca ctctcgagta ctcgatccca 480
   gactctattg cgccgcaggt caacgtgatc cagccaacca cgcgattcgg acagatccgt 540
   gccaaccggg ctacgcatag cagcaagccc aagggtgggc ttgacgagtt ggctatctcg 600
   caggcagcta cggcggatga tgatagcatt tgtgaccaga tcaccacccc acactgtctg 660
   cggaagctgt acaatgtcaa tggctacaag gccgatcccg ctagtggtag caagatcggt 720
   tttgctagtt tcctggagga atacgcgcgg tactctgatc tggtactgtt cgaggagaac 780
   ctggcaccgt ttgcggaggg tgagaacttt actgtcgtca tgtacaacgg cggcaagaat 840
   gaccagaact ccaagagcga cagcggcgag gccaacctcg atctgcagta catcgtggga 900
   atgagcgcgg gcgcgcccgt gaccgagttc agcaccgccg gtcgcgcacc cgtcatcccg 960
   gacctggacc agcccgaccc cagcgccggt accaacgagc cgtacctcga gttcctgcag 1020
   aacgtgctac acatggacca ggagcacctg ccgcaggtga tctctacttc ctacggtgag 1080
   aacgaacaga ccatccccga aaagtacgcc cgcaccgttt gcaacatgta cgcgcagctg 1140
   ggcagccgcg gtgtgtcggt gattttctcg tcgggcgact ccggcgtcgg ctctgcctgt 1200
   atgaccaacg acggtacaaa ccgcacccac ttccccccgc agttcccggc gtcctgcccc 1260
   tgggtgacat cggtcggggc cactgagaag atggcccccg agcaagcgac atatttctcc 1320
   tcgggcggct tctctgacct cttcccgcgc ccaaagtacc aggacgctgc tgtcagcagc 1380
   taccttcaga ccctcggatc ccggtaccag ggcttgtaca acggttccaa ccgtgcattc 1440
   cctgacgtct cggcgcaggg taccaacttt gctgtgtacg acaagggccg tctaggccag 1500
   ttcgatggta cttcttgctc tgctcccgcg tttagcggta tcatcgcctt gctcaacgac 1560
   gtccgtctcc agaacaacaa gcccgtcctg ggcttcttga acccctggtt gtatggcgct 1620
   gggagcaagg gcctgaacga cgtcgtgcac ggtggcagta caggatgcga tggacaggag 1680
   cggtttgcag gaaaggccaa tggaagcccc gtcgtgccgt acgctagctg gaatgctacg 1740
   caaggctggg atccagtcac tggccttgga acgccggatt tcggcaagtt gaaggatttg 1800
   gctctgtcgg cttaa 1815
<210> 74
   <211> 1803
   <212> DNA
   <213> Aspergillus terreus
<400> 74
   auguugcccu cucuuguaaa caacggggcg cugucccugg cugugcuuuc gcugcucacc 60
   ucguccgucg ccggcgaggu guuugagaag cugucggccg ugccgaaagg auggcacuuc 120
   ucccacgcug cccaggccga cgcccccauc aaccugaaga ucgcccugaa gcagcaugau 180
   gucgagggcu ucgagcaggc ccugcuggac auguccaccc cgggccacga gaacuacggc 240
   aagcacuucc acgagcacga cgagaugaaa cgcaugcugc uccccagcga cuccgccguc 300
   gacgccgucc agaccuggcu gaccuccgcc ggcaucaccg acuacgaccu cgacgccgac 360
   uggaucaacc ugcgcaccac cgucgagcac gccaacgccc ugcuggacac gcaguucggc 420
   ugguacgaga acgaagugcg ccacaucacg cgccugcgca cccugcaaua cuccaucccc 480
   gagaccgucg ccgcgcacau caacauggug cagccgacca cgcgcuuugg ccagauccgg 540
   cccgaccgcg cgaccuucca cgcgcaccac accuccgacg cgcgcauccu guccgcccug 600
   gccgccgcca gcaacagcac cagcugcgac ucagucauca cccccaagug ccucaaggac 660
   cucuacaagg ucggcgacua cgaggccgac ccggacucgg gcagccaggu cgccuucgcc 720
   agcuaccucg aggaauacgc ccgcuacgcc gacaugguca aguuccagaa cucgcucgcc 780
   cccuacgcca agggccagaa cuucucgguc guccuguaca acggcggcgu caacgaccag 840
   ucguccagcg ccgacuccgg cgaggccaac cucgaccugc agaccaucau gggccucagc 900
   gcgccgcucc ccaucaccga guacaucacc ggcggccgcg gcaagcucau ccccgaucuc 960
   agccagccca accccaacga caacagcaac gagcccuacc ucgaguuccu ccagaacauc 1020
   cucaagcugg accaggacga gcugccgcag gugaucucga ccuccuacgg cgaggacgag 1080
   cagacaaucc cccguggcua cgccgaaucc gucugcaaca ugcuggccca gcucggcagc 1140
   cgcggcgugu cgguggucuu cucgucaggc gauucgggcg ucggcgccgc cugccagacc 1200
   aacgacggcc gcaaccaaac ccacuucaac ccgcaguucc cggccagcug cccgugggug 1260
   acgucggucg gggccacgac caagaccaac ccggagcagg cgguguacuu cucgucgggc   1320
   ggguucucgg acuucuggaa gcgcccgaag uaccaggacg aggcgguggc cgcguaccug   1380
   gacacgcugg gcgacaaguu cgcggggcug uucaacaagg gcgggcgcgc guucccggac 1440
   gucgcggcgc agggcaugaa cuacgccauc uacgacaagg gcacgcuggg ccggcuggac 1500
   ggcaccucgu gcucggcgcc ggccuucucg gccaucaucu cgcugcugaa cgaugcgcgc 1560
   cugcgcgagg guaagccgac caugggcuuc uugaacccgu ggcuguaugg ugagggccgc 1620
   gaggcgcuga augauguugu cgugggUggg agcaagggcu gugaugggcg cgaccgguuu   1680
   ggcggcaagc ccaaugggag cccugucgug ccuuuugcua gcuggaaugc uacgcagggc 1740
   ugggacccgg uuacugggcu ggggacgccg aacuuugcga agauguugga gcuggcgcca 1800
   uag 1803
<210> 75
   <211> 1806
   <212> DNA
   <213> Penicillium digitatum
<400> 75
   atgattgcat cattattcaa ccgtagggca ttgacgctcg ctttattgtc actttttgca 60
   tcctctgcca cagccgatgt ttttgagagt ttgtctgctg ttcctcaggg atggagatat 120
   tctcgcacac cgagtgctaa tcagcccttg aagctacaga ttgctctggc tcagggagat 180
   gttgctgggt tcgaggcagc tgtgatcgat atgtcaaccc ccgaccaccc cagttacggg 240
   aaccacttca acacccacga ggaaatgaag cggatgctgc agcctagcgc ggagtccgta 300
   gactcgatcc gtaactggct cgaaagtgcc ggtatttcca agatcgaaca ggacgctgac 360
   tggatgacct tctataccac cgtgaagaca gcgaatgagc tgctggcagc caacttccag 420
   ttctacatca atggagtcaa gaaaatagag cgtctccgca cactcaagta ctctgtcccg 480
   gacgctttgg tgtcccacat taacatgatc cagccaacca cccgtttcgg ccagctgcgc 540
   gcccagcgcg ccattttaca caccgaggtc aaggataacg acgaggcttt ccgctcaaat 600
   gccatgtccg ctaatccgga ctgcaacagc atcatcactc cccagtgtct caaggatttg 660
   tacagtatcg gtgactatga ggccgacccc accaatggga acaaggtcgc gtttgccagc 720
   tacctagagg agtatgcccg atactccgat ctcgcattat ttgagaaaaa catcgccccc 780
   tttgccaagg gacagaattt ctccgttgtc cagtataacg gcggtggtaa tgatcaacaa 840
   tcgagcagtg gcagtagtga ggcgaatctt gacttgcagt acatcgttgg agtcagctct 900
   cctgttcccg ttacagagtt tagcactgga ggtcgcggtg aacttgttcc ggatctcgac 960
   cagccgaatc ccaatgacaa caacaacgag ccataccttg aattcctcca gaacgtgctc 1020
   aagttgcaca agaaggacct cccccaggtg atttccacct cttatggcga ggacgagcag 1080
   agcgttccag agaagtacgc ccgcgccgtt tgcaacctgt actcccaact cggtagccgt 1140
   ggtgtgtccg taatcttttc atccggcgac tctggcgttg gcgccgcgtg tcagacgaac 1200
   gacggccgga acgcgaccca cttcccaccc cagttcccgg ccgcctgccc ctgggtgaca 1260
   tcagtcggtg cgacaaccca cactgcgccc gaacgagccg tttacttctc atctggcggt 1320
   ttctccgatc tctgggatcg ccctacgtgg caagaagatg ctgtgagtga gtacctcgag 1380
   aacctgggcg accgctggtc tggcctcttc aaccctaagg gccgtgcctt ccccgacgtc 1440
   gcagcccagg gtgaaaacta cgccatctac gataagggtt ctttgatcag cgtcgatggc 1500
   acctcttgct cggcacctgc gtttgccgga gtcatcgccc tcctcaacga cgcccgcatc 1560
   aaggccaata gaccacccat gggcttcctc aacccttggc tgtactctga aggccgcagc 1620
   ggcctaaacg acattgtcaa cggcggtagc actggctgcg acggtcatgg ccgcttctcc 1680
   ggccccacta acggtggtac gtcgattcca ggtgccagct ggaacgctac taagggctgg 1740
   gaccctgtct ccggtcttgg atcgcccaac tttgctgcca tgcgcaaact cgccaacgct 1800
   gagtag 1806
<210> 76
   <211> 1806
   <212> DNA
   <213> Penicillium oxalicum
<400> 76
   atgcatgttc ctctgttgaa ccaaggcgcg ctgtcgctgg ccgtcgtctc gctgttggcc 60
   tccacggtct cggccgaagt attcgacaag cttgtcgctg tccctgaagg atggcgattc 120
   tcccgcactc ccagtggaga ccagcccatc cgactgcagg ttgccctcac acagggtgac 180
   gttgagggct tcgagaaggc cgttctggac atgtcaactc ccgaccaccc caactatggc 240
   aagcacttca agtcacacga ggaagttaag cgcatgctgc agcctgcagg cgagtccgtc 300
   gaagccatcc accagtggct cgagaaggcc ggcatcaccc acattcaaca ggatgccgac 360
   tggatgacct tctacaccac cgttgagaag gccaacaacc tgctggatgc caacttccag 420
   tactacctca acgagaacaa gcaggtcgag cgtctgcgca ccttggagta ctcggttcct 480
   gacgagctcg tctcgcacat taaccttgtc accccgacca ctcgcttcgg ccagctgcac 540
   gccgagggtg tgacgctgca cggcaagtct aaggacgtcg acgagcaatt ccgccaggct 600
   gctacttccc ctagcagcga ctgcaacagt gctatcaccc cgcagtgcct caaggacctg 660
   tacaaggtcg gcgactacaa ggccagtgcc tccaatggca acaaggtcgc cttcaccagc 720
   tacctggagc agtacgcccg gtactcggac ctggctctgt ttgagcagaa cattgccccc 780
   tatgctcagg gccagaactt caccgttatc cagtacaacg gtggtctgaa cgaccagagc 840
   tcgcctgcgg acagcagcga ggccaacctg gatctccagt acattatcgg aacgagctct 900
   cccgtccccg tgactgagtt cagcaccggt ggtcgtggtc ccttggtccc cgacttggac 960
   cagcctgaca tcaacgacaa caacaacgag ccttacctcg acttcttgca gaatgtcatc 1020
   aagatgagcg acaaggatct tccccaggtt atctccacct cgtacggtga ggacgagcag 1080
   agcgtccccg caagctacgc tcgtagcgtc tgcaacctca tcgctcagct cggcggccgt 1140
   ggtgtctccg tgatcttctc atctggtgat tccggtgtgg gctctgcctg tcagaccaac 1200
   gacggcaaga acaccactcg cttccccgct cagttccccg ccgcctgccc ctgggtgacc 1260
   tctgttggtg ctactaccgg tatctccccc gagcgcggtg tcttcttctc ctccggtggc 1320
   ttctccgacc tctggagccg cccctcgtgg caaagccacg ccgtcaaggc ctaccttcac 1380
   aagcttggca agcgtcaaga cggtctcttc aaccgcgaag gccgtgcgtt ccccgacgtg 1440
   tcagcccagg gtgagaacta cgctatctac gcgaagggtc gtctcggcaa ggttgacggc 1500
   acttcctgct cggctcccgc tttcgccggt ctggtttctc tgctgaacga cgctcgcatc 1560
   aaggcgggca agtccagcct cggcttcctg aacccctggt tgtactcgca ccccgatgcc 1620
   ttgaacgaca tcaccgtcgg tggaagcacc ggctgcgacg gcaacgctcg cttcggtggt 1680
   cgtcccaacg gcagtcccgt cgtcccttac gctagctgga acgctactga gggctgggac 1740
   cccgtcaccg gtctgggtac tcccaacttc cagaagctgc tcaagtctgc cgttaagcag 1800
   aagtaa 1806
<210> 77
   <211> 1806
   <212> DNA
   <213> Penicillium roqueforti
<400> 77
   atgattgcat ccctatttag tcgtggagca ttgtcgctcg cggtcttgtc gcttctcgcg 60
   tcctctgctg cagccgatgt atttgagagt ttgtctgctg ttcctcaagg atggagatat 120
   tctcgcaggc cgcgtgctga tcagcccttg aagttacaga tcgctctgac acagggggat 180
   actgccggct tcgaagaggc tgtgatggag atgtcaaccc ccgatcaccc tagctacggg 240
   caccacttca ccacccacga agaaatgaag cggatgctac agcccagtgc ggagtccgcg 300
   gagtcaatcc gtgactggct cgaaggcgcg ggtattacca ggatcgaaca ggatgcagat 360
   tggatgacct tctacaccac cgtggagacg gcaaatgagc tgctggcagc caatttccag 420
   ttctacgtca gtaatgtcag gcacattgag cgtcttcgca cactcaagta ctcagtcccg 480
   aaggctctgg tgccacacat caacatgatc cagccaacca cccgtttcgg ccagctgcgc 540
   gcccatcggg gcatattaca cggccaggtc aaggaatccg acgaggcttt ccgctcaaac 600
   gccgtgtccg ctcagccgga ttgcaacagt atcatcactc ctcagtgtct caaggatata 660
   tataatatcg gtgattacca ggccaatgat accaatggga acaaggtcgg gtttgccagc 720
   tacctagagg agtatgcacg atactccgat ctggcactat ttgagaaaaa tatcgcgccc 780
   tctgccaagg gccagaactt ctccgtcacc aggtacaacg gcggtcttaa tgatcaaagt 840
   tccagcggta gcagcagcga ggcgaacctg gacttgcagt acattgttgg agtcagctct 900
   cctgttcccg tcaccgaatt tagcgttggc ggccgtggtg aacttgttcc cgatctcgac 960
   cagcctgatc ccaatgataa caacaacgag ccataccttg aattcctcca gaacgtgctc 1020
   aagctggaca aaaaggacct tccccaggtg atttctacct cctatggtga ggacgagcag 1080
   agcattcccg agaagtacgc ccgcagtgtt tgcaacttgt actcgcagct cggtagccgt 1140
   ggtgtatccg tcattttctc atctggcgac tccggcgttg ggtccgcgtg cctgacgaac 1200
   gacggcagga acgcgacccg cttcccaccc cagttccccg ccgcctgccc gtgggtgaca 1260
   tcagtcggcg cgacaaccca taccgcgccc gaacaggccg tgtacttctc gtccggcggc 1320
   ttttccgatc tctgggctcg cccgaaatgg caagaggagg ccgtgagtga gtacctcgag 1380
   atcctgggta accgctggtc tggcctcttc aaccctaagg gtcgtgcctt ccccgatgtc 1440
   acagcccaag gtcgcaatta cgctatatac gataagggct cgttgaccag cgtcgacggc 1500
   acctcctgct cggcacctgc cttcgccgga gtcgtcgccc tcctcaacga cgctcgcctc 1560
   aaagtcaaca aaccaccaat gggcttcctt aatccttggc tgtactcgac agggcgcgcc 1620
   ggcctaaagg acattgtcga tggcggcagc acgggttgcg atggcaagag ccgcttcggt 1680
   ggtgccaata acggtggtcc gtcgatccca ggtgctagct ggaacgctac taagggttgg 1740
   gaccctgttt ctggtctcgg gtcgcccaac tttgctacca tgcgcaagct tgcgaacgct 1800
   gagtag 1806
<210> 78
   <211> 1806
   <212> DNA
   <213> Penicillium rubens Wisconsin
<400> 78
   augauugcau cucuauuuaa ccguggagca uugucgcucg cgguauuguc gcuucucgcg     60
   ucuucggcuu ccgcugaugu auuugagagu uugucugcug uuccucaagg auggagauau    120
   ucucgcagac cgcgugcuga ucagccccug aagcuacaga uugcucuggc acaaggggau    180
   acugccggau ucgaagaggc ugugauggac augucaaccc cugaucaccc cagcuacggg    240
   aaccacuucc acacccacga ggaaaugaag cggaugcugc agcccagcgc ggaguccgca    300
   gacucgaucc gugacuggcu ugaaagugcg gguaucaaua gaauugaaca ggaugccgac    360
   uggaugacau ucuacaccac cgucgagacg gcaaaugagc ugcuggcagc caauuuccag    420
   uucuaugcca acagugccaa gcacauugag cgucuucgca cacuccagua cuccgucccg    480
   gaggcucuga ugccacacau caacaugauc cagccaacca cucguuucgg ccagcugcgc    540
   guccaggggg ccauauugca cacccagguc aaggaaaccg acgaggcuuu ccgcucaaac    600
   gccgugucca cuucaccgga cugcaacagu aucaucacuc cucagugucu caagaauaug    660
   uacaaugugg gugacuacca ggccgacgac gacaauggga acaaggucgg auuugccagc    720
   uaccuagagg aguaugcacg guacuccgau uuggaacuau uugagaaaaa ugucgcaccc    780
   uucgccaagg gccagaacuu cuccgucauc caguauaacg gcggucuuaa cgaucaacac    840
   ucgagugcua gcagcagcga ggcgaaccuu gacuuacagu acauuguugg aguuagcucu    900
   ccuguuccag uuacagaguu uagcguuggc ggucguggug aacuuguucc cgaucuugac    960
   cagccugauc ccaaugauaa caacaacgag ccauaccuug aauuccucca gaacgugcuc   1020
   aagauggaac aacaggaccu cccccaggug auuuccaccu cuuauggcga gaacgagcag   1080
   aguguucccg agaaauacgc ccgcaccgua ugcaacuugu ucucgcagcu uggcagccgu   1140
   gguguguccg ucaucuucgc aucuggcgac uccggcguug gcgccgcgug ccagacgaau    1200
   gacggcagga acgcgacccg cuucccggcc caguucccug cugccugccc augggugaca   1260
   ucggucggcg cgacaaccca caccgcgccc gagaaggccg uguacuucuc guccgguggc   1320
   uucuccgauc uuugggaucg cccgaaaugg caagaagacg ccgugaguga cuaccucgac   1380
   acccugggcg accgcugguc cggccucuuc aauccuaagg gccgugccuu ccccgacguc 1440
   ucagcccaag gucaaaacua cgccauauac gauaagggcu cguugaccag cgucgacggc 1500
   accucgugcu cggcacccgc cuucgccggu gucaucgccc uccucaacga cgcccgccuc 1560
   aaggccaaca aaccacccau gggcuuccuc aaucccuggc uguacucgac aggccgugac 1620
   ggccugaacg acauuguuca uggcggcagc acuggcugug auggcaacgc ccgcuucggc 1680
   ggccccggua acggcagucc gaggguucca ggugccagcu ggaacgcuac uaagggcugg 1740
   gacccuguuu cuggucuugg aucacccaac uuugcuacca ugcgcaagcu cgcgaacggu 1800
   gaguag 1806
<210> 79
   <211> 1785
   <212> DNA
   <213> Neosartorya fischeri
<400> 79
<210> 80
   <211> 1707
   <212> DNA
   <213> Aspergillus fumigatus
<400> 80
   augcuguccu cgacucucua cgcagggugg cuccucuccc ucgcagcccc agcccuuugu      60
   guggugcagg agaagcucuc agcuguuccu aguggcugga cacucaucga ggaugcaucg     120
   gagagcgaca cgaucacucu cucaauugcc cuugcucggc agaaccucga ccagcuugag     180
   uccaagcuga ccacgcuggc gaccccaggg aacccggagu acggcaagug gcuggaccag     240
   uccgacauug agucccuauu uccuacugca agcgaugaug cuguucucca auggcucaag     300
   gcggccggga uuacccaagu gucucgucag ggcagcuugg ugaacuucgc caccacugug     360
   ggaacagcga acaagcucuu ugacaccaag uucucuuacu accgcaaugg ugcuucccag     420
   aaacugcgua ccacgcagua cuccaucccc gaucaccuga cagagucgau cgaucugauu     480
   gcccccacug ucuucuuugg caaggagcag aacagcgcac ugucaucuca cgcagugaag     540
   cuuccagcuc uuccuaggag ggcagccacc aacaguucuu gcgccaaccu gaucaccccc     600
   gacugccuag uggagaugua caaccucggc gacuacaaac cugaugcauc uucgggaagu     660
   cgagucggcu ucgguagcuu cuugaaugag ucggccaacu augcagauuu ggcugcguau     720
   gagcaacucu ucaacauccc accccagaau uucucagucg aauugaucaa cagaggcguc     780
   aaugaucaga auugggccac ugcuucccuc ggcgaggcca aucuggacgu ggaguugauu     840
   guagccguca gccacccccu gccaguagug gaguuuauca cuggcgcccu accuccagua     900
   cuacgaguac uugcucucca aacccaacuc ccaucuuccu caggugauuu ccaacucacu     960
   guucccgagu acuacgccag gagaguuugc aacuugaucg gcuugauggg ucuucguggc    1020
   aucacggugc ucgaguccuc uggugauacc ggaaucggcu cggcaugcau guccaaugac    1080
   ggcaccaaca agccccaauu cacuccuaca uucccuggca ccugccccuu caucaccgca 1140
   guugguggua cucaguccua ugcuccugaa guugcuuggg acggcaguuc cggcggauuc 1200
   agcaacuacu ucagccgucc cugguaccag ucuuucgcgg uggacaacua ccucaacaac 1260
   cacauuacca aggauaccaa gaaguacuau ucgcaguaca ccaacuucaa gggccgugga 1320
   uucccugaug uuuccgccca uaguuugacc ccuuacuacg aggucgucuu gacuggcaaa 1380
   cacuacaagu cuggcggcac auccgccgcc agccccgucu uugccgguau ugucggucug 1440
   cugaacgacg cccgucugcg cgccggcaag uccacucuug gcuuccugaa cccauugcug 1500
   uauagcaucc uggccgaagg auucaccgau aucacugccg gaaguucaau cgguuguaau 1560
   gguaucaacc cacagaccgg aaagccaguu ccugguggug guauuauccc cuacgcucac 1620
   uggaacgcua cugccggcug ggauccuguu acuggccuug ggguuccuga uuucaugaaa 1680
   uugaaggagu ugguucuguc guuguaa 1707
<210> 81
   <211> 1785
   <212> DNA
   <213> Phaeosphaeria nodorum
<400> 81
   atgcagacct tcggtgcttt tctcgtttcc ttcctcgccg ccagcggcct ggccgcggcc 60
   gagcccttcg agaagctctt tagcaccccc gagggctgga agatgcaggg cctcgccacc 120
   aacgagcaga tcgtcaagct ccagatcgcc ctccagcagg gcgacgtggc cggctttgag 180
   cagcacgtca tcgacatcag cacccccagc caccccagct acggcgctca ctacggcagc 240
   cacgaagaga tgaagcgcat gatccagccc agcagcgaga ctgtcgccag cgtcagcgcc 300
   tggctcaagg ccgctggcat caacgacgcc gagatcgaca gcgactgggt caccttcaag 360
   accaccgtcg gcgtcgccaa caagatgctc gacaccaagt tcgcctggta cgtcagcgag 420
   gaagccaagc cccgcaaggt cctccgcacc cttgagtaca gcgtccccga cgacgtcgcc 480
   gagcacatca acctcatcca gcccaccacc cgcttcgccg ccatccgcca gaaccacgag 540
   gtcgcccacg agatcgtcgg cctccagttt gccgccctcg ccaacaacac cgtcaactgc 600
   gacgccacca tcacccccca gtgcctcaag accctctaca agatcgacta caaggccgac 660
   cccaagagcg gcagcaaggt cgccttcgcc agctaccttg agcagtacgc ccgctacaac 720
   gacctcgccc tcttcgagaa ggccttcctg cctgaggccg tcggccagaa cttcagcgtc 780
   gtccagttct ctggcggcct caacgaccag aacaccaccc aggatagcgg cgaggccaac 840
   ctcgacctcc agtacatcgt cggcgtcagc gcccctctgc ccgtcaccga gtttagcact 900
   ggcggccgag gcccttgggt cgccgatctc gatcagcctg acgaggccga cagcgccaac 960
   gagccctacc ttgagttcct ccagggcgtc ctcaagctcc cccagagcga gctgccccag 1020
   gtcatcagca cctcgtacgg cgagaacgag cagagcgtcc ccaagagcta cgccctcagc 1080
   gtctgcaacc tcttcgccca gcttggctct cgcggcgtca gcgtcatctt cagcagcggc 1140
   gatagcggcc ctggcagcgc ctgccagtct aacgacggca agaacaccac caagttccag 1200
   ccccagtacc ctgccgcctg ccccttcgtc actagcgtcg gctctacccg ctacctcaac 1260
   gagactgcca ccggcttcag ctccggcggc ttcagcgact actggaagcg ccccagctac 1320
   caggacgacg ccgtcaaggc ctacttccac cacctcggcg agaagttcaa gccctacttc 1380
   aaccgccacg gccgaggctt ccctgacgtc gccactcagg gctacggctt ccgcgtctac 1440
   gaccagggca agctcaaggg cctccagggc acttctgcca gcgcccctgc cttcgccggc 1500
   gtcattggcc tgctcaacga cgcccgcctc aaggccaaga agcccaccct cggctttctc 1560
   aaccccctgc tctacagcaa cagcgacgcc ctcaacgaca tcgtcctcgg cggctccaag 1620
   ggctgcgacg gccacgctag gtttaacggc cctcccaacg gcagccccgt catcccttac 1680
   gccggctgga acgccactgc cggctgggac cctgttaccg gcctcggcac ccccaacttc 1740
   cccaagctcc tcaaggccgc cgtcccctct cgataccgcg cttaa 1785
<210> 82
   <211> 1833
   <212> DNA
   <213> Trichoderma atroviride
<400> 82
   atgcagacct tcggtgcttt tctcgtttcc ttcctcgccg ccagcggcct ggccgcggcc      60
   aacgctgctg tcctcctcga cagcctcgac aaggtccccg tcggctggca ggctgcttct     120
   gcccctgctc ccagcagcaa gatcaccctc caggtcgccc tcacccagca gaacatcgac     180
   cagcttgaga gcaagctcgc cgccgtcagc acccccaaca gcagcaacta cggcaagtac     240
   ctcgacgtcg acgagatcaa ccagatcttc gcccccagca gcgccagcac tgccgctgtc     300
   gagagctggc tcaagagcta cggcgtcgac tacaaggtcc agggcagcag catctggttc     360
   cagaccgacg tcagcacggc caacaagatg ctcagcacca acttccacac ctacaccgac     420
   agcgtcggcg ccaagaaggt ccgcaccctc cagtacagcg tccccgagac tctcgccgac     480
   cacatcgacc tcatcagccc caccacctac ttcggcacca gcaaggccat gcgagccctc     540
   aagatccaga acgccgccag cgccgtcagc cctctcgctg ctcgacaaga gcccagcagc     600
   tgcaagggca ccatcgagtt cgagaaccgc accttcaacg tctttcagcc cgactgcctc     660
   cgcaccgagt acagcgtcaa cggctacaag cccagcgcca agagcggcag ccgaatcggc     720
   ttcggcagct tcctcaacca gagcgccagc agcagcgacc tcgccctctt cgagaagcac     780
   ttcggcttcg ccagccaggg cttcagcgtc gagctgatca acggcggcag caacccccag      840
   cctcccaccg atgctaacga cggcgaggcc aacctcgacg cccagaacat cgtcagcttc     900
   gtccagcccc tgcccatcac cgagtttatc gctggcggca ccgcccccta cttccccgat 960
   cctgttgagc ctgccggcac ccccgacgag aacgagccct accttgagta ctacgagtac 1020
   ctcctcagca agagcaacaa ggaactcccc caggtcatca ccaacagcta cggcgacgag 1080
   gaacagaccg tcccccaggc ctacgccgtc cgcgtctgca acctcatcgg cctcatgggc 1140
   ctccgcggca tcagcatcct tgagagcagc ggcgacgagg gcgtcggcgc ttcttgcctc 1200
   gccaccaaca gcaccaccac cccccagttc aaccccatct tccccgccac gtgcccctac 1260
   gtcactagcg tcggcggcac cgtcagcttc aaccccgagg tcgcttggga cggcagcagc 1320
   ggcggcttca gctactactt cagccgcccc tggtatcaag aggccgccgt cggcacctac 1380
   ctcaacaagt acgtcagcga ggaaacgaag gaatattaca agagctacgt cgacttcagc 1440
   ggccgaggct tccctgacgt cgccgctcac tctgtcagcc ccgactaccc cgtctttcag 1500
   ggcggcgagc tgactccttc tggcggcact tctgccgcca gccccatcgt cgccagcgtc 1560
   attgccctgc tcaacgacgc ccgactccga gccggcaagc ctgccctcgg ctttctcaac 1620
   cccctcatct acggctacgc ctacaagggc ttcaccgaca tcacctccgg ccaggccgtt     1680
   ggctgcaacg gcaacaacac ccagaccggc ggaccccttc ctggcgctgg cgttatccct     1740
   ggcgccttct ggaacgccac caagggctgg gaccccacca ccggctttgg cgtccccaac 1800
   ttcaagaagc tccttgagct ggtccgctac atc 1833
<210> 83
   <211> 1803
   <212> DNA
   <213> Arthroderma benhamiae
<400> 83
   atgcagacct tcggtgcttt tctcgtttcc ttcctcgccg ccagcggcct ggccgcggcc 60
   aagcctactc ctggcgcttc ccacaaggtc atcgagcacc tcgacttcgt ccccgagggc 120
   tggcagatgg tcggcgctgc tgaccctgcc gccatcatcg acttttggct cgccatcgag 180
   cgcgagaacc ccgagaagct ctacgacacc atctacgacg tcagcacccc cggacgcgcc 240
   cagtacggca agcacctcaa gcgcgaggaa ctcgacgacc tcctccgccc tcgcgccgag 300
   actagcgaga gcatcatcaa ctggctcacc aacggcggcg tcaaccccca gcacattcgc 360
   gacgagggcg actgggtccg cttcagcacc aacgtcaaga ccgccgagac tctcatgaac 420
   acccgcttca acgtctttaa ggacaacctc aacagcgtca gcaagatccg cacccttgag 480
   tacagcgtcc ccgtcgccat cagcgcccac gtccagatga tccagcccac caccctcttc 540
   ggccgccaga agccccagaa cagcctcatc ctcaaccccc tcaccaagga ccttgagagc 600
   atgagcgtcg aagagttcgc cgccagccag tgccgcagcc tcgtcactac tgcctgcctc 660
   cgcgagctgt acggcctcgg cgatcgagtc acccaggccc gcgacgacaa ccgaattggc 720
   gtcagcggct tcctcgaaga gtacgcccag taccgcgacc ttgagctgtt cctcagccgc 780
   ttcgagccca gcgccaaggg cttcaacttc agcgagggcc tgatcgctgg cggcaagaac 840
   acccagggtg gccctggctc tagcaccgag gccaacctcg acatgcagta cgtcgtcggc 900
   ctcagccaca aggccaaggt cacctactac agcactgccg gccgaggccc cctcatccct 960
   gatctctcac agcccagcca ggccagcaac aacaacgagc cctaccttga gcagctccgc 1020
   tacctcgtca agctccccaa gaaccagctc cccagcgtcc tcaccaccag ctacggcgac 1080
   accgagcaga gcctccccgc cagctacacc aaggccacgt gcgacctctt cgcccagctc 1140
   ggcactatgg gcgtcagcgt catcttcagc agcggcgaca ctggccctgg cagctcgtgc 1200
   cagaccaacg acggcaagaa cgccacgcgc ttcaacccca tctaccccgc cagctgcccc 1260
   ttcgtcacca gcattggcgg caccgtcggc accggccctg agcgagctgt cagctttagc 1320
   agcggcggct tcagcgaccg cttccctcgc cctcagtacc aggacaacgc cgtcaaggac 1380
   tacctcaaga tcctcggcaa ccagtggtcc ggcctcttcg accctaacgg ccgagccttc 1440
   cccgacattg ccgcccaggg cagcaactac gccgtctacg acaagggccg catgaccggc 1500
   gttagcggca cttctgcttc cgcccctgct atggccgcca tcattgccca gctcaacgac 1560
   ttccgcctcg ccaagggcag ccccgtcctc ggctttctca acccctggat ctacagcaag 1620
   ggcttcagcg gcttcaccga catcgtcgac ggcggctcta ggggctgcac cggctacgac 1680
   atctacagcg gcctcaaggc caagaaggtc ccctacgcca gctggaacgc caccaagggc 1740
   tgggaccccg tcaccggctt tggcaccccc aacttccagg ccctgaccaa ggtcctgccc 1800
   taa 1803
<210> 84
   <211> 1803
   <212> DNA
   <213> Fusarium graminearum
<400> 84
   atgcagacct tcggtgcttt tctcgtttcc ttcctcgccg ccagcggcct ggccgcggcc      60
   aagagctact ctcaccacgc cgaggccccc aagggctgga aggtcgacga tactgcccgc     120
   gtcgccagca ccggcaagca gcaggtcttt tcgatcgccc tgaccatgca gaacgtcgac     180
   cagcttgaga gcaagctcct cgacctcagc agccccgaca gcaagaacta cggccagtgg     240
   atgagccaga aggacgtcac caccgccttc taccccagca aggaagccgt cagcagcgtc     300
   accaagtggc tcaagagcaa gggcgtcaag cactacaacg tcaacggcgg cttcatcgac     360
   ttcgccctcg acgtgaaggg cgccaacgcc ctcctcgaca gcgactacca gtactacacc     420
   aaggaaggcc agaccaagct ccgcaccctc agctacagca tccccgacga cgtcgccgag     480
   cacgtccagt tcgtcgaccc cagcaccaac ttcggcggca ccctcgcctt tgcccccgtc     540
   actcacccta gccgcaccct caccgagcgc aagaacaagc ccaccaagag caccgtcgac 600
   gccagctgcc agaccagcat cacccccagc tgcctcaagc agatgtacaa catcggcgac 660
   tacaccccca aggtcgagag cggcagcacg atcggcttca gcagcttcct cggcgagagc 720
   gctatctaca gcgacgtctt tctgttcgag gaaaagttcg gcatccccac ccagaacttc 780
   accaccgtcc tcatcaacaa cggcaccgac gaccagaaca ccgcccacaa gaacttcggc 840
   gaggccgacc tcgacgccga gaacatcgtc ggcattgccc accccctgcc cttcacccag 900
   tacatcactg gcggcagccc ccccttcctg cccaacatcg atcagcccac tgccgccgac 960
   aaccagaacg agccctacgt ccccttcttc cgctacctcc tcagccagaa ggaagtcccc 1020
   gccgtcgtca gcaccagcta cggcgacgaa gaggacagcg tcccccgcga gtacgccacc 1080
   atgacctgca acctcatcgg cctgctcggc ctccgcggca tcagcgtcat cttcagcagc 1140
   ggcgacatcg gcgtcggcgc tggctgtctt ggccccgacc acaagaccgt cgagttcaac 1200
   gccatcttcc ccgccacgtg cccctacctc actagcgtcg gcggcacggt cgacgtcacc 1260
   cccgagattg cttgggaggg cagcagcggc ggcttcagca agtacttccc tcgccccagc 1320
   taccaggaca aggccgtcaa gacctacatg aagaccgtca gcaagcagac caagaagtac 1380
   tacggcccct acaccaactg ggagggccga ggctttcctg acgtcgccgg ccacagcgtc 1440
   agccccaact acgaggtcat ctacgccggc aagcagagcg cctctggcgg cacttctgct 1500
   gccgcccctg tctgggctgc catcgtcggc ctgctcaacg acgcccgatt ccgagccggc 1560
   aagcctagcc tcggctggct caaccccctc gtctacaagt acggccccaa ggtcctcacc 1620
   gacatcaccg gcggctacgc cattggctgc gacggcaaca acacccagag cggcaagccc 1680
   gagcctgccg gctctggcat tgtccctggc gcccgatgga acgccactgc cggatgggac 1740
   cctgtcaccg gctacggcac ccccgacttc ggcaagctca aggacctcgt cctcagcttc 1800
   taa 1803
<210> 85
   <211> 1872
   <212> DNA
   <213> Acremonium alcalophilum
<400> 85
   atgcagacct tcggtgcttt tctcgtttcc ttcctcgccg ccagcggcct ggccgcggcc        60
   gccgtcgtca ttcgcgccgc cgtcctcccc gacgccgtca agctgatggg caaggccatg       120
   cccgacgaca ttatttccct ccagttttcc ctgaagcagc agaacatcga ccagctggag       180
   acccgcctcc gcgccgtctc ggaccccagc tcccccgagt acggccagta catgagcgag       240
   tccgaggtca acgagttctt taagccccgc gacgactcgt tcgccgaggt cattgactgg       300
   gtcgccgcca gcggctttca ggacatccac ctgacgcccc aggctgccgc cattaacctc       360
   gccgccaccg tcgagacggc cgaccagctc ctgggcgcca acttcagctg gtttgacgtc 420
   gacggcaccc gcaagctccg caccctggag tacacgatcc ccgaccgcct cgccgaccac 480
   gtcgacctga tttcccccac cacgtacttc ggccgcgccc gactggacgg cccccgcgag 540
   acccccacgc gcctcgacaa gcgccagcgc gaccccgtcg ccgacaaggc ctacttccac 600
   ctcaagtggg accgcggcac cagcaactgc gacctggtca tcacgccccc ctgcctggag 660
   gccgcctaca actacaagaa ctacatgccc gaccccaact cgggcagccg cgtctcgttc 720
   accagctttc tggagcaggc cgcccagcag agcgacctca ccaagttcct ctccctgacg 780
   ggcctcgacc gcctgcgccc ccccagcagc aagcccgcca gcttcgacac ggtcctgatc 840
   aacggcggcg agacccacca gggcacgccc cccaacaaga cctccgaggc caacctcgac 900
   gtccagtggc tggccgccgt cattaaggcc cgactcccca tcacccagtg gattacgggc 960
   ggccgccccc ccttcgtccc caacctccgc ctgcgccacg agaaggacaa cacgaacgag 1020
   ccctacctgg agttctttga gtacctcgtc cgcctgcccg cccgcgacct cccccaggtc 1080
   atctccaact cgtacgccga ggacgagcag accgtccccg aggcctacgc ccgacgcgtc 1140
   tgcaacctca tcggcattat gggcctgcgc ggcgtcaccg tcctcacggc ctccggcgac 1200
   tcgggcgtcg gcgccccctg ccgcgccaac gacggcagcg accgcctgga gttctccccc 1260
   cagtttccca cctcgtgccc ctacatcacc gccgtcggcg gcacggaggg ctgggacccc 1320
   gaggtcgcct gggaggcctc ctcgggcggc ttcagccact actttctccg cccctggtac 1380
   caggccaacg ccgtcgagaa gtacctcgac gaggagctgg accccgccac ccgcgcctac 1440
   tacgacggca acggcttcgt ccagtttgcc ggccgagcct accccgacct gtccgcccac 1500
   agctcctcgc cccgctacgc ctacatcgac aagctcgccc ccggcctgac cggcggcacg 1560
   agcgcctcct gccccgtcgt cgccggcatc gtcggcctcc tgaacgacgc ccgactccgc 1620
   cgcggcctgc ccacgatggg cttcattaac ccctggctgt acacgcgcgg ctttgaggcc 1680
   ctccaggacg tcaccggcgg ccgcgcctcg ggctgccagg gcatcgacct ccagcgcggc 1740
   acccgcgtcc ccggcgccgg catcattccc tgggcctcct ggaacgccac ccccggctgg 1800
   gaccccgcca cgggcctcgg cctgcccgac ttctgggcca tgcgcggcct cgccctgggc 1860
   cgcggcacct aa 1872
<210> 86
   <211> 1905
   <212> DNA
   <213> Sodiomyces alkalinus
<400> 86
   atgcagacct tcggtgcttt tctcgtttcc ttcctcgccg ccagcggcct ggccgcggcc 60
   gccgtcgtca ttcgcgccgc ccccctcccc gagagcgtca agctcgtccg caaggccgcc 120
   gccgaggacg gcattaacct ccagctctcc ctgaagcgcc agaacatgga ccagctggag      180
   aagttcctcc gcgccgtcag cgaccccttt tcccccaagt acggccagta catgtcggac      240
   gccgaggtcc acgagatctt ccgccccacc gaggactcct ttgaccaggt cattgactgg 300
   ctcaccaagt cgggcttcgg caacctgcac atcacgcccc aggctgccgc cattaacgtc 360
   gccaccacgg tcgagaccgc cgaccagctg tttggcgcca acttctcctg gtttgacgtc 420
   gacggcacgc ccaagctccg caccggcgag tacacgatcc ccgaccgcct cgtcgagcac      480
   gtcgacctgg tcagccccac cacgtacttc ggccgcatgc gccccccccc tcgcggcgac      540
   ggcgtcaacg actggatcac cgagaactcg cccgagcagc ccgcccccct gaacaagcgc      600
   gacaccaaga cggagagcga ccaggcccgc gaccacccct cctgggactc gcgcaccccc      660
   gactgcgcca ccatcattac gcccccctgc ctggagacgg cctacaacta caagggctac 720
   atccccgacc ccaagtccgg ctcgcgcgtc agcttcacca gcttcctgga gcaggccgcc      780
   cagcaggccg acctgaccaa gttcctcagc ctgacgcgcc tggagggctt tcgcaccccc      840
   gccagcaaga agaagacctt caagacggtc ctgatcaacg gcggcgagtc ccacgagggc 900
   gtccacaaga agtcgaagac cagcgaggcc aacctcgacg tccagtggct ggccgccgtc 960
   acccagacga agctgcccat cacccagtgg attacgggcg gccgcccccc cttcgtcccc 1020
   aacctccgca tccccacccc cgaggccaac acgaacgagc cctacctgga gttcctggag 1080
   tacctctttc gcctgcccga caaggacctc ccccaggtca tcagcaactc ctacgccgag 1140
   gacgagcaga gcgtccccga ggcctacgcc cgacgcgtct gcggcctcct gggcattatg     1200
   ggcctccgcg gcgtcaccgt cctgacggcc tccggcgact cgggcgtcgg cgccccctgc     1260
   cgcgccaacg acggctcggg ccgcgaggag ttcagccccc agtttcccag ctcctgcccc 1320
   tacatcacca cggtcggcgg cacccaggcc tgggaccccg aggtcgcctg gaagggcagc 1380
   agcggcggct tctccaacta ctttccccgc ccctggtacc aggtcgccgc cgtcgagaag 1440
   tacctggagg agcagctgga ccccgccgcc cgcgagtact acgaggagaa cggcttcgtc 1500
   cgctttgccg gccgagcctt ccccgacctg agcgcccaca gcagcagccc caagtacgcc 1560
   tacgtcgaca agcgcgtccc cggcctcacc ggcggcacgt cggccagctg ccccgtcgtc 1620
   gccggcatcg tcggcctcct gaacgacgcc cgactccgcc gcggcctgcc cacgatgggc 1680
   ttcattaacc cctggctcta cgccaagggc taccaggccc tggaggacgt caccggcggc 1740
   gccgccgtcg gctgccaggg catcgacatt cagacgggca agcgcgtccc cggcgccggc 1800
   atcattcccg gcgccagctg gaacgccacc cccgactggg accccgccac gggcctcggc 1860
   ctgcccaact tctgggccat gcgcgagctc gccctggagg actaa 1905
<210> 87
   <211> 1788
   <212> DNA
   <213> Aspergillus kawachii
<400> 87
   atgcagacct tcggtgcttt tctcgtttcc ttcctcgccg ccagcggcct ggccgcggcc 60
   gtcgtccatg agaagctcgc tgctgtcccc agcggctggc accaccttga ggatgccggc 120
   agcgaccacc agatcagcct ctcgattgcc ctcgcccgca agaacctcga ccagcttgag 180
   agcaagctca aggacctcag cacccctggc gagagccagt acggccagtg gctcgaccaa 240
   gaggaagtcg acaccctgtt ccccgtcgcc agcgacaagg ccgtcatcag ctggctccgc 300
   agcgccaaca tcacccacat tgcccgccag ggcagcctcg tcaacttcgc caccaccgtc 360
   gacaaggtca acaagctcct caacaccacc ttcgcctact accagcgcgg cagctctcag 420
   cgcctccgca ccaccgagta cagcatcccc gacgacctcg tcgacagcat cgacctgatc 480
   agccccacca cgttcttcgg caaggaaaag acctctgccg gcctcaccca gcgcagccag 540
   aaggtcgata accacgtcgc caagcgcagc aacagcagca gctgcgccga caccatcacc 600
   ctcagctgcc tcaaggaaat gtacaacttc ggcaactaca cccccagcgc cagcagcggc 660
   agcaagctcg gcttcgccag cttcctcaac gagagcgcca gctacagcga cctcgccaag 720
   ttcgagcgcc tcttcaacct ccccagccag aacttcagcg tcgagctgat caacggcggc 780
   gtcaacgacc agaaccagag caccgccagc ctcaccgagg ccgacctcga tgtcgagctg 840
   cttgtcggcg tcggccaccc cctgcccgtc accgagttta tcaccagcgg cgagcccccc 900
   ttcatccccg accctgatga gccttctgcc gccgacaacg agaacgagcc ctacctccag 960
   tactacgagt acctcctcag caagcccaac agcgccctgc cccaggtcat cagcaacagc 1020
   tacggcgacg acgagcagac cgtccccgag tactacgcca agcgcgtctg caacctcatc 1080
   ggcctcgtcg gcctccgcgg catcagcgtc cttgagtcta gcggcgacga gggcatcggc 1140
   tctggctgcc gaaccaccga cggcaccaac agcacccagt tcaaccccat cttccccgcc 1200
   acgtgcccct acgtcactgc cgtcggcggc accatgagct acgcccccga gattgcttgg 1260
   gaggccagct ccggcggctt cagcaactac ttcgagcgag cctggttcca gaaggaagcc 1320
   gtccagaact acctcgccaa ccacatcacc aacgagacta agcagtacta cagccagttc 1380
   gccaacttca gcggccgagg cttccccgac gtcagcgccc acagcttcga gcccagctac 1440
   gaggtcatct tctacggcgc tcgctacggc agcggcggca cttctgctgc ctgccccctg 1500
   ttttctgccc tcgtcggcat gctcaacgac gcccgactcc gagccggcaa gtcgaccctc 1560
   ggcttcctca accccctgct ctacagcaag ggctacaagg ccctcaccga cgtcaccgct 1620
   ggccagagca ttggctgcaa cggcatcgac ccccagagcg acgaggctgt cgctggcgct 1680
   ggcatcattc cctgggccca ctggaacgcc accgtcggct gggaccctgt cactggcctt 1740
   ggcctccccg acttcgagaa gctccgccag ctcgtcctca gcctctaa 1788
<210> 88
   <211> 1809
   <212> DNA
   <213> Talaromyces stipitatus
<400> 88
   atgcagacct tcggtgcttt tctcgtttcc ttcctcgccg ccagcggcct ggccgcggcc      60
   gctgctgctc ttgttggcca cgagtctctc gccgccctcc ctgtcggctg ggacaaggtc     120
   agcactcctg ccgctggcac caacatccag ctcagcgtcg ccctcgccct ccagaacatc     180
   gagcagcttg aggaccacct caagagcgtc agcacccccg gctctgccag ctacggccag     240
   tacctcgaca gcgacggcat tgccgcccag tacggccctt ctgacgccag cgtcgaggcc     300
   gtcaccaact ggctcaagga agccggcgtc accgacatct acaacaacgg ccagagcatc     360
   cacttcgcca ccagcgtcag caaggccaac agcctcctcg gcgccgactt caactactac     420
   agcgacggct ccgccaccaa gctccgcacc ctcgcttaca gcgtccccag cgacctgaag     480
   gaagccatcg acctcgtcag ccccaccacc tacttcggca agaccaccgc cagccgcagc     540
   atccaggcct acaagaacaa gcgagccagc accaccagca agagcggcag cagcagcgtc     600
   caggtcagcg cctcttgcca gaccagcatc acccccgcct gcctcaagca gatgtacaac     660
   gtcggcaact acacccccag cgtcgcccac ggctctcgcg ttggcttcgg cagcttcctc     720
   aaccagagcg ccatcttcga cgacctcttc acctacgaga aggtcaacga catccccagc     780
   cagaacttca ccaaggtcat cattgccaac gccagcaaca gccaggacgc cagcgacggc     840
   aactacggcg aggccaacct cgacgtccag aacattgtcg gcatcagcca ccccctgccc     900
   gtcaccgagt ttctcactgg cggcagccca cccttcgtcg ccagcctcga cacccccacc     960
   aaccagaacg agccctacat cccctactac gagtacctcc tcagccagaa gaacgaggac    1020
   ctcccccagg tcatcagcaa cagctacggc gacgacgagc agagcgtccc ctacaagtac    1080
   gccatccgcg cctgcaacct catcggcctc actggcctcc gcggcatcag cgtccttgag    1140
   agcagcggcg atctcggcgt tggcgctggc tgccgatcca acgacggcaa gaacaagacc    1200
   cagttcgacc ccatcttccc cgccacgtgc ccctacgtca ctagcgtcgg cggcacccag    1260
   agcgtcaccc ccgagattgc ttgggtcgct tccagcggcg gcttcagcaa ctacttcccc    1320
   cgcacctggt atcaagagcc cgccatccag acctacctcg gcctcctcga cgacgagact    1380
   aagacctact acagccagta caccaacttc gagggccgag gcttccccga cgtcagcgcc    1440
   cattctctca cccccgacta ccaggtcgtc ggcggaggct accttcagcc ttctggcggc    1500
   acttctgccg ccagccctgt ctttgccggc atcattgccc tgctcaacga cgcccgactc    1560
   gccgctggca agcccaccct cggctttctc aaccccttct tctacctcta cggctacaag    1620
   ggcctcaacg acatcactgg cggccagagc gtcggctgca acggcatcaa cggccagact    1680
   ggcgcccctg ttcccggcgg aggaattgtc cctggcgccg cttggaacag caccaccgga 1740
   tgggaccctg ccaccggcct tggcaccccc gactttcaga agctcaagga actcgtcctc 1800
   agcttctaa 1809
<210> 89
   <211> 1800
   <212> DNA
   <213> Fusarium oxysporum
<400> 89
   atgcagacct tcggtgcttt tctcgtttcc ttcctcgccg ccagcggcct ggccgcggcc 60
   aagtcgtttt cccaccacgc cgaggccccc cagggctggc aggtccagaa gaccgccaag 120
   gtcgcctcca acacgcagca cgtctttagc ctcgccctga ccatgcagaa cgtcgaccag 180
   ctggagtcga agctcctgga cctgagctcc cccgacagcg ccaactacgg caactggctc 240
   agccacgacg agctgacctc cacgttctcg cccagcaagg aggccgtcgc ctcggtcacc 300
   aagtggctga agagcaaggg catcaagcac tacaaggtca acggcgcctt cattgacttt 360
   gccgccgacg tcgagaaggc caacaccctc ctgggcggcg actaccagta ctacacgaag 420
   gacggccaga ccaagctgcg cacgctctcc tactcgatcc ccgacgacgt cgccggccac 480
   gtccagttcg tcgaccccag caccaacttc ggcggcacgg tcgcctttaa ccccgtcccc 540
   cacccctccc gcaccctcca ggagcgcaag gtctccccct ccaagtcgac ggtcgacgcc 600
   tcctgccaga cctcgatcac gcccagctgc ctgaagcaga tgtacaacat tggcgactac 660
   acccccgacg ccaagagcgg ctccgagatc ggcttcagca gcttcctcgg ccaggccgcc 720
   atttacagcg acgtcttcaa gtttgaggag ctcttcggca tccccaagca gaactacacc 780
   acgatcctga ttaacaacgg caccgacgac cagaacacgg cccacggcaa ctttggcgag 840
   gccaacctcg acgccgagaa catcgtcggc attgcccacc ccctgccctt caagcagtac 900
   atcaccggcg gcagcccccc ctttgtcccc aacattgacc agcccacgga gaaggacaac 960
   cagaacgagc cctacgtccc cttctttcgc tacctcctgg gccagaagga cctgcccgcc 1020
   gtcatctcga ccagctacgg cgacgaggag gactccgtcc cccgcgagta cgccaccctc 1080
   acgtgcaaca tgatcggcct cctgggcctg cgcggcatct ccgtcatttt ctcctcgggc 1140
   gacattggcg tcggctcggg ctgcctcgcc cccgactaca agaccgtcga gttcaacgcc 1200
   atctttcccg ccacctgccc ctacctgacg tccgtcggcg gcaccgtcga cgtcacgccc 1260
   gagattgcct gggagggcag ctccggcggc ttctccaagt actttccccg cccctcgtac 1320
   caggacaagg ccatcaagaa gtacatgaag accgtctcga aggagacgaa gaagtactac 1380
   ggcccctaca ccaactggga gggccgcggc ttccccgacg tcgccggcca ctccgtcgcc 1440
   cccgactacg aggtcatcta caacggcaag caggcccgat ccggcggcac cagcgccgcc 1500
   gcccccgtct gggccgccat cgtcggcctc ctgaacgacg cccgattcaa ggccggcaag 1560
   aagagcctgg gctggctcaa ccccctgatc tacaagcacg gccccaaggt cctcaccgac 1620
   atcacgggcg gctacgccat tggctgcgac ggcaacaaca cccagagcgg caagcccgag 1680
   cccgccggct ccggcctggt ccccggcgcc cgatggaacg ccaccgccgg ctgggacccc 1740
   accacgggct acggcacgcc caacttccag aagctcaagg acctcgtcct gtccctctaa 1800
<210> 90
   <211> 1788
   <212> DNA
   <213> Trichoderma virens
<400> 90
   atgcagacct tcggtgcttt tctcgtttcc ttcctcgccg ccagcggcct ggccgcggcc      60
   gtcgtccatg agaagctcgc tgctgtcccc agcggctggc accaccttga ggatgccggc     120
   agcgaccacc agatcagcct ctcgattgcc ctcgcccgca agaacctcga ccagcttgag     180
   agcaagctca aggacctcag cacccctggc gagagccagt acggccagtg gctcgaccaa     240
   gaggaagtcg acaccctgtt ccccgtcgcc agcgacaagg ccgtcatcag ctggctccgc     300
   agcgccaaca tcacccacat tgcccgccag ggcagcctcg tcaacttcgc caccaccgtc     360
   gacaaggtca acaagctcct caacaccacc ttcgcctact accagcgcgg cagctctcag     420
   cgcctccgca ccaccgagta cagcatcccc gacgacctcg tcgacagcat cgacctgatc     480
   agccccacca cgttcttcgg caaggaaaag acctctgccg gcctcaccca gcgcagccag     540
   aaggtcgata accacgtcgc caagcgcagc aacagcagca gctgcgccga caccatcacc     600
   ctcagctgcc tcaaggaaat gtacaacttc ggcaactaca cccccagcgc cagcagcggc     660
   agcaagctcg gcttcgccag cttcctcaac gagagcgcca gctacagcga cctcgccaag     720
   ttcgagcgcc tcttcaacct ccccagccag aacttcagcg tcgagctgat caacggcggc     780
   gtcaacgacc agaaccagag caccgccagc ctcaccgagg ccgacctcga tgtcgagctg     840
   cttgtcggcg tcggccaccc cctgcccgtc accgagttta tcaccagcgg cgagcccccc     900
   ttcatccccg accctgatga gccttctgcc gccgacaacg agaacgagcc ctacctccag     960
   tactacgagt acctcctcag caagcccaac agcgccctgc cccaggtcat cagcaacagc    1020
   tacggcgacg acgagcagac cgtccccgag tactacgcca agcgcgtctg caacctcatc    1080
   ggcctcgtcg gcctccgcgg catcagcgtc cttgagtcta gcggcgacga gggcatcggc    1140
   tctggctgcc gaaccaccga cggcaccaac agcacccagt tcaaccccat cttccccgcc    1200
   acgtgcccct acgtcactgc cgtcggcggc accatgagct acgcccccga gattgcttgg    1260
   gaggccagct ccggcggctt cagcaactac ttcgagcgag cctggttcca gaaggaagcc    1320
   gtccagaact acctcgccaa ccacatcacc aacgagacta agcagtacta cagccagttc    1380
   gccaacttca gcggccgagg cttccccgac gtcagcgccc acagcttcga gcccagctac      1440
   gaggtcatct tctacggcgc tcgctacggc agcggcggca cttctgctgc ctgccccctg      1500
   ttttctgccc tcgtcggcat gctcaacgac gcccgactcc gagccggcaa gtcgaccctc      1560
   ggcttcctca accccctgct ctacagcaag ggctacaagg ccctcaccga cgtcaccgct      1620
   ggccagagca ttggctgcaa cggcatcgac ccccagagcg acgaggctgt cgctggcgct      1680
   ggcatcattc cctgggccca ctggaacgcc accgtcggct gggaccctgt cactggcctt      1740
   ggcctccccg acttcgagaa gctccgccag ctcgtcctca gcctctaa                   1788
<210> 91
   <211> 1770
   <212> DNA
   <213> Trichoderma atroviride
<400> 91
   atgcagacct tcggtgcttt tctcgtttcc ttcctcgccg ccagcggcct ggccgcggcc 60
   gctgtccttg tcgagtctct caagcaggtc cccaacggct ggaacgccgt cagcacccct 120
   gaccccagca ccagcatcgt cctccagatc gccctcgccc agcagaacat cgacgagctt 180
   gagtggcgcc tcgccgccgt gtctaccccc aactctggca actacggcaa gtacctcgac 240
   atcggcgaga tcgagggcat cttcgccccc agcaacgcca gctacaaggc cgtcgcttcc 300
   tggctccaga gccacggcgt caagaacttc gtcaagcagg ccggcagcat ctggttctac 360
   accaccgtca gcaccgccaa caagatgctc agcaccgact tcaagcacta cagcgacccc 420
   gtcggcatcg agaagctccg caccctccag tacagcatcc ccgaggaact cgtcggccac 480
   gtcgacctca tcagccccac cacctacttc ggcaacaacc accctgccac cgcccgcacc 540
   cccaacatga aggccatcaa cgtcacctac cagatcttcc accccgactg cctcaagacc 600
   aagtacggcg tcgacggcta cgccccctca cctcgatgcg gcagccgaat cggcttcggc 660
   agcttcctca acgagactgc cagctacagc gacctcgccc agttcgagaa gtacttcgac 720
   ctccccaacc agaacctcag caccctcctc atcaacggcg ccatcgacgt ccagcccccc 780
   agcaacaaga acgacagcga ggccaacatg gacgtccaga ccatcctcac cttcgtccag 840
   cccctgccca tcaccgagtt cgtcgtcgcc ggcatccccc cctacattcc cgatgccgcc 900
   ctccccattg gcgaccccgt tcagaacgag ccctggcttg agtacttcga gttcctcatg 960
   agccgcacca acgccgagct gccccaggtc attgccaaca gctacggcga cgaggaacag 1020
   accgtccccc aggcctacgc cgtccgcgtc tgcaaccaga ttggcctcct cggcctccgc 1080
   ggcatcagcg tcattgcctc tagcggcgac accggcgtcg gcatgtcttg catggccagc 1140
   aacagcacca ccccccagtt caaccccatg ttccccgcca gctgccccta catcaccacc 1200
   gtcggcggca cccagcacct cgacaacgag atcgcctggg agctgagcag cggcggcttc 1260
   agcaactact tcacccgccc ctggtatcaa gaggacgccg ccaagaccta ccttgagcgc 1320
   cacgtcagca ccgagactaa ggcctactac gagcgctacg ccaacttcct gggccgaggc 1380
   tttcctgacg tcgccgccct cagcctcaac cccgactacc ccgtcatcat cggcggcgag 1440
   cttggcccta acggcggcac ttctgctgcc gcccctgtcg tcgccagcat cattgccctg 1500
   ctcaacgacg cccgcctctg cctcggcaag cctgccctcg gctttctcaa ccccctcatc 1560
   taccagtacg ccgacaaggg cggcttcacc gacatcacca gcggccagtc ttggggctgc 1620
   gccggcaaca ccactcagac tggacctccc cctcctggcg ctggcgtcat tcctggcgct 1680
   cactggaacg ccaccaaggg ctgggacccc gtcaccggct ttggcacccc caacttcaag 1740
   aagctcctca gcctcgccct cagcgtctaa 1770
<210> 92
   <211> 1758
   <212> DNA
   <213> Agaricus bisporus
<400> 92
   atgcagacct tcggtgcttt tctcgtttcc ttcctcgccg ccagcggcct ggccgcggcc      60
   tctcctcttg ctcgacgctg ggacgacttc gccgagaagc acgcctgggt cgaggttcct     120
   cgcggctggg agatggtcag cgaggcccct agcgaccaca ccttcgacct ccgcatcggc     180
   gtcaagagca gcggcatgga acagctcatc gagaacctca tgcagaccag cgaccccacc     240
   cacagccgct acggccagca cctcagcaag gaagaactcc acgacttcgt ccagccccac     300
   cccgactcta ctggcgccgt cgaggcctgg cttgaggact tcggcatcag cgacgacttc     360
   atcgaccgca ccggcagcgg caactgggtc accgtccgag tctctgtcgc ccaggccgag     420
   cgaatgctcg gcaccaagta caacgtctac cgccacagcg agagcggcga gtccgtcgtc     480
   cgcaccatga gctacagcct ccccagcgag ctgcacagcc acatcgacgt cgtcgccccc     540
   accacctact tcggcaccat gaagtcgatg cgcgtcacct cgttcctcca gcccgagatc     600
   gagcccgtcg acccctctgc caagccttct gctgctcccg ccagctgcct cagcaccacc     660
   gtcattaccc ccgactgcct ccgcgacctc tacaacaccg ccgactacgt ccccagcgcc     720
   accagccgca acgccattgg cattgccggc tacctcgacc gcagcaaccg agccgacctc     780
   cagaccttct tccgccgctt tcgccctgac gccgtcggct tcaactacac caccgtccag     840
   ctcaacggcg gaggcgacga ccagaacgac cctggcgtcg aggccaacct cgacatccag     900
   tacgccgctg gcattgcctt ccccaccccc gccacctact ggtctactgg cggcagcccc     960
   cccttcatcc ccgacaccca gacccccacc aacaccaacg agccctacct cgactggatc    1020
   aacttcgtcc tcggccagga tgagatcccc caggtcatca gcaccagcta cggcgacgac    1080
   gagcagaccg tccccgagga ctacgccacc agcgtctgca acctcttcgc ccagcttggc    1140
   tctcgcggcg tcaccgtctt tttcagcagc ggcgacttcg gcgtcggcgg tggcgactgc      1200
   ctcactaacg acggcagcaa ccaggtcctc ttccagcccg ccttccctgc cagctgcccc      1260
   tttgtcactg ccgtcggcgg caccgtccga ctcgaccctg agatcgccgt cagcttcagc      1320
   ggcggtggct tcagccgcta cttcagccgc cccagctacc agaaccagac cgtcgcccag      1380
   ttcgtcagca acctcggcaa caccttcaac ggcctctaca acaagaacgg ccgagcctac      1440
   cccgacctcg ccgctcaggg caacggcttc caggtcgtca tcgacggcat cgtccgatcg      1500
   gtcggcggca cttctgccag cagccctacc gtcgccggca tcttcgccct gctcaacgac      1560
   ttcaagctct ctcgcggcca gagcaccctc ggcttcatca accccctcat ctacagcagc      1620
   gccacctccg gcttcaacga catccgagcc ggcaccaacc ctggctgtgg cacccgaggc      1680
   tttaccgccg gcactggctg ggaccctgtc accggactcg gcacccctga ctttctccgc     1740
   ctccagggcc tcatctaa                                                    1758
<210> 93
   <211> 1812
   <212> DNA
   <213> Magnaporthe oryzae
<400> 93
   atgcagacct tcggtgcttt tctcgtttcc ttcctcgccg ccagcggcct ggccgcggcc      60
   cgcgtctttg attctctccc tcacccccct cgcggctggt cctactctca cgccgctgag      120
   agcaccgagc ccctcaccct ccgaattgcc ctccgccagc agaacgccgc tgcccttgag      180
   caggtcgtcc tccaggtcag caacccccgc cacgccaact acggccagca cctcacccga      240
   gatgagctgc gctcttacac cgcccctacc cctcgcgctg tccgctctgt cactagctgg      300
   ctcgtcgaca acggcgtcga cgactacacc gtcgagcacg actgggtcac cctccgcacc      360
   actgtcggcg ctgccgatcg actcctcggc gccgactttg cctggtacgc tggccctggc      420
   gagactctcc agctccgcac tctcagctac ggcgtggacg acagcgtcgc ccctcacgtc      480
   gatctcgtcc agcccaccac ccgctttggc ggccctgttg gccaggccag ccacatcttc      540
   aagcaggacg acttcgacga gcagcagctc aagaccctca gcgtcggctt ccaggtcatg      600
   gccgacctcc ctgctaacgg ccctggcagc attaaggccg cctgcaacga gagcggcgtc      660
   acccctctct gcctccgcac cctctaccgc gtcaactaca agcccgccac caccggcaac      720
   ctcgtcgcct tcgccagctt ccttgagcag tacgcccgct acagcgacca gcaggccttc      780
   acccagcgag tccttggccc tggcgtcccg ctccagaact tcagcgtcga gactgtcaac      840
   ggcggagcca acgaccagca gagcaagctc gatagcggcg aggccaacct cgacctccag      900
   tacgtcatgg ccatgtccca ccccatcccc atccttgagt acagcactgg cggccgaggc      960
   cccctcgtcc ctactctcga tcagcccaac gccaacaaca gcagcaacga gccctacctt     1020
   gagttcctca cctacctgct cgcccagccc gacagcgcca ttccccagac tctcagcgtg     1080
   agctacggcg aggaagaaca gagcgtcccc cgcgactacg ccatcaaggt ctgcaacatg     1140
   ttcatgcagc tcggcgctcg cggcgtcagc gtcatgttta gcagcggcga tagcggccct     1200
   ggcaacgact gcgtccgagc ctctgacaac gccaccttct tcggcagcac cttccctgcc     1260
   ggctgcccct acgtcactag cgtcggcagc accgtcggct tcgagcctga gcgagccgtc     1320
   agctttagct ccggcggctt cagcatctac cacgcccgac ccgactacca gaacgaggtc     1380
   gtccccaagt acatcgagag catcaaggcc agcggctacg agaagttctt cgacggcaac     1440
   ggccgaggca tccccgatgt cgctgctcag ggcgctcgct tcgtcgtcat cgacaagggc     1500
   cgcgtcagcc tcatcagcgg cactagcgct tccagccccg ccttcgctgg catggtcgcc     1560
   ctcgtcaacg ccgctcgcaa gagcaaggat atgcccgccc tcggcttcct caaccccatg     1620
   ctctaccaga acgctgccgc catgaccgac atcgtcaacg gcgctggcat cggctgccgc     1680
   aagcagcgca ccgagtttcc caacggtgcc cgcttcaacg ccaccgccgg atgggaccct     1740
   gtcactggcc ttggcacccc cctgttcgac aagctcctcg ccgttggcgc tcccggcgtc     1800
   cctaacgcct aa                                                         1812
<210> 94
   <211> 1845
   <212> DNA
   <213> Togninia minima
<400> 94
   atgcagacct tcggtgcttt tctcgtttcc ttcctcgccg ccagcggcct ggccgcggcc      60
   tccgatgtcg tccttgagtc tctccgcgag gtcccccagg gctggaagcg actccgagat     120
   gccgaccccg agcagagcat caagctccgc attgcccttg agcagcccaa cctcgacctc     180
   ttcgagcaga ccctctacga catcagcagc cccgaccacc ccaagtacgg ccagcacctc     240
   aagagccacg agctgcgcga catcatggcc cctcgcgagg aatccactgc cgccgtcatt     300
   gcctggctcc aggatgctgg cctcagcggc agccagatcg aggacgacag cgactggatc     360
   aacatccaga ccaccgtcgc ccaggccaac gacatgctca acaccacctt cggcctcttc     420
   gcccaagagg gcaccgaggt caaccgcatt cgcgccctcg cctacagcgt ccccgaggaa     480
   attgtccccc acgtcaagat gatcgccccc atcatccgct tcggccagct ccgccctcag     540
   atgagccaca tcttcagcca cgagaaggtc gaggaaaccc ccagcatcgg caccatcaag     600
   gccgctgcca tccccagcgt cgacctcaac gtcaccgcct gcaacgccag catcaccccc     660
   gagtgcctcc gcgccctcta caacgtcggc gactacgagg ccgaccccag caagaagtcc     720
   ctcttcggcg tctgcggcta ccttgagcag tacgccaagc acgaccagct cgccaagttc     780
   gagcagacgt acgcccccta cgccatcggc gccgacttca gcgtcgtcac catcaacggc     840
   ggaggcgaca accagaccag caccatcgac gacggcgagg ccaacctcga catgcagtac      900
   gccgtcagca tggcctacaa gacccccatc acctactaca gcactggcgg ccgaggcccc      960
   ctcgtccctg atctcgatca gcccgacccc aacgacgtca gcaacgagcc ctacctcgac     1020
   ttcgtcagct acctcctcaa gctccccgac agcaagctcc cccagaccat caccaccagc     1080
   tacggcgagg acgagcagag cgtcccccgc agctacgtcg agaaggtctg caccatgttc     1140
   ggcgcccttg gcgcccgagg cgtcagcgtc attttcagct ctggcgacac cggcgtcggc     1200
   agcgcctgcc agactaacga cggcaagaac accacccgct ttctgcccat cttccctgcc     1260
   gcctgcccct acgtcactag cgtcggcggc acccgctacg tcgatcctga ggtcgccgtc     1320
   agcttcagca gcggcggctt cagcgacatc ttccccaccc ccctgtacca gaagggcgcc     1380
   gtcagcggct acctcaagat cctcggcgac cgctggaagg gcctctacaa ccctcacggc     1440
   cgaggcttcc ctgacgtcag cggccagtct gtccgctacc acgtctttga ctacggcaag     1500
   gacgtcatgt acagcggcac cagcgccagc gcccccatgt ttgctgctct cgtcagcctc     1560
   ctcaacaacg cccgcctcgc caagaagctc ccccctatgg gcttcctcaa cccctggctc     1620
   tacaccgtcg gcttcaacgg cctcaccgac atcgtccacg gcggctctac tggctgcacc     1680
   ggcaccgatg tctacagcgg cctgcctacc cccttcgtcc cctacgcctc ttggaacgcc     1740
   accgtcggct gggaccctgt cactggcctt ggcacccccc tgttcgacaa gctcctcaac     1800
   ctcagcaccc ccaacttcca cctcccccac atcggcggcc actaa                    1845
<210> 95
   <211> 1848
   <212> DNA
   <213> Bipolaris maydis
<400> 95
   atgcagacct tcggtgcttt tctcgtttcc ttcctcgccg ccagcggcct ggccgcggcc 60
   tctaccactt ctcacgtcga gggcgaggtc gtcgagcgcc ttcatggcgt ccctgagggc 120
   tggtcacagg tcggcgctcc caaccccgac cagaagctcc gcttccgcat tgccgtccgc 180
   agcgccgaca gcgagctgtt cgagcgcacc ctcatggaag tcagcagccc cagccacccc 240
   cgctacggcc agcacctcaa gcgccacgag ctgaaggacc tcatcaagcc tcgcgccaag 300
   agcaccagca acatcctcaa ctggctccaa gagagcggca tcgaggcccg cgacatccag 360
   aacgacggcg agtggatcag cttctacgcc cccgtcaagc gagccgagca gatgatgagc 420
   accaccttca agacctacca gaacgaggcc cgagccaaca tcaagaagat ccgcagcctc 480
   gactacagcg tccccaagca catccgcgac gacatcgaca tcatccagcc caccacgcgc 540
   ttcggccaga tccagcctga gcgcagccag gtctttagcc aagaggaagt ccccttcagc 600
   gccctcgtcg tcaacgccac gtgcaacaag aagatcaccc ccgactgcct cgccaacctc 660
   tacaacttca aggactacga cgccagcgac gccaacgtca cgatcggcgt cagcggcttc 720
   cttgagcagt acgcccgctt cgacgacctc aagcagttca tcagcacctt ccagcccaag 780
   gccgctggct ccaccttcca ggtcaccagc gtcaacgctg gccccttcga ccagaacagc 840
   accgcctcta gcgtcgaggc caacctcgac atccagtaca ccaccggcct cgtcgccccc 900
   gacatcgaga ctcgctactt caccgtcccc ggacgcggca tcctcatccc cgacctcgac 960
   cagcctaccg agagcgacaa cgccaacgag ccctacctcg actacttcac ctacctcaac 1020
   aaccttgagg acgaggaact ccccgacgtc ctcaccacca gctacggcga gagcgagcag 1080
   agcgtccctg ccgagtacgc caagaaggtc tgcaacctca tcggccagct cggcgctcgc 1140
   ggcgtcagcg tcattttcag cagcggcgac accggccctg gcagcgcctg ccagactaac 1200
   gacggcaaga acaccacccg ctttctgccc atcttccccg ccagctgccc ctacgtcact 1260
   agcgtcggcg gcactgtcgg cgtcgagcct gagaaggccg tcagctttag cagcggcggc 1320
   ttcagcgacc tctggccccg acctgcctac caagagaagg ccgtgagcga gtaccttgag 1380
   aagctcggcg accgctggaa cggcctctac aaccctcagg gccgaggctt ccctgacgtc 1440
   gctgctcagg gccagggctt ccaggtcttt gacaagggcc gcctcatctc ggtcggcggc 1500
   acatctgctt ccgcccctgt ctttgccagc gtcgtcgccc tcctcaacaa cgcccgaaag 1560
   gctgccggaa tgagcagcct cggcttcctc aacccctgga tctacgagca gggctacaag 1620
   ggcctcaccg acatcgtcgc tggcggctct actggctgca ccggccgctc tatctacagc 1680
   ggcctccctg cccccctggt cccttacgct tcttggaacg ccaccgaggg ctgggacccc 1740
   gtcactggct atggcacccc cgacttcaag cagctcctca ccctcgccac cgcccccaag 1800
   tctggcgagc gacgagttcg acgaggcggc cttggaggcc aggcttaa 1848
<210> 96
   <211> 1791
   <212> DNA
   <213> Arthroderma benhamiae
<400> 96
   atgcgtcttc tcaaatttgt gtgcctgttg gcatcagttg ccgccgcaaa gcctactcca      60
   ggggcgtcac acaaggtcat tgaacatctt gactttgttc cagaaggatg gcagatggtt     120
   ggtgccgcgg accctgctgc tatcattgat ttctggcttg ccatcgagcg cgaaaaccca     180
   gaaaagctct acgacaccat ctatgacgtc tccacccctg gacgcgcaca atatggcaaa     240
   catttgaagc gtgaggaatt ggatgactta ctacgcccaa gggcagagac gagtgagagc     300
   atcatcaact ggctcaccaa tggtggagtc aacccacaac atattcggga tgaaggggac     360
   tgggtcagat tctctaccaa tgtcaagact gccgaaacgt tgatgaatac ccgcttcaac     420
   gtcttcaagg acaacctaaa ttccgtttca aaaattcgaa ctttggagta ttccgtccct     480
   gtagctatat cagctcatgt ccaaatgatc cagccaacta ccttatttgg acgacagaag 540
   ccacagaaca gtttgatcct aaaccccttg accaaggatc tagaatccat gtccgttgaa 600
   gaatttgctg cttctcagtg caggtcctta gtgactactg cctgccttcg agaattgtac 660
   ggacttggtg accgtgtcac tcaggctagg gatgacaacc gtattggagt atccggcttt 720
   ttggaggagt acgcccaata ccgcgatctt gagctcttcc tctctcgctt tgagccatcc 780
   gccaaaggat ttaatttcag tgaaggcctt attgccggag gaaagaacac tcagggtggt 840
   cctggaagct ctactgaggc caaccttgat atgcaatatg tcgtcggtct gtcccacaag 900
   gcaaaggtca cctattactc caccgctggc cgtggcccat taattcccga tctatctcag 960
   ccaagccaag cttcaaacaa caacgaacca taccttgaac agctgcggta cctcgtaaag 1020
   ctccccaaga accagcttcc atctgtattg acaacttcct atggagacac agaacagagc 1080
   ttgcccgcca gctataccaa agccacttgc gacctctttg ctcagctagg aactatgggt 1140
   gtgtctgtta tcttcagcag tggtgatacc gggcccggaa gctcatgcca gaccaacgat 1200
   ggcaagaatg cgactcgctt caaccctatc tacccagctt cttgcccgtt tgtgacctcc 1260
   atcggtggaa ccgttggtac cggtcctgag cgtgcagttt cattctcctc tggtggcttc 1320
   tcagacaggt tcccccgccc acaatatcag gataacgctg ttaaagacta cctgaaaatt 1380
   ttgggcaacc agtggagcgg attgtttgac cccaacggcc gtgctttccc agatatcgca 1440
   gctcagggat caaattatgc tgtctatgac aagggaagga tgactggagt ctccggcacc 1500
   agtgcatccg cccctgccat ggctgccatc attgcccagc ttaacgattt ccgactggca 1560
   aagggctctc ctgtgctggg attcttgaac ccatggatat attccaaggg tttctctggc 1620
   tttacagata ttgttgatgg cggttccagg ggttgcactg gttacgatat atacagcggc 1680
   ttgaaagcga agaaggttcc ctacgcaagc tggaatgcaa ctaagggatg ggacccagta 1740
   acgggatttg gtactcccaa cttccaagct ctcactaaag tgctgcccta a 1791
<210> 97
   <211> 1803
   <212> DNA
   <213> Artificial Sequence
<220>
   <223> TRI045 synthetic gene optimized for expression
<400> 97
   atgcagacct tcggtgcttt tctcgtttcc ttcctcgccg ccagcggcct ggccgcggcc      60
   aagcctactc ctggcgcttc ccacaaggtc atcgagcacc tcgacttcgt ccccgagggc     120
   tggcagatgg tcggcgctgc tgaccctgcc gccatcatcg acttttggct cgccatcgag     180
   cgcgagaacc ccgagaagct ctacgacacc atctacgacg tcagcacccc cggacgcgcc     240
   cagtacggca agcacctcaa gcgcgaggaa ctcgacgacc tcctccgccc tcgcgccgag     300
   actagcgaga gcatcatcaa ctggctcacc aacggcggcg tcaaccccca gcacattcgc     360
   gacgagggcg actgggtccg cttcagcacc aacgtcaaga ccgccgagac tctcatgaac     420
   acccgcttca acgtctttaa ggacaacctc aacagcgtca gcaagatccg cacccttgag    480
   tacagcgtcc ccgtcgccat cagcgcccac gtccagatga tccagcccac caccctcttc     540
   ggccgccaga agccccagaa cagcctcatc ctcaaccccc tcaccaagga ccttgagagc     600
   atgagcgtcg aagagttcgc cgccagccag tgccgcagcc tcgtcactac tgcctgcctc     660
   cgcgagctgt acggcctcgg cgatcgagtc acccaggccc gcgacgacaa ccgaattggc     720
   gtcagcggct tcctcgaaga gtacgcccag taccgcgacc ttgagctgtt cctcagccgc     780
   ttcgagccca gcgccaaggg cttcaacttc agcgagggcc tgatcgctgg cggcaagaac     840
   acccagggtg gccctggctc tagcaccgag gccaacctcg acatgcagta cgtcgtcggc     900
   ctcagccaca aggccaaggt cacctactac agcactgccg gccgaggccc cctcatccct     960
   gatctctcac agcccagcca ggccagcaac aacaacgagc cctaccttga gcagctccgc    1020
   tacctcgtca agctccccaa gaaccagctc cccagcgtcc tcaccaccag ctacggcgac    1080
   accgagcaga gcctccccgc cagctacacc aaggccacgt gcgacctctt cgcccagctc    1140
   ggcactatgg gcgtcagcgt catcttcagc agcggcgaca ctggccctgg cagctcgtgc    1200
   cagaccaacg acggcaagaa cgccacgcgc ttcaacccca tctaccccgc cagctgcccc    1260
   ttcgtcacca gcattggcgg caccgtcggc accggccctg agcgagctgt cagctttagc    1320
   agcggcggct tcagcgaccg cttccctcgc cctcagtacc aggacaacgc cgtcaaggac    1380
   tacctcaaga tcctcggcaa ccagtggtcc ggcctcttcg accctaacgg ccgagccttc    1440
   cccgacattg ccgcccaggg cagcaactac gccgtctacg acaagggccg catgaccggc    1500
   gttagcggca cttctgcttc cgcccctgct atggccgcca tcattgccca gctcaacgac    1560
   ttccgcctcg ccaagggcag ccccgtcctc ggctttctca acccctggat ctacagcaag    1620
   ggcttcagcg gcttcaccga catcgtcgac ggcggctcta ggggctgcac cggctacgac    1680
   atctacagcg gcctcaaggc caagaaggtc cctacgccca gctggaacgc caccaagggc    1740
   tgggaccccg tcaccggctt tggcaccccc aacttccagg ccctgaccaa ggtcctgccc    1800
   taa                                                                  1803
<210> 98
   <211> 580
   <212> PRT
   <213> Arthroderma benhamiae
<400> 98
   Lys Pro Thr Pro Gly Ala Ser His Lys Val Ile Glu His Leu Asp Phe
   1 5 10 15
   Val Pro Glu Gly Trp Gln Met Val Gly Ala Ala Asp Pro Ala Ala Ile
   20 25 30
   Ile Asp Phe Trp Leu Ala Ile Glu Arg Glu Asn Pro Glu Lys Leu Tyr
   35 40 45
   Asp Thr Ile Tyr Asp Val Ser Thr Pro Gly Arg Ala Gln Tyr Gly Lys
   50 55 60
   His Leu Lys Arg Glu Glu Leu Asp Asp Leu Leu Arg Pro Arg Ala Glu
   65 70 75 80
   Thr Ser Glu Ser Ile Ile Asn Trp Leu Thr Asn Gly Gly Val Asn Pro
   85 90 95
   Gln His Ile Arg Asp Glu Gly Asp Trp Val Arg Phe Ser Thr Asn Val
   100 105 110
   Lys Thr Ala Glu Thr Leu Met Asn Thr Arg Phe Asn Val Phe Lys Asp
   115 120 125
   Asn Leu Asn Ser Val Ser Lys Ile Arg Thr Leu Glu Tyr Ser Val Pro
   130 135 140
   Val Ala Ile Ser Ala His Val Gln Met Ile Gln Pro Thr Thr Leu Phe
   145 150 155 160
   Gly Arg Gln Lys Pro Gln Asn Ser Leu Ile Leu Asn Pro Leu Thr Lys
   165 170 175
   Asp Leu Glu Ser Met Ser Val Glu Glu Phe Ala Ala Ser Gln Cys Arg
   180 185 190
   Ser Leu Val Thr Thr Ala Cys Leu Arg Glu Leu Tyr Gly Leu Gly Asp
   195 200 205
   Arg Val Thr Gln Ala Arg Asp Asp Asn Arg Ile Gly Val Ser Gly Phe
   210 215 220
   Leu Glu Glu Tyr Ala Gln Tyr Arg Asp Leu Glu Leu Phe Leu Ser Arg
   225 230 235 240
   Phe Glu Pro Ser Ala Lys Gly Phe Asn Phe Ser Glu Gly Leu Ile Ala
   245 250 255
   Gly Gly Lys Asn Thr Gln Gly Gly Pro Gly Ser Ser Thr Glu Ala Asn
               260                 265                 270
   Leu Asp Met Gln Tyr Val Val Gly Leu Ser His Lys Ala Lys Val Thr
           275                 280                 285
   Tyr Tyr Ser Thr Ala Gly Arg Gly Pro Leu Ile Pro Asp Leu Ser Gln
       290                 295                 300
   Pro Ser Gln Ala Ser Asn Asn Asn Glu Pro Tyr Leu Glu Gln Leu Arg
   305                 310                 315                 320
   Tyr Leu Val Lys Leu Pro Lys Asn Gln Leu Pro Ser Val Leu Thr Thr
                   325                 330                 335
   Ser Tyr Gly Asp Thr Glu Gln Ser Leu Pro Ala Ser Tyr Thr Lys Ala
               340                 345                 350
   Thr Cys Asp Leu Phe Ala Gln Leu Gly Thr Met Gly Val Ser Val Ile
           355                 360                 365
   Phe Ser Ser Gly Asp Thr Gly Pro Gly Ser Ser Cys Gln Thr Asn Asp
       370                 375                 380
   Gly Lys Asn Ala Thr Arg Phe Asn Pro Ile Tyr Pro Ala Ser Cys Pro
   385                 390                 395                 400
   Phe Val Thr Ser Ile Gly Gly Thr Val Gly Thr Gly Pro Glu Arg Ala
                   405                 410                 415
   Val Ser Phe Ser Ser Gly Gly Phe Ser Asp Arg Phe Pro Arg Pro Gln
               420                 425                 430
   Tyr Gln Asp Asn Ala Val Lys Asp Tyr Leu Lys Ile Leu Gly Asn Gln
           435                 440                 445
   Trp Ser Gly Leu Phe Asp Pro Asn Gly Arg Ala Phe Pro Asp Ile Ala
       450                 455                 460
   Ala Gln Gly Ser Asn Tyr Ala Val Tyr Asp Lys Gly Arg Met Thr Gly
   465                 470                 475                 480
   Val Ser Gly Thr Ser Ala Ser Ala Pro Ala Met Ala Ala Ile Ile Ala
                   485                 490                 495
   Gln Leu Asn Asp Phe Arg Leu Ala Lys Gly Ser Pro Val Leu Gly Phe
               500                 505                 510
   Leu Asn Pro Trp Ile Tyr Ser Lys Gly Phe Ser Gly Phe Thr Asp Ile
   515 520 525
   Val Asp Gly Gly Ser Arg Gly Cys Thr Gly Tyr Asp Ile Tyr Ser Gly
   530 535 540
   Leu Lys Ala Lys Lys Val Pro Tyr Ala Ser Trp Asn Ala Thr Lys Gly
   545 550 555 560
   Trp Asp Pro Val Thr Gly Phe Gly Thr Pro Asn Phe Gln Ala Leu Thr
   565 570 575
   Lys Val Leu Pro
   580
<210> 99
   <211> 390
   <212> PRT
   <213> Arthroderma benhamiae
<400> 99
   Cys Arg Ser Leu Val Thr Thr Ala Cys Leu Arg Glu Leu Tyr Gly Leu
   1 5 10 15
   Gly Asp Arg Val Thr Gln Ala Arg Asp Asp Asn Arg Ile Gly Val Ser
   20 25 30
   Gly Phe Leu Glu Glu Tyr Ala Gln Tyr Arg Asp Leu Glu Leu Phe Leu
   35 40 45
   Ser Arg Phe Glu Pro Ser Ala Lys Gly Phe Asn Phe Ser Glu Gly Leu
   50 55 60
   Ile Ala Gly Gly Lys Asn Thr Gln Gly Gly Pro Gly Ser Ser Thr Glu
   65 70 75 80
   Ala Asn Leu Asp Met Gln Tyr Val Val Gly Leu Ser His Lys Ala Lys
   85 90 95
   Val Thr Tyr Tyr Ser Thr Ala Gly Arg Gly Pro Leu Ile Pro Asp Leu
   100 105 110
   Ser Gln Pro Ser Gln Ala Ser Asn Asn Asn Glu Pro Tyr Leu Glu Gln
   115 120 125
   Leu Arg Tyr Leu Val Lys Leu Pro Lys Asn Gln Leu Pro Ser Val Leu
   130 135 140
   Thr Thr Ser Tyr Gly Asp Thr Glu Gln Ser Leu Pro Ala Ser Tyr Thr
   145 150 155 160
   Lys Ala Thr Cys Asp Leu Phe Ala Gln Leu Gly Thr Met Gly Val Ser
   165 170 175
   Val Ile Phe Ser Ser Gly Asp Thr Gly Pro Gly Ser Ser Cys Gln Thr
   180 185 190
   Asn Asp Gly Lys Asn Ala Thr Arg Phe Asn Pro Ile Tyr Pro Ala Ser
   195 200 205
   Cys Pro Phe Val Thr Ser Ile Gly Gly Thr Val Gly Thr Gly Pro Glu
   210 215 220
   Arg Ala Val Ser Phe Ser Ser Gly Gly Phe Ser Asp Arg Phe Pro Arg
   225 230 235 240
   Pro Gln Tyr Gln Asp Asn Ala Val Lys Asp Tyr Leu Lys Ile Leu Gly
   245 250 255
   Asn Gln Trp Ser Gly Leu Phe Asp Pro Asn Gly Arg Ala Phe Pro Asp
   260 265 270
   Ile Ala Ala Gln Gly Ser Asn Tyr Ala Val Tyr Asp Lys Gly Arg Met
   275 280 285
   Thr Gly Val Ser Gly Thr Ser Ala Ser Ala Pro Ala Met Ala Ala Ile
   290 295 300
   Ile Ala Gln Leu Asn Asp Phe Arg Leu Ala Lys Gly Ser Pro Val Leu
   305 310 315 320
   Gly Phe Leu Asn Pro Trp Ile Tyr Ser Lys Gly Phe Ser Gly Phe Thr
   325 330 335
   Asp Ile Val Asp Gly Gly Ser Arg Gly Cys Thr Gly Tyr Asp Ile Tyr
   340 345 350
   Ser Gly Leu Lys Ala Lys Lys Val Pro Tyr Ala Ser Trp Asn Ala Thr
   355 360 365
   Lys Gly Trp Asp Pro Val Thr Gly Phe Gly Thr Pro Asn Phe Gln Ala
   370 375 380
   Leu Thr Lys Val Leu Pro
   385 390
<210> 100
   <211> 5
   <212> PRT
   <213> Artificial Sequence
<220>
   <223> Peptide substrate sequence
<400> 100
   Ala Ala Pro Pro Ala
   1 5
<210> 101
   <211> 5
   <212> PRT
   <213> Artificial Sequence
<220>
   <223> Peptide fragment of beta-lactoglobulin
<400> 101
   Ile Ile Ala Glu Lys
   1 5
<210> 102
   <211> 7
   <212> PRT
   <213> Artificial Sequence
<220>
   <223> Peptide fragment of beta-lactoglobulin
<400> 102
   Ala Leu Pro Met His Ile Arg
   1 5
<210> 103
   <211> 9
   <212> PRT
   <213> Artificial Sequence
<220>
   <223> Peptide fragment of beta-lactoglobulin
<400> 103
   Trp Glu Asn Gly Glu Cys Ala Gln Lys
   1 5
<210> 104
   <211> 20
   <212> PRT
   <213> Artificial Sequence
<220>
   <223> Peptide fragment of beta-lactoglobulin
<400> 104
   Val Tyr Val Glu Glu Leu Lys Pro Thr Pro Glu Gly Asp Leu Glu Ile
   1               5                   10                  15
   Leu Leu Gln Lys
               20
<210> 105
   <211> 17
   <212> PRT
   <213> Artificial Sequence
<220>
   <223> Peptide fragment of beta-lactoglobulin
<400> 105
   Glu Glu Leu Lys Pro Thr Pro Glu Gly Asp Leu Glu Ile Leu Leu Gln
   1               5                   10                  15
   Lys
<210> 106
   <211> 26
   <212> PRT
   <213> Artificial Sequence
<220>
   <223> Peptide fragment of beta-lactoglobulin
<400> 106
   Val Ala Gly Thr Trp Tyr Ser Leu Ala Met Ala Ala Ser Asp Ile Ser
   1               5                   10                  15
   Leu Leu Asp Ala Gln Ser Ala Pro Leu Arg
               20                  25
<210> 107
   <211> 7
   <212> PRT
   <213> Artificial Sequence
<220>
   <223> tryptic fragment of beta-casein
<400> 107
   Gly Pro Phe Pro Ile Ile Val
   1               5
<210> 108
   <211> 8
   <212> PRT
   <213> Artificial Sequence
<220>
   <223> tryptic fragment of beta-casein
<400> 108
   His Lys Glu Met Pro Phe Pro Lys
   1 5
<210> 109
   <211> 6
   <212> PRT
   <213> Artificial Sequence
<220>
   <223> Sequence motif
<220>
   <221> MISC_FEATURE
   <222> (1)..(1)
   <223> Xaa may be Gly, Thr, Ser or Val
<400> 109
   Xaa Glu Ala Asn Leu Asp
   1 5
<210> 110
   <211> 5
   <212> PRT
   <213> Artificial Sequence
<220>
   <223> Sequence motif
<220>
   <221> MISC_FEATURE
   <222> (1)..(1)
   <223> Xaa may be Ile, Leu or Val
<220>
   <221> MISC_FEATURE
   <222> (3)..(3)
   <223> Xaa may be Ser or Thr
<220>
   <221> MISC_FEATURE
   <222> (4)..(4)
   <223> Xaa may be Ile or Val
<400> 110
   Xaa Thr Xaa Xaa Gly
   1 5
<210> 111
   <211> 5
   <212> PRT
   <213> Artificial Sequence
<220>
   <223> Sequence motif
<400> 111
   Gln Asn Phe Ser Val
   1 5
<210> 112
   <211> 844
   <212> PRT
   <213> Lactobacillus helveticus
<400> 112
   Met Ala Val Lys Arg Phe Tyr Lys Thr Phe His Pro Glu His Tyr Asp
   1               5                   10                  15
   Leu Arg Ile Asn Val Asn Arg Lys Asn Lys Thr Ile Asn Gly Thr Ser
               20                  25                  30
   Thr Ile Thr Gly Asp Val Ile Glu Asn Pro Val Phe Ile Asn Gln Lys
           35                  40                  45
   Phe Met Thr Ile Asp Ser Val Lys Val Asp Gly Lys Asn Val Asp Phe
       50                  55                  60
   Asp Val Ile Glu Lys Asp Glu Ala Ile Lys Ile Lys Thr Gly Val Thr
   65                  70                  75                  80
   Gly Lys Ala Val Ile Glu Ile Ala Tyr Ser Ala Pro Leu Thr Asp Thr
                   85                  90                  95
   Met Met Gly Ile Tyr Pro Ser Tyr Tyr Glu Leu Glu Gly Lys Lys Lys
               100                 105                 110
   Gln Ile Ile Gly Thr Gln Phe Glu Thr Thr Phe Ala Arg Gln Ala Phe
           115                 120                 125
   Pro Cys Val Asp Glu Pro Glu Ala Lys Ala Thr Phe Ser Leu Ala Leu
       130                 135                 140
   Lys Trp Asp Glu Gln Asp Gly Glu Val Ala Leu Ala Asn Met Pro Glu
   145                 150                 155                 160
   Val Glu Val Asp Lys Asp Gly Tyr His His Phe Glu Glu Thr Val Arg
   165 170 175
   Met Ser Ser Tyr Leu Val Ala Phe Ala Phe Gly Glu Leu Gln Ser Lys
   180 185 190
   Thr Thr His Thr Lys Asp Gly Val Leu Ile Gly Val Tyr Ala Thr Lys
   195 200 205
   Ala His Lys Pro Lys Glu Leu Asp Phe Ala Leu Asp Ile Ala Lys Arg
   210 215 220
   Ala Ile Glu Phe Tyr Glu Glu Phe Tyr Gln Thr Lys Tyr Pro Leu Pro
   225 230 235 240
   Gln Ser Leu Gln Leu Ala Leu Pro Asp Phe Ser Ala Gly Ala Met Glu
   245 250 255
   Asn Trp Gly Leu Val Thr Tyr Arg Glu Ala Tyr Leu Leu Leu Asp Pro
   260 265 270
   Asp Asn Thr Ser Leu Glu Met Lys Lys Leu Val Ala Thr Val Ile Thr
   275 280 285
   His Glu Leu Ala His Gln Trp Phe Gly Asp Leu Val Thr Met Lys Trp
   290 295 300
   Trp Asp Asn Leu Trp Leu Asn Glu Ser Phe Ala Asn Met Met Glu Tyr
   305 310 315 320
   Leu Ser Val Asp Gly Leu Glu Pro Asp Trp His Ile Trp Glu Met Phe
   325 330 335
   Gln Thr Ser Glu Ala Ala Ser Ala Leu Asn Arg Asp Ala Thr Asp Gly
   340 345 350
   Val Gln Pro Ile Gln Met Glu Ile Asn Asp Pro Ala Asp Ile Asp Ser
   355 360 365
   Val Phe Asp Gly Ala Ile Val Tyr Ala Lys Gly Ser Arg Met Leu Val
   370 375 380
   Met Val Arg Ser Leu Leu Gly Asp Asp Ala Leu Arg Lys Gly Leu Lys
   385 390 395 400
   Tyr Tyr Phe Asp His His Lys Phe Gly Asn Ala Thr Gly Asp Asp Leu
   405 410 415
   Trp Asp Ala Leu Ser Thr Ala Thr Asp Leu Asp Ile Gly Lys Ile Met
   420 425 430
   His Ser Trp Leu Lys Gln Pro Gly Tyr Pro Val Val Asn Ala Phe Val
   435 440 445
   Ala Glu Asp Gly His Leu Lys Leu Thr Gln Lys Gln Phe Phe Ile Gly
   450 455 460
   Glu Gly Glu Asp Lys Gly Arg Gln Trp Gln Ile Pro Leu Asn Ala Asn
   465 470 475 480
   Phe Asp Ala Pro Lys Ile Met Ser Asp Lys Glu Ile Asp Leu Gly Asn
   485 490 495
   Tyr Lys Val Leu Arg Glu Glu Ala Gly His Pro Leu Arg Leu Asn Val
   500 505 510
   Gly Asn Asn Ser His Phe Ile Val Glu Tyr Asp Lys Thr Leu Leu Asp
   515 520 525
   Asp Ile Leu Ser Asp Val Asn Glu Leu Asp Pro Ile Asp Lys Leu Gln
   530 535 540
   Leu Leu Gln Asp Leu Arg Leu Leu Ala Glu Gly Lys Gln Ile Ser Tyr
   545 550 555 560
   Ala Ser Ile Val Pro Leu Leu Val Lys Phe Ala Asp Ser Lys Ser Ser
   565 570 575
   Leu Val Ile Asn Ala Leu Tyr Thr Thr Ala Ala Lys Leu Arg Gln Phe
   580 585 590
   Val Glu Pro Glu Ser Asn Glu Glu Lys Asn Leu Lys Lys Leu Tyr Asp
   595 600 605
   Leu Leu Ser Lys Asp Gln Val Ala Arg Leu Gly Trp Glu Val Lys Pro
   610 615 620
   Gly Glu Ser Asp Glu Asp Val Gln Ile Arg Pro Tyr Glu Leu Ser Ala
   625 630 635 640
   Ser Leu Tyr Ala Glu Asn Ala Asp Ser Ile Lys Ala Ala His Gln Ile
   645 650 655
   Phe Thr Glu Asn Glu Asp Asn Leu Glu Ala Leu Asn Ala Asp Ile Arg
   660 665 670
   Pro Tyr Val Leu Ile Asn Glu Val Lys Asn Phe Gly Asn Ala Glu Leu
   675 680 685
   Val Asp Lys Leu Ile Lys Glu Tyr Gln Arg Thr Ala Asp Pro Ser Tyr
   690 695 700
   Lys Val Asp Leu Arg Ser Ala Val Thr Ser Thr Lys Asp Leu Ala Ala
   705 710 715 720
   Ile Lys Ala Ile Val Gly Asp Phe Glu Asn Ala Asp Val Val Lys Pro
   725 730 735
   Gln Asp Leu Cys Asp Trp Tyr Arg Gly Leu Leu Ala Asn His Tyr Gly
   740 745 750
   Gln Gln Ala Ala Trp Asp Trp Ile Arg Glu Asp Trp Asp Trp Leu Asp
   755 760 765
   Lys Thr Val Gly Gly Asp Met Glu Phe Ala Lys Phe Ile Thr Val Thr
   770 775 780
   Ala Gly Val Phe His Thr Pro Glu Arg Leu Lys Glu Phe Lys Glu Phe
   785 790 795 800
   Phe Glu Pro Lys Ile Asn Val Pro Leu Leu Ser Arg Glu Ile Lys Met
   805 810 815
   Asp Val Lys Val Ile Glu Ser Lys Val Asn Leu Ile Glu Ala Glu Lys
   820 825 830
   Asp Ala Val Asn Asp Ala Val Ala Lys Ala Ile Asp
   835 840
<210> 113
   <211> 7
   <212> PRT
   <213> Artificial Sequence
<220>
   <223> Protein sequence cleaved by tripeptidyl peptidase
<400> 113
   Val Ile Pro Val Pro Gln Lys
   1 5
<210> 114
   <211> 6
   <212> PRT
   <213> Artificial Sequence
<220>
   <223> Protein sequence cleaved by tripeptidyl peptidase
<400> 114
   Gly Pro Phe Pro Val Ile
   1               5
<210> 115
   <211> 8
   <212> PRT
   <213> Artificial Sequence
<220>
   <223> Peptide substrate
<400> 115
   Arg Gly Pro Phe Pro Ile Ile Val
   1               5
<210> 116
   <211> 5
   <212> PRT
   <213> Artificial Sequence
<220>
   <223> Peptide substrate
<400> 116
   Ala Ala Phe Pro Ala
   1               5
<210> 117
   <211> 525
   <212> PRT
   <213> Artificial Sequence
<220>
   <223> Enzyme polypetide sequence (not to be combined with)
<400> 117
   Met Arg Thr Ala Ala Ala Ser Leu Thr Leu Ala Ala Thr Cys Leu Phe
   1               5                   10                  15
   Glu Leu Ala Ser Ala Leu Met Pro Arg Ala Pro Leu Ile Pro Ala Met
               20                  25                  30
   Lys Ala Lys Val Ala Leu Pro Ser Gly Asn Ala Thr Phe Glu Gln Tyr
           35                  40                  45
   Ile Asp His Asn Asn Pro Gly Leu Gly Thr Phe Pro Gln Arg Tyr Trp
       50                  55                  60
   Tyr Asn Pro Glu Phe Trp Ala Gly Pro Gly Ser Pro Val Leu Leu Phe
   65                  70                  75                  80
   Thr Pro Gly Glu Ser Asp Ala Ala Asp Tyr Asp Gly Phe Leu Thr Asn
                   85                  90                  95
   Lys Thr Ile Val Gly Arg Phe Ala Glu Glu Ile Gly Gly Ala Val Ile
               100                 105                 110
   Leu Leu Glu His Arg Tyr Trp Gly Ala Ser Ser Pro Tyr Pro Glu Leu
           115                 120                 125
   Thr Thr Glu Thr Leu Gln Tyr Leu Thr Leu Glu Gln Ser Ile Ala Asp
       130                 135                 140
   Leu Val His Phe Ala Lys Thr Val Asn Leu Pro Phe Asp Glu Ile His
   145                 150                 155                 160
   Ser Ser Asn Ala Asp Asn Ala Pro Trp Val Met Thr Gly Gly Ser Tyr
                   165                 170                 175
   Ser Gly Ala Leu Ala Ala Trp Thr Ala Ser Ile Ala Pro Gly Thr Phe
               180                 185                 190
   Trp Ala Tyr His Ala Ser Ser Ala Pro Val Gln Ala Ile Tyr Asp Phe
           195                 200                 205
   Trp Gln Tyr Phe Val Pro Val Val Glu Gly Met Pro Lys Asn Cys Ser
       210                 215                 220
   Lys Asp Leu Asn Arg Val Val Glu Tyr Ile Asp His Val Tyr Glu Ser
   225                 230                 235                 240
   Gly Asp Ile Glu Arg Gln Gln Glu Ile Lys Glu Met Phe Gly Leu Gly
                   245                 250                 255
   Ala Leu Lys His Phe Asp Asp Phe Ala Ala Ala Ile Thr Asn Gly Pro
               260                 265                 270
   Trp Leu Trp Gln Asp Met Asn Phe Val Ser Gly Tyr Ser Arg Phe Tyr
           275                 280                 285
   Lys Phe Cys Asp Ala Val Glu Asn Val Thr Pro Gly Ala Lys Ser Val
       290                 295                 300
   Pro Gly Pro Glu Gly Val Gly Leu Glu Lys Ala Leu Gln Gly Tyr Ala
   305                 310                 315                 320
   Ser Trp Phe Asn Ser Thr Tyr Leu Pro Gly Ser Cys Ala Glu Tyr Lys
   325 330 335
   Tyr Trp Thr Asp Lys Asp Ala Val Asp Cys Tyr Asp Ser Tyr Glu Thr
   340 345 350
   Asn Ser Pro Ile Tyr Thr Asp Lys Ala Val Asn Asn Thr Ser Asn Lys
   355 360 365
   Gln Trp Thr Trp Phe Leu Cys Asn Glu Pro Leu Phe Tyr Trp Gln Asp
   370 375 380
   Gly Ala Pro Lys Asp Glu Ser Thr Ile Val Ser Arg Ile Val Ser Ala
   385 390 395 400
   Glu Tyr Trp Gln Arg Gln Cys His Ala Tyr Phe Pro Glu Val Asn Gly
   405 410 415
   Tyr Thr Phe Gly Ser Ala Asn Gly Lys Thr Ala Glu Asp Val Asn Lys
   420 425 430
   Trp Thr Lys Gly Trp Asp Leu Thr Asn Thr Thr Arg Leu Ile Trp Ala
   435 440 445
   Asn Gly Gln Phe Asp Pro Trp Arg Asp Ala Ser Val Ser Ser Lys Thr
   450 455 460
   Arg Pro Gly Gly Pro Leu Gln Ser Thr Glu Gln Ala Pro Val His Val
   465 470 475 480
   Ile Pro Gly Gly Phe His Cys Ser Asp Gln Trp Leu Val Tyr Gly Glu
   485 490 495
   Ala Asn Ala Gly Val Gln Lys Val Ile Asp Glu Glu Val Ala Gln Ile
   500 505 510
   Lys Ala Trp Val Ala Glu Tyr Pro Lys Tyr Arg Lys Pro
   515 520 525
<210> 118
   <211> 33
   <212> PRT
   <213> Artificial Sequence
<220>
   <223> alpha-2-gliadin sequence
<400> 118
   Leu Gln Leu Gln Pro Phe Pro Gln Pro Gln Leu Pro Tyr Pro Gln Pro
   1               5                   10                  15
   Gln Leu Pro Tyr Pro Gln Pro Gln Leu Pro Tyr Pro Gln Pro Gln Pro
               20                  25                  30
   Phe
<210> 119
   <211> 21
   <212> PRT
   <213> Artificial Sequence
<220>
   <223> Intermediate alpha-2-gliadin peptide product
<400> 119
   Tyr Pro Gln Pro Gln Leu Pro Tyr Pro Gln Pro Gln Leu Pro Tyr Pro
   1               5                   10                  15
   Gln Pro Gln Pro Phe
               20
<210> 120
   <211> 178
   <212> PRT
   <213> Bos taurus
<400> 120
   Met Lys Cys Leu Leu Leu Ala Leu Ala Leu Thr Cys Gly Ala Gln Ala
   1               5                   10                  15
   Leu Ile Val Thr Gln Thr Met Lys Gly Leu Asp Ile Gln Lys Val Ala
               20                  25                  30
   Gly Thr Trp Tyr Ser Leu Ala Met Ala Ala Ser Asp Ile Ser Leu Leu
           35                  40                  45
   Asp Ala Gln Ser Ala Pro Leu Arg Val Tyr Val Glu Glu Leu Lys Pro
       50                  55                  60
   Thr Pro Glu Gly Asp Leu Glu Ile Leu Leu Gln Lys Trp Glu Asn Gly
   65                  70                  75                  80
   Glu Cys Ala Gln Lys Lys Ile Ile Ala Glu Lys Thr Lys Ile Pro Ala
                   85                  90                  95
   Val Phe Lys Ile Asp Ala Leu Asn Glu Asn Lys Val Leu Val Leu Asp
   100 105 110
   Thr Asp Tyr Lys Lys Tyr Leu Leu Phe Cys Met Glu Asn Ser Ala Glu
   115 120 125
   Pro Glu Gln Ser Leu Ala Cys Gln Cys Leu Val Arg Thr Pro Glu Val
   130 135 140
   Asp Asp Glu Ala Leu Glu Lys Phe Asp Lys Ala Leu Lys Ala Leu Pro
   145 150 155 160
   Met His Ile Arg Leu Ser Phe Asn Pro Thr Gln Leu Glu Glu Gln Cys
   165 170 175
   His Ile
<210> 121
   <211> 224
   <212> PRT
   <213> Bos taurus
<400> 121
   Met Lys Val Leu Ile Leu Ala Cys Leu Val Ala Leu Ala Leu Ala Arg
   1               5                   10                  15
   Glu Leu Glu Glu Leu Asn Val Pro Gly Glu Ile Val Glu Ser Leu Ser
               20                  25                  30
   Ser Ser Glu Glu Ser Ile Thr Arg Ile Asn Lys Lys Ile Glu Lys Phe
           35                  40                  45
   Gln Ser Glu Glu Gln Gln Gln Thr Glu Asp Glu Leu Gln Asp Lys Ile
       50                  55                  60
   His Pro Phe Ala Gln Thr Gln Ser Leu Val Tyr Pro Phe Pro Gly Pro
   65                  70                  75                  80
   Ile Pro Asn Ser Leu Pro Gln Asn Ile Pro Pro Leu Thr Gln Thr Pro
                   85                  90                  95
   Val Val Val Pro Pro Phe Leu Gln Pro Glu Val Met Gly Val Ser Lys
               100                 105                 110
   Val Lys Glu Ala Met Ala Pro Lys His Lys Glu Met Pro Phe Pro Lys
           115                 120                 125
   Tyr Pro Val Glu Pro Phe Thr Glu Ser Gln Ser Leu Thr Leu Thr Asp
       130                 135                 140
   Val Glu Asn Leu His Leu Pro Leu Pro Leu Leu Gln Ser Trp Met His
   145                 150                 155                 160
   Gln Pro His Gln Pro Leu Pro Pro Thr Val Met Phe Pro Pro Gln Ser
                   165                 170                 175
   Val Leu Ser Leu Ser Gln Ser Lys Val Leu Pro Val Pro Gln Lys Ala
               180                 185                 190
   Val Pro Tyr Pro Gln Arg Asp Met Pro Ile Gln Ala Phe Leu Leu Tyr
           195                 200                 205
   Gln Glu Pro Val Leu Gly Pro Val Arg Gly Pro Phe Pro Ile Ile Val
       210                 215                 220
<210> 122
   <211> 9
   <212> PRT
   <213> Artificial Sequence
<220>
   <223> sequence of bradykinin peptide
<400> 122
   Arg Pro Pro Gly Phe Ser Pro Phe Arg
   1               5
<210> 123
   <211> 4
   <212> PRT
   <213> Artificial Sequence
<220>
   <223> Peptide substrate sequence
<220>
   <221> MISC_FEATURE
   <222> (3)..(3)
   <223> Xaa may be any amino acid except Cys and Met
<400> 123
   Ala Ala Xaa Phe
   1

## Description

### FIELD OF THE INVENTION

The present invention relates to proline tolerant tripeptidyl peptidases for use in the preparation of hydrolysates and in foods comprising said proline tolerant tripeptidyl peptidases or hydrolysates.

### BACKGROUND

Proteases (synonymous with peptidases) are enzymes that are capable of cleaving peptide bonds between amino acids in substrate peptides, oligopeptides and/or proteins.

Proteases are grouped into 7 families based on their catalytic reaction mechanism and the amino acid residue involved in the active site for catalysis. The serine proteases, aspartic acid proteases, cysteine proteases and metalloprotease are the 4 major families, whilst the threonine proteases, glutamic acid proteases and ungrouped proteases make up the remaining 3 families.

The substrate specificity of a protease is usually defined in terms of preferential cleavage of bonds between particular amino acid residues in a substrate. Typically, amino acid positions in a substrate peptide are defined relative to the location of the scissile bond (i.e. the position at which a protease cleaves):

NH₂ -...... P3-P2-P1*P1'-P2'-P3'......-COOH

Illustrated using the hypothetical peptide above, the scissile bond is indicated by the asterisk (*) whilst amino acid residues are represented by the letter 'P', with the residues N-terminal to the scissile bond beginning at P1 and increasing in number when moving away from the scissile bond towards the N-terminus. Amino acid residues C-terminal to the scissile bond begin at P1' and increase in number moving towards the C-terminal residue.

Proteases can be also generally subdivided into two broad groups based on their substrate-specificity. The first group is that of the endoproteases, which are proteolytic peptidases capable of cleaving internal peptide bonds of a peptide or protein substrate and tending to act away from the N-terminus or C-terminus. Examples of endoproteases include trypsin, chymotrypsin and pepsin. In contrast, the second group of proteases is the exopeptidases which cleave peptide bonds between amino acid residues located towards the C or N-terminus of a protein or peptide substrate.

Certain enzymes of the exopeptidase group may have tripeptidyl peptidase activity. Such enzymes are therefore capable of cleaving 3 amino acid fragments (tripeptides) from the unsubstituted N-terminus of substrate peptides, oligopeptides and/or proteins. Tripeptidyl peptidases are known to cleave tripeptide sequences from the N-terminus of a substrate except peptide bonds with proline at the P1 and/or P1' position. Alternatively tripeptidyl peptidases may be proline-specific and only capable of cleaving substrates having a proline residue N-terminal to the scissile bond (i.e. in the P1 position).

Both exopeptidases and endoproteases have many applications both in the food and feed industries and in the production of hydrolysates.

A tripeptidyl aminopeptidase, a DNA construct encoding the tripeptidyl aminopeptidase, a method of producing tripeptidyl aminopeptidase and methods of reducing the tripeptidyl aminopeptidase production in cells in which tripeptidyl aminopeptidase production is undesirable are described in US5821104. Gene sequences that encode proteases obtainable from *Aspergillus niger* as well as methods and uses thereof are described in WO 02/068623.

### SUMMARY OF THE INVENTION

According to a first aspect of the invention there is provided a method for the production of a hydrolysate comprising:
(a) admixing at least one protein or a portion thereof with:
   (A) at least one endoprotease; and
   (B) at least one proline tolerant tripeptidyl peptidase having exopeptidase activity wherein said proline tolerant tripeptidyl peptidase is capable of cleaving tri-peptides from the N-terminus of peptides having:
      Proline at P1'; and
      an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and
(b) recovering the hydrolysate,
   wherein the at least one proline tolerant tripeptidyl peptidase:
   (a) comprises the amino acid sequence SEQ ID No. 3, SEQ ID No. 30 or a functional fragment thereof that retains said peptidase activity;
   (b) comprises an amino acid sequence having at least 80% identity to SEQ ID No. 3, SEQ ID No. 30 or a functional fragment thereof that retains said peptidase activity;
   (c) is encoded by a nucleotide sequence comprising the sequence SEQ ID No. 57;
   (d) is encoded by a nucleotide sequence comprising at least 80% sequence identity to SEQ ID No. 57;
   (e) is encoded by a nucleotide sequence which hybridises to SEQ ID No. 57 under high stringency conditions;
   (f) is encoded by a nucleotide sequence which differs from SEQ ID No. 57 due to degeneracy of the genetic code; or
   (g) is encoded by a nucleotide sequence comprising SEQ ID No. 57 or a nucleotide sequence having at least 90% identity thereto or a sequence which hybridises to SEQ ID No. 57 under high stringency conditions.

In a second aspect there is provided the use of at least one endoprotease and at least one proline tolerant tripeptidyl peptidase or fermentate comprising a proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
in the manufacture of a hydrolysate for reducing the immunogenicity in a subject predisposed to having an immune reaction to an untreated hydrolysate or for reducing bitterness of the hydrolysate, wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

In a third aspect there is provided a hydrolysate comprising at least one endoprotease and a proline tolerant tripeptidyl peptidase predominantly having exopeptidase activity wherein said proline tolerant tripeptidyl peptidase is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
wherein the at least one proline tolerant tripeptidyl peptidase:
(a) comprises the amino acid sequenceSEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

According to a fourth aspect there is provided a composition comprising at least one proline tolerant tripeptidyl peptidase predominantly having exopeptidase wherein said proline tolerant tripeptidyl peptidase is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
and one or more ingredients selected from the group consisting of: polyols, such as glycerol and/or sorbitol; sugars, such as glucose, fructose, sucrose, maltose, lactose and trehalose; salts, such as NaCl, KCI, CaCl₂, Na₂SO₄ or other food grade salts; a preservative, e.g. sodium benzoate and/or potassium sorbate; or combinations thereof,
wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

In a fifth aspect there is provided a method for producing a feedstuff or foodstuff comprising contacting a feed component or food component with a hydrolysate or a composition of the invention.

In a sixth aspect there is provided a food additive composition or feed additive composition comprising at least one endoprotease and at least one proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
or a hydrolysate of the invention;
optionally further comprising one or more ingredients selected from the group consisting of: polyols, such as glycerol and/or sorbitol; sugars, such as glucose, fructose, sucrose, maltose, lactose and trehalose; salts, such as NaCl, KCI, CaCl₂, Na₂SO₄ or other food grade salts; a preservative, e.g. sodium benzoate and/or potassium sorbate; or combinations thereof,
wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

In a seventh aspect there is provided a food additive or feed additive composition comprising a hydrolysate according to the invention.

In an eighth aspect there is provided a foodstuff or feedstuff comprising: at least one endoprotease and at least one proline tolerant tripeptidyl peptidase wherein said proloine tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; wherein the at least one proline tolerant tripeptidyl peptidase:
comprises an amino acid sequence selected from SEQ ID No. 3, SEQ ID No. 30 or a functional fragment thereof that retains said peptidase ativity or an amino acid sequence having at least 80% identity thereto that retains said peptidase activity; or a hydrolysate of the invention and optionally at least one food or feed ingredient.

In a ninth aspect there is provided a kit comprising at least one proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
at least one endoprotease; and instructions for co-administering same, wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

In a tenth aspect the invention provides a nonfood product comprising the hydrolysate of the invention, wherein the nonfood product is a cosmetic, a lotion, or a cleanser for use on human skin.

In any of the embodiments described herein, hydrolysates produced by the protein hydrolysis processes described herein can be removed or permeated by ultrafiltration or any suitable membrane separation known in the art.

### BRIEF DESCRIPTION OF THE DRAWINGS

Embodiments of the invention will now be described, by way of example only, with reference to accompanying drawings, in which:
**Figure 1** shows a plasmid map of the expression vector pTTT-pyrG-TRI083.
**Figure 2** shows a pH profile for a proline tolerant tripeptidyl peptidase.
**Figure 3** shows a graph displaying the activity of a proline tolerant tripeptidyl peptidase at various temperatures.
**Figure 4** shows enzyme activity when Alphalase® AFP (herein referred to as AFP) (an acid protease) is used in combination with a proline tolerant tripeptidyl peptidase at different dosages of each enzyme.
**Figure 5** shows the ability of a proline tolerant tripeptidyl peptidase to cleave the substrate AAPPA over time.
**Figure 6** shows the production of the cleavage product AAP from an AAPPA substrate over time.
**Figure 7** shows a plasmid map of the expression vector pTTT-pyrG13-TRI071. The endogenous signal sequence was replaced by the secretion signal sequence from the *Trichoderma reesei* acidic fungal protease (AFP) and an intron from a *Trichoderma reesei* glycoamylase gene (TrGA1) (see lower portion of Figure 7).
**Figure 8** shows dose response of Alphalase® AFP on protein hydrolysis of corn soy feed. Dashed line represents the control where only pepsin and pancreatin were used.
**Figure 9** shows the dependence of DH on enzyme composition in the feed sample.
**Figure 10** shows the effect of feed treatment by Alphalase® AFP and proline tolerant tripeptidyl peptidase at different conditions. Solid bars represent the treatment at 40°C for 100 min. Empty bars represent the treatment at 40°C for 200 min.
**Figure 11** shows the effect of commerical proteases compared to a proline tolerant tolerant tripeptidyl peptidase on ileal N digestibility %.
**Figure 12** shows the effect of commerical proteases compared to a proline tolerant tripeptidyl peptidase on ileal digestiblity of energy (MJ/kg).
**Figure 13** shows the increase of serine equivalents of a 15% (w/w) WPI hydrolysis employing an endopeptidase and different endopeptidase/exopeptidases combinations (Endopeptidase (Endo): Alphalase® FP2, PepN: 197 nkat_{H-Ala-*p*NA}/mL in stock solution; a proline tolerant tripeptidyl peptidase (TRI083) 4,375 nkat_{H-Ala-Ala-Ala-*p*NA}/mL).
**Figure 14** shows the increase of serine equivalents of a 15 % (w/w) WPI hydrolysis employing an endopeptidase and tripeptidyl peptidase (Endopeptidase (Endo): Alphalase® FP2, a proline tolerant tripeptidylpeptidase (TRI071=3PP_Fo) 4,375 nkat_{H-Ala-Ala-Ala-*p*NA}/mL).
**Figure 15** shows Michaelis-Menten plots of *T. reesei* proline tolerant tripeptidyl peptidase analysed using H-Ala-Ala-Ala-pNA analysed with and without pre-hydrolysed H-Ala-Ala-Ala-OH. The pre-hydrolysed inhibitor added contains Ala-Ala-Ala, Ala-Ala and Ala. The concentration is given as the concentration of H-Ala-Ala-Ala-OH before hydrolysis.
**Figure 16** shows the digestion of a fragment of β-lactoglobulin (peptide IIAEK) by a proline tolerant tripeptidyl peptidase measured with liquid chromatography-mass spectrometry (LC-MS).
**Figure 17** shows the digestion of a fragment of β-lactoglobulin (peptide ALPMHIR) by a proline tolerant tripeptidyl peptidase measured with LC-MS.
**Figure 18** shows the digestion of a fragment of β-lactoglobulin (peptide WENGECAQK) by a proline tolerant tripeptidyl peptidase measured with LC-MS.
**Figure 19** shows the digestion of a fragment of β-lactoglobulin (peptide VYVEELKPTPEGDLEILLQK) by a proline tolerant tripeptidyl peptidase measured with LC-MS.
**Figure 20** shows the further digestion of a proline tolearnt tripeptidyl petpidase digestion product EELKPTPEGDLEILLQK of beta-lactoglobulin by a proline tolerant tripeptidyl peptidase measured with LC-MS.
**Figure 21** shows the digestion of a fragment of β-lactoglobulin (peptide VAGTWYSLAMAASDISLLDAQSAPLR) by a proline tolerant tripeptidyl peptidase measured with LC-MS.
**Figure 22** shows the digestion of a tryptic fragment of β-casein (GPFPIIV) by a proline tolerant tripeptidyl peptidase measured with LC-MS.
**Figure 23** shows the digestion of a tryptic fragment of β-casein (HKEMPFPK) by a proline tolerant tripeptidyl peptidase measured with LC-MS.
**Figure 24** shows the digestion of a bradykinin peptide by a proline tolerant tripeptidyl peptidase.
**Figure 25** shows alignments between a number of proline tolerant tripeptidyl peptidase amino acid sequences. The xEANLD, y'Tzx'G and QNFSV motifs are shown (boxed).

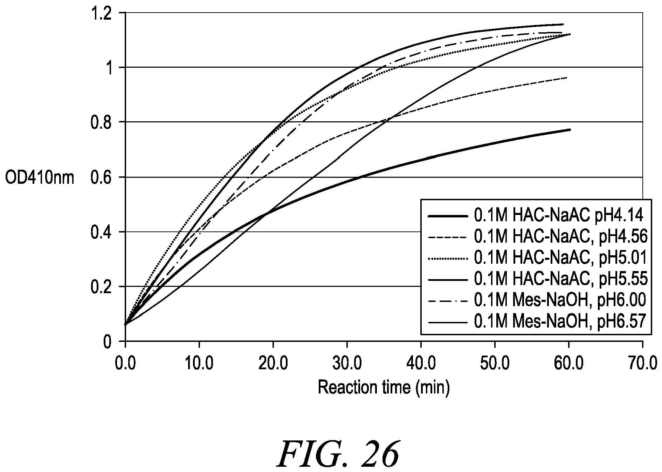

**Figure 26** shows the effect of pH on TRI045 (a tripeptidyl peptidase having pre-pro sequence SEQ ID No. 98 and mature protein SEQ ID No. 99) activity using AAF-pNA as substrate (values are the average of one test with 0.8 µl TRI045 (n=2).

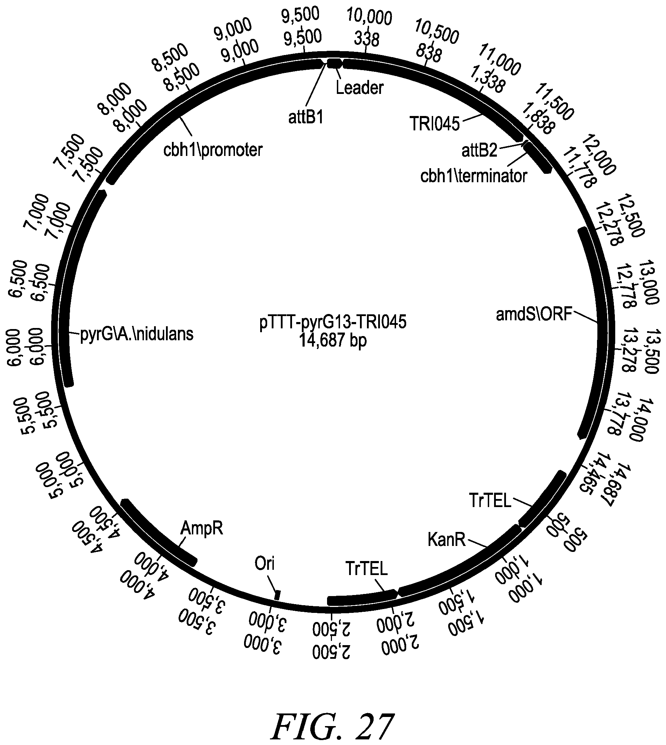

**Figure 27** shows a plasmid map of the expression vector pTTT-pyrG13-TRI045.

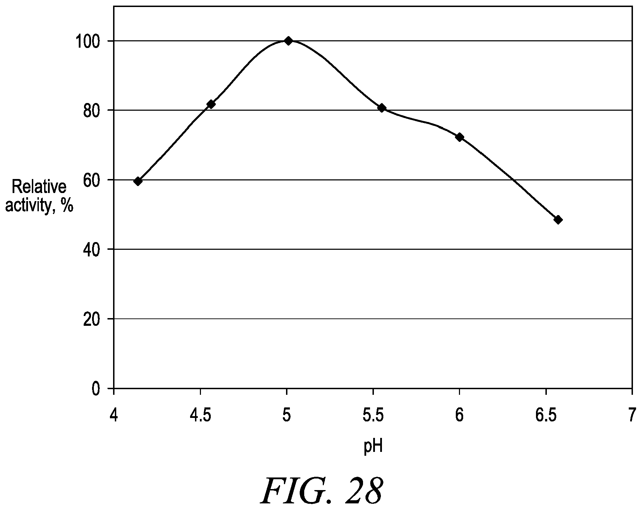

**Figure 28** shows a pH profile for the tripeptidyl peptidase TRI045.

### DETAILED DESCRIPTION

A seminal finding of the present invention is that a tripeptidyl peptidase can have exopeptidase activity on a substrate having proline at P1 and/or P1' as well as any other amino acid at P1 and/or P1'. This is highly surprising as tripeptidyl peptidases that have been documented in the art typically are inhibited when proline is at P1 or are active when proline is at P1 but inactive when an amino acid other than proline is present at position P1 in the substrate, this is sometimes referred to herein as a proline-specific tripeptidyl peptidase. The inventors have shown for the first time that a proline tolerant tripeptidyl peptidase according to the present invention is highly advantageous for use in the preparation of hydrolysates and confers advantages to subjects fed the hydrolysate or a food and/or feed comprising the hydrolysate and/or a food or feed additive composition comprising the proline tolerant tripeptidyl peptidase (optionally in combination with at least one endoprotease). Alternatively or additionally, the hydrolysate produced using an endoprotease and tripeptidyl peptidase may also have reduced bitterness when compared to an untreated hydrolysate. Advantageously, a proline tolerant tripeptidyl peptidase taught for use in the present invention is capable of acting on a wide range of peptide and/or protein substrates and due to having such a broad substrate-specificity is not readily inhibited from cleaving substrates enriched in certain amino acids (e.g. proline and/or lysine and/or arginine and/or glycine). The use of such a proline tolerant tripeptidyl peptidase therefore may efficiently and/or rapidly breakdown protein substrates (e.g. present in a substrate for preparation of a hydrolysate).

Based on these findings, described herein is a method for the production of a hydrolysate comprising:
(a) admixing at least one protein or a portion thereof with:
   (A) at least one endoprotease; and
   (B)
      (a') at least one proline tolerant tripeptidyl peptidase or fermentate comprising a proline tolerant tripeptidyl peptidase predominantly having exopeptidase activity wherein said proline tolerant tripeptidyl peptidase is capable of cleaving tri-peptides from the N-terminus of peptides having:
         Proline at P1; and
         an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
      (b') at least one proline tolerant tripeptidyl peptidase having exopeptidase activity wherein said proline tolerant tripeptidyl peptidase is capable of cleaving tri-peptides from the N-terminus of peptides having:
         Proline at P1'; and
         an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and
(b) recovering the hydrolysate.

Suitably the method may comprise a further step of admixing the hydrolysate recovered in step (b) with at least one food or feed ingredient.

Suitably step (A) and step (B) of the method for production of a hydrolysate may be carried out simultaneously (e.g. at the same time).

Suitably, the protein or portion thereof may be admixed with the endoprotease before adding the proline tolerant tripeptidyl peptidase. Suitably the protein or portion thereof may be admixed with the endoprotease before adding the proline tolerant tripeptidyl peptidase and one or more further protease(s) as detailed herein.

Described herein is a method for the production of a hydrolysate comprising:
(a) admixing at least one protein or a portion thereof selected from the group consisting of: a plant protein, a milk-based protein, or an egg protein with:
   (i) at least one endopeptidase;
   (ii) at least one exo-tripeptidyl peptidase of the S53 family; and
   (iii) one or more aminopeptidase, and
(b) recovering the hydrolysate.

Suitably, (i) (ii) and/or (iii) may be admixed simultaneously with the protein or portion thereof. Alternatively, the order of admixing in step (a) may be carried out in a specific order namely (i) may be carried out before (ii) and/or (iii).

Described herein is the use of (i) at least one endopeptidase; (ii) at least one exo-tripeptidyl peptidase of the S53 family; and (iii) one or more aminopeptidase, in the manufacture of a hydrolysate from at least one protein or a portion thereof selected from the group consisting of: a plant protein, a milk based protein, or an egg protein, for reducing the immunogenicity in a subject predisposed to having an immune reaction to an untreated hydrolysate or for reducing bitterness of the hydrolysate.

In any of the embodiments described herein ultrafiltration or membrane separation can be used to improve the protein hydrolysis processes described herein. This can increase the production of short peptides with highly reduced allergenicity.

In one embodiment the at least one protein for use in the methods and/or uses of the present invention may be selected from the group consisting of: a plant protein, preferably wherein the protein is one or more of a gliadin, an immunogenic fragment of a gliadin, a grain protein, gluten, a soy protein.

In another embodiment the at least one protein for use in the methods and/or uses of the present invention may be selected from the group consisting of: a milk-based protein, preferably wherein the protein is one or more of a casein, e.g. beta-casein; a lactoglobulin, e.g. beta-lactoglobulin; or a whey protein.

Suitably the at least one protein for use in the methods and/or uses of the present invention may be an egg protein.

The term "exo-tripeptidyl peptidase of the S53 family" as used herein refers to a protease predominantly having exopeptidase activity as well as the ability to cleave tripeptides from the N-terminus of a protein and/or peptide substrate. The S53 family peptidases broadly encompass a class of serine proteases. Although the S53 family includes both endoproteases and exopeptidases it is intended herein that this definition refers only to those tripeptidyl peptidases predominantly having exopeptidase activity.

An "exo-tripeptidyl peptidase of the S53 family" has an activity of at least about 50nkat per mg of protein in the "Exopeptidase Broad-Specificity Assay" (EBSA) taught herein. Suitably an "exo-tripeptidyl peptidase of the S53 family" in accordance with the present invention has an activity of between about 50-2000 nkat per mg of protein in the EBSA activity assay taught herein.

The combination of an endoprotease, an exo-tripeptidyl peptidase of the S53 family and an aminopeptidase may be referred to herein as a "triple enzyme composition". The term "triple enzyme composition" refers to a combination of at least: an endoprotease, an exo-tripeptidyl peptidase of the S53 family and an aminopeptidase. In other words the term "triple enzyme composition" may also include one or more further enzyme activities and/or further constituents.

The exo-tripeptidyl peptidase of the S53 family is a proline tolerant tripeptidyl peptidase.

The term "aminopeptidase" as used herein refers to an exopeptidase which is able to cleave single amino acids from the N-terminus of a protein and/or peptide substrate.

The aminopeptidase may be obtainable (e.g. obtained) from *Lactobacillus.*

Suitably the aminopeptidase may be obtainable from *Lactobacillus helveticus.* More suitably the aminopeptidase may be obtainable from *Lactobacillus helveticus* ATCC 12046.

The aminopeptidase for use in combination with an endoprotease and exo-tripeptidyl peptidase in accordance with the present invention may be an aminopeptidase N (e.g. PepN) (EC 3.4.11.2).

In one embodiment the aminopeptidase used in combination with an endoprotease and exo-tripeptidyl peptidase of the S53 family may comprise the sequence shown as:

The term "admixing" as used herein refers to the mixing of one or more ingredients and/or enzymes where the one or more ingredients or enzymes are added in any order and in any combination. Suitably, admixing may relate to mixing one or more ingredients and/or enzymes simultaneously or sequentially.

In one embodiment the one or more ingredients and/or enzymes may be mixed sequentially. Preferably, the one or more ingredients and/or enzymes may be mixed simultaneously.

The term "recovering a hydrolysate" as used herein refers to the isolation of a hydrolysate. In some embodiments this may involve separating the hydrolysed matter from unhydrolysed protein and/or peptide substrates. In other embodiments it may additionally or alternatively involve separating the hydrolysed matter away from a proline tolerant tripeptidyl peptidase and/or endoprotease or triple enzyme composition of the invention used for preparing said hydrolysate. In one embodiment the hydrolysate may comprise hydrolysed matter with a purity of at least 80%, more suitably at least 90%, even more suitably at least 95%. Preferably the hydrolysate may comprise hydrolysed matter with a purity of at least about 99%.

In one embodiment a proline tolerant tripeptidyl peptidase or triple enzyme composition for use in the methods and/or uses of the present invention may be incubated with a substrate (e.g. a protein and/or peptide substrate) at a temperature of at least about 40ºC. In other words the method of the present invention may be carried out at a temperature of at least about 40ºC.

Suitably the proline tolerant tripeptidyl peptidase or triple enzyme composition may be incubated with a substrate at a temperature of at least about 45ºC, suitably at least about 50°C.

Preferably the proline tolerant tripeptidyl peptidase or triple enzyme composition may be incubated with a substrate at a temperature of at least about 55ºC.

In another embodiment the proline tolerant tripeptidyl peptidase or triple enzyme composition for use in the methods and/or uses of the present invention may be incubated with a substrate (e.g. a protein and/or peptide substrate) at a temperature of between about 40ºC to about 70 ºC. In other words the method of the present invention may be carried out at a temperature of between about 40ºC to about 70ºC.

Suitably the proline tolerant tripeptidyl peptidase or triple enzyme composition may be incubated with a substrate at a temperature of between about 40ºC to about 60ºC; even more suitably at a temperature of between about 45ºC to about 65ºC.

Preferably the proline tolerant tripeptidyl peptidase or triple enzyme composition may be incubated with a substrate at a temperature of between about 50ºC to about 60ºC.

The proline tolerant tripeptidyl peptidase for use in the methods and/or uses or comprised in any of the products of the present invention is capable of cleaving tripeptides from the N-terminus of peptides having proline at P1'; and an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

Additionally, the proline tolerant tripeptidyl peptidase for use in the methods and/or uses or comprised in any of the products of the present invention may be capable of cleaving tri-peptides from the N-terminus of peptides having proline at P1; and an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1.

The term "proline tolerant tripeptidyl peptidase" as used herein relates to an exopeptidase which can cleave tripeptides from the N-terminus of a peptide, oligopeptide and/or protein substrate. A "proline tolerant tripeptidyl peptidase", also referred to herein as 3PP, is capable of cleaving peptide bonds where proline is at position P1' as well as cleaving peptide bonds where an amino acid other than proline is at P1' and may additionally be capable of cleaving peptide bonds where proline is at position P1 as well as cleaving peptide bonds where an amino acid other than proline is at P1.

In one embodiment the proline tolerant tripeptidyl peptidase and/or exo-tripeptidyl peptidase of the S53 family is not an endoprotease.

In another embodiment the proline tolerant tripeptidyl peptidase and/or exo-tripeptidyl peptidase of the S53 family is not an enzyme which cleaves tetrapeptides from the N-terminus of a substrate.

In a further embodiment the proline tolerant tripeptidyl peptidase and/or exo-tripeptidyl peptidase of the S53 family is not an enzyme which cleaves dipeptides from the N-terminus of a substrate.

In a yet further embodiment the proline tolerant tripeptidyl peptidase and/or exo-tripeptidyl peptidase of the S53 family is not an enzyme which cleaves single amino acids from the N-terminus of a substrate.

In one embodiment the proline tolerant tripeptidyl peptidase may be capable of cleaving peptide bonds where proline is at position P1 as well as cleaving peptide bonds where an amino acid other than proline is at P1.

The proline tolerant tripeptidyl peptidase is capable of cleaving peptide bonds where proline is at position P1' as well as cleaving peptide bonds where an amino acid other than proline is at P1'.

Suitably, the proline tolerant tripeptidyl peptidase may also be able to cleave peptide bonds where the proline present at position P1 and/or P1' is present in its *cis* or *trans* configuration. Suitably an "amino acid other than proline" may be an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids.

In another embodiment the "amino acid other than proline" may be an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine or valine.

Suitably, in such an embodiment synthetic amino acids may be excluded.

Preferably, the proline tolerant tripeptidyl peptidase may be able to cleave peptide bonds where proline is present at position P1 and P1'.

It is surprising that a tripeptidyl peptidase can act on a substrate having proline at position P1 and/or P1'. It is even more surprising that in addition to this activity a tripeptidyl peptidase may also have activity when an amino acid other than proline is present at position P1 and/or P1'.

In addition to having activity on any of the various substrates as described above the proline tolerant tripeptidyl peptidase for use in the present invention may additionally be tolerant of proline at one or more positions selected from the group consisting of: P2, P2', P3 and P3'. Suitably the proline tolerant tripeptidyl peptidase in addition to having the activities described above may be tolerant of proline at position P2, P2', P3 and P3'.

This is advantageous as it allows the efficient cleavage of peptide and/or protein substrates having stretches of proline and allows cleavage of a wide range of peptide and/or protein substrates.

Preferably the proline tolerant tripeptidyl peptidase may have a high activity on peptides and/or proteins having one or more of lysine, arginine or glycine in the P1 position. Without wishing to be bound by theory peptide and/or protein substrates comprising these amino acids at the P1 position may be difficult to digest for many tripeptidyl peptidases and/or proteases in general and upon encountering such residues cleavage of the peptide and/or protein substrate by a tripeptidyl peptidase and/or protease may halt or slow. Advantageously, by using a proline tolerant tripeptidyl peptidase of the invention it is possible to digest protein and/or peptide substrates comprising lysine, arginine and/or glycine at P1 efficiently.

Suitably the proline tolerant tripeptidyl peptidase may have a preferential activity on peptides and/or proteins having lysine at the P1 position. Advantageously this allows the efficient cleavage of substrates having high lysine content, such as whey protein.

In one embodiment the proline tolerant tripeptidyl peptidase or exo-tripeptidyl peptidase of the S53 family may comprise a catalytic triad of the amino acids serine, aspartate and histidine.

The proline tolerant tripeptidyl peptidase or exo-tripeptidyl peptidase of the S53 family for use in the present invention may be a thermostable proline tolerant tripeptidyl peptidase or exo-tripeptidyl peptidase of the S53 family.

The term "thermostable" means that an enzyme retains its activity when heated to temperatures of up to about 60°C. Suitably "thermostable" may mean that an enzyme retains its activity when heated to about 65°C, more suitably about 70°C.

Advantageously, a thermostable proline tolerant tripeptidyl peptidase or exo-tripeptidyl peptidase of the S53 family is less prone to being denatured and/or will retain its activity for a longer period of time in e.g. an animal when compared to a non-thermostable variant.

The proline tolerant tripeptidyl peptidase or exo-tripeptidyl peptidase of the S53 family may have activity in a range of about pH 2 to about pH 7. Suitably, the proline tolerant tripeptidyl peptidase or exo-tripeptidyl peptidase of the S53 family may have activity in a range of about pH 4 to about pH 7, more suitably in a range of about pH 4.5 to about pH 6.5.

Suitably the method of the present invention, in particular the hydrolysis step, may be carried out at a pH of between 2 to about 7.

In one embodiment the method of the present invention, in particular the hydrolysis step may be carried out at a pH of between about 4 to about 7, e.g. 4.5 to 6.5.

Using a proline tolerant tripeptidyl peptidase or exo-tripeptidyl peptidase of the S53 family having activity in a pH range between about pH 4 to about pH 7 is advantageous as it allows the proline tolerant tripeptidyl peptidase or exo-tripeptidyl peptidase of the S53 family to be used with one more endoproteases having activity in this pH range.

When a proline tolerant tripeptidyl peptidase or exo-tripeptidyl peptidase of the S53 family having activity in a pH range between about pH 4 to about pH 7 is used, suitably it may be used in combination with a neutral or an alkaline endoprotease.

Advantageously this means that changing the pH of the reaction medium comprising the protein and/or peptide substrate for hydrolysate production is not necessary between enzyme treatments. In other words it allows the proline tolerant tripeptidyl peptidase and the endoprotease or the constituents of the triple enzyme composition to be added to a reaction simultaneously, which may make the process for producing the hydrolysate quicker and/or more efficient and/or more cost-effective. Moreover, this allows for a more efficient reaction as at lower pH values the substrate may precipitate out of solution and therefore not be cleaved.

Any suitable alkaline endoprotease may be used in the present invention. Suitably, the alkaline endoprotease may be one or more selected from the group consisting of: a trypsin, a chymotrypsin, or a subtilisin.

In another embodiment the proline tolerant tripeptidyl peptidase or exo-tripeptidyl peptidase of the S53 family may have activity at an acidic pH (suitably, the proline tolerant tripeptidyl peptidase may have optimum activity at acidic pH). The proline tolerant tripeptidyl peptidase or exo-tripeptidyl peptidase of the S53 family may have activity at a pH of less than about pH 6, more suitably less than about pH 5. Preferably, the proline tolerant tripeptidyl peptidase may have activity at a pH of between about 2.5 to about pH 4.0, more suitably at between about 3.0 to about 3.3.

Suitably the method of the present invention, in particular the hydrolysis step, may be carried out at a pH of between 2 to about 4, e.g. 3 to 3.3. In one embodiment the proline tolerant tripeptidyl peptidase or exo-tripeptidyl peptidase of the S53 family may have activity at a pH around 2.5.

A proline tolerant tripeptidyl peptidase or exo-tripeptidyl peptidase of the S53 family having activity at an acidic pH can be used in combination with an acid endoprotease and advantageously does not require the pH of the reaction medium comprising the protein and/or peptide substrate for hydrolysate production to be changed between enzyme treatments. In other words it allows the proline tolerant tripeptidyl peptidase and the endoprotease or the constituents of the triple enzyme composition to be added to a reaction simultaneously, which may make the process for producing the hydrolysate quicker and/or more efficient and/or more cost-effective.

At least one endoprotease is used in combination with the proline tolerant tripeptidyl peptidase or exo-tripeptidyl peptidase of the S53 family for any of the applications herein. For example at least one endoprotease is comprised in the composition and/or food additive composition and/or nonfood product of the invention.

The term "endoprotease" as used herein is synonymous with the term "endopeptidase" and refers to an enzyme which is a proteolytic peptidase capable of cleaving internal peptide bonds of a peptide or protein substrate (e.g. not located towards the C or N-terminus of the peptide or protein substrate). Such endoproteases may be defined as ones that tend to act away from the N-terminus or C-terminus.

In one embodiment the endoprotease may be one or more selected from the group consisting of: a serine protease, an aspartic acid protease, a cysteine protease, a metalloprotease, a threonine protease, a glutamic acid protease and a protease selected from the family of ungrouped proteases.

In one embodiment the endoprotease may be one or more selected from the group consisting of: an acid fungal protease, a subtilisin, a chymotrypsin, a trypsin and a pepsin or from the group of commercial protease products Alphalase® AFP, Alphalase® FP2, Alphalase® NP, FoodPro® Alkaline Protease, FoodPro® PXT, FoodPro® PBR , FoodPro® 30L, FoodPro® PHT or FoodPro® 51FP.

In one embodiment, the endoprotease may be an acid endoprotease. Suitably, the endoprotease may be an acid fungal protease.

Advantageously, the use of an endoprotease in combination with a proline tolerant tripeptidyl peptidase or exo-tripeptidyl peptidase of the S53 family can increase the efficiency of substrate cleavage. Without wishing to be bound by theory, it is believed that an endoprotease is able to cleave a peptide and/or protein substrate at multiple regions away from the C or N-terminus, thereby producing more N-terminal ends for the proline tolerant tripeptidyl peptidase to use as a substrate, thereby advantageously increasing reaction efficiency and/or reducing reaction times and/or more effectively removing antigens (and thereby reducing immunogenicity).

The proline tolerant tripeptidyl peptidase or exo-tripeptidyl peptidase of the S53 family for use in accordance with the present invention may be an "in-food" proline tolerant tripeptidyl peptidase.

The term "in-food" or "in-feed" as used herein means that the enzyme (e.g. the proline tolerant tripeptidyl peptidase and/or endoprotease and/or exo-tripeptidyl peptidase of the S53 family and/or triple enzyme composition) is functional, preferably primarily functional, more preferably solely functional, in the gastrointestinal tract (GIT) of a subject. In other words, the term "in-food" as used herein means that the enzyme is substantially inactive (or is inactive) in the composition and/or food additive composition and/or feed additive composition and/or foodstuff and/or feedstuff prior to consumption of the composition and/or food additive composition and/or feed additive composition and/or foodstuff and/or feedstuff by a subject. There term "primarily functional" means that the enzyme mainly functions on its substrate once it enters the GIT. In other words, prior to entering the GIT the level of enzyme activity defined as the amount of cleavage of peptide and/or protein substrates to tripeptides is less than about 20%, suitably less than about 10%, preferably less than about 5%, of the level of enzyme activity after it enters the GIT.

The term "solely functional" as used herein means that the enzyme is inactive before entering the GIT and is activated upon entering the GIT.

The term "inactive" as used herein means that the enzyme is not active. This may mean that the enzyme's activity is somehow inhibited or that the enzyme is in an environment in which it is inactive or that the enzyme is presented to its substrate immediately prior to feeding to the animal such that there is not enough time to be active. The "inactivity" of the enzyme may be in any event reversible once it enters the GIT of a subject.

Therefore, suitably the proline tolerant tripeptidyl peptidase (optionally in combination with an endoprotease) and/or exo-tripeptidyl peptidase of the S53 family and/or triple enzyme composition may be admixed with the at least one protein or portion thereof immediately prior to consumption of same by the subject.

The term "substantially inactive" as used herein means that the enzyme has low activity compared with its activity once it has entered the GIT. For instance, substantially inactive may mean that the enzyme in the feed additive composition and/or food additive composition and/or feed additive composition and/or foodstuff and/or feedstuff has less than 10% of its activity when compared with its activity in the GIT.

Maintaining the "in-food" enzyme in an inactive or substantially inactive state in the composition and/or food additive composition and/or feed additive composition and/or foodstuff and/or feedstuff can be achieved in a number of ways known to one skilled in the art.

By way of example only maintaining the water content (wt %) of the composition and/or food additive composition and/or feed additive composition and/or foodstuff and/or feedstuff at less than 15%, preferably less than 10%, is sufficient to ensure that the in-food enzyme is inactive or substantially inactive in the composition and/or food additive composition and/or feed additive composition and/or foodstuff and/or feedstuff.

In one embodiment the composition and/or food additive composition and/or feed additive composition and/or foodstuff and/or feedstuff, post-admixing the in-food enzyme, are (maintained and/or stored) in a dry state or substantially dry state.

The term "dry state" as used herein means that the proline tolerant tripeptidyl peptidase (optionally in combination with an endoprotease) and/or the composition and/or food additive composition and/or feed additive composition and/or foodstuff and/or feedstuff contains no or only a very low amount of water. In other words the term "dry state" as used herein may mean that the proline tolerant tripeptidyl peptidase (optionally in combination with an endoprotease) and/or exo-tripeptidyl peptidase of the S53 family and/or triple enzyme composition and/or the composition and/or food additive composition and/or feed additive composition and/or foodstuff and/or feedstuff comprises less than 5%, preferably less than 1%, water content (wt %).

In one embodiment the proline tolerant tripeptidyl peptidase and/or endoprotease and/or exo-tripeptidyl peptidase of the S53 family and/or triple enzyme composition for use in the methods and/or uses and/or products of the present invention may in a dry or substantially dry state.

In another embodiment a proline tolerant tripeptidyl peptidase for use in any of the methods and/or uses and/or products of the invention may be admixed with a composition comprising at least one protein or at least a portion of a protein, wherein the composition, the proline tolerant tripeptidyl peptidase or combinations thereof and/or exo-tripeptidyl peptidase of the S53 family and/or triple enzyme composition are in a dry or substantially dry state when admixed. Suitably an endoprotease may be further admixed.

The term "dry or substantially dry state" when used herein means that the composition, proline tolerant tripeptidyl peptidase, endoprotease or combinations thereof contains only a very low amount of water. In other words the term "substantially dry state" as used herein may mean that the composition, proline tolerant tripeptidyl peptidase, endoprotease or combinations thereof and/or exo-tripeptidyl peptidase of the S53 family and/or triple enzyme composition comprises less than 15%, preferably less than 10%, water content (wt %).

In one embodiment, the composition, proline tolerant tripeptidyl peptidase, endoprotease or combinations thereof and/or exo-tripeptidyl peptidase of the S53 family and/or triple enzyme composition may be dried prior to, during or after (preferably prior to) use in the methods and/or uses of the invention.

In another embodiment the composition, proline tolerant tripeptidyl peptidase, endoprotease or combinations thereof and/or exo-tripeptidyl peptidase of the S53 family and/or triple enzyme composition either before or after use in the methods and/or uses of the invention comprises less than 15 wt % moisture content.

In another embodiment the composition, proline tolerant tripeptidyl peptidase, endoprotease or combinations thereof and/or exo-tripeptidyl peptidase of the S53 family and/or triple enzyme composition either before or after use in the methods and/or uses of the invention comprises less than 10 wt % moisture content; moresuitably less than 5 wt % moisture content, and even more suitably less than 1 wt % moisture content.

The proline tolerant tripeptidyl peptidase (e.g. "in-food" proline tolerant tripeptidyl peptidase), endoprotease or combinations thereof may be maintained in an inactive or substantially inactive state in the composition and/or food additive composition and/or feed additive composition and/or foodstuff and/or feedstuff by physically preventing the enzyme from interacting with its substrate. For example the proline tolerant tripeptidyl peptidase (e.g. "in-food" proline tolerant tripeptidyl peptidase), endoprotease or combinations thereof and/or exo-tripeptidyl peptidase of the S53 family and/or triple enzyme composition may be encapsulated prior to its use in the methods and/or uses composition and/or food additive composition and/or feed additive composition and/or foodstuff and/or feedstuff of the invention.

When the proline tolerant tripeptidyl peptidase (e.g. "in-food" proline tolerant tripeptidyl peptidase), endoprotease or combinations thereof and/or exo-tripeptidyl peptidase of the S53 family and/or triple enzyme composition is physically prevented from interacting with its substrate in the composition and/or food additive composition and/or feed additive composition and/or foodstuff and/or feedstuff, then once in the GIT the physical barrier is removed thus allowing the interaction of the proline tolerant tripeptidyl peptidase (e.g. "in-food" proline tolerant tripeptidyl peptidase), endoprotease or combinations thereof and/or exo-tripeptidyl peptidase of the S53 family and/or triple enzyme composition with its substrate.

By way of example only, the encapsulation may be removed by passage of the encapsulated proline tolerant tripeptidyl peptidase (e.g. "in-food" proline tolerant tripeptidyl peptidase), endoprotease or combinations thereof and/or exo-tripeptidyl peptidase of the S53 family and/or triple enzyme composition through the gizzard, proventriculus or stomach of a subject. The gizzard, proventriculus or stomach of a subject is at very low (acidic) pH (e.g. pH 2-4). This acidity can be used to activate encapsulated enzymes.

In one embodiment the enzyme may be encapsulated by a polymer, such as chitin or chitosans, gelatin, gum arabic or wax for example. By way of example only the polymer may be a gelatin or gum arabic as taught in Xue et al Food Funct. 2013, Apr 25; 6 Feb (epub); 4 (4) 610-7. Alternatively, the polymer may a chitosan-based hydrogel as taught in Zhang et al Biomacromolecules 2011,12,2894-2901.

In one embodiment the proline tolerant tripeptidyl peptidase (e.g., "in-food" proline tolerant tripeptidyl peptidase), endoprotease or combinations thereof and/or exo-tripeptidyl peptidase of the S53 family and/or triple enzyme composition may be activated by consumption of the enzyme by a subject.

The term "inactive" as used herein may mean that the enzyme is presented to its substrate immediately prior to consumption of the enzyme by a subject such that there is not enough time to be active before it enters the GIT of the subject.

In some embodiment inactivation of the enzyme is a permanent deactivation, e.g. by heat, chemical or pH denaturing. Thus in such embodiments the "inactive" may be permanently inactive, e.g. including in the GIT of a subject.

The present enzymes, including combinations of 3PP and an endoprotease, are also useful in a starch conversion process, particularly in a saccharification and fermentation process of gelatinized, raw, and or granular starch that has undergone liquefaction. The desired end-product (often referred to as an "end-of-fermentation" or "EOF" product) may be any product that may be produced by the enzymatic conversion of the starch substrate. For example, the desired product may be a syrup rich in glucose and maltose, which can be used in other processes, such as the preparation of high-fructose corn syrup (HFCS), or which can be converted into a number of other useful products, such as an ascorbic acid intermediates (*e.g.,* gluconate; 2-keto-L-gulonic acid; 5-keto-gluconate; and 2,5-diketogluconate); 1,3-propanediol; amino acids (*e.g.,* tyrosine, serine, lysine, glutamic acid, glycine, phenylalanine and tryptophan); organic acids (*e.g.,* lactate, pyruvate, succinate, citrate, isocitrate, gluconate, itaconate, and oxaloacetate); antibiotics; antimicrobials; enzymes; vitamins; hormones; ethanol, butanol, and other alcohols; glucono delta-lactone; sodium erythorbate; omega-3 fatty acid; isoprene; and other biochemicals and biomaterials. One skilled in the art is aware of various fermentation conditions that may be used in the production of these EOF products.

Those of skill in the art are well aware of available methods that may be used to prepare starch substrates for conversion. Useful starch substrates may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. The starch substrate can be a crude starch from milled whole grain, which contains non-starch fractions, *e.g.,* germ residues and fibers. Liquefaction generally involves gelatinization of starch simultaneously with or followed by the addition of an α-amylase, although additional liquefaction-inducing enzymes optionally may be added. In some cases, liquefaction of starch is performed at or below the gelatinization temperature typically requires similar classes of enzymes with different performance criteria. The liquefied starch can be saccharified into a syrup with a lower degree of polymerization (DP) using α-amylases, optionally in the presence of other enzymes. The exact composition of the products of saccharification depends on the combination of enzymes used, as well as the type of granular starch processed.

The present enzymes may be added during liquefaction and/or saccharification as an isolated enzyme solution, dried or granular enzyme, clarified broth, ultrafiltrate concentrate, or whole cell broth, optionally as part of a blend. The present enzymes can be also added in the form of a cultured cell material produced by host cells expressing the enzymes. The present enzymes may also be secreted by a host cell into the reaction medium during the fermentation or simultaneous saccharification and fermentation (SSF) process, such that the enzyme is provided continuously into the reaction (see, below). The host cell producing and secreting the present enzymes may also express an additional enzyme, such as a glucoamylase and/or α-amylase. The host cell can be engineered to express a broad spectrum of various saccharolytic enzymes.

The soluble starch hydrolysate produced by treatment with amylase can be converted into high fructose starch-based syrup, such as high fructose corn syrup (HFCS). This conversion can be achieved using a glucose isomerase, particularly a glucose isomerase immobilized on a solid support.

Soluble starch hydrolysate, particularly a glucose rich syrup, can be fermented by contacting the starch hydrolysate with a fermenting organism. Ethanologenic microorganisms include yeast, such as *Saccharomyces cerevisiae* and bacteria, *e.g., Zymomonas mobilis,* expressing alcohol dehydrogenase and pyruvate decarboxylase. Commercial sources of yeast include ETHANOL REDO (LeSaffre); THERMOSACCÒ (Lallemand); RED STARO (Red Star); FERMIOLÒ (DSM Specialties); and SUPERSTARTÒ (Alltech). Microorganisms that produce other EOF (such as those mentioned, above) are also known in the art. As mentioned, above, the saccharification and fermentation processes may be carried out as an SSF process, wherein the fermenting organism expresses the present enzymes, optionally with one or more additional enzymes, such as a glucoamylase and/or α-amylase. Fermentation may comprise subsequent enrichment, purification, and recovery of the EOF.

### HYDROLYSATES

Disclosed herein is a hydrolysate obtainable (e.g. obtained) by a method of the invention.

Suitably such a hydrolysate may be enriched in tripeptides. Suitably, the hydrolysate may be enriched in one or more of a tripeptide having a proline at its N-terminus and/or a tripeptide having a proline at its N-terminus and C-terminus.

Preferably such a hydrolysate may be enriched in one or more tripeptides having a proline at its N-terminus and a proline at its C-terminus.

Alternatively or additionally, the hydrolysate may comprise a proline tolerant tripeptidyl peptidase (optionally in combination with an endoprotease) and/or exo-tripeptidyl peptidase of the S53 family and/or triple enzyme composition described herein, such as any one of the amino acid sequences described herein or a proline tolerant tripeptidyl peptidase obtainable (e.g. obtained) by any of the methods herein.

The term "hydrolysate" as used herein has its usual meaning in the art and refers to a product resulting from the treatment of a protein or portion thereof with a proline tolerant tripeptidyl peptidase and an endoprotease. The extent of proteolytic cleavage of the protein or portion thereof can range from minimal (e.g. cleavage of a single peptide bond on a single protein) to extensive depending on, for example, the conditions of the treatment, such as the length of the treatment, the temperature, the concentration of the protein, and the purity, concentration, and activity of the proline tolerant tripeptidyl peptidase and endoprotease.

A "hydrolysate" therefore typically comprises a mixture of short peptides obtainable by cleaving a peptide and/or protein substrate with at least one protease (suitably a proline tolerant tripeptidyl peptidase). Suitably such a hydrolysate may be substantially enriched in tripeptides, and optionally also in single amino acids and/or dipeptides.

Advantageously, enrichment in tripeptides, dipeptides and/or single amino acids may result in more efficient uptake of the hydrolysate by an animal and/or human.

Hydrolysates with a high content of tripeptides as well as dipeptides are nutritionally advantageous because the intestinal absorption of di- and tripeptides happens via the PepT1 receptors, which function independently from the uptake of free amino acids (Gilbert et al. 2008, J ANIM SCI, 86:2135-2155).

The term "substantially enriched in tripeptides" as used herein means that of the total peptide concentration measured by any method known in the art (e.g. liquid chromatography-mass spectrometry (LC-MS)) at least about 20% (suitably at least about 30%) of those peptides are tripeptides. Suitably, at least about 40% of those peptides are tripeptides, more suitably at least about 50%.

In one embodiment the term "substantially enriched in tripeptides" as used herein means that of the total peptide concentration measured by any method known in the art (e.g. liquid chromatography-mass spectrometry (LC-MS)) at least about 70% of those peptides are tripeptides.

Advantageously ultrafiltration can be used during protein hydrolysis. Enzymatic membrane reactors have at least one ultrafiltration membrane coupled to the hydrolysis reactor. Thereby ultrafiltration can be used during the hydrolysis to remove enzyme inhibiting peptides and/or to produce peptide fractions with molecular masses determined by the molecular weight cutoff of the membrane. This is useful for production of short peptides with highly reduced allergenicity. Enzyme membrane reactors can also facilitate higher conversion of the protein substrate into the desired peptides and re-use of the proteolytic enzymes applied. Examples of use of enzyme membrane reactor are given in Eisele et al. (2013), European Food Research and Technology 236, 483-490 and Cheison SC, Wang Z, Xu SY (2007) J Agric Food Chem 55, 3896-3904.

In one embodiment the hydrolysate may comprise less than about 20%, suitably less than about 10% of the full-length starting substrate (e.g. at least one protein). Suitably, the hydrolysate may comprise less than about 5%, more suitably less than about 1% of the full-length starting substrate (e.g. at least one protein).

In some embodiments the hydrolysate may comprise no, or substantially no, full-length starting substrate.

The term "substantially no" as used in this context may mean less than about 0.5%, suitably less than about 0.1% of full-length starting substrate.

Where an endoprotease, exo-tripeptidyl peptidase of the S53 family and aminopeptidase have been used in the manufacture of a hydrolysate it is believed that such a hydrolysate will be enriched in single amino acids, dipeptides and tripeptides.

In one embodiment the single amino acids, dipeptides and tripeptides present in such a hydrolysate may be quantified in terms of molarity of each stated. In one embodiment the molar ratio of single amino acids, dipeptides and tripeptides in a hydrolysate may be at least about 20% single amino acids to at least about 10% dipeptides to at least about 10% tripeptides.

In another embodiment the molar ratio of single amino acids, dipeptides and tripeptides in a hydrolysate may be at least about 10% single amino acids to at least about 20% dipeptides to at least about 20% tripeptides.

The hydrolysate obtainable according to the methods of the present invention or for use in any of the applications taught herein may have a reduced immunogenicity in a subject predisposed to having an immune response to the at least one protein or a portion thereof that formed the substrate for digestion for the production of the hydrolysate.

The hydrolysate is produced by admixing at least one protein or a portion thereof with an endoprotease and a proline tolerant tripeptidyl peptidase.

The protein or portion thereof used as the substrate in manufacture of the hydrolysate may be an animal protein or a plant protein (e.g. a vegetable protein).

Suitably the protein or portion thereof may be one or more selected from the group consisting of: a gliadin, a beta-casein, a beta-lactoglobulin or an immunogenic fragment of a gliadin, a beta-casein, a beta-lactoglobulin, glycinin, beta-conglycinin, cruciferin, napin, collagen, whey protein, fish protein, meat protein, egg protein, soy protein, a hordein and a grain protein.

In one embodiment, preferably the protein or portion thereof is a plant protein, a milk based protein, an egg protein or a combination thereof.

In one embodiment, preferably the protein or portion thereof is a plant protein, preferably wherein the protein is one or more of a gliadin, an immunogenic fragment of a gliadin, a grain protein, gluten, a soy protein.

In one embodiment, preferably the protein or portion thereof is a milk-based protein, preferably wherein the protein is one or more of a casein, e.g. beta-casein; a lactoglobulin, e.g. beta-lactoglobulin; or a whey protein.

In one embodiment, preferably the protein or portion thereof is an egg protein.

The protein or portion thereof may be comprised in corn, soybean meal, corn dried distillers grains with solubles (DDGS), wheat, wheat proteins (including gluten), wheat byproducts, wheat bran, corn by products including corn gluten meal, barley, oat, rye, triticale, full fat soy, animal by-product meals, an alcohol-soluble protein (preferably a zein (e.g. a maize zein maize) and/or a kafirin (e.g. from sorghum)), a protein from oil seeds (preferably from soybean seed proteins, sun flower seed proteins, rapeseed proteins, canola seed proteins or combinations thereof) or a combination thereof.

In one embodiment the protein or portion thereof may be a protein-meal. In one embodiment this may be a protein-meal from fish or a protein-meal from an animal (e.g. such as a non-human mammal).

In some embodiments the protein or a portion thereof for use in accordance with the present invention may be an animal by-product.

Such by-products may include tissues from animal production and processing which are not utilized in human food and are processed into an array of protein meals used in animal feeds and pet food. In one embodiment the animal protein by-products may be meat and bone meal, meat meal, blood meal, poultry by-product meal, poultry meal, feather meal, and fish meal.

In another embodiment the protein or a portion thereof for use in the present invention may be a microbial protein. Microbial proteins, such as e.g. yeast extracts, are typically made by extracting the cell contents from microbial cultures; they may be used as food additives or flavourings, or as nutrients for microbial culture media.

In yet further embodiments the protein or a portion thereof for use in the present invention may be an invertebrate protein, suitably an insect protein.

Insects/invertebrates possess enormous biodiversity and represent a large biomass (95% of the animal kingdom), and thus offer alternative protein sources.

In one embodiment the protein or portion thereof may be one or more selected from the group consisting of: a wheat protein, portions of a wheat protein, a dairy protein and portions of a dairy protein.

The wheat protein or portion thereof may be obtainable from wheat, wheat products (e.g. wheat flour), wheat byproducts and/or wheat bran. Suitably, the wheat protein may be one or more selected from the group consisting of: gliadin, portions of gliadin, gluten and portions of gluten.

The dairy protein or a portion thereof may be milk protein. Suitably the milk protein may be one or more selected from the group consisting of: a beta-casein, a beta-lactoglobulin and a whey protein.

The "portion thereof" in relation to a protein or portion thereof used for the manufacture of a hydrolysate may be an immunogenic fragment of a protein. An "immunogenic fragment" is any portion that is capable of eliciting an immune response in a sensitive individual. The "immunogenic fragment" or "portion thereof" is a region of a full-length protein comprising or consisting of at least 10 amino acids, suitably at least 20 amino acids, more suitably at least 30 amino acids.

In some embodiments the immunogenic fragment or portion thereof may be at least about 50 amino acids; more suitably at least about 100 amino acids, and even more suitably at least about 200 amino acids.

As used herein, "milk protein" encompasses any naturally-occurring protein in the normal secretion of the mammary gland of a postpartum female mammal, or products derived therefrom, such as fractions thereof, or components made therefrom or thereof. The milk can be from any mammalian species including but not limited to cow, goat, sheep, buffalo, yak, camel, llama, alpaca, and human. Milk proteins from those mammals whose milk is used commercially or widely in various cultures and countries are preferred. It is to be noted that "milk protein" as used herein encompasses both the singular and the plural of the word "protein", thus, the term "milk protein" may refer to a single protein, or any mixture of one or more proteins, except as otherwise indicated.

The term "whey protein" encompasses any protein found in any amount in "whey"; the liquid by-product of cheese making that is separated from the curd. The whey resulting from the production of many cheeses is particularly low in micellar milk proteins, such as caseins, but relatively enriched in soluble proteins such as alpha lactalbumin and beta-lactoglobulin. As with "milk protein" above, the term "whey protein" as used herein encompasses both the singular and the plural of the word "protein", thus, the term "whey protein" also may refer to a single protein, or any mixture of one or more whey proteins, except as otherwise indicated. It will be understood by the skilled artisan that whey proteins are in fact a subclass of milk proteins, and thus the term "milk protein" may include one or more whey proteins, except as otherwise indicated herein. Whey compositions may include, for example, milk, cream, and cheese whey. Whey derived from any cheese type may be used. Whey protein may be derived from any methods such as filtration, dialysis, evaporation, and reverse osmosis of cheese whey, or by any other process which results in the proteins typically described as "whey proteins".

In a preferred embodiment, the hydrolysate obtainable (e.g. obtained) by the method of the present invention may be a milk protein hydrolysate, a wheat protein hydrolysate (e.g. a gliadin and/or gluten hydrolysate), a soy protein hydrolysate or combinations thereof. Therefore, described herein is the use of at least one endoprotease and at least one proline tolerant tripeptidyl peptidase or fermentate comprising a proline tolerant tripeptidyl peptidase wherein said proline tolerant tripeptidyl peptidase predominantly has exopeptidase activity and is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
or obtainable by any one of methods of the invention; in the manufacture of a hydrolysate for reducing the immunogenicity in a subject predisposed to having an immune reaction to an untreated hydrolysate.

Suitably, the proline tolerant tripeptidyl peptidase may be capable of cleaving tri-peptides from the N-terminus of peptides having: Proline at P1; and an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

As used herein, "reduced immunogenicity" refers to any reduction, decrease, or amelioration of a measurable immunological response. The measurement of such response may be assessed *in vitro* or *in vivo.* For example, the response may be measured directly or indirectly in a biological sample comprising tissue, cells, or fluid, or the like, or any combination thereof from an individual or it may be assessed in the individual, either directly or indirectly. While in various embodiments provided herein, any mathematical decrease (or reduction) in such response, whether measured *in vitro* or *in vivo,* will suffice, it is preferred that the decrease be a more substantial one. Of course, the skilled artisan will appreciate that biological data such as a measurement of an immunological response, are subject to potentially large variation within an individual, and from individual to individual. For example where the response is in a "sensitive" individual, the immune response is preferably substantially reduced (e.g. by at least about 50%, 60%, or 70%, or even about 80% or more). More preferably, only small or minimal differences are seen in such the sensitive individual with the protein hydrolysates described herein, as compared to a measure of the immunological response from an individual who is not sensitive to one or more proteins or portions thereof. This is particularly preferable where the immunological response is deemed adverse, for example an allergic response. In such cases the decrease in the sensitive individual's immunological response (or measure thereof) may be at least about 85% to about 90%, more preferably about 90% to about 95%, or even more. In some case, a sensitive individual's response to the protein hydrolysates described herein is not significantly different, statistically, from the response of an individual who is not sensitive. In yet other cases, the reduction in immunological response may be many-fold over that seen with the unhydrolysed protein in a "sensitive" individual. For example, there may be about a 10-fold to 100-fold or even 1000-fold reduction in response. More preferably reductions of about 1000-fold to 10,000-fold or even 100,000-fold or greater reduction in a measurement of an immunological response from an individual consuming or exposed to the protein hydrolysate compositions as disclosed herein, as compared to that individual's response to the unmodified proteins.

As used herein, a "sensitive" individual is an individual predisposed to having an immune response or reaction to the protein in an unhydrolysed form. Such immune response or reaction as a direct or indirect result of the consumption of, or exposure to, for example one or more protein or portion thereof, is a measure of the immunogenicity of those proteins. Such proteins will demonstrate little to no immunogenicity in an individual who is not predisposed to having such an immune response to the protein, such an individual is sometimes referred to herein as "insensitive" or "not sensitive" to the one or more proteins or portions thereof. Preferably, such an individual will not have a significant immune reaction (immunological response) to either the exposure to or consumption of the protein.

Sensitive individuals having a reaction to wheat proteins (in particular gluten and/or gliadin) may present with symptoms of coeliac disease (e.g. celiac sprue). Symptoms include pain and discomfort in the digestive tract, chronic constipation and diarrheoa, failure to thrive (in children), anaemia and fatigue, but these may be absent, and symptoms in other organ systems have been described. Vitamin deficiencies are often noted in people with coeliac disease owing to the reduced ability of the small intestine to properly absorb nutrients from food. Without wishing to be bound by theory it is believed that upon exposure to gliadin that the enzyme tissue transglutaminase modifies the gliadin resulting in cross-reaction of the immune system with the bowel tissue causing an inflammatory reaction in the affected individual.

Sensitive individuals having a reaction to milk proteins may present with symptoms of a milk allergy which is caused by an adverse immune reaction to one or more of the proteins or immunogenic fragments thereof in the milk. The disorder can be either antibody-mediated or non-antibody-mediated. Antibody-mediated milk allergies are typically rapid in onset and may result in the individual displaying gastrointestinal, dermatological and/or respiratory symptoms. Such symptoms may further manifest as: skin rashes, hives, vomiting, and gastric distress, such as diarrheoa, rhinitis, stomach pain, wheezing, or anaphylactic reactions. Non-antibody-mediated is typically believed to be mediated by T-lymphocytes and not caused by antibodies. Symptoms of this form are typically gastrointestinal and dermatological.

Other sensitive individuals may have a reaction to soy which results in a range of symptoms, the most severe being anaphylaxis.

The hydrolysates and/or food and/or feed and/or comprising such hydrolysates or compositions of the invention may be particularly suitable for administering to a subject suffering from coeliac disease, a milk protein allergy and/or a soy protein allergy.

Advantageously, the endoprotease in combination with a proline tolerant tripeptidyl peptidase is capable of cleaving protein substrates associated with causing an immune response in sensitive individuals suffering from a disease, such as a milk protein allergy and/or a soy protein allergy.

Therefore disclosed herein is a food additive or feed additive composition comprising a hydrolysate produced in accordance with the methods and/or uses of the present invention.

Described herein is the use of at least one endoprotease and at least one proline tolerant tripeptidyl peptidase or fermentate comprising a proline tolerant tripeptidyl peptidase wherein said proline tolerant tripeptidyl peptidase predominantly has exopeptidase activity and is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
or obtainable by any one of methods of the invention; in the manufacture of a hydrolysate for reducing bitterness of the hydrolysate.

When the protein or portion thereof substrate for hydrolysate production is rich in hydrophobic L-amino acids the protein hydrolysate may have a bitter taste. Without wishing to be bound by theory it is believed that the bitterness of a peptide is dependent on its peptide length and the average hydrophobicity of the L-amino acid residues therein.

The "reduced bitterness" of a hydrolysate can be measured objectively using a tasting panel of individuals who are asked to rate the bitterness of a hydrolysate. A bitterness index can be used to rate the bitterness of the hydrolysate. For example, a bitterness index can be used that rates substances relative to quinine which is given a reference index of 1, alternatively a bitterness index may be used having a scale from 0 (not bitter) to 10 (bitter). Suitably any tasting of hydrolysates may be done using the appropriate controls, such as blind testing. Additionally or alternatively, the cleavage of known bitter peptides may be monitored via LC-MS or other suitable techniques known in the art.

The endoprotease and proline tolerant tripeptidyl peptidase may be used to cleave bitter peptides.

Advantageously, this can be achieved due to the tolerance of a wide variety of amino acids at positions P1, P2, P3, P1', P2' and/or P3' by the proline tolerant tripeptidyl peptidase.

The proline tolerant tripeptidyl peptidase may be capable of cleaving a protein having the sequence GPFPIIV, VIPVPQK and/or GPFPVI. GPFPVI is known to be a highly bitter peptide (Matoba et al (1970), Agric. Biol. Chem. 34, 321).

Surprisingly, the proline tolerant tripeptidyl peptidase may be capable of cleaving peptides having proline at the P1 and P1' position, thus facilitating the cleavage of such bitter peptides.

In one embodiment debittered hydrolysates may be used in the preparation of food and or foodstuffs.

Described herein, the hydrolysate may comprise a proline tolerant tripeptidyl peptidase comprising one or more amino acid sequence selected from:
SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

Described herein, the proline tolerant tripeptidyl peptidase may comprise one or more amino acid sequence selected from SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28 or a functional fragment thereof or an amino acid sequence having at least 70% identity therewith.

Described herein, the proline tolerant tripeptidyl peptidase may comprise one or more amino acid sequence selected from SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

### KITS

Described herein is a kit comprising at least one proline tolerant tripeptidyl peptidase wherein said proline tolerant tripeptidyl peptidase predominantly has exopeptidase activity and is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
or obtainable by any one of methods of the invention; at least one endoprotease; and instructions for co-administering same.

Described herein, the proline tolerant tripeptidyl peptidase may be capable of cleaving tripeptides from the N-terminus of peptides having:
(i)
   (A) Proline at P1; and
   (B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and
(ii)
   (a') Proline at P1'; and
   (b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

More suitably, the proline tolerant tripeptidyl peptidase may be capable of cleaving tripeptides from the N-terminus of peptides having proline at position P1 and at position P1'. Described herein is a kit comprising:
(i) at least one endopeptidase;
(ii) at least one exo-tripeptidyl peptidase of the S53 family; and (iii) one or more aminopeptidase, and instructions for co-administering same
The endoprotease may be compartmentalised separately to the proline tolerant tripeptidyl peptidase or the two enzymes may be mixed.

The proline tolerant tripeptidyl peptidase and/or endoprotease may be formulated in any manner as described herein or known to the person skilled in the art.

The term "co-administering" as used herein means administering one or more ingredients and/or enzyme either separately (e.g. sequentially) or together (e.g. simultaneously).

### ACTIVITY AND ASSAYS

The proline tolerant tripeptidyl peptidase for use in the present invention predominantly has exopeptidase activity.

The term "exopeptidase" activity as used herein means that the proline tolerant tripeptidyl peptidase is capable of cleaving tri-peptides from the N-terminus of a substrate, such as a protein and/or peptide substrate.

The term "predominantly has exopeptidase activity" as used herein means that the tripeptidyl peptidase has no or substantially no endoprotease activity.

"Substantially no endoprotease activity" means that the proline tolerant tripeptidyl peptidase or exo-peptidase of the S53 family has less than about 100U endoprotease activity in the "Endoprotease Assay" taught herein when compared to 1000nkat of exopeptidase activity in the "Exopeptidase Broad-Specificity Assay (EBSA)" taught herein. Suitably, "substantially no endoprotease activity" means that the proline tolerant tripeptidyl peptidase has less than about 100U endoprotease activity in the "Endoprotease Assay" taught herein when compared to 1000nkat of exopeptidase activity in the "Exopeptidase Broad-Specificity Assay" taught herein.

Preferably the proline tolerant tripeptidyl peptidase or exo-peptidase of the S53 family may have less than about 10U endoprotease activity in the "Endoprotease Assay" taught herein when compared to 1000 nkat of exopeptidase activity in the "Exopeptidase Broad-Specificity Assay" taught herein, more preferably less than about 1U endoprotease activity in the "Endoprotease Assay" taught herein when compared to 1000 nkat of exopeptidase activity in the "Exopeptidase Broad-Specificity Assay" taught herein. Even more preferably the proline tolerant tripeptidyl peptidase or exo-tripeptidyl peptidase may have less than about 0.1U endoprotease activity in the "Endoprotease Assay" taught herein when compared to 1000 nkat of exopeptidase activity in the "Exopeptidase Broad-Specificity Assay" taught herein.

### "ENDOPROTEASE ASSAY"

### Azoscasein assay for endoprotease activity

A modified version of the endoprotease assay described by Iversen and Jorgensen, 1995 (Biotechnology Techniques 9, 573-576) is used. An enzyme sample of 50 µl is added to 250 µl of azocasein (0.25% w/v; from Sigma) in 4 times diluted Mcllvaine buffer, pH 5 and incubated for 15 min at 40°C with shaking (800 rpm). The reaction is terminated by adding 50 µl of 2 M trichloroacetic acid (TCA) (from Sigma Aldrich, Denmark) and centrifugation for 5 min at 20,000 g. To a 195 µl sample of the supernatant 65 µl of 1 M NaOH is added and absorbance at 450 nm is measured. One unit of endoprotease activity is defined as the amount which yields an increase in absorbance of 0.1 in 15 min at 40°C at 450 nm.

### "EXOPEPTIDASE ASSAY"

There are two parts to the assay:

### Part 1 - "Exopeptidase Broad-Specificity Assay" (EBSA)

10 µL of the chromogenic peptide solution (10 mM H-Ala-Ala-Ala-pNA dissolved in dimethyl sulfoxide (DMSO); MW = 387.82; Bachem, Switzerland) were added to 130 µl Na-acetate (20 mM, adjusted to pH 4.0 with acetic acid) in a microtiter plate and heated for 5 minutes at 40°C. 10 µL of appropriately diluted enzyme was added and the absorption was measured in a MTP reader (Versa max, Molecular Devices, Denmark) at 405 nm. One katal of proteolytic activity was defined as the amount of enzyme required to release 1 mole of p-nitroaniline per second.

### Part 2 (i) - P1 Proline Assay

(a) Dissolve the substrate H-Arg-Gly-Pro-Phe-Pro-Ile-Ile-Val (MW = 897.12; from Schafer-N, Copenhagen in 10 times diluted Mcllvain buffer, pH=4.5 at 1 mg/ml concentration.
(b) Incubate 1000 ul of the substrate solution with 10 ug of proline tolerant tripeptidyl peptidase solution at 40°C.
(c) Take 100 ul samples at seven time points (0, 30, 60, 120, 720 and 900 min), dilute with 50ul 5% TFA, heat inactivate (10 min at 80°C) and keep at -20°C until LC-MS analysis;
(d) Perform LC-MC/MS analysis using an Agilent 1100 Series Capillary HPLC system (Agilent Technologies, Santa Clara, CA) interfaced to a LTQ Orbitrap Classic hybrid mass spectrometer (Thermo Scientific, Bremen, Germany);
(e) Load samples onto a 50 mm Fortis™ C18 column with an inner diameter of 2.1 mm and a practical size of 1.7 µm
(f) Perform separation at a flow rate of 200µL/min using a 14 min gradient of 2-28% Solvent B (H2O/CH3CN/ HCOOH (50/950/0.65 v/v/v)) into the lonMAX source- The LTQ Orbitrap Classic instrument was operated in a data-dependent MS/MS mode;
(g) Measure the peptide masses by the Orbitrap (obtain MS scans with a resolution of 60.000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of ±10 ppm relative to masses on the list;
(h) Use the open source program Skyline 1.4.0.4421 (available from MacCoss Lab Software, University of Washington, Department of Genome Sciences, 3720 15^{th} Ave NE Seattle, Washington, US) to access the RAW files and extract MS1 intensities to build chromatograms. Set the precursor isotopic import filter to a count of three, (M, M+1, and M+2) at a resolution of 60,000 and use the most intense charge state;
(i) Peptide sequences of the substrate and cleavage products were typed into Skyline and intensities were calculated in each sample (0, 30, 60, 120, 720 and 900 min hydrolysis).
(j) One unit of activity is defined as the amount of enzyme which in this assay will hydrolyse 50% of the substrate within 720 min while releasing Arg-Gly-Pro.

### Part 2 (ii) - P1' Proline Assay

(a) Dissolve the peptide H-Ala-Ala-Phe-Pro-Ala-NH2 (MW = 474.5; from Schafer-N, Copenhagen) in 10 times diluted Mcllvain buffer, pH=4.5 at 0.1 mg/ml concentration.
(b) Incubate 1000 ul of the substrate solution with 10 ug proline tolerant tripeptidyl peptidase solution at 40°C.
(c) Take 100 ul samples at seven time points (0, 30, 60, 120, 720 and 900 min), dilute with 50ul 5% TFA, heat inactivate (10 min at 80°C) and keep at -20°C until LC-MS analysis;
(d) Perform LC-MC/MS analysis using a Agilent 1100 Series Capillary HPLC system (Agilent Technologies, Santa Clara, CA) interfaced to a LTQ Orbitrap Classic hybrid mass spectrometer (Thermo Scientific, Bremen, Germany);
(e) Load samples onto a 50 mm Fortis™ C18 column with an inner diameter of 2.1 mm and a practical size of 1.7 µm
(f) Perform separation at a flow rate of 200µL/min using a 14 min gradient of 2-28% Solvent B (H2O/CH3CN/ HCOOH (50/950/0.65 v/v/v)) into the lonMAX source- The LTQ Orbitrap Classic instrument was operated in a data-dependent MS/MS mode;
(g) Measure the peptide masses by the Orbitrap (obtain MS scans with a resolution of 60.000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of ±10 ppm relative to masses on the list.
(h) Use the open source program Skyline 1.4.0.4421 (available from MacCoss Lab Software, University of Washington, Department of Genome Sciences, 3720 15^{th} Ave NE Seattle, Washington, US) to access the RAW files and extract MS1 intensities to build chromatograms. Set the precursor isotopic import filter to a count of three, (M, M+1, and M+2) at a resolution of 60,000 and use the most intense charge state;
(i) Peptide sequences of the substrate as well as cleavage products were typed into Skyline and intensities were calculated in each sample.
(j) One unit of activity is defined as the amount of enzyme which in this assay will hydrolyse 50% of the substrate within 720 min while releasing Ala-Ala-Phe.

In one embodiment a proline tolerant tripeptidyl peptidase in accordance with the present invention has an activity of at least 50 nkat in Part 1 of the activity taught herein and at least 100U activity in Part 2(i) or Part 2(ii) of the assay taught herein per mg of protein.

In one embodiment a proline tolerant tripeptidyl peptidase in accordance with the present invention has an activity of between about 50-2000 nkat in Part 1 of the activity taught herein and between about 1-500 units activity in Part 2(i) or Part 2(ii) of the assay taught herein per mg of protein. Note the protein measurement is described in Example 2.

### "P1 AND P1' PROLINE ACTIVITY ASSAY"

Suitably the tripeptidyl peptidase for use in the present invention may be able to cleave substrates having proline at position P1 and P1'. This can be assessed using the assay taught below.

In this assay a tripeptidyl peptidase is examined for its ability to hydrolyse a synthetic substrate AAPPA by LC-MS and label free quantification.
(a) Dissolve the peptide H-AAPPA-NH2 (MW=424.3, from Schafer-N, Copenhagen) in 20 mM MES buffer, pH=4.0 (1 mg/ml);
(b) Incubate 1000 ul of the H-AAPPA-NH2 solution with 200 ul proline tolerant tripeptidyl peptidase solution (40ug/ml) (substrate/enzyme 100:0.8) at room temperature;
(c) Take 100 ul samples at seven time points (0, 5, 15, 60, 180, 720 and 1440 min), dilute with 50ul 5% TFA, heat inactivate (10 min at 80°C) and keep at -20°C until LC-MS analysis;
(d) Perform Nano LC-MS/MS analyses using an Easy LC system (Thermo Scientific, Odense, DK) interfaced to a LTQ Orbitrap Classic hybrid mass spectrometer (Thermo Scientific, Bremen, Germany);
(e) Load samples onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle;
(f) Perform separation at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK) - operate the LTQ Orbitrap Classic instrument in a data-dependent MS/MS mode;
(g) Measure the peptide masses by the Orbitrap (obtain MS scans with a resolution of 60 000 at m/z 400), and select up to 2 of the most intense peptide m/z and subject to fragmentation using CID in the linear ion trap (LTQ). Enable dynamic exclusion with a list size of 500 masses, duration of 40 s, and an exclusion mass width of ±10 ppm relative to masses on the list;
(h) Use the open source program Skyline 1.4.0.4421 (available from MacCoss Lab Software, University of Washington, Department of Genome Sciences, 3720 15^{th} Ave NE Seattle, Washington, US) to access the RAW files which program can use the MS1 intensities to build chromatograms. Set the precursor isotopic import filter to a count of three, (M, M+1, and M+2) at a resolution of 60,000 and use the most intense charge state;
(i) Peptide sequences of the substrate as well as cleavage products were typed into Skyline and intensities were calculated in each sample.
(j) One unit of activity is defined as the amount of enzyme which in this assay will hydrolyse 50% of the substrate within 24 h while releasing AAP.

In one embodiment a proline tolerant tripeptidyl peptidase for use in the present invention has an activity of at least 50 nkat in Part 1 of the activity taught herein and at least 100 U activity in Part 2(i) or Part 2(ii) of the assay taught herein per mg of protein. In one embodiment a proline tolerant tripeptidyl peptidase for use in the present invention has an activity of between about 50-2000 nkat in Part 1 of the activity taught herein and between about 1-500 units activity in Part 2(i) or Part 2(ii) of the assay taught herein per mg of protein (protein concentration is calculated as in Example 2).

In one embodiment a proline tolerant tripeptidyl peptidase for use in the present invention may have at least 10 U activity in the "P1 and P1' Proline Activity Assay" taught herein per mg of protein.

In one embodiment a proline tolerant tripeptidyl peptidase for use in the present invention has an activity of between about 1 U - 500 U activity in the "P1 and P1' Proline Activity Assay" taught herein per mg of protein.

In addition to the above, the proline tolerant tripeptidyl peptidase may also have activity in accordance with Part 1 of the "Exopeptidase Activity Assay" taught above.

In one embodiment the proline tolerant tripeptidyl peptidase for use in the present invention may have at least 10 U activity in the "P1 and P1' Proline Activity Assay" taught herein and at least 50 nkatal in Part 1 of the "Exopeptidase Activity Assay" taught herein per mg of protein. In another embodiment a proline tolerant tripeptidyl peptidase for use in the present invention has an activity of between about 1 U - 500 U activity in the "P1 and P1' Proline Activity Assay" taught herein and between about 50 U - 2000 U katal in Part 1 of the "Exopeptidase Activity Assay" taught herein per mg of protein.

### AMINO ACID AND NUCLEOTIDE SEQUENCES

The proline tolerant tripeptidyl peptidase for use in accordance with the present invention may be obtainable (e.g. obtained) from any source so long as it has the activity described in the claims.

In one embodiment the proline tolerant tripeptidyl peptidase for use in accordance with the present invention may be obtainable (e.g. obtained) from *Trichoderma.*

Suitably from *Trichoderma reesei,* more suitably, *Trichoderma reesei* QM6A.

Suitably from *Trichoderma virens,* more suitably, *Trichoderma virens* Gv29-8.

Suitably from *Trichoderma atroviride.* More suitably, *Trichoderma atroviride* IMI 206040.

In one embodiment the proline tolerant tripeptidyl peptidase for use in accordance with the present invention may be obtainable (e.g. obtained) from *Aspergillus.*

Suitably from *Aspergillus fumigatus,* more suitably *Aspergillus fumigatus* CAE17675.

Suitably from *Aspergillus kawachii,* more suitably from *Aspergillus kawachii* IFO 4308.

Suitably from *Aspergillus nidulans,* more suitably from *Aspergillus nidulans* FGSC A4.

Suitably from *Aspergillus oryzae,* more suitably *Aspergillus oryzae* RIB40.

Suitably from *Aspergillus ruber,* more suitably *Aspergillus ruber* CBS135680.

Suitably from *Aspergillus terreus,* more suitably from *Aspergillus terreus* NIH2624.

In one embodiment the proline tolerant tripeptidyl peptidase for use in accordance with the present invention may be obtainable (e.g. obtained) from *Bipolaris,* suitably from *Bipolaris maydis,* more suitably *Bipolaris maydis* C5.

In one embodiment the proline tolerant tripeptidyl peptidase for use in accordance with the present invention may be obtainable (e.g. obtained) from *Togninia,* suitably from *Togninia minima* more suitably *Togninia minima* UCRPA7.

In one embodiment the proline tolerant tripeptidyl peptidase for use in accordance with the present invention may be obtainable (e.g. obtained) from *Talaromyces,* suitably from *Talaromyces stipitatus* more suitably *Talaromyces stipitatus* ATCC 10500.

In one embodiment the proline tolerant tripeptidyl peptidase for use in accordance with the present invention may be obtainable (e.g. obtained) from *Arthroderma,* suitably from *Arthroderma benhamiae* more suitably *Arthroderma benhamiae* CBS 112371.

In one embodiment the proline tolerant tripeptidyl peptidase for use in accordance with the present invention may be obtainable (e.g. obtained) from *Magnaporthe,* suitably from *Magnaporthe oryzae* more suitably *Magnaporthe oryzae* 70-1.

In another embodiment the proline tolerant tripeptidyl peptidase for use in accordance with the present invention may be obtainable (e.g. obtained) from *Fusarium.*

Suitably from *Fusarium oxysporum,* more suitably from *Fusarium oxysporum f. sp.* cubense race 4.

Suitably from *Fusarium graminearum,* more suitably *Fusarium graminearum* PH-1.

In a further embodiment the proline tolerant tripeptidyl peptidase for use in accordance with the present invention may be obtainable (e.g. obtained) from *Phaeosphaeria,* suitably from *Phaeosphaeria nodorum* more suitably *Phaeosphaeria nodorum* SN15.

In a yet further embodiment the proline tolerant tripeptidyl peptidase for use in accordance with the present invention may be obtainable (e.g. obtained) from *Agaricus,* suitably from *Agaricus bisporus* more suitably *Agaricus bisporus var. burnettii* JB137-S8.

In a yet further embodiment the proline tolerant tripeptidyl peptidase for use in accordance with the present invention may be obtainable (e.g. obtained) from *Acremonium,* suitably from *Acremonium alcalophilum.*

In a yet further embodiment the proline tolerant tripeptidyl peptidase for use in accordance with the present invention may be obtainable (e.g. obtained) from *Sodiomyces,* suitably from *Sodiomyces alkalinus.*

In one embodiment the proline tolerant tripeptidyl peptidase for use in accordance with the present invention may be obtainable (e.g. obtained) from *Penicillium.*

Suitably the proline tolerant tripeptidyl peptidase may be obtainable from *Penicillium digitatum,* more suitably from *Penicillium digitatum* Pd1.

Suitably the proline tolerant tripeptidyl peptidase may be obtainable from *Penicillium oxalicum,* more suitably from *Penicillium oxalicum* 114-2.

Suitably the proline tolerant tripeptidyl peptidase may be obtainable from *Penicillium roqueforti,* more suitably from *Penicillium roqueforti* FM164.

Suitably the proline tolerant tripeptidyl peptidase may be obtainable from *Penicillium rubens,* more suitably from *Penicillium rubens* Wisconsin 54-1255.

In another embodiment the proline tolerant tripeptidyl peptidase for use in accordance with the present invention may be obtainable (e.g. obtained) from *Neosartorya.*

Suitably the proline tolerant tripeptidyl peptidase may be obtainable from *Neosartorya fischeri,* more suitably from *Neosartorya fischeri* NRRL181.

In one embodiment the tripeptidyl peptidase (e.g. proline tolerant tripeptidyl peptidase) for use in accordance with the present invention is not obtainable (e.g. obtained) from *Aspergillus niger.*

| **SEQ ID No.:** | **Sequence** | **Origin** |
|---|---|---|
| 1 | | Trichoderma reesei QM6a |
| 2 | | Trichoderma reesei QM6a |
| 3 | | Aspergillus oryzae RIB40 |
| 4 | | Phaeosphaer ia nodorum SN15 |
| 5 | | Trichoderma atroviride IMI 206040 |
| 6 | | Arthroderma benhamiae CBS 112371 |
| 7 | | Fusarium graminearum PH-1 |
| 8 | | Acremonium alcalophilum |
| 9 | | Sodiomyces alkalinus |
| 10 | | Aspergillus kawachii IFO 4308 |
| 11 | | Talaromyces stipitatus ATCC 10500 |
| 12 | | Fusarium oxysporum f. sp. cubense race 4 |
| 13 | | Trichoderma virens Gv29-8 |
| 14 | | Trichoderma atroviride IMI 206040 |
| 15 | | Agaricus bisporus var. burnettii JB137-S8 |
| 16 | | Magnaporthe oryzae 70-15 |
| 17 | | Togninia minima UCRPA7 |
| 18 | | Bipolaris maydis C5 |
| 19 | | Aspergillus kawachii IFO 4308 |
| 20 | | Aspergillus nidulans FGSC A4 |
| 21 | | Aspergillus ruber CBS 135680 |
| 22 | | Aspergillus terreus NIH2624 |
| 23 | | Penicillium digitatum Pd1 |
| | | |
| 24 | | Penicillium oxalicum 114-2 |
| 25 | | Penicillium roqueforti FM164 |
| 26 | | Penicillium rubens Wisconsin 54-1255 |
| 27 | | Neosartorya fischeri NRRL 181 |
| 28 | | Aspergillus fumigatus CAE17675 |
| 29 | | Trichoderma reesei QM6a |
| 30 | | Aspergillus oryzae RIB40 |
| 31 | | Phaeosphaer ia nodorum SN15 |
| 32 | | Trichoderma atroviride IMI 206040 |
| 33 | | Arthroderma benhamiae CBS 112371 |
| 34 | | Fusarium graminearum PH-1 |
| 35 | | Acremonium alcalophilum |
| 36 | | Sodiomyces alkalinus |
| 37 | | Aspergillus kawachii IFO 4308 |
| 38 | | Talaromyces stipitatus ATCC 10500 |
| 39 | | Fusarium oxysporum f. sp. cubense race 4 |
| 40 | | Trichoderma virens Gv29-8 |
| 41 | | Trichoderma atroviride IMI 206040 |
| 42 | | Agaricus bisporus var. burnettii JB137-S8 |
| 43 | | Magnaporthe oryzae 70-15 |
| 44 | | Togninia minima UCRPA7 |
| 45 | | Bipolaris maydis C5 |
| 46 | | Aspergillus kawachii IFO 4308 |
| 47 | | Aspergillus nidulans FGSC A4 |
| 48 | | Aspergillus ruber CBS 135680 |
| 49 | | Aspergillus terreus NIH2624 |
| 50 | | Penicillium digitatum Pd1 |
| 51 | | Penicillium oxalicum 114-2 |
| 52 | | Penicillium roqueforti FM164 |
| 53 | | Penicillium rubens Wisconsin 54-1255 |
| 54 | | Neosartorya fischeri NRRL 181 |
| 55 | | Aspergillus fumigatus CAE17675 |
| | | |
| 56 | | Trichoderma reesei QM6a |
| 57 | | Aspergillus oryzae RIB40 |
| 58 | | Phaeosphaer ia nodorum SN15 |
| 59 | | Trichoderma atroviride IMI 206040 |
| 60 | | Arthroderma benhamiae CBS 112371 |
| 61 | | Fusarium graminearum PH-1 |
| 62 | | Aspergillus kawachii IFO 4308 |
| 63 | | Talaromyces stipitatus ATCC 10500 |
| 64 | | Fusarium oxysporum f. sp. cubense race 4 |
| 65 | | Trichoderma virens Gv29-8 |
| 66 | | Trichoderma atroviride IMI 206040 |
| 67 | | Agaricus bisporus var. burnettii JB137-S8 |
| 68 | | Magnaporthe oryzae 70-15 |
| 69 | | Togninia minima UCRPA7 |
| 70 | | Bipolaris maydis C5 |
| 71 | | Aspergillus kawachii IFO 4308 |
| 72 | | Aspergillus nidulans FGSC A4 |
| 73 | | Aspergillus ruber CBS 135680 |
| 74 | | Aspergillus terreus NIH2624 |
| 75 | | Penicillium digitatum Pd1 |
| 76 | | Penicillium oxalicum 114-2 |
| 77 | | Penicillium roqueforti FM164 |
| 78 | | Penicillium rubens Wisconsin 54-1255 |
| 79 | | Neosartorya fischeri NRRL 181 |
| 80 | | Aspergillus fumigatus CAE17675 |
| 81 | | Phaeosphaer ia nodorum SN15 |
| 82 | | Trichoderma atroviride IMI 206040 |
| 83 | | Arthroderma benhamiae CBS 112371 |
| 84 | | Fusarium graminearum PH-1 |
| 85 | | Acremonium alcalophilum |
| 86 | | Sodiomyces alkalinus |
| 87 | | Aspergillus kawachii IFO 4308 |
| 88 | | Talaromyces stipitatus ATCC 10500 |
| 89 | | Fusarium oxysporum f. sp. cubense race 4 |
| 90 | | Trichoderma virens Gv29-8 |
| 91 | | Trichoderma atroviride IMI 206040 |
| 92 | | Agaricus bisporus var. burnettii JB137-S8 |
| 93 | | Magnaporthe oryzae 70-15 |
| 94 | | Togninia minima UCRPA7 |
| 95 | | Bipolaris maydis C5 |

| **SEQ ID No.:** | **Description** | **Sequence** | **Origin** |
|---|---|---|---|
| 96 | TRI045 Genomic sequence CDS | | Human skin fungus *Arthroderma benhamiae* |
| | | | |
| 97 | TRI045 Synthetic Gene optimized for expression in trichoderma with trichoderma signal sequence underlined | | Human skin fungus *Arthroderma benhamiae* |
| | | | |
| 98 | TRI045 pre_pro amino acid sesquence | | Human skin fungus *Arthroderma benhamiae* |
| 99 | TRI045 mature Interpro domain IPR000209 Peptidase S8/S53 dom | | Human skin fungus *Arthroderma benhamiae* |

Described herein, the at least one proline tolerant tripeptidyl peptidase may:
(a) comprise the amino acid sequence SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof;
(b) comprise an amino acid having at least 70% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof;
(c) be encoded by a nucleotide sequence comprising the sequence SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97;
(d) be encoded by a nucleotide sequence comprising at least about 70% sequence identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97;
(e) be encoded by a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 under medium stringency conditions; or
(f) be encoded by a nucleotide sequence which differs from SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 due to degeneracy of the genetic code.

The proline tolerant tripeptidyl peptidase may be expressed as a polypeptide sequence which undergoes further post-transcriptional and/or post-translational modification.

Described herein, the proline tolerant tripeptidyl peptidase may comprise the amino acid sequence SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 98 or a functional fragment thereof.

Described herein, the proline tolerant tripeptidyl peptidase comprise an amino acid having at least 70% identity to SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 98 or a functional fragment thereof.

Described herein, the proline tolerant tripeptidyl peptidase may comprise the amino acid sequence SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 98 or a functional fragment thereof.

Described herein, the proline tolerant tripeptidyl peptidase comprise an amino acid having at least 70% identity to SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 98 or a functional fragment thereof;

In another embodiment the proline tolerant tripeptidyl peptidase may be a "mature" proline tolerant tripeptidyl peptidase which has undergone post-transcriptional and/or post-translational modification (e.g. post-translational cleavage). Suitably such modification may lead to an activation of the enzyme.

Described herein, the proline tolerant tripeptidyl peptidase may comprise the amino acid sequence SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 99 or a functional fragment thereof.

Described herein, the proline tolerant tripeptidyl peptidase comprise an amino acid having at least 70% identity to SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 99 or a functional fragment thereof.

Described herein, the proline tolerant tripeptidyl peptidase may comprise the amino acid sequence SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 99 or a functional fragment thereof.

Described herein, the proline tolerant tripeptidyl peptidase comprise an amino acid having at least 70% identity to SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42 SEQ ID No. 43, SEQ ID No. 44, SEQ ID No. 45, SEQ ID No. 99 or a functional fragment thereof.

The term "functional fragment" is a portion of an amino acid sequence that retains its peptidase enzyme activity. Therefore, a functional fragment of a proline tolerant tripeptidyl peptidase is a portion of a proline tolerant tripeptidyl peptidase predominantly having exopeptidase activity wherein said proline tolerant tripeptidyl peptidase is capable of cleaving tri-peptides from the N-terminus of peptides having
(i)
   (A) Proline at P1; and
   (B) An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; and/or
(ii)
   (a') Proline at P1'; and
   (b') An amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'. Alternatively or additionally a functional fragment of a proline tolerant tripeptidyl peptidase is a portion of a proline tolerant tripeptidyl peptidase predominantly having exopeptidase activity and capable of cleaving tri-peptides from the N-terminus of peptides having proline at P1 and P1'.

The "portion" is any portion that still has the activity as defined above; suitably a portion may be at least 50 amino acids in length, more suitably at least 100. In other embodiments the portion may be about 150 or about 200 amino acids in length.

In one embodiment the functional fragment may be portion of a proline tolerant tripeptidyl peptidase following post transcriptional and/or post-translational modification (e.g. cleavage). Described herein, the functional fragment may comprise a sequence shown as: SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55 or SEQ ID No. 99.

Described herein, the proline tolerant tripeptidyl peptidase may comprise one or more amino acid sequence selected from SEQ ID No. 1, or a functional fragment thereof.

Described herein, the proline tolerant tripeptidyl peptidase may comprise an amino acid having at least 70% identity to SEQ ID No. 1 or a functional fragment thereof.

Described herein, the proline tolerant tripeptidyl peptidase may comprise one or more amino acid sequence selected from SEQ ID No. 2, or a functional fragment thereof.

Described herein, the proline tolerant tripeptidyl peptidase may comprise an amino acid having at least 70% identity to SEQ ID No. 2 or a functional fragment thereof.

The proline tolerant tripeptidyl peptidase may comprise one or more amino acid sequence selected from SEQ ID No. 3 or a functional fragment thereof.

Described herein, the proline tolerant tripeptidyl peptidase may comprise an amino acid having at least 70% identity to SEQ ID No. 3 or a functional fragment thereof.

Described herein, the proline tolerant tripeptidyl peptidase may comprise one or more amino acid sequence selected from SEQ ID No. 4 or a functional fragment thereof.

Described herein, the proline tolerant tripeptidyl peptidase may comprise an amino acid having at least 70% identity to SEQ ID No. 4 or a functional fragment thereof.

Described herein, the proline tolerant tripeptidyl peptidase may comprise one or more amino acid sequence selected from SEQ ID No. 5 or a functional fragment thereof.

Described herein, the proline tolerant tripeptidyl peptidase may comprise an amino acid having at least 70% identity to SEQ ID No. 5 or a functional fragment thereof.

Described herein, the proline tolerant tripeptidyl peptidase may comprise one or more amino acid sequence selected from SEQ ID No.6 or a functional fragment thereof.

Described herein, the proline tolerant tripeptidyl peptidase may comprise an amino acid having at least 70% identity to SEQ ID No.6 or a functional fragment thereof.

Described herein, the proline tolerant tripeptidyl peptidase may comprise one or more amino acid sequence selected from SEQ ID No. 7 or a functional fragment thereof.

Described herein, the proline tolerant tripeptidyl peptidase may comprise an amino acid having at least 70% identity to SEQ ID No. 7 or a functional fragment thereof.

Described herein, the proline tolerant tripeptidyl peptidase may comprise one or more amino acid sequence selected from SEQ ID No. 8 or a functional fragment thereof.

Described herein, the proline tolerant tripeptidyl peptidase may comprise an amino acid having at least 70% identity to SEQ ID No. 8 or a functional fragment thereof.

Described herein, the proline tolerant tripeptidyl peptidase may comprise one or more amino acid sequence selected from SEQ ID No. 9 or a functional fragment thereof.

Described herein, the proline tolerant tripeptidyl peptidase may comprise an amino acid having at least 70% identity to SEQ ID No. 9 or a functional fragment thereof.

Described herein, the proline tolerant tripeptidyl peptidase may comprise one or more amino acid sequence selected from SEQ ID No. 10 or a functional fragment thereof.

Described herein, the proline tolerant tripeptidyl peptidase may comprise an amino acid having at least 70% identity to SEQ ID No. 10 or a functional fragment thereof.

Described herein, the proline tolerant tripeptidyl peptidase may comprise one or more amino acid sequence selected from SEQ ID No. 11 or a functional fragment thereof.

Described herein, the proline tolerant tripeptidyl peptidase may comprise an amino acid having at least 70% identity to SEQ ID No. 11 or a functional fragment thereof.

Described herein, the proline tolerant tripeptidyl peptidase may comprise one or more amino acid sequence selected from SEQ ID No. 12 or a functional fragment thereof.

Described herein, the proline tolerant tripeptidyl peptidase may comprise an amino acid having at least 70% identity to SEQ ID No. 12 or a functional fragment thereof.

Described herein, the proline tolerant tripeptidyl peptidase may comprise one or more amino acid sequence selected from SEQ ID No. 13 or a functional fragment thereof.

Described herein, the proline tolerant tripeptidyl peptidase may comprise an amino acid having at least 70% identity to SEQ ID No. 13 or a functional fragment thereof.

Described herein, the proline tolerant tripeptidyl peptidase may comprise one or more amino acid sequence selected from SEQ ID No. 14 or a functional fragment thereof.

Described herein, the proline tolerant tripeptidyl peptidase may comprise an amino acid having at least 70% identity to SEQ ID No. 14 or a functional fragment thereof.

Described herein, the proline tolerant tripeptidyl peptidase may comprise one or more amino acid sequence selected from SEQ ID No. 15 or a functional fragment thereof.

Described herein, the proline tolerant tripeptidyl peptidase may comprise an amino acid having at least 70% identity to SEQ ID No. 15 or a functional fragment thereof.

Described herein, the proline tolerant tripeptidyl peptidase may comprise one or more amino acid sequence selected from SEQ ID No. 16 or a functional fragment thereof.

Described herein, the proline tolerant tripeptidyl peptidase may comprise an amino acid having at least 70% identity to SEQ ID No. 16 or a functional fragment thereof.

Described herein, the proline tolerant tripeptidyl peptidase may comprise one or more amino acid sequence selected from SEQ ID No. 17 or a functional fragment thereof.

Described herein, the proline tolerant tripeptidyl peptidase may comprise an amino acid having at least 70% identity to SEQ ID No. 17 or a functional fragment thereof.

Described herein, the proline tolerant tripeptidyl peptidase may comprise one or more amino acid sequence selected from SEQ ID No. 18 or a functional fragment thereof.

Described herein, the proline tolerant tripeptidyl peptidase may comprise an amino acid having at least 70% identity to SEQ ID No. 18 or a functional fragment thereof.

Described herein, the proline tolerant tripeptidyl peptidase may comprise one or more amino acid sequence selected from SEQ ID No. 19 or a functional fragment thereof.

Described herein, the proline tolerant tripeptidyl peptidase may comprise an amino acid having at least 70% identity to SEQ ID No. 19 or a functional fragment thereof.

Described herein, the proline tolerant tripeptidyl peptidase may comprise one or more amino acid sequence selected from SEQ ID No. 20 or a functional fragment thereof.

Described herein, the proline tolerant tripeptidyl peptidase may comprise an amino acid having at least 70% identity to SEQ ID No. 20 or a functional fragment thereof.

Described herein, the proline tolerant tripeptidyl peptidase may comprise one or more amino acid sequence selected from SEQ ID No. 21 or a functional fragment thereof.

Described herein, the proline tolerant tripeptidyl peptidase may comprise an amino acid having at least 70% identity to SEQ ID No. 21 or a functional fragment thereof.

Described herein, the proline tolerant tripeptidyl peptidase may comprise one or more amino acid sequence selected from SEQ ID No. 22 or a functional fragment thereof.

Described herein, the proline tolerant tripeptidyl peptidase may comprise an amino acid having at least 70% identity to SEQ ID No. 22 or a functional fragment thereof.

Described herein, the proline tolerant tripeptidyl peptidase may comprise one or more amino acid sequence selected from SEQ ID No. 23 or a functional fragment thereof.

Described herein, the proline tolerant tripeptidyl peptidase may comprise an amino acid having at least 70% identity to SEQ ID No. 23 or a functional fragment thereof.

Described herein, the proline tolerant tripeptidyl peptidase may comprise one or more amino acid sequence selected from SEQ ID No. 24 or a functional fragment thereof.

Described herein, the proline tolerant tripeptidyl peptidase may comprise an amino acid having at least 70% identity to SEQ ID No. 24 or a functional fragment thereof.

Described herein, the proline tolerant tripeptidyl peptidase may comprise one or more amino acid sequence selected from SEQ ID No. 25 or a functional fragment thereof.

Described herein, the proline tolerant tripeptidyl peptidase may comprise an amino acid having at least 70% identity to SEQ ID No. 25 or a functional fragment thereof.

Described herein, the proline tolerant tripeptidyl peptidase may comprise one or more amino acid sequence selected from SEQ ID No. 26 or a functional fragment thereof.

Described herein, the proline tolerant tripeptidyl peptidase may comprise an amino acid having at least 70% identity to SEQ ID No. 26 or a functional fragment thereof.

Described herein, the proline tolerant tripeptidyl peptidase may comprise one or more amino acid sequence selected from SEQ ID No. 27 or a functional fragment thereof.

Described herein, the proline tolerant tripeptidyl peptidase may comprise an amino acid having at least 70% identity to SEQ ID No. 27 or a functional fragment thereof.

Described herein, the proline tolerant tripeptidyl peptidase may comprise one or more amino acid sequence selected from SEQ ID No. 28, or a functional fragment thereof.

Described herein, the proline tolerant tripeptidyl peptidase may comprise an amino acid having at least 70% identity to SEQ ID No. 28 or a functional fragment thereof.

Described herein, the proline tolerant tripeptidyl peptidase may comprise one or more amino acid sequence selected from SEQ ID No. 29, or a functional fragment thereof.

Described herein, the proline tolerant tripeptidyl peptidase may comprise an amino acid having at least 70% identity to SEQ ID No. 29 or a functional fragment thereof.

The proline tolerant tripeptidyl peptidase may comprise one or more amino acid sequence selected from SEQ ID No. 30 or a functional fragment thereof.

Described herein, the proline tolerant tripeptidyl peptidase may comprise an amino acid having at least 70% identity to SEQ ID No. 30 or a functional fragment thereof.

Described herein, the proline tolerant tripeptidyl peptidase may comprise one or more amino acid sequence selected from SEQ ID No. 31 or a functional fragment thereof.

Described herein, the proline tolerant tripeptidyl peptidase may comprise an amino acid having at least 70% identity to SEQ ID No. 31 or a functional fragment thereof.

Described herein, the proline tolerant tripeptidyl peptidase may comprise one or more amino acid sequence selected from SEQ ID No. 32 or a functional fragment thereof.

Described herein, the proline tolerant tripeptidyl peptidase may comprise an amino acid having at least 70% identity to SEQ ID No. 32 or a functional fragment thereof.

Described herein, the proline tolerant tripeptidyl peptidase may comprise one or more amino acid sequence selected from SEQ ID No.33 or a functional fragment thereof.

Described herein, the proline tolerant tripeptidyl peptidase may comprise an amino acid having at least 70% identity to SEQ ID No.33 or a functional fragment thereof.

Described herein, the proline tolerant tripeptidyl peptidase may comprise one or more amino acid sequence selected from SEQ ID No. 34 or a functional fragment thereof.

Described herein, the proline tolerant tripeptidyl peptidase may comprise an amino acid having at least 70% identity to SEQ ID No. 34 or a functional fragment thereof.

Described herein, the proline tolerant tripeptidyl peptidase may comprise one or more amino acid sequence selected from SEQ ID No. 35 or a functional fragment thereof.

Described herein, the proline tolerant tripeptidyl peptidase may comprise an amino acid having at least 70% identity to SEQ ID No. 35 or a functional fragment thereof.

Described herein, the proline tolerant tripeptidyl peptidase may comprise one or more amino acid sequence selected from SEQ ID No. 36 or a functional fragment thereof.

Described herein, the proline tolerant tripeptidyl peptidase may comprise an amino acid having at least 70% identity to SEQ ID No. 36 or a functional fragment thereof.

Described herein, the proline tolerant tripeptidyl peptidase may comprise one or more amino acid sequence selected from SEQ ID No. 37 or a functional fragment thereof.

Described herein, the proline tolerant tripeptidyl peptidase may comprise an amino acid having at least 70% identity to SEQ ID No. 37 or a functional fragment thereof.

Described herein, the proline tolerant tripeptidyl peptidase may comprise one or more amino acid sequence selected from SEQ ID No. 38 or a functional fragment thereof.

Described herein, the proline tolerant tripeptidyl peptidase may comprise an amino acid having at least 70% identity to SEQ ID No. 38 or a functional fragment thereof.

Described herein, the proline tolerant tripeptidyl peptidase may comprise one or more amino acid sequence selected from SEQ ID No. 39 or a functional fragment thereof.

Described herein, the proline tolerant tripeptidyl peptidase may comprise an amino acid having at least 70% identity to SEQ ID No. 39 or a functional fragment thereof.

Described herein, the proline tolerant tripeptidyl peptidase may comprise one or more amino acid sequence selected from SEQ ID No. 40 or a functional fragment thereof.

Described herein, the proline tolerant tripeptidyl peptidase may comprise an amino acid having at least 70% identity to SEQ ID No. 40 or a functional fragment thereof.

Described herein, the proline tolerant tripeptidyl peptidase may comprise one or more amino acid sequence selected from SEQ ID No. 41 or a functional fragment thereof.

Described herein, the proline tolerant tripeptidyl peptidase may comprise an amino acid having at least 70% identity to SEQ ID No. 41 or a functional fragment thereof.

Described herein, the proline tolerant tripeptidyl peptidase may comprise one or more amino acid sequence selected from SEQ ID No. 42 or a functional fragment thereof.

Described herein, the proline tolerant tripeptidyl peptidase may comprise an amino acid having at least 70% identity to SEQ ID No. 42 or a functional fragment thereof.

Described herein, the proline tolerant tripeptidyl peptidase may comprise one or more amino acid sequence selected from SEQ ID No. 43 or a functional fragment thereof.

Described herein, the proline tolerant tripeptidyl peptidase may comprise an amino acid having at least 70% identity to SEQ ID No. 43 or a functional fragment thereof.

Described herein, the proline tolerant tripeptidyl peptidase may comprise one or more amino acid sequence selected from SEQ ID No. 44 or a functional fragment thereof.

Described herein, the proline tolerant tripeptidyl peptidase may comprise an amino acid having at least 70% identity to SEQ ID No. 44 or a functional fragment thereof.

Described herein, the proline tolerant tripeptidyl peptidase may comprise one or more amino acid sequence selected from SEQ ID No. 45 or a functional fragment thereof.

Described herein, the proline tolerant tripeptidyl peptidase may comprise an amino acid having at least 70% identity to SEQ ID No. 45 or a functional fragment thereof.

Described herein, the proline tolerant tripeptidyl peptidase may comprise one or more amino acid sequence selected from SEQ ID No. 46 or a functional fragment thereof.

Described herein, the proline tolerant tripeptidyl peptidase may comprise an amino acid having at least 70% identity to SEQ ID No. 46 or a functional fragment thereof.

Described herein, the proline tolerant tripeptidyl peptidase may comprise one or more amino acid sequence selected from SEQ ID No. 47 or a functional fragment thereof.

Described herein, the proline tolerant tripeptidyl peptidase may comprise an amino acid having at least 70% identity to SEQ ID No. 47 or a functional fragment thereof.

Described herein, the proline tolerant tripeptidyl peptidase may comprise one or more amino acid sequence selected from SEQ ID No. 48 or a functional fragment thereof.

Described herein, the proline tolerant tripeptidyl peptidase may comprise an amino acid having at least 70% identity to SEQ ID No. 48 or a functional fragment thereof.

Described herein, the proline tolerant tripeptidyl peptidase may comprise one or more amino acid sequence selected from SEQ ID No. 49 or a functional fragment thereof.

Described herein, the proline tolerant tripeptidyl peptidase may comprise an amino acid having at least 70% identity to SEQ ID No. 49 or a functional fragment thereof.

Described herein, the proline tolerant tripeptidyl peptidase may comprise one or more amino acid sequence selected from SEQ ID No. 50 or a functional fragment thereof.

Described herein, the proline tolerant tripeptidyl peptidase may comprise an amino acid having at least 70% identity to SEQ ID No. 50 or a functional fragment thereof.

Described herein, the proline tolerant tripeptidyl peptidase may comprise one or more amino acid sequence selected from SEQ ID No. 51 or a functional fragment thereof.

Described herein, the proline tolerant tripeptidyl peptidase may comprise an amino acid having at least 70% identity to SEQ ID No. 51 or a functional fragment thereof.

Described herein, the proline tolerant tripeptidyl peptidase may comprise one or more amino acid sequence selected from SEQ ID No. 52 or a functional fragment thereof.

Described herein, the proline tolerant tripeptidyl peptidase may comprise an amino acid having at least 70% identity to SEQ ID No. 52 or a functional fragment thereof.

Described herein, the proline tolerant tripeptidyl peptidase may comprise one or more amino acid sequence selected from SEQ ID No. 53 or a functional fragment thereof.

Described herein, the proline tolerant tripeptidyl peptidase may comprise an amino acid having at least 70% identity to SEQ ID No. 53 or a functional fragment thereof.

Described herein, the proline tolerant tripeptidyl peptidase may comprise one or more amino acid sequence selected from SEQ ID No. 54 or a functional fragment thereof.

Described herein, the proline tolerant tripeptidyl peptidase may comprise an amino acid having at least 70% identity to SEQ ID No. 54 or a functional fragment thereof.

Described herein, the proline tolerant tripeptidyl peptidase may comprise one or more amino acid sequence selected from SEQ ID No. 55 or a functional fragment thereof.

Described herein, the proline tolerant tripeptidyl peptidase may comprise an amino acid having at least 70% identity to SEQ ID No. 55 or a functional fragment thereof.

Described herein, the proline tolerant tripeptidyl peptidase may comprise one or more amino acid sequence selected from SEQ ID No. 98 or a functional fragment thereof.

Described herein, the proline tolerant tripeptidyl peptidase may comprise an amino acid sequence having at least 70% identity to SEQ ID No. 98 or a functional fragment thereof.

Described herein, the proline tolerant tripeptidyl peptidase may comprise one or more amino acid sequence selected from SEQ ID No. 99 or a functional fragment thereof.

Described herein, the proline tolerant tripeptidyl peptidase may comprise an amino acid sequence having at least 70% identity to SEQ ID No. 99 or a functional fragment thereof.

Described herein, the proline tolerant tripeptidyl peptidase may comprise an amino acid sequence having at least 80% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No.
50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence having at least 85% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence having at least 90% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence having at least 95% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44,
SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence having at least 97% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence having at least 99% identity to SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof.

Described herein, the proline tolerant tripeptidyl peptidase may comprise an amino acid sequence selected from one more of the group consisting of: SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98 and SEQ ID No. 99.

Described herein, when used for food applications, the proline tolerant tripeptidyl peptidase may comprise an amino acid sequence selected from one more of the group consisting of: SEQ ID No. 1, SEQ ID No. 2, SEQ ID No. 3, SEQ ID No. 29 and SEQ ID No. 30, or a sequence having at least 70% identity thereto; more suitably a sequence having at least 80% thereto or at least 90% thereto.

Described herein, it may be suitable that the proline tolerant tripeptidyl peptidase may comprise an amino acid sequence selected from the group consisting of SEQ ID No. 3, SEQ ID No. 4, SEQ ID No. 30 and SEQ ID No. 31, or a sequence having at least 70% identity thereto. Suitably a sequence having at least 80% thereto or at least 90% thereto. Advantageously these particular amino acid sequences may be particularly suited to cleaving peptide and/or protein substrates enriched in lysine, arginine and/or glycine. Particularly where lysine, arginine and/or glycine are present at the P1 position.

Described herein, when used for feed applications, suitably, the proline tolerant tripeptidyl peptidase may comprise an amino acid sequence selected from one more of the group consisting of: SEQ ID No. 1, SEQ ID No. 2, SEQ ID No. 5, SEQ ID No. 6, SEQ ID No. 7, SEQ ID No. 29, SEQ ID No. 32, SEQ ID No. 33, SEQ ID No. 34, SEQ ID No. 98 and SEQ ID No. 99, or a sequence having at least 70% identity thereto. Suitably a sequence having at least 80% thereto or at least 90% thereto.

Described herein, the proline tolerant tripeptidyl peptidase may have the sequence SEQ ID No. 1, SEQ ID No. 2 or SEQ ID No. 29.

The proline tolerant tripeptidyl peptidase may comprise one or more of the sequence motifs selected from the group consisting of: xEANLD, y'Tzx'G and QNFSV.

Suitably, the proline tolerant tripeptidyl peptidase may comprise xEANLD.

x may be one or more amino acid selected from the group consisting of: G, T, S and V.

In another embodiment the proline tolerant tripeptidyl peptidase may comprise y'Tzx'G.

y' may be one or more amino acid selected from the group consisting of: I, L and V.

z may be one or more amino acid selected from the group consisting of: S and T.

x' may be one or more amino acid selected from the group consisting of: I and V.

In another embodiment the proline tolerant tripeptidyl peptidase may comprise the sequence motif QNFSV.

In a further embodiment the proline tolerant tripeptidyl peptidase may comprise the sequence motifs xEANLD and y'Tzx'G or xEANLD and QNFSV.

In a yet further embodiment the proline tolerant tripeptidyl peptidase may comprise the sequence motifs y'Tzx'G and QNFSV.

Suitably the proline tolerant tripeptidyl peptidase may comprise the sequence motifs xEANLD, y'Tzx'G and QNFSV.

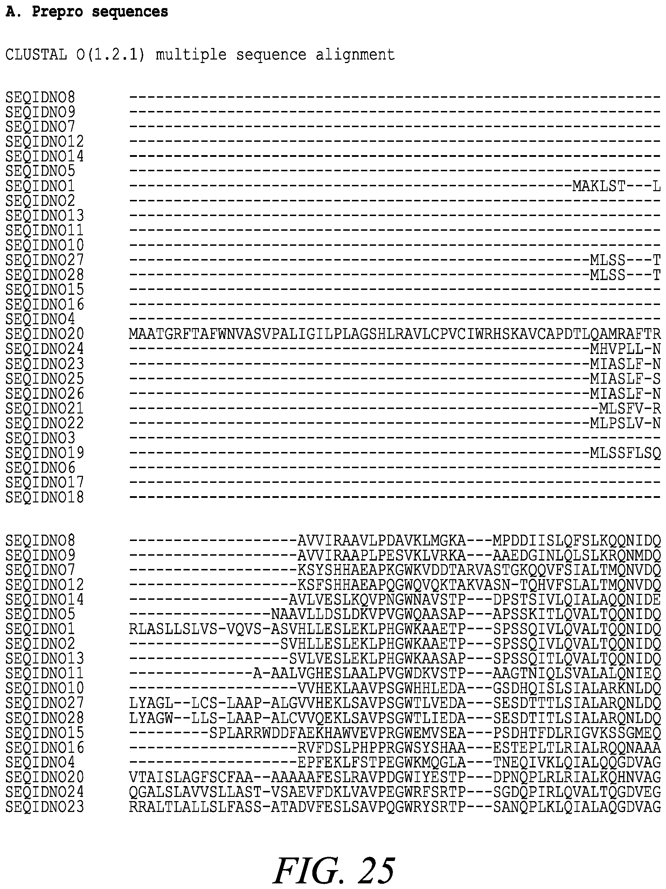

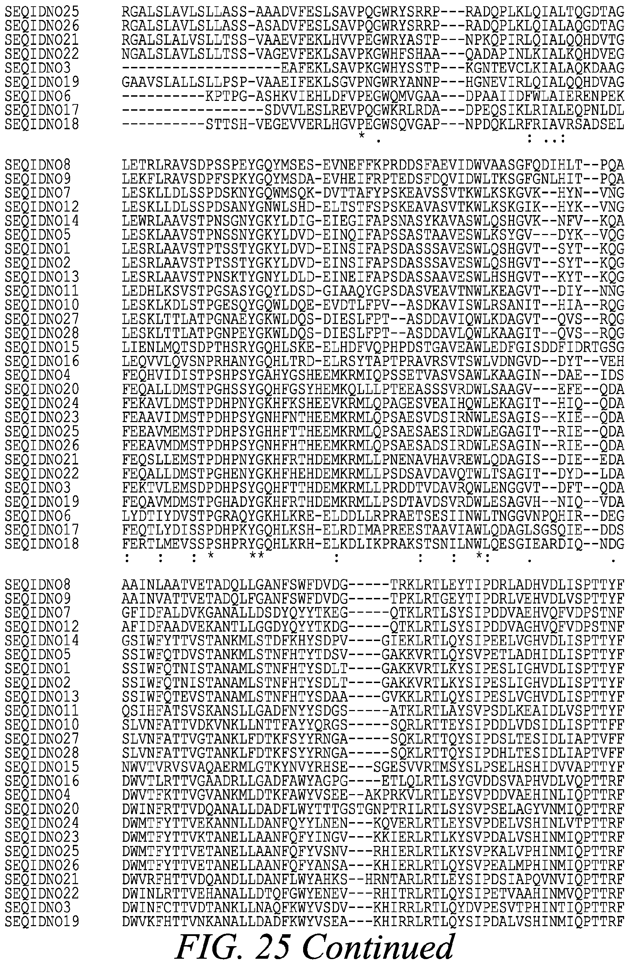

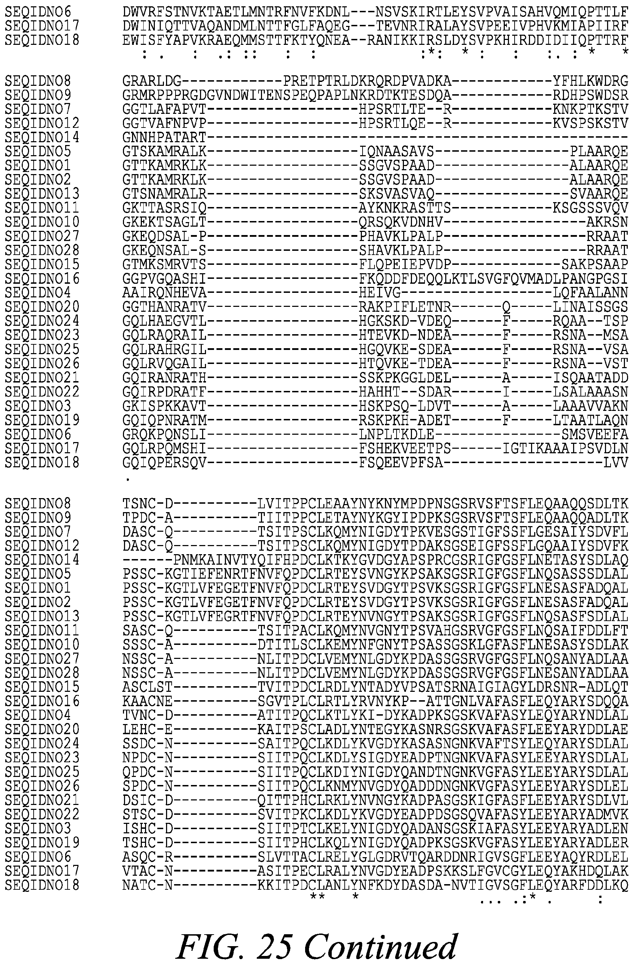

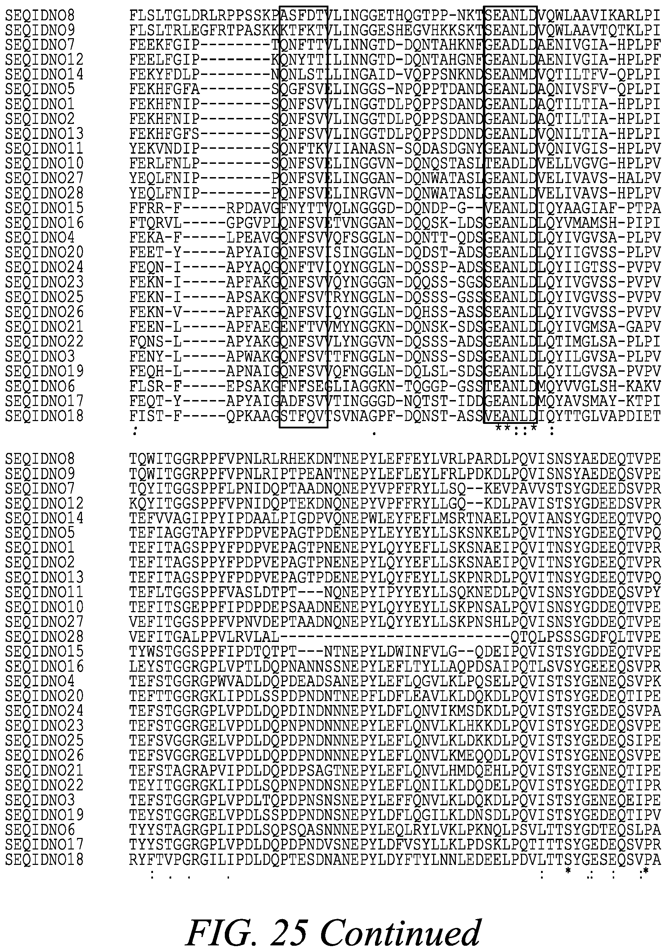

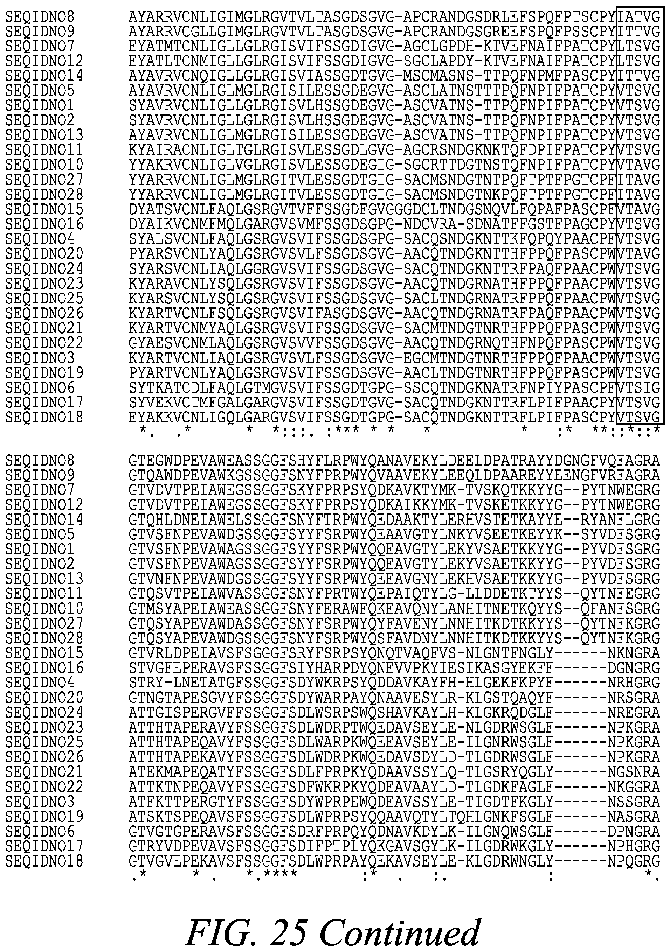

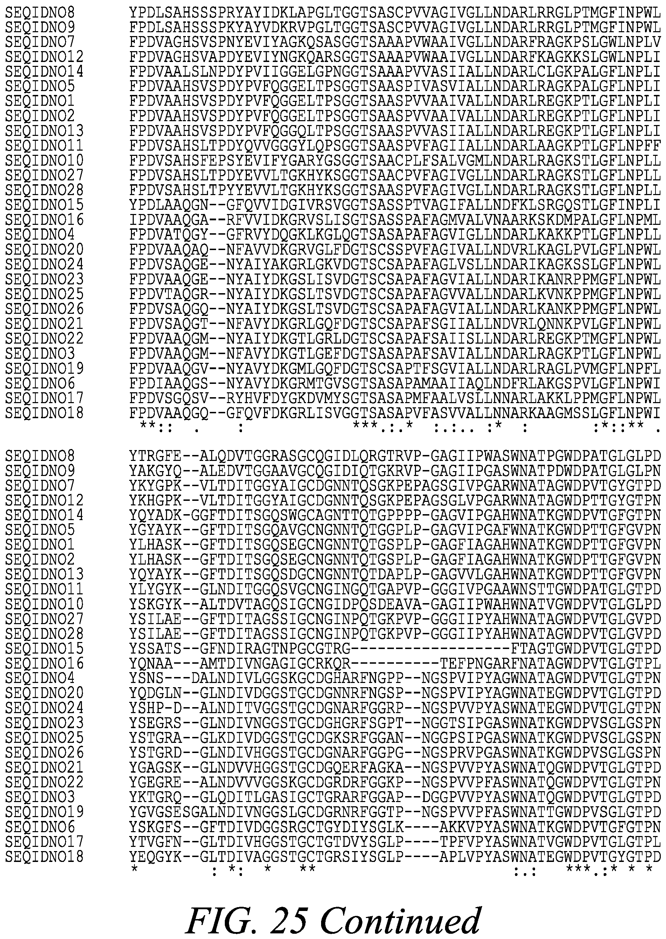

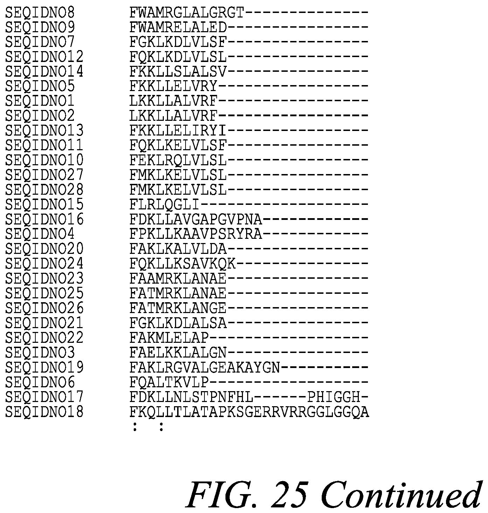

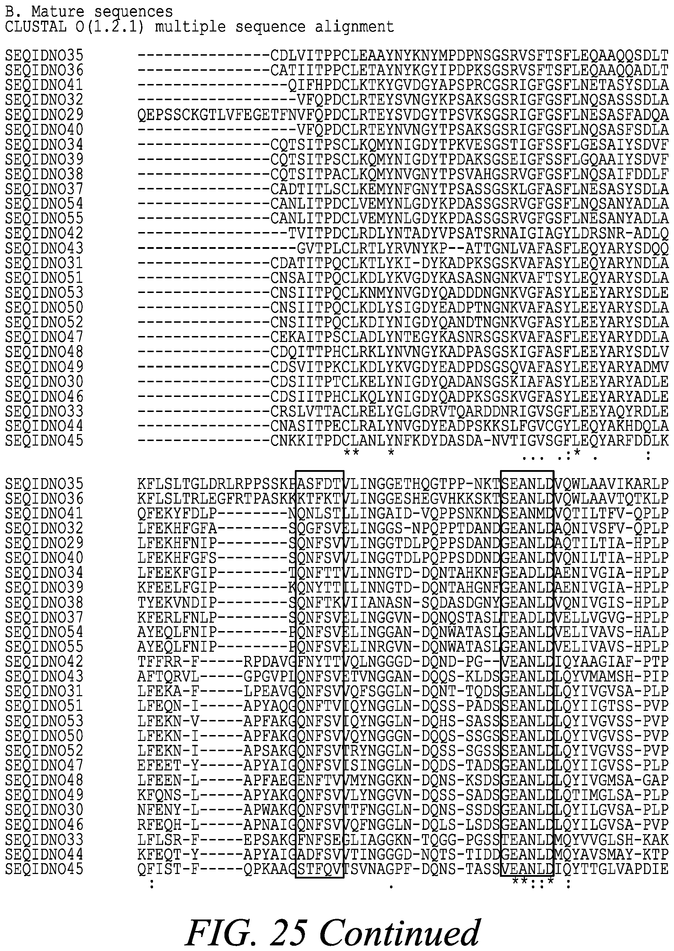

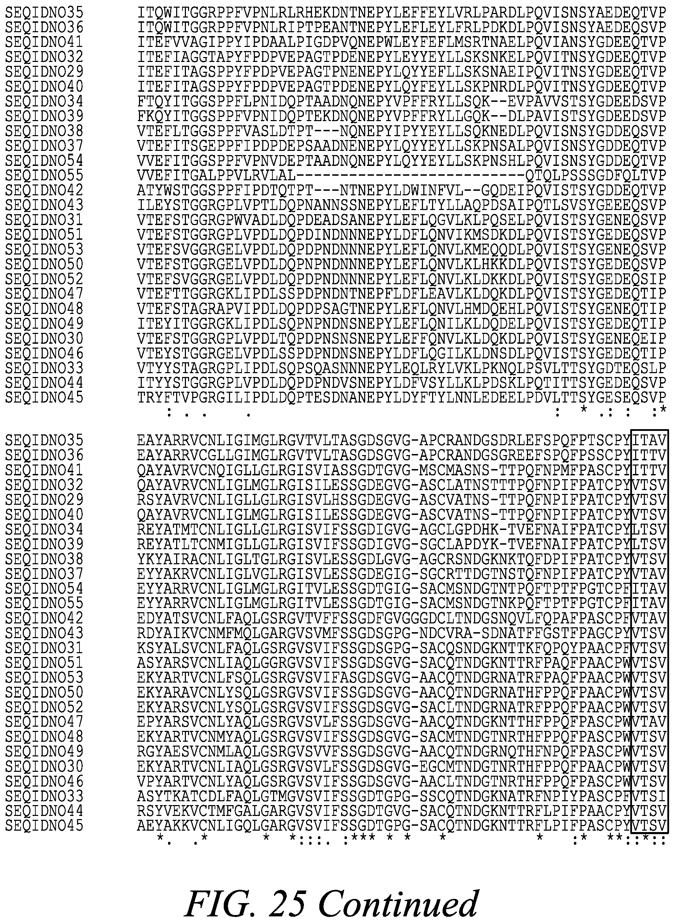

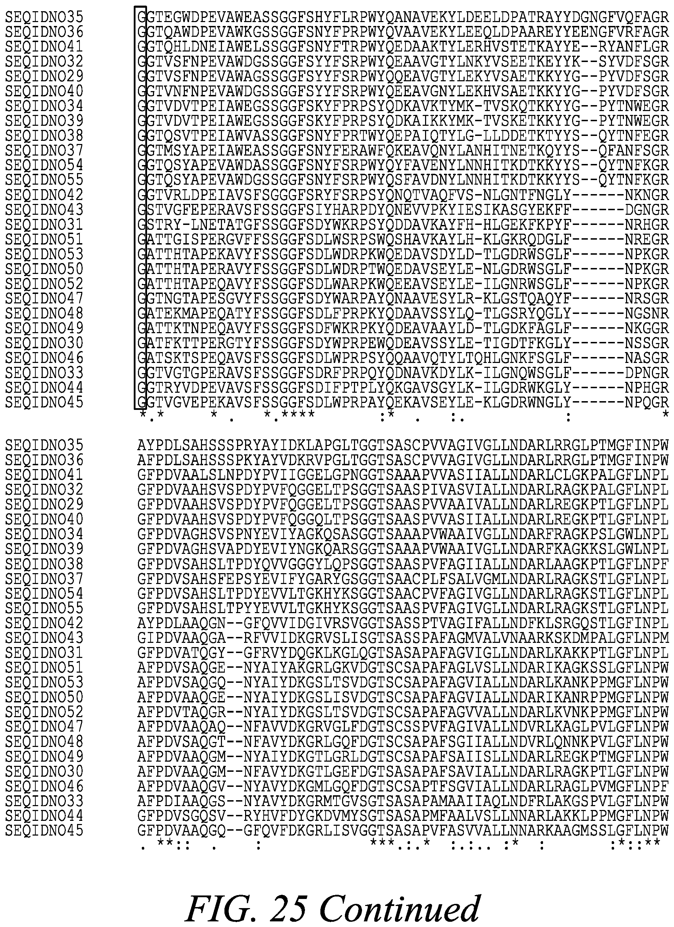

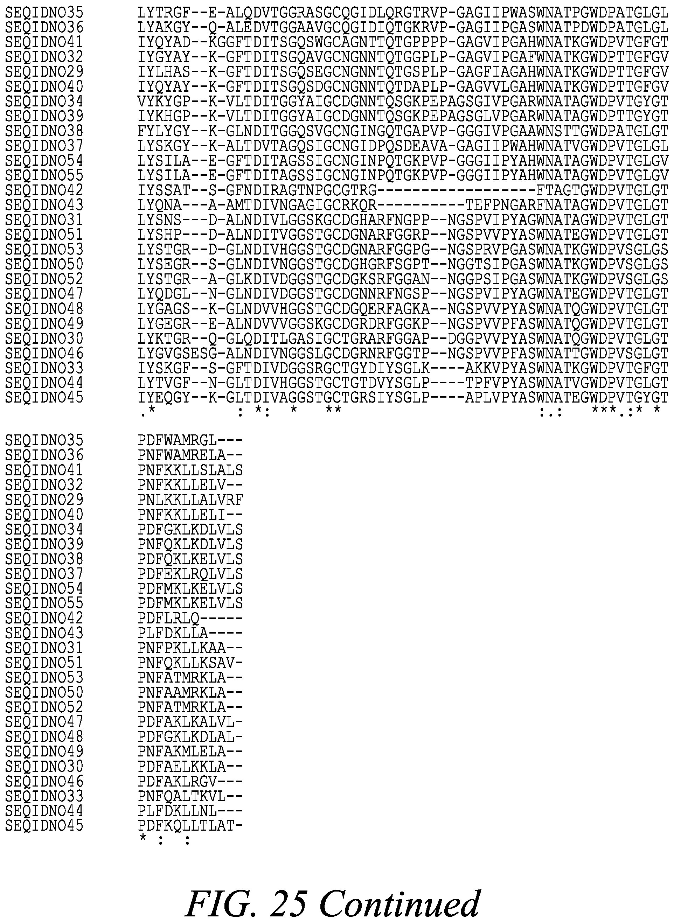

One or more of the motifs are present in the proline tolerant tripeptidyl peptidases for use in the present invention. Figure 25 indicates the positioning of these motifs.

Described herein, the proline tolerant tripeptidyl peptidase may be encoded by a nucleotide sequence shown as SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96, SEQ ID No. 97 or a nucleotide sequence having at least 70% identity thereto. Suitably a sequence having at least 80% thereto or at least 90% thereto.

Described herein, the proline tolerant tripeptidyl peptidase may be encoded by a nucleotide sequence having at least 95% sequence identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79 or SEQ ID No. 80, more preferably at least 99% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97.

Described herein, the proline tolerant tripeptidyl peptidase may be encoded by a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No.
96 or SEQ ID No. 97 under medium stringency conditions. Described herein, a nucleotide sequence which hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97 under high stringency conditions.

Described herein, the proline tolerant tripeptidyl peptidase may be encoded by a nucleotide sequence which differs from SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 96 or SEQ ID No. 97 due to degeneracy of the genetic code.

Described herein is an isolated nucleic acid comprising:
(a) a nucleotide sequence as shown herein as SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97;
(b) a nucleotide sequence which has at least about 70% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97; or
(c) a sequence that hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 under medium stringency conditions; or
(d) a nucleotide sequence which differs from
   SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 due to degeneracy of the genetic code.

Described herein, the nucleotide sequence may be a nucleotide sequence having at least about 80% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97, more suitably at least about 90% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97.

Described herein, may be a nucleotide sequence having at least about 95% identity, suitably at least about 99% identity to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97.

Described herein, the isolated nucleic acid may comprise a nucleotide sequence that hybridises to SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 under high stringency conditions. Described herein, the isolated nucleotide sequence comprising a nucleotide sequence shown as SEQ ID No. 56, SEQ ID No. 57, SEQ ID No. 58, SEQ ID No. 59, SEQ ID No. 60, SEQ ID No. 61, SEQ ID No. 62, SEQ ID No. 63, SEQ ID No. 64, SEQ ID No. 65, SEQ ID No. 66, SEQ ID No. 67, SEQ ID No. 68, SEQ ID No. 69, SEQ ID No. 70, SEQ ID No. 71, SEQ ID No. 72, SEQ ID No. 73, SEQ ID No. 74, SEQ ID No. 75, SEQ ID No. 76, SEQ ID No. 77, SEQ ID No. 78, SEQ ID No. 79, SEQ ID No. 80, SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 96 or SEQ ID No. 97 may be a DNA, cDNA, synthetic DNA and/or RNA sequence.

Preferably the sequence is a DNA sequence, more preferably a cDNA sequence coding for the proline tolerant tripeptidyl peptidase for use in the present invention. Described herein is an isolated nucleic acid comprising:
(a) a nucleotide sequence as shown herein as SEQ ID No. 56
(b) a nucleotide sequence which has at least about 70% identity to SEQ ID No. 56; or
(c) a sequence that hybridises to SEQ ID No. 56 under medium stringency conditions; or
(c) a nucleotide sequence which differs from SEQ ID No. 56 due to degeneracy of the genetic code.

Described herein, the nucleotide sequence may be a nucleotide sequence having at least about 80% identity to SEQ ID No. 56, more suitably at least about 90% identity to SEQ ID No. 56.

Described herein, the nucleotide sequence may be a nucleotide sequence having at least bout 95% identity, suitably at least about 99% identity to SEQ ID No. 56.

Described herein, the isolated nucleic acid may comprise a nucleotide sequence that hybridises to SEQ ID No. 56 under high stringency conditions.

Described herein, the isolated nucleic acid may be comprised in a vector (e.g. a plasmid). Described herein is a host cell comprising an isolated nucleic acid sequence or vector according to the invention.

Described herein, the host cell may be a *Trichoderma* host cell, preferably a *Trichoderma reesei* host cell.

In one aspect, preferably the amino acid and/or nucleotide sequence for use in the present invention is in an isolated form. The term "isolated" means that the sequence is at least substantially free from at least one other component with which the sequence is naturally associated in nature and as found in nature. The amino acid and/or nucleotide sequence for use in the present invention may be provided in a form that is substantially free of one or more contaminants with which the substance might otherwise be associated. Thus, for example it may be substantially free of one or more potentially contaminating polypeptides and/or nucleic acid molecules.

In one aspect, preferably the amino acid and/or nucleotide sequence for use in the present invention is in a purified form. The term "purified" means that a given component is present at a high level. The component is desirably the predominant component present in a composition. Preferably, it is present at a level of at least about 90%, or at least about 95% or at least about 98%, said level being determined on a dry weight/dry weight basis with respect to the total composition under consideration.

### ENZYMES

Described herein, the tripeptidyl peptidase or exo-tripeptidyl peptidase of the S53 family may have be selected from any of the enzyme sequences selected below.

Described herein, the enzyme may be a tripeptidyl peptidase comprising SEQ ID No. 1, a functional fragment thereof or a sequence having at least 70% identity to SEQ ID No. 1. Suitably the enzyme may have at least 80% or 85% identity thereto. Preferably at least 90% or 95% identity thereto. More preferably at least 97% or 99% identity thereto.

Described herein, the may be a tripeptidyl peptidase comprising SEQ ID No. 2, a functional fragment thereof or a sequence having at least 70% identity to SEQ ID No. 2. Suitably the enzyme may have at least 80% or 85% identity thereto. Preferably at least 90% or 95% identity thereto. More preferably at least 97% or 99% identity thereto.

In one embodiment the enzyme for use in the present invention may be a tripeptidyl peptidase comprising SEQ ID No. 3, a functional fragment thereof or a sequence having at least 80% identity to SEQ ID No. 3. Suitably the enzyme may have at least 85% identity thereto. Preferably at least 90% or 95% identity thereto. More preferably at least 97% or 99% identity thereto.

Described herein, the enzyme may be a tripeptidyl peptidase comprising SEQ ID No. 4, a functional fragment thereof or a sequence having at least 70% identity to SEQ ID No. 4. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 5, a functional fragment thereof or a sequence having at least 70% identity to SEQ ID No. 5. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 6, a functional fragment thereof or a sequence having at least 70% identity to SEQ ID No. 6. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 7, a functional fragment thereof or a sequence having at least 70% identity to SEQ ID No. 7. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 8, a functional fragment thereof or a sequence having at least 70% identity to SEQ ID No. 8. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 9, a functional fragment thereof or a sequence having at least 70% identity to SEQ ID No. 9. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 10, a functional fragment thereof or a sequence having at least 70% identity to SEQ ID No. 10. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 11, a functional fragment thereof or a sequence having at least 70% identity to SEQ ID No. 11. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 12, a functional fragment thereof or a sequence having at least 70% identity to SEQ ID No. 12. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 13, a functional fragment thereof or a sequence having at least 70% identity to SEQ ID No. 13. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 14, a functional fragment thereof or a sequence having at least 70% identity to SEQ ID No. 14. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 15, a functional fragment thereof or a sequence having at least 70% identity to SEQ ID No. 15. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 16, a functional fragment thereof or a sequence having at least 70% identity to SEQ ID No. 16. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 17, a functional fragment thereof or a sequence having at least 70% identity to SEQ ID No. 17. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 18, a functional fragment thereof or a sequence having at least 70% identity to SEQ ID No. 18. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 19, a functional fragment thereof or a sequence having at least 70% identity to SEQ ID No. 19. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 20, a functional fragment thereof or a sequence having at least 70% identity to SEQ ID No. 20. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 21, a functional fragment thereof or a sequence having at least 70% identity to SEQ ID No. 21. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 22, a functional fragment thereof or a sequence having at least 70% identity to SEQ ID No. 22. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 23, a functional fragment thereof or a sequence having at least 70% identity to SEQ ID No. 23. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 24, a functional fragment thereof or a sequence having at least 70% identity to SEQ ID No. 24. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 25, a functional fragment thereof or a sequence having at least 70% identity to SEQ ID No. 25. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 26, a functional fragment thereof or a sequence having at least 70% identity to SEQ ID No. 26. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 27, a functional fragment thereof or a sequence having at least 70% identity to SEQ ID No. 27. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 28, a functional fragment thereof or a sequence having at least 70% identity to SEQ ID No. 28. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 29, a functional fragment thereof or a sequence having at least 70% identity to SEQ ID No. 29. Suitably the enzyme may have at least 80% or 85% identity thereto. Preferably at least 90% or 95% identity thereto. More preferably at least 97% or 99% identity thereto.

In one embodiment the enzyme for use in the present invention may be a tripeptidyl peptidase comprising SEQ ID No. 30, a functional fragment thereof or a sequence having at least 80% identity to SEQ ID No. 30. Suitably the enzyme may have at least 85% identity thereto. Preferably at least 90% or 95% identity thereto. More preferably at least 97% or 99% identity thereto.

Described herein, the enzyme may be a tripeptidyl peptidase comprising SEQ ID No. 31, a functional fragment thereof or a sequence having at least 70% identity to SEQ ID No. 31. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 32, a functional fragment thereof or a sequence having at least 70% identity to SEQ ID No. 32. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 33, a functional fragment thereof or a sequence having at least 70% identity to SEQ ID No. 33. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 34, a functional fragment thereof or a sequence having at least 70% identity to SEQ ID No. 34. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 35, a functional fragment thereof or a sequence having at least 70% identity to SEQ ID No. 35. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 36, a functional fragment thereof or a sequence having at least 70% identity to SEQ ID No. 36. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 37, a functional fragment thereof or a sequence having at least 70% identity to SEQ ID No. 37. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 38, a functional fragment thereof or a sequence having at least 70% identity to SEQ ID No. 38. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 39, a functional fragment thereof or a sequence having at least 70% identity to SEQ ID No. 39. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 40, a functional fragment thereof or a sequence having at least 70% identity to SEQ ID No. 40. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 41, a functional fragment thereof or a sequence having at least 70% identity to SEQ ID No. 41. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 42, a functional fragment thereof or a sequence having at least 70% identity to SEQ ID No. 42. Suitably the enzyme may have at least 80% or 85% identity thereto. Preferably at least 90% or 95% identity thereto.

Described herein, the enzyme may be a tripeptidyl peptidase comprising SEQ ID No. 43, a functional fragment thereof or a sequence having at least 70% identity to SEQ ID No. 43. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 44, a functional fragment thereof or a sequence having at least 70% identity to SEQ ID No. 44. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 45, a functional fragment thereof or a sequence having at least 70% identity to SEQ ID No. 45. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 46, a functional fragment thereof or a sequence having at least 70% identity to SEQ ID No. 46. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 47, a functional fragment thereof or a sequence having at least 70% identity to SEQ ID No. 47. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 48, a functional fragment thereof or a sequence having at least 70% identity to SEQ ID No. 48. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 49, a functional fragment thereof or a sequence having at least 70% identity to SEQ ID No. 49. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 50, a functional fragment thereof or a sequence having at least 70% identity to SEQ ID No. 50. Suitably the enzyme may have at least 80% or 85% identity thereto. Preferably at least 90% or 95% identity thereto.

Described herein, the enzyme may be a tripeptidyl peptidase comprising SEQ ID No. 51, a functional fragment thereof or a sequence having at least 70% identity to SEQ ID No. 51. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 52, a functional fragment thereof or a sequence having at least 70% identity to SEQ ID No. 52. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 53, a functional fragment thereof or a sequence having at least 70% identity to SEQ ID No. 53. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 54, a functional fragment thereof or a sequence having at least 70% identity to SEQ ID No. 54. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 55, a functional fragment thereof or a sequence having at least 70% identity to SEQ ID No. 55. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 98, a functional fragment thereof or a sequence having at least 70% identity to SEQ ID No. 98. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase comprising SEQ ID No. 99, a functional fragment thereof or a sequence having at least 70% identity to SEQ ID No. 99. Suitably the enzyme may have at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 56 or a sequence having at least 70% identity thereto. Suitably by a sequence having at least 80% or 85% identity thereto. Preferably at least 90% or 95% identity thereto. More preferably at least 97% or 99% identity thereto.

In one embodiment the enzyme for use in the present invention may be a tripeptidyl peptidase encoded by a nucleotide sequence comprising the sequence shown as SEQ ID No. 57 or a sequence having at least 80% identity thereto. Suitably by a sequence having at least 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 58 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 59 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 60 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 61 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme for may be a tripeptidyl peptidase encoded by a nucleotide sequence comprising the sequence shown as SEQ ID No. 62 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 63 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 64 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 65 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 66 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 67 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 68 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 69 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 70 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 71 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 72 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 73 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 74 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 75 or a sequence having at least 70% identity thereto.

Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 76 or a sequence having at least 70% identity thereto.

Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 77 or a sequence having at least 70% identity thereto.

Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 78 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 79 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 80 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 81 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 82 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 83 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 84 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 85 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 86 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 87 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 88 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 89 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 90 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 91 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 92 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 93 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 94 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 95 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 96 or a sequence having at least 70% identity thereto. Suitably by a sequence having at least 80% or 85% identity thereto. Preferably at least 90% or 95% identity thereto. More preferably at least 97% or 99% identity thereto.

Described herein, the enzyme may be a tripeptidyl peptidase encoded by a nucleotide sequence comprising the sequence shown as SEQ ID No. 97 or a sequence having at least 70% identity thereto. Suitably by a sequence having at least 80% or 85% identity thereto. Preferably at least 90% or 95% identity thereto. More preferably at least 97% or 99% identity thereto.

### NUCLEOTIDE SEQUENCE

Disclosed herein is the use of nucleotide sequences encoding proteins having the specific properties as defined herein.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. Preferably it means DNA, more preferably cDNA sequence coding for the present invention.

Preferably the nucleotide sequence does not include the native nucleotide sequence when in its natural environment and when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence can be isolated and/or purified post expression of a nucleotide sequence in its native organism. Preferably, however, the amino acid sequence may be expressed by a nucleotide sequence in its native organism but wherein the nucleotide sequence is not under the control of the promoter with which it is naturally associated within that organism.

Typically, the nucleotide sequence is prepared using recombinant DNA techniques (i.e. recombinant DNA). However, in an alternative embodiment of the invention, the nucleotide sequence could be synthesised, in whole or in part, using chemical methods well known in the art (see Caruthers MH et al., (1980) Nuc Acids Res Symp Ser 215-23 and Horn T et al., (1980) Nuc Acids Res Symp Ser 225-232).

### PREPARATION OF THE NUCLEOTIDE SEQUENCE

A nucleotide sequence encoding either a protein which has the specific properties as defined herein or a protein which is suitable for modification may be identified and/or isolated and/or purified from any cell or organism producing said protein. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used. Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar plates containing a substrate for enzyme (i.e. maltose), thereby allowing clones expressing the enzyme to be identified.

In a yet further alternative, the nucleotide sequence encoding the enzyme may be prepared synthetically by established standard methods, e.g. the phosphoroamidite method described by Beucage S.L. etal., (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K etal., (Science (1988) 239, pp 487-491).

### AMINO ACID SEQUENCES

Also disclosed herein is the use of amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The protein may be used in conjunction with other proteins, particularly enzymes. Thus the present disclosure also covers a combination of proteins wherein the combination comprises the protein/enzyme of the present disclosure and another protein/enzyme, which may be another protein/enzyme according to the present disclosure. This aspect is discussed in a later section.

Preferably the amino acid sequence is not a native enzyme. In this regard, the term "native enzyme" means an entire enzyme that is in its native environment and when it has been expressed by its native nucleotide sequence.

### ISOLATED

In one aspect, preferably the amino acid sequence, or nucleic acid, or enzyme for use according to the present invention is in an isolated form. The term "isolated" means that the sequence or enzyme or nucleic acid is at least substantially free from at least one other component with which the sequence, enzyme or nucleic acid is naturally associated in nature and as found in nature. The sequence, enzyme or nucleic acid for use according to the present invention may be provided in a form that is substantially free of one or more contaminants with which the substance might otherwise be associated. Thus, for example it may be substantially free of one or more potentially contaminating polypeptides and/or nucleic acid molecules.

### PURIFIED

In one aspect, preferably the sequence, enzyme or nucleic acid for use according to the present invention is in a purified form. The term "purified" means that the given component is present at a high level. The component is desirably the predominant component present in a composition. Preferably, it is present at a level of at least about 80% said level being determined on a dry weight/dry weight basis with respect to the total composition under consideration. Suitably it may be present at a level of at least about 90%, or at least about 95, or at least about 98% said level being determined on a dry weight/dry weight basis with respect to the total composition under consideration.

### SEQUENCE IDENTITY OR SEQUENCE HOMOLOGY

The present disclosure also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid or a nucleotide sequence which may be at least 85 or 90% identical, preferably at least 95 or 98% identical to the subject sequence. Typically, the homologues will comprise the same active sites etc. as the subject amino acid sequence for instance. Although homology can also be considered in terms of similarity (i.e. amino acid residues having similar chemical properties/functions), in the context of the present invention it is preferred to express homology in terms of sequence identity.

In one embodiment, a homologous sequence is taken to include an amino acid sequence or nucleotide sequence which has one or several additions, deletions and/or substitutions compared with the subject sequence.

In one aspect the present disclosure relates to a protein whose amino acid sequence is represented herein or a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

Suitably, the degree of identity with regard to an amino acid sequence is determined over at least 20 contiguous amino acids, preferably over at least 30 contiguous amino acids, preferably over at least 40 contiguous amino acids, preferably over at least 50 contiguous amino acids, preferably over at least 60 contiguous amino acids, preferably over at least 100 contiguous amino acids, preferably over at least 200 contiguous amino acids.

In one aspect the present disclosure relates to a nucleic acid sequence (or gene) encoding a protein whose amino acid sequence is represented herein or encoding a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In the present context, a homologous sequence is taken to include a nucleotide sequence which may be at least 85 or 90% identical, preferably at least 95 or 98% identical to a nucleotide sequence encoding a polypeptide of the present disclosure (the subject sequence).

Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. Calculation of maximum % homology or % identity therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov), FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60), such as for example in the GenomeQuest search tool (www.genomequest.com).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| FOR BLAST | |
|---|---|
| GAP OPEN | 9 |
| GAP EXTENSION | 2 |

| FOR CLUSTAL | DNA | PROTEIN |
|---|---|---|
| Weight Matrix | IUB | Gonnet 250 |
| GAP OPENING | 15 | 10 |
| GAP EXTEND | 6.66 | 0.1 |

In one embodiment, CLUSTAL may be used with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence is determined over at least 100 contiguous nucleotides, preferably over at least 200 contiguous nucleotides, preferably over at least 300 contiguous nucleotides, preferably over at least 400 contiguous nucleotides, preferably over at least 500 contiguous nucleotides, preferably over at least 600 contiguous nucleotides, preferably over at least 700 contiguous nucleotides, preferably over at least 800 contiguous nucleotides.

Suitably, the degree of identity with regard to a nucleotide sequence may be determined over the whole sequence.

Suitably, the degree of identity with regard to a protein (amino acid) sequence is determined over at least 100 contiguous amino acids, preferably over at least 200 contiguous amino acids, preferably over at least 300 contiguous amino acids.

Suitably, the degree of identity with regard to an amino acid or protein sequence may be determined over the whole sequence taught herein.

In the present context, the term "query sequence" means a homologous sequence or a foreign sequence, which is aligned with a subject sequence in order to see if it falls within the scope of the present invention. Accordingly, such query sequence can for example be a prior art sequence or a third party sequence.

In one preferred embodiment, the sequences are aligned by a global alignment program and the sequence identity is calculated by identifying the number of exact matches identified by the program divided by the length of the subject sequence.

In one embodiment, the degree of sequence identity between a query sequence and a subject sequence is determined by 1) aligning the two sequences by any suitable alignment program using the default scoring matrix and default gap penalty, 2) identifying the number of exact matches, where an exact match is where the alignment program has identified an identical amino acid or nucleotide in the two aligned sequences on a given position in the alignment and 3) dividing the number of exact matches with the length of the subject sequence.

In yet a further preferred embodiment, the global alignment program is selected from the group consisting of CLUSTAL and BLAST (preferably BLAST) and the sequence identity is calculated by identifying the number of exact matches identified by the program divided by the length of the subject sequence.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | GAP |
| | | I L V |
| | Polar - uncharged | C S T M |
| | | N Q |
| | Polar - charged | D E |
| | | K R |
| AROMATIC | | H F W Y |

The present disclosure also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by synthetic amino acids (e.g. unnatural amino acids) include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. It is to be understood that the nucleotide sequences described herein may be modified by any method available in the art. Such modifications may be carried out in order to enhance the *in vivo* activity or life span of nucleotide sequences of the present disclosure.

The present disclosure also encompasses the use of nucleotide sequences that are complementary to the sequences presented herein, or any derivative, fragment or derivative thereof. If the sequence is complementary to a fragment thereof then that sequence can be used as a probe to identify similar coding sequences in other organisms etc. Polynucleotides which are not 100% homologous to the sequences of the present disclosure can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the disclosure.

Variants and strain/species homologues may also be obtained using degenerate PCR which will use primers designed to target sequences within the variants and homologues encoding conserved amino acid sequences within the sequences of the present disclosure. Conserved sequences can be predicted, for example, by aligning the amino acid sequences from several variants/homologues. Sequence alignments can be performed using computer software known in the art. For example the GCG Wisconsin PileUp program is widely used. The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) for use according to the invention may be used to produce a primer, e.g. a PCR primer, a primer for an alternative amplification reaction, a probe e.g. labelled with a revealing label by conventional means using radioactive or nonradioactive labels, or the polynucleotides may be cloned into vectors. Such primers, probes and other fragments will be at least 15, preferably at least 20, for example at least 25, 30 or 40 nucleotides in length, and are also encompassed by the term polynucleotides as used herein.

Polynucleotides such as DNA polynucleotides and probes may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

### HYBRIDISATION

The present disclosure also encompasses the use of sequences that are complementary to the nucleic acid sequences as described herein or sequences that are capable of hybridising either to the sequences as described herein or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the sequences presented herein, or any derivative, fragment or derivative thereof.

The term "variant" also encompasses sequences that are complementary to sequences that are capable of hybridising to the nucleotide sequences presented herein.

Preferably, the term "variant" encompasses sequences that are complementary to sequences that are capable of hybridising under medium stringency conditions (e.g. 50°C and 0.2xSSC {1xSSC = 0.15 M NaCl, 0.015 M Na₃citrate pH 7.0}) to the nucleotide sequences presented herein.

More preferably, the term "variant" encompasses sequences that are complementary to sequences that are capable of hybridising under high stringency conditions (e.g. 65°C and 0.1xSSC {1xSSC = 0.15 M NaCl, 0.015 M Na₃citrate pH 7.0}) to the nucleotide sequences presented herein.

The present disclosure also relates to the use of nucleotide sequences that can hybridise to the nucleotide sequences as described herein (including complementary sequences of those presented herein).

The present disclosure also relates to the use of nucleotide sequences that are complementary to sequences that can hybridise to the nucleotide sequences as described herein (including complementary sequences of those presented herein).

Also disclosed herein is the use of polynucleotide sequences that are capable of hybridising to the nucleotide sequences presented herein under conditions of intermediate to maximal stringency.

In a preferred aspect, the nucleotide sequences can hybridise to the nucleotide sequenceas described herein, or the complement thereof, under medium stringency conditions (e.g. 50°C and 0.2xSSC {1xSSC = 0.15 M NaCl, 0.015 M Na₃citrate pH 7.0}).

In a more preferred aspect, the nucleotide sequences can hybridise to the nucleotide sequenceas described herein, or the complement thereof, under high stringent conditions (e.g. 65°C and 0.1xSSC {1xSSC = 0.15 M NaCl, 0.015 M Na₃citrate pH 7.0}).

Preferably hybridisation is analysed over the whole of the sequences taught herein.

### MOLECULAR EVOLUTION

As a non-limiting example, it is possible to produce numerous site directed or random mutations into a nucleotide sequence, either in vivo or in vitro, and to subsequently screen for improved functionality of the encoded polypeptide by various means.

In addition, mutations or natural variants of a polynucleotide sequence can be recombined with either the wildtype or other mutations or natural variants to produce new variants. Such new variants can also be screened for improved functionality of the encoded polypeptide. The production of new preferred variants can be achieved by various methods well established in the art, for example the Error Threshold Mutagenesis (WO 92/18645), oligonucleotide mediated random mutagenesis (US 5,723, 323), DNA shuffling (US 5,605,793), exo-mediated gene assembly WO00/58517. The application of these and similar random directed molecular evolution methods allows the identification and selection of variants of the enzymes disclosed herein which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate.

### SITE-DIRECTED MUTAGENESIS

Once a protein-encoding nucleotide sequence has been isolated, or a putative protein-encoding nucleotide sequence has been identified, it may be desirable to mutate the sequence in order to prepare a protein of the present disclosure.

Mutations may be introduced using synthetic oligonucleotides. These oligonucleotides contain nucleotide sequences flanking the desired mutation sites.

A suitable method is disclosed in Morinaga et al., (Biotechnology (1984) 2, p646-649). Another method of introducing mutations into enzyme-encoding nucleotide sequences is described in Nelson and Long (Analytical Biochemistry (1989), 180, p 147-151).

### RECOMBINANT

In one aspect the sequence for use in the present invention is a recombinant sequence - i.e. a sequence that has been prepared using recombinant DNA techniques.

These recombinant DNA techniques are within the capabilities of a person of ordinary skill in the art. Such techniques are explained in the literature, for example, J. Sambrook, E. F. Fritsch, and T. Maniatis, 1989, Molecular Cloning: A Laboratory Manual, Second Edition, Books 1-3, Cold Spring Harbor Laboratory Press.

### SYNTHETIC

In one aspect the sequence for use in the present invention is a synthetic sequence - i.e. a sequence that has been prepared by *in vitro* chemical or enzymatic synthesis. It includes, but is not limited to, sequences made with optimal codon usage for host organisms - such as the methylotrophic yeasts *Pichia* and *Hansenula.*

Proteins and/or peptides for use in the present invention may also be of a synthetic origin.

### EXPRESSION OF ENZYMES

Described herein is a method for the expression of a proline tolerant tripeptidyl peptidase predominantly having exopeptidase activity wherein said proline tolerant tripeptidyl peptidase is capable of cleaving tri-peptides from the N-terminus of peptides having the consensus sequence cleavage sites:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1':
   said method comprising:
   (a) transforming a host cell with a nucleic acid or a vector comprising a nucleotide sequence encoding a proline tolerant tripeptidyl peptidase;
   (b) expressing the nucleic acid sequence or vector of step (a); and
   (c) obtaining the proline tolerant tripeptidyl peptidase or a fermentate comprising said proline tolerant tripeptidyl peptidase.

Described herein, the method may further comprise isolating and/or purifying and/or packaging the proline tolerant tripeptidyl peptidase.

The nucleic acid may be any nucleic acid encoding a proline tolerant tripeptidyl peptidase having the activity detailed herein. Suitably, the nucleic acid may be any one of the nucleic acids detailed herein. Suitably, the nucleic acid may be an isolated nucleic acid of the invention.

As used herein the term "fermentate" refers to the mixture of constituents present following (e.g. at the end of) the culturing of the host cell which includes the tripeptidyl peptidase (e.g. proline tolerant tripeptidyl peptidase) and/or exo-tripeptidyl peptidase of the S53 family. The fermentate may comprises as well as the tripeptidyl peptidase in accordance with the present disclosure other components such as particulate matter, solids, substrates not utilised during culturing, debris, media, cell waste, etc. In one aspect, bacterial cells (and, preferably, spores) are removed from the fermentate and/or inactivated to provide a cell-free fermentate.

The nucleotide sequence for use in the present invention may be incorporated into a recombinant replicable vector. The vector may be used to replicate and express the nucleotide sequence, in protein/enzyme form, in and/or from a compatible host cell.

Expression may be controlled using control sequences e.g. regulatory sequences.

The protein produced by a host recombinant cell by expression of the nucleotide sequence may be secreted or may be contained intracellularly depending on the sequence and/or the vector used. The coding sequences may be designed with signal sequences which direct secretion of the substance coding sequences through a particular prokaryotic or eukaryotic cell membrane.

The term "expression vector" means a construct capable of *in vivo* or *in vitro* expression.

In one embodiment the proline tolerant tripeptidyl peptidase and/or endoprotease for use in the present invention may be encoded by a vector. In other words the vector may comprise a nucleotide sequence encoding the proline tolerant tripeptidyl peptidase.

Preferably, the expression vector is incorporated into the genome of a suitable host organism. The term "incorporated" preferably covers stable incorporation into the genome. The nucleotide sequence for use in the present invention may be present in a vector in which the nucleotide sequence is operably linked to regulatory sequences capable of providing for the expression of the nucleotide sequence by a suitable host organism.

The vectors for use in the present invention may be transformed into a suitable host cell as described below to provide for expression of a polypeptide for use in the present invention.

The choice of vector e.g. a plasmid, cosmid, or phage vector will often depend on the host cell into which it is to be introduced.

The vectors for use in the present invention may contain one or more selectable marker genes- such as a gene, which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect, transform, transduce or infect a host cell.

Thus, described herein is a method of making nucleotide sequences for use in the present invention by introducing a nucleotide sequence of the present invention into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

The nucleotide sequence and/or vector encoding the proline tolerant tripeptidyl peptidase and/or the endoprotease may be codon optimised for expression in a particular host organism.

The nucleotide sequence and/or vector encoding the proline tolerant tripeptidyl peptidase and/or the endoprotease may be codon optimised for expression in a prokaryotic or eukaryotic cell. Suitably, the nucleotide sequence and/or vector encoding the proline tolerant tripeptidyl peptidase and/or the endoprotease may be codon optimised for expression in a fungal host organism (e.g. *Trichoderma,* preferably *Trichoderma reesei*).

Codon optimisation refers to a process of modifying a nucleic acid sequence for enhanced expression in a host cell of interest by replacing at least one codon (e.g. at least about more than 1, 2, 3, 4, 5, 10, 15, 20, 25, 50, 60, 70, 80 or 100 codons) of the native sequence with codons that are more frequently used in the genes of the host cell, whilst maintaining the native amino acid sequence. Various species exhibit particular bias for certain codons of a particular amino acid. Codon bias (differences in codon usage between organisms) often correlates with the efficiency of translation of messenger RNA (mRNA), which is in tum believed to be dependent on, amongst other things, the properties of the codons being translated and the availability of particular transfer RNA (tRNA) molecules. The predominance of selected tRNAs in a cell is generally a reflection of the codons used most frequently in peptide synthesis.

Accordingly, genes can be tailored for optimal gene expression in a given organism based on codon optimisation. A nucleotide sequence and/vector that has undergone this tailoring can be referred to therefore as a "codon optimised" nucleotide sequence and/or vector.

Codon usage tables are readily available, for example, at the "Codon Usage Database", and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimising a particular sequence for expression in a particular host cell are also available, such as Gene Forge (Aptagen; Jacobus, Pa.). In some embodiments, one or more codons (e.g. 1, 2, 3, 4, 5, 10, 15, 20, 25, 50, or more, or all codons) in a sequence encoding a proline tolerant tripeptidyl peptidase and/or endoprotease for use in the present invention correspond to the most frequently used codon for a particular amino acid.

In one embodiment the nucleotide sequence encoding the proline tolerant tripeptidyl peptidase may be a nucleotide sequence which has been codon optimised for expression in *Trichoderma reesei.*

Described herein, the codon optimised sequence may comprise a nucleotide sequence shown as SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 97 or a nucleotide sequence having at least 70% identity thereto. Suitably a sequence having at least 80% thereto or at least 90% thereto.

Preferably the codon optimised sequence may comprise a nucleotide sequence having at least 95% sequence identity to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, more preferably at least 99% identity to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97.

Described herein, the proline tolerant tripeptidyl peptidase may be encoded by a nucleotide sequence which hybridises to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95, SEQ ID No. 97 under medium stringency conditions. Suitably, a nucleotide sequence which hybridises to SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97 under high stringency conditions.

Described herein, the proline tolerant tripeptidyl peptidase may be encoded by a nucleotide sequence which differs from SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97 due to degeneracy of the genetic code.

Described herein is a vector (e.g. plasmid) comprising one or more of the sequences selected from the group consisting of: SEQ ID No. 81, SEQ ID No. 82, SEQ ID No. 83, SEQ ID No. 84, SEQ ID No. 85, SEQ ID No. 86, SEQ ID No. 87, SEQ ID No. 88, SEQ ID No. 89, SEQ ID No. 90, SEQ ID No. 91, SEQ ID No. 92, SEQ ID No. 93, SEQ ID No. 94, SEQ ID No. 95 or SEQ ID No. 97.

### REGULATORY SEQUENCES

In some applications, the nucleotide sequence for use in the present invention is operably linked to a regulatory sequence which is capable of providing for the expression of the nucleotide sequence, such as by the chosen host cell. By way of example, the present disclosure covers a vector comprising the nucleotide sequence for use in the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme of the present invention may also be achieved by the selection of heterologous regulatory regions, e.g. promoter, secretion leader and terminator regions.

Preferably, the nucleotide sequence according to the present invention is operably linked to at least a promoter.

Other promoters may even be used to direct expression of the polypeptide for use in the present invention.

Examples of suitable promoters for directing the transcription of the nucleotide sequence in a bacterial, fungal or yeast host are well known in the art.

The promoter can additionally include features to ensure or to increase expression in a suitable host. For example, the features can be conserved regions such as a Pribnow Box or a TATA box.

### CONSTRUCTS

The term "construct" - which is synonymous with terms such as "conjugate", "cassette" and "hybrid" - includes a nucleotide sequence for use according to the present invention directly or indirectly attached to a promoter.

An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker, which allows for the selection of the genetic construct.

For some applications, preferably the construct of the present invention comprises at least the nucleotide sequence of the present invention operably linked to a promoter.

### HOST CELLS

The term "host cell" - in relation to the present invention includes any cell that comprises either the nucleotide sequence or an expression vector as described above and which is used in the recombinant production of a protein having the specific properties as defined herein.

Thus, described herein are host cells transformed or transfected with a nucleotide sequence that expresses the protein for use in the present invention. The cells will be chosen to be compatible with the said vector and may for example be prokaryotic (for example bacterial), fungal, yeast or plant cells.

Examples of suitable bacterial host organisms are gram positive or gram negative bacterial species.

Depending on the nature of the nucleotide sequence encoding the polypeptide for use in the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells - such as yeast, fungal and plant host cells - may provide for post-translational modifications (e.g. myristoylation, glycosylation, truncation, lipidation and tyrosine, serine or threonine phosphorylation) as may be needed to confer optimal biological activity on recombinant expression products for use in the present invention. The host cell may be a protease deficient or protease minus strain. This may for example be the protease deficient strain *Aspergillus oryzae* JaL 125 having the alkaline protease gene named "alp" deleted. This strain is described in WO97/35956.

### ORGANISM

The term "organism" in relation to the present invention includes any organism that could comprise the nucleotide sequence coding for the polypeptide according to the present invention and/or products obtained therefrom, and/or wherein a promoter can allow expression of the nucleotide sequence for use in the present invention when present in the organism.

Suitable organisms may include a prokaryote, fungus, yeast or a plant.

The term "transgenic organism" in relation to the present invention includes any organism that comprises the nucleotide sequence coding for the polypeptide according to the present invention and/or the products obtained therefrom, and/or wherein a promoter can allow expression of the nucleotide sequence for use in the present invention within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism described herein includes an organism comprising any one of, or combinations of, the nucleotide sequence coding for the polypeptide as described herein, constructs as described herein, vectors as described herein, plasmids as described herein, cells as described herein, tissues as described herein, or the products thereof.

For example the transgenic organism may also comprise the nucleotide sequence coding for the polypeptide as described herein under the control of a heterologous promoter.

### TRANSFORMATION OF HOST CELLS/ORGANISM

As indicated earlier, the host organism can be a prokaryotic or a eukaryotic organism. Examples of suitable prokaryotic hosts include *E*. *coli* and *Bacillus subtilis.*

Teachings on the transformation of prokaryotic hosts are well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

Filamentous fungi cells may be transformed using various methods known in the art - such as a process involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

### TRANSFORMED FUNGUS

A host organism may be a fungus - such as a mould. Examples of suitable such hosts include any member belonging to the genera Thermomyces, Acremonium, Aspergillus, Penicillium, Mucor, Neurospora, Trichoderma and the like.

In one embodiment, the host organism may be a filamentous fungus.

Transforming filamentous fungi is discussed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings which may also be utilised in transforming filamentous fungi are reviewed in US-A-5674707.

In addition, gene expression in filamentous fungi is taught in in Punt et al. (2002) Trends Biotechnol 2002 May;20(5):200-6, Archer & Peberdy Crit Rev Biotechnol (1997) 17(4):273-306.

Described herein is the production of transgenic filamentous fungi according to the present invention prepared by use of these standard techniques.

Described herein, the host organism is a *Trichoderma* host organism, e.g. a *Trichoderma reesei* host organism.

Described herein, the host organism can be of the genus *Aspergillus,* such as *Aspergillus niger.*

A transgenic *Aspergillus* can also be prepared by following, for example, the teachings of Turner G. 1994 (Vectors for genetic manipulation. In: Martinelli, S.D., Kinghorn J.R. (Editors) Aspergillus: 50 years on. Progress in industrial microbiology vol 29. Elsevier Amsterdam 1994. pp. 641-666).

### TRANSFORMED YEAST

Described herein, the transgenic organism can be a yeast.

A review of the principles of heterologous gene expression in yeast are provided in, for example, Methods Mol Biol (1995), 49:341-54, and Curr Opin Biotechnol (1997) Oct;8(5) :554-60.

In this regard, yeast - such as the species *Saccharomyces cerevisiae or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

### CULTURING AND PRODUCTION

Host cells transformed with the nucleotide sequence as described herein may be cultured under conditions conducive to the production of the encoded polypeptide and which facilitate recovery of the polypeptide from the cells and/or culture medium.

The medium used to cultivate the cells may be any conventional medium suitable for growing the host cell in questions and obtaining expression of the polypeptide.

The protein produced by a recombinant cell may be displayed on the surface of the cell.

The protein may be secreted from the host cells and may conveniently be recovered from the culture medium using well-known procedures.

### SECRETION

Often, it is desirable for the protein to be secreted from the expression host into the culture medium from where the protein may be more easily recovered. Described herein, the secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*gla*A - both 18 and 24 amino acid versions e.g. from *Aspergillus*), the a-factor gene (yeasts e.g. *Saccharomyces, Kluyveromyces* and *Hansenula*) or the α-amylase gene (*Bacillus*)*.*

By way of example, the secretion of heterologous proteins in *E. coli* is reviewed in Methods Enzymol (1990) 182:132-43.

### POST-TRANSCRIPTION AND POST-TRANSLATIONAL MODIFICATIONS

Suitably the proline tolerant tripeptidyl peptidase and/or the endoprotease for use in the present invention may be encoded by any one of the nucleotide sequences taught herein. Depending upon the host cell used post-transcriptional and/or post-translational modifications may be made. It is envisaged that the enzymes (e.g. the proline tolerant tripeptidyl peptidase and/or the endoprotease) for use in the present methods and/or uses encompasses enzymes (e.g. the proline tolerant tripeptidyl peptidase and/or the endoprotease) which have undergone post-transcriptional and/or post-translational modification.

One non-limiting example of a post-transcriptional and/or post-translational modifications is "clipping" or "cleavage" of a polypeptide (e.g. of the proline tolerant tripeptidyl peptidase and/or the endoprotease).

In some embodiments the polypeptide (e.g. the tripeptidyl peptidase for use in the present invention e.g. proline tolerant tripeptidyl peptidase and/or the endoprotease) may be clipped or cleaved. This may result in the conversion of the proline tolerant tripeptidyl peptidase and/or the endoprotease from an inactive or substantially inactive state to an active state (i.e. capable of performing the activity described herein).

The proline tolerant tripeptidyl peptidase may be a pro-peptide which undergoes further post-translational modification to a mature peptide, i.e. a polypeptide which has the proline tolerant tripeptidyl peptidase activity.

By way of example only SEQ ID No. 1 is the same as SEQ ID No. 29 except that SEQ ID No. 1 has undergone post-translational and/or post-transcriptional modification to remove some amino acids, more specifically 197 amino acids from the N-terminus. Therefore the polypeptide shown herein as SEQ ID No. 1 could be considered in some circumstances (i.e. in some host cells) as a pro-peptide - which is further processed to a mature peptide (SEQ ID No. 29) by post-translational and/or post-transcriptional modification. The precise modifications, e.g. cleavage site(s), in respect of the post-translational and/or post-transcriptional modification may vary slightly depending on host species. In some host species there may be no post translational and/or post-transcriptional modification, hence the pro-peptide would then be equivalent to the mature peptide (i.e. a polypeptide which has the tripeptidyl peptidase activity of the present invention). Without wishing to be bound by theory, the cleavage site(s) may be shifted by a few residues (e.g. 1, 2 or 3 residues) in either direction compared with the cleavage site shown by reference to SEQ ID No. 29 compared with SEQ ID No.1. In other words, rather than cleavage at position 197 (R) for example, the cleavage may be at position 196-A, 195-A, 194-A, 198Q, 199E, 200P for example. In addition or alternatively, the cleavage may result in the removal of about 197 amino acids, in some embodiments the cleavage may result in the removal of between 194 and 200 residues.

Other examples of post-transcriptional and/or post-translational modifications include but are not limited to myristoylation, glycosylation, truncation, lipidation and tyrosine, serine or threonine phosphorylation. The skilled person will appreciate that the type of post-transcriptional and/or post-translational modifications that may occur to a protein (e.g. the proline tolerant tripeptidyl peptidase and/or the endoprotease) may depend on the host organism in which the protein (e.g. the proline tolerant tripeptidyl peptidase and/or the endoprotease) is expressed.

### DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

### FUSION PROTEINS

The amino acid sequence for use according to the present invention may be produced as a fusion protein, for example to aid in extraction and purification. Examples of fusion protein partners include glutathione-S-transferase (GST), 6xHis, GAL4 (DNA binding and/or transcriptional activation domains) and (β-galactosidase). It may also be convenient to include a proteolytic cleavage site between the fusion protein partner and the protein sequence of interest to allow removal of fusion protein sequences.

Preferably, the fusion protein will not hinder the activity of the protein sequence.

Gene fusion expression systems in *E. coli* have been reviewed in Curr Opin Biotechnol (1995) 6(5):501-6.

In another embodiment of the invention, the amino acid sequence may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

### GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

### DOSAGES

The proline tolerant tripeptidyl peptidase and/or the endoprotease and/or exo-tripeptidyl peptidase of the S53 family for use in the methods and/or uses of the present invention may be dosed in any suitable amount.

In one embodiment the proline tolerant tripeptidyl peptidase and/or exo-tripeptidyl peptidase of the S53 family may be dosed in an amount of about 5 mg to 3 g of enzyme per kg of protein substrate and/or food and/or feed additive composition.

In the preparation of a hydrolyzate suitably the enzyme proline tolerant tripeptidyl peptidase and/or exo-tripeptidyl peptidase of the S53 family may be dosed in an amount of 5 mg to 3 g of enzyme per kg of protein substrate.

In one embodiment suitably the enzyme proline tolerant tripeptidyl peptidase and/or exo-tripeptidyl peptidase of the S53 family may be dosed in an amount of 25mg to 1000mg of enzyme per kg of protein substrate.

In one embodiment the proline tolerant tripeptidyl peptidase may be dosed in an amount of about 0.01 mg -100 mg; 0.5 mg -100 mg; 1 mg -50 mg; 5 mg -100 mg; 5 mg - 20 mg, 10 mg-100 mg; 0.05 mg - 50 mg; or 0.10 mg -10 mg of enzyme per kg of feed additive composition. In certain embodiments the proline tolerant tripeptidyl peptidase may be dosed in an amount of about 0.01 g to 1000 g of enzyme per kg of feed additive composition, such as 0.1 g to 500 g, such as 0.5 g to 700 g, such as in an amount of about 0.01 g - 200 g, 0.01 g -100 g; 0.5 g -100 g; 1 g - 50 g; 5 g -100 g; 5 g - 20 g, 5 g - 15 g, 10 g -100 g; 0.05 g - 50 g; or 0.10 g -10 g of enzyme per kg of feed additive composition.

In one preferred embodiment, the proline tolerant tripeptidyl peptidase may be dosed in an amount of about 5 mg - 20 mg of enzyme per kg of feed additive composition,
The exact amount will depend on the particular type of composition employed and on the specific protease activity per mg of protein.

In another embodiment the proline tolerant tripeptidyl peptidase and/or exo-tripeptidyl peptidase of the S53 family may be dosed in an amount of about 1 mg to about 1 kg of enzyme per kg of food and/or feed and/or feedstuff and/or premix. Suitably the proline tolerant tripeptidyl peptidase and/or exo-tripeptidyl peptidase of the S53 family may be dosed at about 1 mg to about 250 g per kg of food and/or feed and/or feedstuff and/or premix. Preferably at about 1 mg to about 100 g (more preferably at about 1 mg to about 1 g) per kg of food and/or feed and/or feedstuff and/or premix.

The endoprotease may be dosed in an amount of about 50 to about 3000 mg of enzyme per kg of protein substrate, e.g. 0.05 to 3 g of enzyme per metric ton (MT) of protein substrate. Suitably, the endoprotease may be dosed in an amount of less than about 4.0 g of enzyme per MT of protein substrate.

In another embodiment the endoprotease may be dosed at between about 0.5 g and about 5.0 g of enzyme per MT of protein substrate. Suitably the endoprotease may be dosed at between about 0.5 g and about 3.0 g of enzyme per MT of protein substrate. More suitably, the endoprotease may be dosed at about 1.0 g to about 2.0 g of enzyme per MT of protein substrate.

In one embodiment the aminopeptidase may be dosed in an amount of between about 0.5 mg to about 2 g of enzyme per kg of protein substrate and/or food and/or feed additive composition. Suitably the aminopeptidase may be dosed in an amount of between about 1 mg to about 2 g of enzyme per kg of protein substrate and/or food and/or feed additive composition. More suitably in an amount of between about 5 mg to about 1.5 g of enzyme per kg of protein substrate and/or food and/or feed additive composition.

In the preparation of a hydrolysate the aminopeptidase may be dosed in an amount of between about 0.5 mg to about 2 g of enzyme per kg of protein substrate. Suitably the aminopeptidase may be dosed in an amount of between about 1 mg to about 2 g of enzyme per kg of protein substrate. More suitably in an amount of between about 5 mg to about 1.5 g of enzyme per kg of protein substrate.

In one embodiment the aminopeptidase may be dosed in an amount of between about 5 mg to about 500 mg of enzyme per kg of protein substrate. Suitably the aminopeptidase may be dosed in an amount of between about 50 mg to about 500 mg of enzyme per kg of protein substrate. Suitably the aminopeptidase may be dosed in an amount of between about 100 mg to about 450 mg of enzyme per kg of protein substrate.

### SUBJECT

The term "subject" may be used to refer to an "animal" or a "human".

Suitably the subject may be a "sensitive individual" predisposed to having an immune reaction to an untreated hydrolysate comprising one or more particular proteins or portions thereof. For example, the subject may be a sensitive individual having: a gluten (e.g. gliadin) allergy, a milk protein allergy and/or a soy protein allergy.

The term "animal", as used herein, means an animal that is to be or has been administered with a feed additive composition according to the present invention or a feedstuff comprising said feed additive composition according to the present invention.

Preferably, the animal is a mammal, a ruminant animal, monogastric animal, fish or crustacean including for example livestock or a domesticated animal (e.g. a pet).

In one embodiment the "animal" is livestock.

The term "livestock", as used herein refers to any farmed animal. Preferably, livestock is one or more of cows or bulls (including calves), pigs (including piglets, swine, growing pigs, sows), poultry (including broilers, chickens, egg layers and turkeys), birds, fish (including freshwater fish, such as salmon, cod, trout and carp, e.g. koi carp, and marine fish, such as sea bass), crustaceans (such as shrimps, mussels and scallops), horses (including race horses), sheep (including lambs).

In another embodiment the "animal" is a domesticated animal or pet or an animal maintained in a zoological environment.

The term "domesticated animal or pet or animal maintained in a zoological environment" as used herein refers to any relevant animal including canines (e.g. dogs), felines (e.g. cats), rodents (e.g. guinea pigs, rats, mice), birds, fish (including freshwater fish and marine fish), and horses.

In one embodiment the animal is a monogastric animal. In a preferred embodiment the monogastric animal may be poultry or pig (or a combination thereof).

In another embodiment the animal is a ruminant animal.

The term animal is not intended to refer to a human being.

### COMPOSITIONS

Described herein is a general composition comprising at least one proline tolerant tripeptidyl peptidase predominantly having exopeptidase wherein said proline tolerant tripeptidyl peptidase is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

Described herein is a food additive composition comprising at least one proline tolerant tripeptidyl peptidase wherein said proline tolerant tripeptidyl peptidase predominantly has exopeptidase activity and is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

Described herein is a feed additive composition comprising at least one proline tolerant tripeptidyl peptidase wherein said proline tolerant tripeptidyl peptidase predominantly has exopeptidase activity and is capable of cleaving tri-peptides from the N-terminus of peptides having:
(i) Proline at P1; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1; or
(ii) Proline at P1'; and
   an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'.

Alternatively or additionally the composition and/or food additive composition and/or feed additive composition may comprise a proline tolerant tripeptidyl peptidase obtainable by any one of the methods as described herein.

In another embodiment there is provided a food additive and/or feed additive composition comprising a hydrolysate of the invention. Such a food and/or feed additive composition comprises a proline tolerant tripeptidyl peptidase in combination with an endoprotease.

The enzymes such as the proline tolerant tripeptidyl peptidase for use in the methods and/or uses of the present invention and/or the exo-tripeptidyl peptidase and/or the composition and/or food additive and/or feed additive composition may be formulated in any appropriate manner known in the art.

In some embodiments further ingredients may be admixed with the tripeptidyl peptidase (e.g. proline tolerant tripeptidyl peptidase) such as salts such as Na₂SO₄, maltodextrin, limestone (calcium carbonate), cyclodextrin, wheat or a wheat component, starch, Talc, PVA, polyols such as sorbitol and glycerol, benzoate, sorbiate, sugars such as sucrose and glucose, propylene glycol, 1,3-propane diol, parabens, sodium chloride, citrate, acetate, sodium acetate, phosphate, calcium, metabisulfite, formate or mixtures thereof.

The food additive composition or feed additive composition according to the present invention comprises the proline tolerant tripeptidyl peptidase as defined in the claims or fermentate according to the present invention and further comprises one or more ingredients selected from the group consisting of: a salt, polyol including sorbitol and glycerol, wheat or a wheat component, sodium acetate, sodium acetate trihydrate, potassium sorbate Talc, PVA, benzoate, sorbiate, 1,3-propane diol, glucose, parabens, sodium chloride, citrate, metabisulfite, formate or a combination thereof.

In one embodiment the salt may be selected from the group consisting of: Na₂SO₄, NaH₂PO₄, Na₂HPO₄, Na₃PO₄, (NH₄)H₂PO₄, K₂HPO₄, KH₂PO₄, K₂SO₄, KHSO₄, ZnSO₄, MgSO₄, CuSO₄, Mg(NO₃)₂, (NH₄)₂SO₄, sodium borate, magnesium acetate, sodium citrate or combinations thereof.

Typical liquid formulations of food grade enzymes may include the following components (% is in w/w): enzyme of interest 0.2% - 30%, preferably 2% - 20%.

The stability of the enzyme formulation might also be increased by using salts like NaCl, KCI, CaCI2, Na2SO4 or other food grade salts in concentrations from about 0.1% to about 20% (suitably from about 0.1% to about 5%). Without wishing to be bound by theory, it is believed that the high salt concentrations might again be a way of achieving microbial stability either alone or in combination with further ingredients. The mechanism of action may be due to lower water activity or a specific action between a certain enzyme and a salt. Therefore in some embodiments the proline tolerant tripeptidyl peptidase may be admixed with at least one salt.

Suitably the preservative may be sodium benzoate and/or potassium sorbate. These preservatives can be typically used in a combined concentration of about 0.1 - 1%, suitably about 0.2 - 0.5%. Sodium benzoate is most efficient at pH < 5.5 and sodium sorbate at pH < 6.

Suitably the sugar is sorbitol.

Suitably the salt is sodium sulphate.

In one embodiment the one or more ingredients (e.g. used for the formulation of the composition and/or food additive composition and/or feed additive composition) may be selected from the group consisting of: polyols, such as glycerol and/or sorbitol; sugars, such as glucose, fructose, sucrose, maltose, lactose and trehalose; salts, such as NaCl, KCI, CaCI2, Na₂SO₄ or other food grade salts; a preservative, e.g. sodium benzoate and/or potassium sorbate; or combinations thereof.

Suitably the one or more ingredients (e.g. used for the formulation of the composition and/or food additive composition and/or feed additive composition) may be selected from the group consisting of: a wheat carrier, sorbitol and sodium sulphate.

Suitably, the proline tolerant tripeptidyl peptidase and/or exo-tripeptidyl peptidase of the S53 family and/or the composition and/or food additive and/or feed additive composition may be admixed with a wheat carrier.

Suitably, the proline tolerant tripeptidyl peptidase and/or exo-tripeptidyl peptidase of the S53 family and/or the composition and/or food additive and/or feed additive composition may be admixed with sorbitol.

Suitably the proline tolerant tripeptidyl peptidase and/or exo-tripeptidyl peptidase of the S53 family and/or the composition and/or food additive and/or feed additive composition may be admixed with sodium sulphate.

In a preferred embodiment the composition and/or food additive and/or feed additive composition may further comprise any endoprotease detailed herein.

### FORMS

The feed additive composition of the present invention and other components and/or the feedstuff comprising same may be used in any suitable form.

The feed additive composition of the present invention may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, feed additive composition of the present invention may be mixed with feed or administered in the drinking water.

Suitable examples of forms include one or more of: powders, pastes, boluses, pellets, tablets, pills, granules, capsules, ovules, solutions or suspensions, which may contain flavouring or colouring agents, for immediate-, delayed-, modified-, sustained-, pulsed- or controlled-release applications.

By way of example, if the composition of the present invention is used in a solid, e.g. pelleted form, it may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

### COMBINATION WITH OTHER COMPONENTS

The proline tolerant tripeptidyl peptidase and endoprotease and/or exo-tripeptidyl peptidase of the S53 family and/or three enzyme composition for use in the present invention and/or the composition and/or food and/or feed additive composition and/or hydrolysate of the present invention may be used in combination with other components.

The proline tolerant tripeptidyl peptidase and endoprotease and/or exo-tripeptidyl peptidase of the S53 family and/or three enzyme composition for use in the present invention and/or the composition and/or food and/or feed additive composition and/or hydrolysate of the present invention and another component which is suitable for animal consumption and is capable of providing a medical or physiological benefit to the consumer.

In one embodiment the "another component" may be one or more enzymes.

Suitable additional enzymes for use in the present invention may be one or more of the enzymes selected from the group consisting of: endoglucanases (E.C. 3.2.1.4); celliobiohydrolases (E.C. 3.2.1.91), β-glucosidases (E.C. 3.2.1.21), cellulases (E.C. 3.2.1.74), lichenases (E.C. 3.1.1.73), lipases (E.C. 3.1.1.3), lipid acyltransferases (generally classified as E.C. 2.3.1.x), phospholipases (E.C. 3.1.1.4, E.C. 3.1.1.32 or E.C. 3.1.1.5), phytases (e.g. 6-phytase (E.C. 3.1.3.26) or a 3-phytase (E.C. 3.1.3.8), alpha-amylases (E.C. 3.2.1.1), xylanases (E.C. 3.2.1.8, E.C. 3.2.1.32, E.C. 3.2.1.37, E.C. 3.1.1.72, E.C. 3.1.1.73), glucoamylases (E.C. 3.2.1.3), proteases (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)) and/or mannanases (e.g. a β-mannanase (E.C. 3.2.1.78)).

Suitably the other component may be a phytase (e.g. a 6-phytase (E.C. 3.1.3.26) or a 3-phytase (E.C. 3.1.3.8)).

In one embodiment (particularly for feed applications) the other component may be one or more of the enzymes selected from the group consisting of xylanases (E.C. 3.2.1.8, E.C. 3.2.1.32, E.C. 3.2.1.37, E.C. 3.1.1.72, E.C. 3.1.1.73), an amylase (including α-amylases (E.C. 3.2.1.1), G4-forming amylases (E.C. 3.2.1.60), β-amylases (E.C. 3.2.1.2) and γ-amylases (E.C. 3.2.1.3); and/or a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In one embodiment (particularly for feed applications) the other component may be a combination of an amylase (e.g. α-amylases (E.C. 3.2.1.1)) and a protease (e.g. subtilisin (E.C. 3.4.21.62)).

In one embodiment (particularly for feed applications) the other component may be a β-glucanase, e.g. an endo-1,3(4)-β-glucanases (E.C. 3.2.1.6).

In one embodiment (particularly for feed applications) the other component may be a mannanases (e.g. a β-mannanase (E.C. 3.2.1.78)).

In one embodiment (particularly for feed applications) the other component may be a lipase (E.C. 3.1.1.3), a lipid acyltransferase (generally classified as E.C. 2.3.1.x), or a phospholipase (E.C. 3.1.1.4, E.C. 3.1.1.32 or E.C. 3.1.1.5), suitably a lipase (E.C. 3.1.1.3).

In one embodiment (particularly for feed applications) the other component may be a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In another embodiment the other component for combining with a proline tolerant tripeptidyl peptidase (optionally in combination with an endoprotease) and/or for combining with an endoprotease in combination with an exo-tripeptidyl peptidase of the S53 family and aminopeptidase may be a further protease. Suitably, the further protease may be selected from the group consisting of: an aminopeptidase and a carboxypeptidase.

The term "carboxypeptidase" as used herein has its usual meaning in the art and refers to an exopeptidase that is capable of cleaving *n* amino acids from the C-terminus of a peptide and/or protein substrate. In one embodiment *n* may be at least 1, suitably *n* may be at least 2. In other embodiments *n* may be at least 3, suitably at least 4.

In other embodiments, the proline tolerant tripeptidyl peptidase (optionally in combination with an endoprotease) and/or three enzyme composition may be used with one or more further exopeptidase.

In one embodiment the proline tolerant tripeptidyl peptidase (optionally in combination with an endoprotease) and/or three enzyme composition is not combined with a proline-specific exopeptidase.

In a particularly preferred embodiment the proline tolerant tripeptidyl peptidase and/or three enzyme composition may not be combined with an enzyme having the following polypeptide sequence:

In one embodiment the additional component may be a stabiliser or an emulsifier or a binder or carrier or an excipient or a diluent or a disintegrant.

The term "stabiliser" as used here is defined as an ingredient or combination of ingredients that keeps a product (e.g. a feed product) from changing over time.

The term "emulsifier" as used herein refers to an ingredient (e.g. a feed ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers.

As used herein the term "binder" refers to an ingredient (e.g. a feed ingredient) that binds the product together through a physical or chemical reaction. During "gelation" for instance, water is absorbed, providing a binding effect. However, binders can absorb other liquids, such as oils, holding them within the product. In the context of the present invention binders would typically be used in solid or low-moisture products for instance baking products: pastries, doughnuts, bread and others. Examples of granulation binders include one or more of: polyvinylpyrrolidone, hydroxypropylmethylcellulose (HPMC), hydroxypropylcellulose (HPC), sucrose, maltose, gelatin and acacia.

"Carriers" mean materials suitable for administration of the enzyme and include any such material known in the art such as, for example, any liquid, gel, solvent, liquid diluent, solubilizer, or the like, which is non-toxic and which does not interact with any components of the composition in a deleterious manner.

Described herein is a method for preparing a composition (e.g. a feed additive composition) comprising admixing feed additive of the present invention (and preferably corn or a corn by-product) with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

Examples of "excipients" include one or more of: microcrystalline cellulose and other celluloses, lactose, sodium citrate, calcium carbonate, dibasic calcium phosphate, glycine, starch, milk sugar and high molecular weight polyethylene glycols.

Examples of "disintegrants" include one or more of: starch (preferably corn, potato or tapioca starch), sodium starch glycollate, croscarmellose sodium and certain complex silicates. Examples of "diluents" include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes) to the feed additive composition or feed additive composition or hydrolysate of the present invention.

In one embodiment preferably the feed additive composition, or feed ingredient, or feed or feedstuff or premix according to the present invention does not comprise chromium or organic chromium.

In one embodiment preferably the feed additive composition, or feed ingredient, or feed or feedstuff or premix according to the present invention does not contain sorbic acid.

### PACKAGING

In one embodiment the proline tolerant tripeptidyl peptidase and endoprotease and/or the exo-tripeptidyl peptidase of the S53 family and/or three enzyme composition for use in the present invention and/or the composition and/or food and/or feed additive composition and/or hydrolysate and/or foodstuff and/or feedstuff according to the present invention is packaged, preferably in a bag, such as a paper bag, or alternatively may be sealed in a container. Any suitable container may be used.

### FOODSTUFF

The term "foodstuff" is used synonymously herein with "food".

Here, the term "foodstuff" is used to refer to food for humans.

The food may be in the form of a solution or as a solid - depending on the use and/or the mode of application and/or the mode of administration.

When used as - or in the preparation of - a food - such as functional food - the hydrolysate and/or composition and/or food additive composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In one embodiment the present invention provides a foodstuff comprising a hydrolysate according to the invention. The foodstuff comprises a proline tolerant tripeptidyl peptidase as defined in the claims in combination with an endoprotease.

Optionally, the foodstuff may further comprise at least one food ingredient.

Described herein, the foodstuff may comprise at least one proline tolerant tripeptidyl peptidase comprising an amino acid sequence selected from:
SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

Described herein, the proline tolerant tripeptidyl peptidase may comprise one or more amino acid sequence selected from SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28 or a functional fragment thereof or an amino acid sequence having at least 70% identity therewith.

Described herein, the proline tolerant tripeptidyl peptidase may comprise one or more amino acid sequence selected from SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

Described herein is a method for the production of a foodstuff comprising contacting a food component with a hydrolysate of the invention or a composition and/or food additive composition of the invention.

Where a food component is contacted with a composition and/or food additive composition, suitably the food component may also be contacted with an endoprotease.

The composition of the present invention can be used in the preparation of food products such as one or more of: jams, marmalades, jellies, dairy products (such as milk or cheese), meat products, poultry products, fish products and bakery products.

In one embodiment, the composition of the present invention can be used in the preparation of protein rich waste streams. The term "protein rich waste streams" as used herein may include substrates which are byproducts from plant or animal processing, e.g. soybean processing, byproducts from rendering facilities (e.g. waste from abattoirs and/or fish processing facilities).

By way of example, the composition of the present invention can be used as ingredients to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, breakfast cereals, instant noodles and cup noodles, instant soups and cup soups, balanced foods and drinks, sweeteners, texture improved snack bars, fibre bars, bake stable fruit fillings, care glaze, chocolate bakery filling, cheese cake flavoured filling, fruit flavoured cake filling, cake and doughnut icing, heat stable bakery filling, instant bakery filling creams, filing for cookies, ready-to-use bakery filling, reduced calorie filling, adult nutritional beverage, acidified soy/juice beverage, aseptic/retorted chocolate drink, bar mixes, beverage powders, calcium fortified soy/plaim and chocolate milk, calcium fortified coffee beverage, powdered shakes (e.g. protein supplement shakes or health shakes or sports shakes or elderly person shakes)), a sports nutrition product, a performance food, a baby food, a food for elderly, a food for people in medical care, satiety supplements.

A composition according to the present invention can further be used as an ingredient in food products such as American cheese sauce, anti-caking agent for grated and shredded cheese, chip dip, cream cheese, dry blended whip topping fat free sour cream, freeze/thaw dairy whipping cream, freeze/thaw stable whipped tipping, low fat and lite natural cheddar cheese, low fat Swiss style yoghurt, aerated frozen desserts, and novelty bars, hard pack ice cream, label friendly, improved economics and indulgence of hard pack ice cream, low fat ice cream: soft serve, barbecue sauce, cheese dip sauce, cottage cheese dressing, dry mix Alfredo sauce, mix cheese sauce, dry mix tomato sauce and others.

For certain aspects, preferably the foodstuff is a beverage.

Preferably the foodstuff may be a bakery product - such as bread, Danish pastry, biscuits or cookies.

Described herein is a method of preparing a food or a food ingredient, the method comprising admixing 5-KGA produced by the process of the present invention or the composition according to the present invention with another food ingredient. The method for preparing or a food ingredient is also another aspect of the present invention.

The foodstuff according to the invention may be a dairy product, a whey-protein product, a bakery product, a fermentation product, a performance food, a baby food, a beverage, a shake, a casing, a sports nutrition product, a performance food, a baby food, a food for elderly, or a food for people in medical care.

Suitably the dairy product may be a milk-based product. Such milk-based products may comprise one or more milk proteins or fragments thereof.

Preferably the dairy (e.g. milk-based product) may be an infant formula.

Suitably the bakery product may be a bread product.

Suitably a fermentation product may be a soy-based fermentation product.

Suitably a casing may be a beverage (preferably a casing for beer) or dairy casing.

### FOOD INGREDIENT

The composition and/or food additive composition of the present invention may be used as a food ingredient.

As used herein the term "food ingredient" includes a formulation which is or can be added to functional foods or foodstuffs as a nutritional supplement and/or fiber supplement. The term food ingredient as used here also refers to formulations which can be used at low levels in a wide variety of products that require gelling, texturising, stabilising, suspending, film-forming and structuring, retention of juiciness, improved mouthfeel, improvements in peptide content, improvements in nutritional components, without adding viscosity.

The food ingredient may be in the form of a solution or as a solid - depending on the use and/or the mode of application and/or the mode of administration.

### FOOD SUPPLEMENTS

The hydrolysate and/or composition and/or food additive composition of the present invention may be - or may be added to - food supplements.

### FUNCTIONAL FOODS

The composition of the present invention may be - or may be added to - functional foods. As used herein, the term "functional food" means food which is capable of providing not only a nutritional effect and/or a taste satisfaction, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

Surveys have suggested that consumers place the most emphasis on functional food claims relating to heart disease. Preventing cancer is another aspect of nutrition which interests consumers a great deal, but interestingly this is the area that consumers feel they can exert least control over. In fact, according to the World Health Organization, at least 35% of cancer cases are diet-related. Furthermore claims relating to osteoporosis, gut health and obesity effects are also key factors that are likely to incite functional food purchase and drive market development.

### FEED

The feed additive composition of the present invention may be used as - or in the preparation of - a feed.

In one embodiment the present invention provides a feedstuff comprising a hydrolysate according to the invention. The feedstuff comprises a proline tolerant tripeptidyl peptidase as defined in the claims in combination with an endoprotease.

Optionally, the feedstuff may further comprise at least one feed ingredient.

Described herein, the feedstuff may comprise at least one proline tolerant tripeptidyl peptidase comprising an amino acid sequence selected from:
SEQ ID No. 29, SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

Described herein, the proline tolerant tripeptidyl peptidase may comprise one or more amino acid sequence selected from SEQ ID No. 1, SEQ ID No. 2, SEQ ID No. 3, SEQ ID No. 4, SEQ ID No. 5, SEQ ID No.6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28 or a functional fragment thereof or an amino acid sequence having at least 70% identity therewith.

Described herein, the proline tolerant tripeptidyl peptidase may comprise one or more amino acid sequence selected from SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42, SEQ ID No. 43, SEQ ID No. 44, SEQ ID No. 45, SEQ ID No. 46, SEQ ID No. 47, SEQ ID No. 48, SEQ ID No. 49, SEQ ID No. 50, SEQ ID No. 51, SEQ ID No. 52, SEQ ID No. 53, SEQ ID No. 54, SEQ ID No. 55, SEQ ID No. 98, SEQ ID No. 99 or a functional fragment thereof or an amino acid sequence having at least 70% identity therewith.

Described herein is a method for the production of a feedstuff comprising contacting a feed component with a hydrolysate of the invention or a composition and/or feed additive composition of the invention.

Where a feed component is contacted with a composition and/or feed additive composition, suitably the feed component may also be contacted with an endoprotease. The term "feed" is used synonymously herein with "feedstuff".

The feed may be in the form of a solution or as a solid - depending on the use and/or the mode of application and/or the mode of administration.

When used as - or in the preparation of - a feed - such as functional feed - the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In a preferred embodiment the feed additive composition of the present invention is admixed with a feed component to form a feedstuff.

The term "feed component" as used herein means all or part of the feedstuff. Part of the feedstuff may mean one constituent of the feedstuff or more than one constituent of the feedstuff, e.g. 2 or 3 or 4. In one embodiment the term "feed component" encompasses a premix or premix constituents.

In one embodiment a feed additive composition comprising a proline tolerant tripeptidyl peptidase and one or more ingredients selected from the group consisting of: polyols, such as glycerol and/or sorbitol; sugars, such as glucose, fructose, sucrose, maltose, lactose and trehalose; salts, such as NaCl, KCI, CaCl₂, Na₂SO₄ or other food grade salts; a preservative, e.g. sodium benzoate and/or potassium sorbate; or combinations thereof (optionally in combination with an endoprotease) may be admixed with at least one protein or portion thereof is an animal protein or a vegetable protein (e.g. selected from one or more of a gliadin, a beta-casein, a beta-lactoglobulin or an immunogenic fragment of a gliadin, a beta-casein, a beta-lactoglobulin, glycinin, beta-conglycinin, cruciferin, napin, collagen, whey protein, fish protein, meat protein, egg protein, soy protein a hordein or grain protein), preferably comprised in corn, soybean meal, corn dried distillers grains with solubles (DDGS), wheat, wheat proteins including gluten, wheat by products, wheat bran, corn by products including corn gluten meal, barley, oat, rye, triticale, full fat soy, animal by-product meals, an alcohol-soluble protein (preferably a zein (e.g. a maize zein maize) and/or a kafirin (e.g. from sorghum)), a protein from oil seeds (preferably from soybean seed proteins, sun flower seed proteins, rapeseed proteins, canola (rapeseed) seed proteins or combinations thereof) or a combination thereof.

Preferably the feed may be a fodder, or a premix thereof, a compound feed, or a premix thereof. In one embodiment the feed additive composition according to the present invention may be admixed with a compound feed, a compound feed component or to a premix of a compound feed or to a fodder, a fodder component, or a premix of a fodder.

The term fodder as used herein means any food which is provided to an animal (rather than the animal having to forage for it themselves). Fodder encompasses plants that have been cut.

The term fodder includes hay, straw, silage, compressed and pelleted feeds, oils and mixed rations, and also sprouted grains and legumes.

Fodder may be obtained from one or more of the plants selected from: alfalfa (Lucerne), barley, birdsfoot trefoil, brassicas, Chau moellier, kale, rapeseed (canola), rutabaga (swede), turnip, clover, alsike clover, red clover, subterranean clover, white clover, grass, false oat grass, fescue, Bermuda grass, brome, heath grass, meadow grasses (from naturally mixed grassland swards, orchard grass, rye grass, Timothy-grass, corn (maize), millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, rye, maize, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

Any feedstuff of the present invention may comprise one or more feed materials selected from the group comprising a) cereals, such as small grains (e.g., wheat, barley, rye, oats and combinations thereof) and/or large grains such as maize or sorghum; b) by products from plants, such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof; c) protein obtained from sources such as soya, sunflower, peanut, lupin, peas, fava beans, cotton, canola (rapeseed), fish meal, dried plasma protein, meat and bone meal, potato protein, whey, copra, sesame; d) oils and fats obtained from vegetable and animal sources; e) minerals and vitamins.

A feedstuff of the present invention may contain at least 30%, at least 40%, at least 50% or at least 60% by weight corn and soybean meal or corn and full fat soy, or wheat meal or sunflower meal.

In addition or in the alternative, a feedstuff of the present invention may comprise at least one high fibre feed material and/or at least one by-product of the at least one high fibre feed material to provide a high fibre feedstuff. Examples of high fibre feed materials include: wheat, barley, rye, oats, by products from plants (e.g. cereals), such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof. Some protein sources may also be regarded as high fibre: protein obtained from sources such as sunflower, lupin, fava beans and cotton.

In the present invention the feed may be one or more of the following: a compound feed and premix, including pellets, nuts or (cattle) cake; a crop or crop residue: corn, soybeans, sorghum, oats, barley, corn stover, copra, straw, chaff, sugar beet waste; fish meal; freshly cut grass and other forage plants; meat and bone meal; molasses; oil cake and press cake; oligosaccharides; conserved forage plants: hay and silage; seaweed; seeds and grains, either whole or prepared by crushing, milling etc.; sprouted grains and legumes; yeast extract.

The term "feed" in the present invention also encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

The term "feed" in the present invention also encompasses in some embodiments fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the colour of ornamental fish.

The term "feed" in the present invention also encompasses in some embodiment bird food. Bird food includes food that is used both in birdfeeders and to feed pet birds. Typically bird food comprises of a variety of seeds, but may also encompass suet (beef or mutton fat).

As used herein the term "contacting" refers to the indirect or direct application of the composition of the present invention to the product (e.g. the feed). Examples of the application methods which may be used, include, but are not limited to, treating the product in a material comprising the feed additive composition, direct application by mixing the feed additive composition with the product, spraying the feed additive composition onto the product surface or dipping the product into a preparation of the feed additive composition.

In one embodiment the feed additive composition of the present invention is preferably admixed with the product (e.g. feedstuff). Alternatively, the feed additive composition may be included in the emulsion or raw ingredients of a feedstuff.

For some applications, it is important that the composition is made available on or to the surface of a product to be affected/treated. This allows the composition to impart one or more of the following favourable characteristics: biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen digestibility (e.g. ileal nitrogen digestibility) and digestible energy (e.g. ileal digestible energy) nitrogen retention, carcass yield, growth rate, weight gain, body weight, mass, feed efficiency, body fat percentage, body fat distribution, growth, egg size, egg weight, egg mass, egg laying rate, lean gain, bone ash %, bone ash mg, back fat %, milk output, milk fat %, reproductive outputs such as litter size, litter survivability, hatchability % and environmental impact, e.g. manure output and/or nitrogen excretion.

The feed additive compositions of the present invention may be applied to intersperse, coat and/or impregnate a product (e.g. feedstuff or raw ingredients of a feedstuff) with a controlled amount of enzyme(s).

Preferably, the feed additive composition of the present invention will be thermally stable to heat treatment up to about 70°C; up to about 85°C; or up to about 95°C. The heat treatment may be performed for up to about 1 minute; up to about 5 minutes; up to about 10 minutes; up to about 30 minutes; up to about 60 minutes. The term thermally stable means that at least about 75% of the enzyme components that were present/active in the additive before heating to the specified temperature are still present/active after it cools to room temperature. Preferably, at least about 80% of the enzyme components that were present and active in the additive before heating to the specified temperature are still present and active after it cools to room temperature.

In a particularly preferred embodiment the feed additive composition is homogenized to produce a powder.

In an alternative preferred embodiment, the feed additive composition is formulated to granules as described in WO2007/044968 (referred to as TPT granules).

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme. Preferably, the salt used for the salt coating has a water activity greater than 0.25 or constant humidity greater than 60 % at 20°C.

Preferably, the salt coating comprises a Na₂SO₄.

The method of preparing a feed additive composition may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour.

It will be understood that the feed additive composition of the present invention is suitable for addition to any appropriate feed material.

As used herein, the term feed material refers to the basic feed material to be consumed by an animal. It will be further understood that this may comprise, for example, at least one or more unprocessed grains, and/or processed plant and/or animal material such as soybean meal or bone meal.

As used herein, the term "feedstuff" refers to a feed material to which one or more feed additive compositions have been added.

It will be understood by the skilled person that different animals require different feedstuffs, and even the same animal may require different feedstuffs, depending upon the purpose for which the animal is reared.

Preferably, the feedstuff may comprise feed materials comprising maize or corn, wheat, barley, triticale, rye, rice, tapioca, sorghum, and/ or any of the by-products, as well as protein rich components like soybean mean, rape seed meal, canola meal, cotton seed meal, sunflower seed mean, animal-by-product meals and mixtures thereof. More preferably, the feedstuff may comprise animal fats and / or vegetable oils.

Optionally, the feedstuff may also contain additional minerals such as, for example, calcium and/or additional vitamins.

Preferably, the feedstuff is a corn soybean meal mix.

Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff may be a feedstuff for a monogastric animal, such as poultry (for example, broiler, layer, broiler breeders, turkey, duck, geese, water fowl), swine (all age categories), a pet (for example dogs, cats) or fish, preferably the feedstuff is for poultry.

By way of example only a feedstuff for chickens, e.g. broiler chickens may be comprises of one or more of the ingredients listed in the table below, for example in the %ages given in the table below:

| **Ingredients** | **Starter (%)** | **Finisher (%)** |
|---|---|---|
| Maize | 46.2 | 46.7 |
| Wheat Middlings | 6.7 | 10.0 |
| Maize DDGS | 7.0 | 7.0 |
| Soyabean Meal 48%CP | 32.8 | 26.2 |
| An/Veg Fat blend | 3.0 | 5.8 |
| L-Lysine HCl | 0.3 | 0.3 |
| DL-methionine | 0.3 | 0.3 |
| L-threonine | 0.1 | 0.1 |
| Salt | 0.3 | 0.4 |
| Limestone | 1.1 | 1.1 |
| Dicalcium Phosphate | 1.2 | 1.2 |
| Poultry Vitamins and Micro-minerals | 0.3 | 0.3 |

By way of example only the diet specification for chickens, such as broiler chickens, may be as set out in the Table below:

| **Diet specification** | | |
|---|---|---|
| Crude Protein (%) | 23.00 | 20.40 |
| Metabolizable Energy Poultry (kcal/kg) | 2950 | 3100 |
| Calcium (%) | 0.85 | 0.85 |
| Available Phosphorus (%) | 0.38 | 0.38 |
| Sodium (%) | 0.18 | 0.19 |
| Dig. Lysine (%) | 1.21 | 1.07 |
| Dig. Methionine (%) | 0.62 | 0.57 |
| Dig. Methionine + Cysteine (%) | 0.86 | 0.78 |
| Dig. Threonine (%) | 0.76 | 0.68 |

By way of example only a feedstuff laying hens may be comprises of one or more of the ingredients listed in the table below, for example in the %ages given in the table below:

| **Ingredient** | **Laying phase (%)** |
|---|---|
| Maize | 10.0 |
| Wheat | 53.6 |
| Maize DDGS | 5.0 |
| Soybean Meal 48%CP | 14.9 |
| Wheat Middlings | 3.0 |
| Soybean Oil | 1.8 |
| L-Lysine HCl | 0.2 |
| DL-methionine | 0.2 |
| L-threonine | 0.1 |
| Salt | 0.3 |
| Dicalcium Phosphate | 1.6 |
| Limestone | 8.9 |
| Poultry Vitamins and Micro-minerals | 0.6 |

By way of example only the diet specification for laying hens may be as set out in the Table below:

| **Diet specification** | |
|---|---|
| Crude Protein (%) | 16.10 |
| Metabolizable Energy Poultry (kcal/kg) | 2700 |
| Lysine (%) | 0.85 |
| Methionine (%) | 0.42 |
| Methionine + Cysteine (%) | 0.71 |
| Threonine (%) | 0.60 |
| Calcium (%) | 3.85 |
| Available Phosphorus (%) | 0.42 |
| Sodium (%) | 0.16 |

By way of example only a feedstuff for turkeys may be comprises of one or more of the ingredients listed in the table below, for example in the %ages given in the table below:

| **Ingredient** | **Phase 1 (%)** | **Phase 2 (%)** | **Phase 3 (%)** | **Phase 4 (%)** |
|---|---|---|---|---|
| Wheat | 33.6 | 42.3 | 52.4 | 61.6 |
| Maize DDGS | 7.0 | 7.0 | 7.0 | 7.0 |
| Soyabean Meal 48%CP | 44.6 | 36.6 | 27.2 | 19.2 |
| Rapeseed Meal | 4.0 | 4.0 | 4.0 | 4.0 |
| Soyabean Oil | 4.4 | 4.2 | 3.9 | 3.6 |
| L-Lysine HCl | 0.5 | 0.5 | 0.4 | 0.4 |
| DL-methionine | 0.4 | 0.4 | 0.3 | 0.2 |
| L-threonine | 0.2 | 0.2 | 0.1 | 0.1 |
| Salt | 0.3 | 0.3 | 0.3 | 0.3 |
| Limestone | 1.0 | 1.1 | 1.1 | 1.0 |
| Dicalcium Phosphate | 3.5 | 3.0 | 2.7 | 2.0 |
| Poultry Vitamins and Micro-minerals | 0.4 | 0.4 | 0.4 | 0.4 |

By way of example only the diet specification for turkeys may be as set out in the Table below:

| **Diet specification** | | | | |
|---|---|---|---|---|
| Crude Protein (%) | 29.35 | 26.37 | 22.93 | 20.00 |
| Metabolizable Energy Poultry (kcal/kg) | 2.850 | 2.900 | 2.950 | 3.001 |
| Calcium (%) | 1.43 | 1.33 | 1.22 | 1.02 |
| Available Phosphorus (%) | 0.80 | 0.71 | 0.65 | 0.53 |
| Sodium (%) | 0.16 | 0.17 | 0.17 | 0.17 |
| Dig. Lysine (%) | 1.77 | 1.53 | 1.27 | 1.04 |
| Dig. Methionine (%) | 0.79 | 0.71 | 0.62 | 0.48 |
| Dig. Methionine + Cysteine (%) | 1.12 | 1.02 | 0.90 | 0.74 |
| Dig. Threonine (%) | 1.03 | 0.89 | 0.73 | 0.59 |

By way of example only a feedstuff for piglets may be comprises of one or more of the ingredients listed in the table below, for example in the %ages given in the table below:

| **Ingredient** | **Phase 1 (%)** | **Phase 2 (%)** |
|---|---|---|
| Maize | 20.0 | 7.0 |
| Wheat | 25.9 | 46.6 |
| Rye | 4.0 | 10.0 |
| Wheat middlings | 4.0 | 4.0 |
| Maize DDGS | 6.0 | 8.0 |
| Soyabean Meal 48% CP | 25.7 | 19.9 |
| Dried Whey | 10.0 | 0.0 |
| Soyabean Oil | 1.0 | 0.7 |
| L-Lysine HCl | 0.4 | 0.5 |
| DL-methionine | 0.2 | 0.2 |
| L-threonine | 0.1 | 0.2 |
| L-tryptophan | 0.03 | 0.04 |
| Limestone | 0.6 | 0.7 |
| Dicalcium Phosphate | 1.6 | 1.6 |
| Swine Vitamins and Micro-minerals | 0.2 | 0.2 |
| Salt | 0.2 | 0.4 |

By way of example only the diet specification for piglets may be as set out in the Table below:

| **Diet specification** | | |
|---|---|---|
| Crude Protein (%) | 21.50 | 20.00 |
| Swine Digestible Energy (kcal/kg) | 3380 | 3320 |
| Swine Net Energy (kcal/kg) | 2270 | 2230 |
| Calcium (%) | 0.80 | 0.75 |
| Digestible Phosphorus (%) | 0.40 | 0.35 |
| Sodium (%) | 0.20 | 0.20 |
| Dig. Lysine (%) | 1.23 | 1.14 |
| Dig. Methionine (%) | 0.49 | 0.44 |
| Dig. Methionine + Cysteine (%) | 0.74 | 0.68 |
| Dig. Threonine (%) | 0.80 | 0.74 |

By way of example only a feedstuff for grower/finisher pigs may be comprises of one or more of the ingredients listed in the table below, for example in the %ages given in the table below:

| **Ingredient** | **Grower/ Finisher (%)** |
|---|---|
| Maize | 27.5 |
| Soyabean Meal 48% CP | 15.4 |
| Maize DDGS | 20.0 |
| Wheat bran | 11.1 |
| Rice bran | 12.0 |
| Canola seed meal | 10.0 |
| Limestone | 1.6 |
| Dicalcium phosphate | 0.01 |
| Salt | 0.4 |
| Swine Vitamins and Micro-minerals | 0.3 |
| Lysine-HCl | 0.2 |
| Vegetable oil | 0.5 |

By way of example only the diet specification for grower/finisher pigs may be as set out in the Table below:

| **Diet specification** | |
|---|---|
| Crude Protein (%) | 22.60 |
| Swine Metabolizable Energy (kcal/kg) | 3030 |
| Calcium (%) | 0.75 |
| Available Phosphorus (%) | 0.29 |
| Digestible Lysine (%) | 1.01 |
| Dig. Methionine + Cysteine (%) | 0.73 |
| Digestible Threonine (%) | 0.66 |

### MEAT BASED FOOD/FEED PRODUCT

The proline tolerant tripeptidyl peptidase and endoprotease and/or composition and/or food/feed composition and/or hydrolysate may be used in the manufacture of a meat based food/feed product.

A "meat based food product" and "meat based feed product" according to the present invention is any product based on meat.

The meat based food product is suitable for human and/or animal consumption as a food and/or a feed. In one embodiment of the invention the meat based food product is a feed product for feeding animals, such as for example a pet food product. In another embodiment of the invention the meat based food product is a food product for humans.

A meat based food/feed product may comprise non-meat ingredients such as for example water, salt, flour, milk protein, vegetable protein, starch, hydrolysed protein, phosphate, acid, spices, colouring agents and/or texturising agents.

A meat based food/feed product in accordance with the present invention preferably comprises between 5-90% (weight/weight) meat. In some embodiments the meat based food product may comprise at least 30% (weight/weight) meat, such as at least 50%, at least 60% or at least 70% meat.

In some embodiments the meat based food/feed product is a cooked meat, such as ham, loin, picnic shoulder, bacon and/or pork belly for example.

The meat based food/feed product may be one or more of the following:
Dry or semi-dry cured meats - such as fermented products, dry-cured and fermented with starter cultures, for example dry sausages, salami, pepperoni and dry ham;
Emulsified meat products (e.g. for cold or hot consumption), such as mortadella, frankfurter, luncheon meat and pâté;
Fish and seafood, such as shrimps, salmon, reformulated fish products, frozen cold-packed fish;
Fresh meat muscle, such as whole injected meat muscle, for example loin, shoulder ham, marinated meat;
Ground and/or restructured fresh meat - or reformulated meat, such as upgraded cut-away meat by cold setting gel or binding, for example raw, uncooked loin chops, steaks, roasts, fresh sausages, beef burgers, meat balls, pelmeni;
Poultry products - such as chicken or turkey breasts or reformulated poultry, e.g. chicken nuggets and/or chicken sausages;
Retorted products - autoclaved meat products, for example picnic ham, luncheon meat, emulsified products.

In one embodiment of the present invention the meat based food/feed product is a processed meat product, such as for example a sausage, bologna, meat loaf, comminuted meat product, ground meat, bacon, polony, salami or pate.

A processed meat product may be for example an emulsified meat product, manufactured from a meat based emulsion, such as for example mortadella, bologna, pepperoni, liver sausage, chicken sausage, wiener, frankfurter, luncheon meat, meat pate.

The meat based emulsion may be cooked, sterilised or baked, e.g. in a baking form or after being filled into a casing of for example plastic, collagen, cellulose or a natural casing. A processed meat product may also be a restructured meat product, such a for example restructured ham. A meat product of the invention may undergo processing steps such as for example salting, e.g. dry salting; curing, e.g. brine curing; drying; smoking; fermentation; cooking; canning; retorting; slicing and/or shredding.

In another embodiment the food/feed product may be an emulsified meat product.

### MEAT

The term "meat" as used herein means any kind of tissue derived from any kind of animal. The term meat as used herein may be tissue comprising muscle fibres derived from an animal. The meat may be an animal muscle, for example a whole animal muscle or pieces cut from an animal muscle.

In another embodiment the meat may comprise inner organs of an animal, such as heart, liver, kidney, spleen, thymus and brain for example.

The term meat encompasses meat which is ground, minced or cut into smaller pieces by any other appropriate method known in the art.

The meat may be derived from any kind of animal, such as from cow, pig, lamb, sheep, goat, chicken, turkey, ostrich, pheasant, deer, elk, reindeer, buffalo, bison, antelope, camel, kangaroo; any kind of fish e.g. sprat, cod, haddock, tuna, sea eel, salmon, herring, sardine, mackerel, horse mackerel, saury, round herring, Pollack, flatfish, anchovy, pilchard, blue whiting, pacific whiting, trout, catfish, bass, capelin, marlin, red snapper, Norway pout and/or hake; any kind of shellfish, e.g. clam, mussel, scallop, cockle, periwinkle, snail, oyster, shrimp, lobster, langoustine, crab, crayfish, cuttlefish, squid, and/or octopus.

In one embodiment the meat is beef, pork, chicken, lamb and/or turkey.

### BIOPHYSICAL CHARACTERISTIC

Feeding an animal a tripeptidyl peptidase for use in the present invention and/or a feedstuff and/or feed additive composition and/or composition of the invention may improve a biophysical characteristic of animal so fed.

Suitably the method and/or use may further comprising administering to an animal at least one feed component, at least one mineral, at least one vitamin or combinations thereof. Alternatively or additionally the method and/or use may further comprise administering to an animal at least one endoprotease.

As used herein, "biophysical characteristic" means any biophysical property of an animal which improves its health and/or performance and/or output.

By way of example, the biophysical characteristic may be one or more selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen digestibility (e.g. ileal nitrogen digestibility) and digestible energy (e.g. ileal digestible energy), nitrogen retention, carcass yield, growth rate, weight gain, body weight, mass, feed efficiency, body fat percentage, body fat distribution, growth, egg size, egg weight, egg mass, egg laying rate, lean gain, bone ash %, bone ash mg, back fat %, milk output, milk fat %, reproductive outputs such as litter size, litter survivability, hatchability % and environmental impact, e.g. manure output and/or nitrogen excretion.

Suitably the biophysical characteristic may be one or more selected from the group consisting of: feed conversion ratio, nitrogen digestibility (e.g. ileal nitrogen digestibility) and digestible energy (e.g. ileal digestible energy).

Described herein, the biophysical characteristic may be the ability to digest a protein. Described herein, the biophysical characteristic of the animal means the performance of the animal.

Suitably, administering to an animal a feed additive composition and/or feed and/or feedstuff and/or feed ingredient and/or premix of the invention may not substantially increase the incidence of necrotic enteritis in the animal when compared to an animal not fed with the feed additive composition and/or feed and/or feedstuff and/or feed ingredient and/or premix of the invention.

The term "substantially increase the incidence of necrotic enteritis" as used herein means that the incidence is not increased by more than about 20%, suitably not increased by more than about 10%. Preferably it is meant that the incidence of necrotic enteritis is not increased by more than about 5%, more preferably more than about 1%.

### PERFORMANCE

As used herein, "performance of the animal" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention.

Preferably "performance of the animal" is determined by feed efficiency and/or weight gain of the animal and/or by the feed conversion ratio.

By "improved performance of the animal" it is meant that there is increased feed efficiency, and/or increased weight gain and/or reduced feed conversion ratio and/or improved digestibility of nutrients or energy in a feed and/or by improved nitrogen retention in the subject resulting from the use of a hydrolysate of the present invention or a composition (e.g. food or feed additive composition) of the present invention in a feed compared with feeding the animal a feed not comprising a hydrolysate or a composition prepared in accordance with the present invention.

Preferably, by "improved animal performance" it is meant that there is increased feed efficiency and/or increased weight gain and/or reduced feed conversion ratio.

As used herein, the term "feed efficiency" refers to the amount of weight gain in an animal that occurs when the animal is fed ad-libitum or a specified amount of food during a period of time.

By "increased feed efficiency" it is meant that the use of a hydrolysate of the present invention or a composition (e.g. food or feed additive composition) according the present invention in feed results in an increased weight gain per unit of feed intake compared with an animal not fed with a hydrolysate or a composition prepared in accordance with the present invention.

### FEED CONVERSION RATIO (FCR)

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a hydrolysate of the present invention or a composition (e.g. food or feed additive composition) in feed results in a lower amount of feed being required to be fed to an animal to increase the weight of the animal by a specified amount compared to the amount of feed required to increase the weight of the animal by the same amount without said hydrolysate or a composition prepared in accordance with the present invention.

### NUTRIENT DIGESTIBILITY

Nutrient digestibility as used herein means the fraction of a nutrient that disappears from the gastro-intestinal tract or a specified segment of the gastrointestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Suitably use of a proline tolerant tripeptidyl peptidase according to the methods and/or uses or any of the aspects of the present invention (optionally in combination with at least one endoprotease) increases protein and/or amino acid digestibility in an animal fed with the feed additive composition and/or feed ingredient and/or feed and/or feedstuff and/or premix and/or hydrolysate of the invention.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

### NITROGEN RETENTION

Nitrogen retention as used herein means as subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

### CARCASS YIELD AND MEAT YIELD

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

### WEIGHT GAIN

Described herein is a method of increasing weight gain in a subject, e.g. poultry or swine, comprising feeding said subject a feedstuff comprising a feed additive composition according to the present invention.

An "increased weight gain" refers to an animal having increased body weight on being fed feed comprising a hydrolysate of the present invention or a composition (e.g. food or feed additive composition) according to the present invention compared with an animal being fed a feed not comprising a hydrolysate or a composition prepared in accordance with the present invention.

### NONFOOD PRODUCTS

The present invention provides a nonfood product comprising the hydrolysate of the invention.

The hydrolysate obtainable (e.g. obtained) may be used in the manufacture of a topically applied product, such as a lotion, cream, ointment, rub, cleanser, or the like. Accordingly, such products comprising the hydrolyzed protein compositions described herein are herein contemplated. Such products are useful for example for therapeutic purposes, for example, to provide relief from dry skin, itching, discomfort, and the like.

These products preferably comprise, in addition to the hydrolyzed protein component, a lipid, wax, oil, water in oil emulsion, oil-in-water emulsion, or the like, as a base. Typically, they may further comprise one or more fragrance components, as well as other ingredients such as surfactants or emulsifiers.

Cosmetic products and other appearance aids or beauty aids comprising the milk or whey protein hydrolyzates described herein are also provided.

In one embodiment, the cosmetic product may be applied to the face, cheeks, lips, or eyes of a person. In another embodiment the product may be used anywhere on the body to help improve the cosmetic appearance of the skin or, for example, to diminish the appearance of wrinkles moles, freckles, scars, blemishes, and the like.

### ADVANTAGES

The inventors have shown for the first time that a proline tolerant tripeptidyl peptidase according to the present invention is highly advantageous for use in the preparation of hydrolysates and confers advantages to subjects fed the hydrolysate or a food and/or feed comprising the hydrolysate and/or a food or feed additive composition comprising the proline tolerant tripeptidyl peptidase (optionally in combination with at least one endoprotease).

Alternatively or additionally, the hydrolysate produced using an endoprotease and tripeptidyl peptidase may also have reduced bitterness when compared to an untreated hydrolysate. Advantageously, a proline tolerant tripeptidyl peptidase taught for use in the present invention is capable of acting on a wide range of peptide and/or protein substrates and due to having such a broad substrate-specificity is not readily inhibited from cleaving substrates enriched in certain amino acids (e.g. proline and/or lysine and/or arginine and/or glycine). The use of such a proline tolerant tripeptidyl peptidase therefore may efficiently and/or rapidly breakdown protein substrates (e.g. present in a substrate for preparation of a hydrolysate). The proline tolerant tripeptidyl peptidase may have a preferential activity on peptides and/or proteins having one or more of lysine, arginine or glycine in the P1 position. Without wishing to be bound by theory peptide and/or protein substrates comprising these amino acids at the P1 position may be difficult to digest for many tripeptidyl peptidases and/or proteases in generally and upon encountering such residues cleavage of the peptide and/or protein substrate by a tripeptidyl peptidase and/or protease may halt or slow. Advantageously, by using a proline tolerant tripeptidyl peptidase of the invention it is possible to digest protein and/or peptide substrates comprising lysine, arginine and/or glycine at P1 efficiently and/or without significantly slowing the cleavage reaction.

Also disclosed are proline tolerant tripeptidyl peptidase that, in addition to having the activities described above, may be tolerant of proline at position P2, P2', P3 and P3'. This is advantageous as it allows the efficient cleavage of peptide and/or protein substrates having stretches of proline and allows cleavage of a wide range of peptide and/or protein substrates.

Advantageously the proline tolerant tripeptidyl peptidase may have a preferential activity on peptides and/or proteins having lysine at the P1 position, this allows the efficient cleavage of substrates having high lysine content, such as whey protein.

Also disclosed are thermostable proline tolerant tripeptidyl peptidases which are less prone to being denatured and/or will therefore retain activity for a longer period of time when compared to a non-thermostable variant.

Advantageously the proline tolerant tripeptidyl peptidase may have activity in a pH range of about pH 7 and can therefore be used with an alkaline endoprotease. This means that changing the pH of the reaction medium comprising the protein and/or peptide substrate for hydrolysate production is not necessary between enzyme treatments. In other words it allows the proline tolerant tripeptidyl peptidase and the endoprotease to be added to a reaction simultaneously, which may make the process for producing the hydrolysate quicker and/or more efficient and/or more cost-effective. Moreover, this allows for a more efficient reaction as at lower pH values the substrate may precipitate out of solution and therefore not be cleaved.

A proline tolerant tripeptidyl peptidase having activity at an acidic pH can be used in combination with an acid endoprotease and advantageously does not require the pH of the reaction medium comprising the protein and/or peptide substrate for hydrolysate production to be changed between enzyme treatments. In other words it allows the proline tolerant tripeptidyl peptidase and the endoprotease to be added to a reaction simultaneously, which may make the process for producing the hydrolysate quicker and/or more efficient and/or more cost-effective.

Advantageously, the endoprotease in combination with a proline tolerant tripeptidyl peptidase is capable of cleaving protein substrates associated with causing an immune response in sensitive individuals suffering from a disease, such as a milk protein allergy and/or a soy protein allergy.

Advantageously, the use of an endoprotease in combination with a proline tolerant tripeptidyl peptidase can increase the efficiency of substrate cleavage. Without wishing to be bound by theory, it is believed that an endoprotease is able to cleave a peptide and/or protein substrate at multiple regions away from the C or N-terminus, thereby producing more N-terminal ends for the proline tolerant tripeptidyl peptidase to use as a substrate, thereby advantageously increasing reaction efficiency and/or reducing reaction times.

Many current feeding practices involve administering an alkaline protease active at a high pH (e.g. pH 8) to animals. Alkaline proteases are therefore only active lower down (e.g. later) in the gastrointestinal tract of an animal where the gastrointestinal tract becomes more alkaline, such as in the later part of the small intestine and the large intestine and caecum. Without wishing to be bound by theory, it is believed that producing oligopeptides in the later parts of the gastrointestinal tract increases populations of microbes which utilise the oligopeptides which in turn can lead to enteric disease challenges and/or reduced nutrients available for uptake by the animal. Additionally, later in the gastrointestinal tract (i.e. lower down) the mucosa is less well-protected than in the upper portions (e.g. the gizzard, proventriculus or stomach) and so is more easily damaged leading to inflammation. Advantageously, the use of a proline tolerant tripeptidyl peptidase having activity at an acid pH alleviates this problem as it is capable of digesting its substrate in the upper gastrointestinal tract thereby not substantially increasing populations of microbes and/or increasing the amount of nutrient (e.g. amino acids/peptides) available for uptake by an animal and/or reducing inflammation.

Advantageously, the use of an endoprotease in combination with a proline tolerant tripeptidyl peptidase can increase the efficiency of substrate cleavage. Without wishing to be bound by theory, it is believed that an endoprotease is able to cleave a peptide and/or protein substrate at multiple regions away from the C or N-terminus, thereby producing more N-terminal ends for the proline tolerant tripeptidyl peptidase to use as a substrate, thereby advantageously increasing reaction efficiency and/or reducing reaction times.

The use of an acid endoprotease and a proline tolerant tripeptidyl peptidase having activity at an acid pH is highly advantageous as the two enzymes can co-operate to digest a peptide and/or protein substrate in the upper gastrointestinal tract (e.g. gizzard, proventriculus or stomach) of an animal and can be active in combination with other endogenous proteases (e.g. pepsin, trypsin or chymotrypsin) present in the animal.

Advantageously feeding a proline tolerant tripeptidyl peptidase to an animal results in increased body weight gain and/or a reduction in feed conversion ratio and/or increased nitrogen digestibility (e.g. ileal nitrogen digestibility) and/or increased energy digestion (e.g. ileal energy digestion).

Use of an endoprotease, an exo-tripeptidyl peptidase of the S53 family (e.g. such as a proline tolerant tripeptidyl peptidase) and an aminopeptidase optionally in combination with one or more further components has many advantages:
- it allows for the efficient production of single amino acids and/or dipeptides and/or tripeptides which can efficiently be absorbed by a subject (e.g. due to having a better osmotic potential for uptake);
- a protein and/or peptide substrate may be more efficiently and/or more quickly digested;
- reduced end-point inhibition (i.e. inhibition by its reaction products) of a the proline tolerant tripeptidyl peptidase, particularly when used *in vitro,* such as in the manufacture of a hydrolysate by digesting the tripeptides into single amino acids and/or dipeptides; and/or
- synergistic and/or additive activity on substrates containing high levels of proline, lysine, arginine and/or glycine.

### ADDITIONAL DEFINITIONS

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a proline tolerant tripeptidyl peptidase", "an endoprotease", "exo-tripeptidyl peptidase of the S53 family", "aminopeptidase" or "an enzyme" includes a plurality of such candidate agents and reference to "the feed", "the feedstuff", "the premix" or "the feed additive composition" includes reference to one or more feeds, feedstuffs, premixes and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention will now be described, by way of example only, with reference to the following Figures and Examples.

### EXAMPLES

### EXAMPLE 1

### Cloning and expression of proline tolerant tripeptidyl peptidases in Trichoderma reesei.

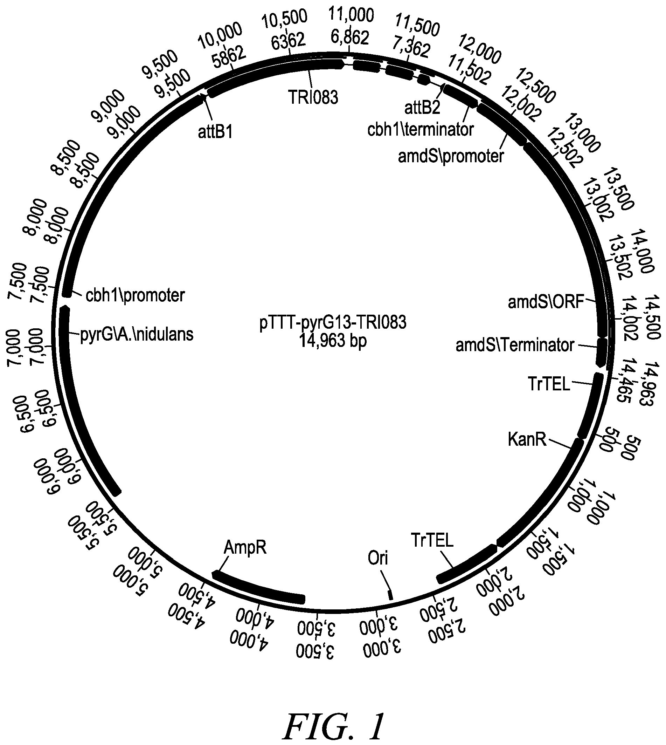

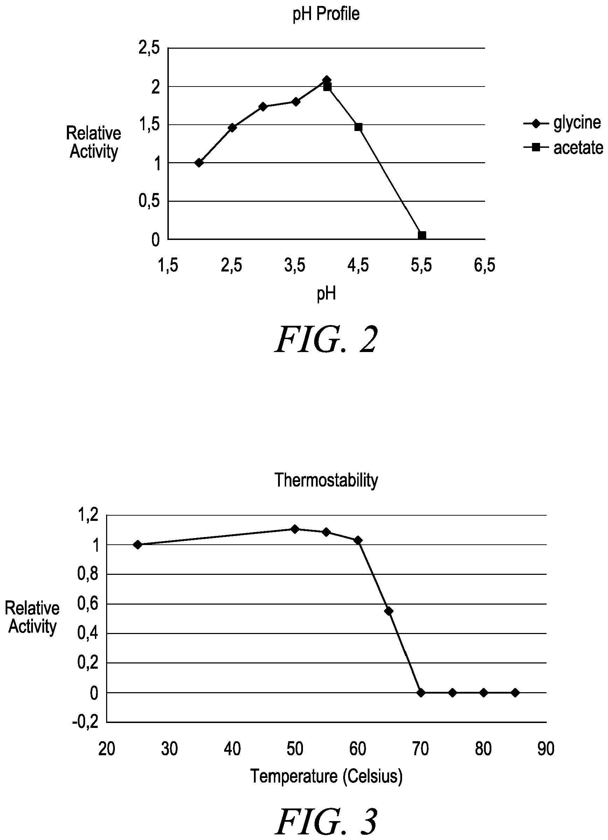

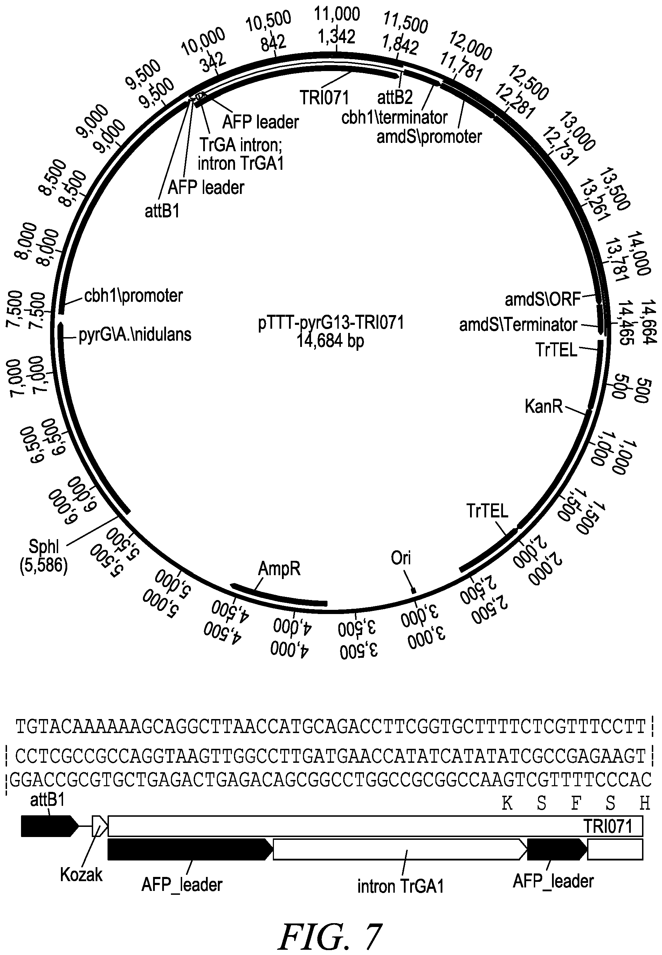

Synthetic genes encoding proline tolerant tripeptidyl peptidases were generated using preferred codons for expression in *Trichoderma reesei* except for TRI079 (SEQ ID No. 57) and TRI083 (SEQ ID No. 56) that were generated as genomic sequences. The predicted secretion signal sequences (SignalP 4.0: Discriminating signal peptides from transmembrane regions. Thomas Nordahl Petersen, Søren Brunak, Gunnar von Heijne & Henrik Nielsen. Nature Methods, 8:785-786, 2011) were replaced (except for TRI079 and TRI083) by the secretion signal sequence from the Trichoderma reesei acidic fungal protease (AFP) and an intron from a Trichoderma reesei glucoamylase gene (TrGA1) (see Figure 7 lower panel). Synthetic genes were introduced into the destination vector pTTT-pyrG13 (as described in US8592194 B2) using LR Clonase™ enzyme mix (Life Technologies) resulting in the construction of expression vectors pTTT-pyrG13 for the proline tolerant tripeptidyl peptidases herein. Expression vectors encoding SEQ ID No's 1, 2 and 29 are shown in Figure 1 and encoding SEQ ID No's 12 and 39 are shown in Figure 7. Expression vectors encoding SEQ ID No's 96 or 97 (TRI045) are shown in Figure 2.5-10 µg of the expression vectors were transformed individually into a suitable Trichoderma reesei strain using PEG mediated protoplast transformation essentially as described in (US8592194 B2). Germinating spores were harvested by centrifugation, washed and treated with 45 mg/ml of lysing enzyme solution (*Trichoderma harzianum,* Sigma L1412) to lyse the fungal cell walls. Further preparation of protoplasts was performed by a standard method, as described by Penttilä et al. (Gene 61 (1987) 155-164).

Spores were harvested using a solution of 0.85% NaCl, 0.015% Tween 80. Spore suspensions were used to inoculate liquid cultures
Cultures were grown for 7 days at 28°C and 80% humidity with shaking at 180 rpm. Culture supernatants were harvested by vacuum filtration and used to measure expression and enzyme performance.

### EXAMPLE 2

### Purification and characterization

### A. Purification of proline tolerant tripeptidyl peptidase

Desalting of samples was performed on PD10 column (GE Life Sciences, USA) equilibrated with 20 mM Na-acetate, pH 4.5 (buffer A). For ion exchange chromatography on Source S15 HR25/5 (GE Life Sciences, USA) the column was equilibrated with buffer A. The desalted sample (7 ml) was applied to the column at a flow rate of 6 ml/min and the column was washed with buffer A. The bound proteins were eluted with a linier gradient of 0-0.35 M NaCl in 20 mM Na-acetate, pH 4.5 (35 min). During the entire run 10 ml fractions were collected. The collected samples were assay for tripeptidyl amino-activity as described below. Protein concentration was calculated based on the absorbance measure at 280 nm and the theoretical absorbance of the protein calculated using the ExPASy ProtParam tool (http://web.expasy.org/cgi-bin/protparam/protparam).

### B. Determination of proline tolerant tripeptidyl peptidase and endopeptidase activity

The chromogenic peptide H-Ala-Ala-Ala-pNA (MW = 387.82; Bachem, Switzerland) was used to determine the activity of proline tolerant tripeptidyl peptidase in the samples produced as described above. The assay was conducted as follows, 10 µL of the chromogenic peptide solution (10 mM dissolved in dimethlyl sulfoxide; DMSO) were added to 130 µl Na-acetate (20 mM, adjusted to pH 4.0 with acetic acid) in a microtiter plate and heated for 5 minutes at 40°C. 10 µL of appropriately diluted enzyme was added and the absorption was measured in an MTP reader (Versa max, Molecular Devices, Denmark) at 405 nm. One katal of proteolytic activity was defined as the amount of enzyme required to release 1 mole of p-nitroaniline per second.

Azoscasein assay for endoprotease activity.

A modified version of the endoprotease assay described by Iversen and Jorgensen, 1995 is used. An enzyme sample of 50 µl is added to 250 µl of azocasein (0.25% w/v; from Sigma) in 4 times diluted Mcllvaine buffer, pH 5 and incubated for 15 min at 40°C with shaking (800 rpm). The reaction is terminated by adding 50 µl of 2 M TCA and centrifugation for 5 min at 20,000 g. To a 195 µl sample of the supernatant 65 µl of 1 M NaOH is added and absorbance at 450 nm is measured. One unit of endoprotease activity is defined as the amount which yields an increase in absorbance of 0.1 in 15 min at 40°C.

The proline tolerant tripeptidyl peptidase samples produced as described in Example 1 were found to be essentially free of endopeptidase side-activity. Upon purification as described in Example 2A, substantially no endopeptidase side activity was detected.

### C. pH profile

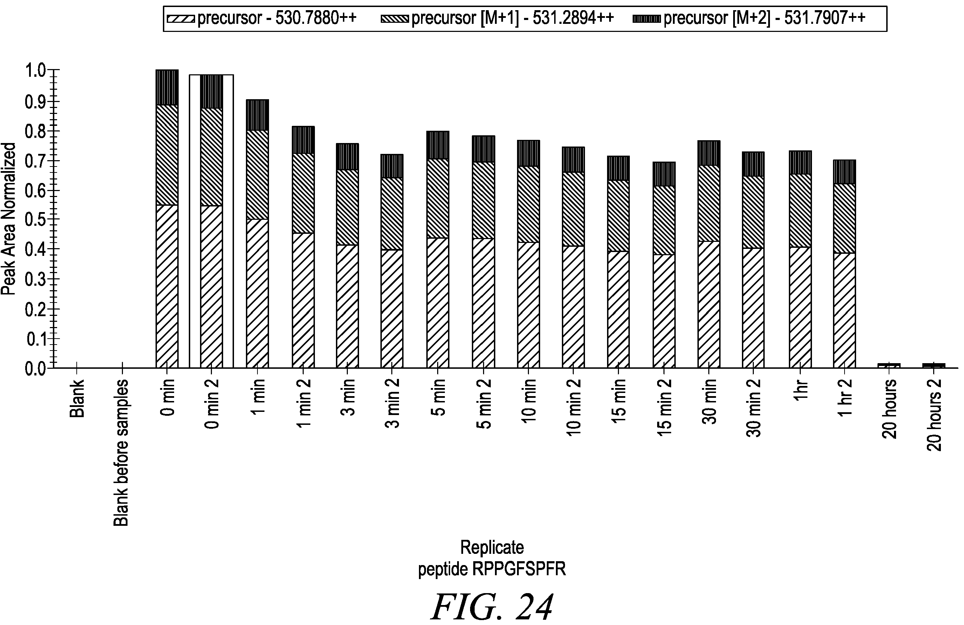

The proline tolerant tripeptidyl peptidase assay described with H-Ala-Ala-Ala-pNA substrate above with modification of using the buffers 20 mM Na-glycine (pH 2.0, 2.5, 3.0, 3.5 and 4.0) or 20 mM Na-acetate buffer (pH 4.0, 4.5 and 5.5) was used to determine the pH profile of proline tolerant tripeptidyl peptidase TRI083 (Figure 2) and TRI045 (Figure 24). Optimum pH of TRI083 and TRI045 was observed to be 4.0.

### D. Temperature profile

The proline tolerant tripeptidyl peptidase assay described above was used at temperatures 25, 50, 55, 60, 65, 70, 75, 80 and 85°C. The optimum temperature of proline tolerant tripeptidyl peptidase TRI083 was found to be 50°C, whereas no activity was found at 70°C and higher temperatures (Figure 3).

### Protein hydrolysis using a proline tolerant tripeptidyl peptidase in combination with an endoprotease (Alphalase® AFP)

The enzymes: Alphalase® AFP and the proline tolerant tripeptidyl peptidase TRI083 expressed as described in Example 1.

Assay buffer: 50 mM NaOAc, pH 4.0, 3% dimethylhemoglobin (Sigma Aldrich), 37 ºC, 1h incubation (100 µl reaction mixture per MTP well). Stop/colour reagent: 0.05% trinitrobenzenesulfonic acid in 125 mM Na borate pH 8.6 (200 µl per well).

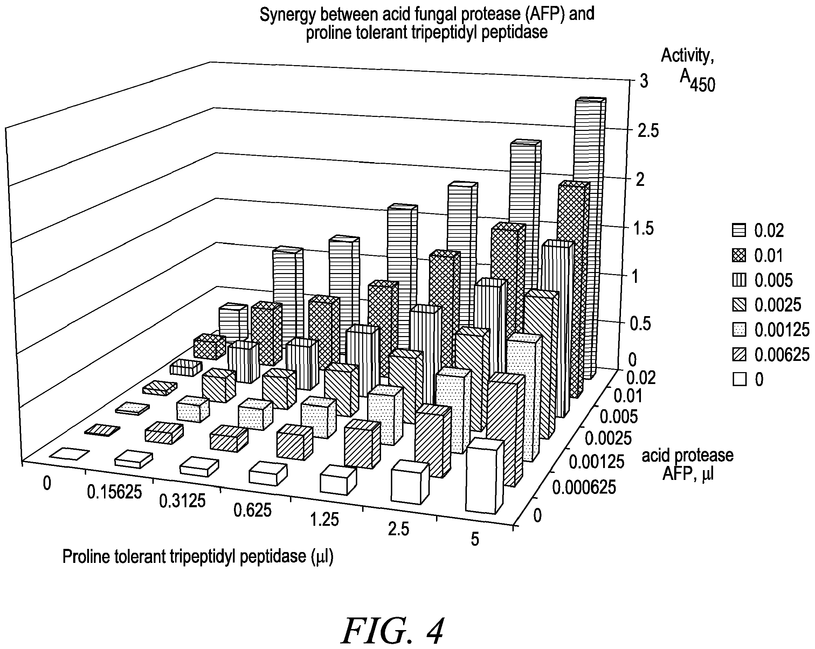

The plate was read at 450 nm using a Versa max microplate reader (Molecular Devices) after about 20 min incubation with stop/colour reagent. The results of the assay (Figure 4) show a synergistic effect when an endoprotease (Alphalase® AFP) is used in combination with the proline tolerant tripeptidyl peptidase.

### Hydrolysis of whey protein (WPI) employing proline tolerant tripeptidyl peptidase in combination with an endopeptidase

For whey protein hydrolysis, Lacprodan-9224 (Aria Food Ingredients, Denmark) was employed. A 20% (w/w) WPI suspension was prepared in H₂Od and adjusted to pH 4.0 with acetic acid (Sigma-Aldrich, Denmark). To prevent microbial growth, 0.0285% (w/w) NaN₃ was added. A volume of 200 µl of the WPI suspension was transferred into each of the 96 wells of a microtiter plate (MTP; VWR, Denmark). Subsequently, 5 µl of each consecutively diluted peptidase, Alphalase® FP2 and proline tolerant tripeptidyl peptidase (3PP = TRI083; 0 - 8750 nkat H-Ala-Ala-Ala-pNA/mL activity in stock solution added), were added to each well, resulting in dosages as shown in Table 1. The MTP was sealed and placed in an iEMS incubator/shaker (Thermo Scientific, Denmark) at 40°C. After 24 h of incubation and shaking at 400 rpm, the hydrolyses were stopped by addition of 20 µl of 2 M trichloroacetic acid (TCA; Sigma-Aldrich, Denmark). As a reference, a plain 20% WPI (w/w) suspension was used which was treated in the same manner as the WPI hydrolyses. Unhydrolysed, precipitated WPI was removed by filtration (0.22 µm). On the filtered WPI hydrolysate o-phthaldehyde (OPA) derivatization was conducted according to Nielsen et al. (2001) with minor modifications. A sample volume of 25 µl was transferred to a well and 175 µl of OPA-reagent, dissolved in phosphate buffer pH 11, was added subsequently. The measured absorptions at 340 nm in an MTP reader (Molecular Devices, Denmark) were transformed into the relative degree of hydrolysis (DH) in per cent.

A 20% (w/w) WPI suspension hydrolysed in 6 M HCI (Sigma-Aldrich, Denmark) at 100°C for 24 h served as reference (100% DH).

As shown in table 1, the combination of proline tolerant tripeptidyl peptidase and endopeptidase Alphalase® FP2 gave the best degree of hydrolysis. The supplementation of endopeptidase with the proline tolerant tripeptidyl peptidase resulted in generally higher DH than without the addition of TRI083 (SEQ ID No. 1, SEQ ID No. 2 or SEQ ID No. 29). For example, the addition of 5 ul 2,188 nkat/ml TRI083 to 0.6% Alphalase® FP2 resulted in an increased degree of hydrolyses of 25.0%, whereas the supplementation of 1.2% (w/w) Alphalase® FP2 with 5 ul 8,375 nkat/ml TRI083 resulted in an increase of 21.4% in DH.

**Table 1: Degree of hydrolysis of 20% (w/w) WPI hydrolysed with an endopeptidase in combination with a proline tolerant tripeptidyl peptidase (3PP)**

| TRI083 dosage [nkat/mL stock solution] | | | | |
|---|---|---|---|---|
| Alphalase® FP2 dosage_{applied WPI} [%] | 0.00 | 2,188 | 4,375 | 8,375 |
| 0.0 | 0.0 | 0.5 | -0.9 | 1.4 |
| 0.6 | 8.8 | 11.1 | 11.0 | 11.5 |
| 1.2 | 11.2 | 12.2 | 12.9 | 13.6 |
| 4.6 | 17.8 | 19.4 | 20.2 | 20.2 |

### Hydrolysis of 33-mer gliadin peptide

The proline tolerant tripeptidyl peptidase was examined for its ability to hydrolyse a synthetic substrate from alpha gliadin (alpha-2-gliadin) by LC-MS and label-free quantification. It was found to cleave the substrate into tri-peptides irrespective of high proline content in the substrate.

### Experimental set-up

The 33-mer of gliadin (alpha-2-gliadin) (aa56-88) H-LQLQPFPQPQLPYPQPQLPYPQPQLPYPQPQPF-OH (Zedira GmbH; D-64293 Darmstadt, Germany) C190H273N43O47 (MW=3911.46) (1mg/ml; 0.26 mM) was incubated at 24 °C in the presence of proline tolerant tripeptidyl peptidase (0.01 mg/ml TRI083) in a total volume of 1000 ul buffer (pH=4.5) (the ratio substrate/enzyme 100:1, w/w). Aliquots (100 ul) of enzyme reaction were stopped with 20 ul 5% trifluoroacetic acid (TFA) after 0, 1, 3, 5, 10, 15 and 30 minutes, respectively. The samples were then transferred to new vials and analyzed on the LTQ Orbitrap mass spectrometer.

### Data Acquisition, label free quantification and MS/MS data analysis

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ±10 ppm relative to masses on the list.

The RAW files were accessed with the open source program Skyline 1.4.0.4421 (available from MacCoss Lab Software, University of Washington, Department of Genome Sciences, 3720 15^{th} Ave NE Seattle, Washington, US) which can use the MS1 intensities to build chromatograms. The precursor isotopic import filter was set to a count of three, (M, M+1, and M+2) at a resolution of 60,000 and the most intense charge state was used. Peptide sequences of the two substrates as well as their cleavage products were typed into Skyline and intensities were calculated in each sample.

The LC-MS/MS data was manually annotated using GPMAW to calculate theoretical values of fragmentation.

The triple charged mass of the intact alpha-2-gliadin peptide was isolated and followed over time. The intact peptide was not detectable after 3 min and was very quickly hydrolyzed (Table 2). Full hydrolysis of the 33-mer alpha-2-gliadin peptide would give the following tri-peptides: LQL' QPF' PQP' QLP' YPQ' PQL' PYP' QPQ' LPY' PQP' QPF. The intermediate product YPQPQLPYPQPQLPYPQPQPF resulting from cleaving off the four tri-peptides LQL, QPF, PQP and QLP from the alpha-2-gliadin substrate was found to accumulate and then to decrease (Table 2).

**Table 2: Relative MS peak intensities of alpha-2-gliadin and derived peptides**

| | 0 min | 1 min | 3min | 5 min | 10 min | 15 min | 30 min |
|---|---|---|---|---|---|---|---|
| Alpha-2 gliadin | 100 | 10 | 0 | 0 | 0 | 0 | 0 |
| YPQPQLPYPQPQLPYPQPQPF | 79 | 100 | 52 | 23 | 23 | 23 | 20 |
| LQL | 3 | 91 | 88 | 94 | 94 | 100 | 100 |
| QPF | 0 | 4 | 55 | 7 | 129 | 26 | 100 |
| PQL | 8 | 21 | 30 | 32 | 42 | 58 | 100 |
| PYP | 4 | 7 | 12 | 15 | 28 | 48 | 101 |
| LPY | 5 | 21 | 46 | 62 | 90 | 100 | 94 |

The accumulation of most of the expected tri-peptides was detected. The underlined tri-peptides were found and confirmed based their MS/MS fragmentation: LQL' *QPF* PQP' QLP' YPQ' PQL' PYP' QPQ' LPY' PQP' *QPF,* whereas the *QPF* tri-peptides were found only based on their mass.

In conclusion, proline tolerant tripeptidyl peptidase was found to cleave the substrate alpha-2-gliadin consecutively into tri-peptides irrespective of a high proline content. During the hydrolysis proline was present in P3, P2, P1, P1', P2' and P3' positions, respectively.

This is in contrast to that previously found tripeptidyl peptidase do not cleave proline in P1 or P1' positions (US7972808B2, US5821104, Reichard et al. 2006, Applied and Environmental Microbiology 72, 1739-1748).

### Cleavage of AAPPA peptide

Proline tolerant tripeptidyl peptidase was examined for its ability to hydrolyse a synthetic substrate AAPPA by LC-MS and label free quantification. The peptide H-AAPPA-NH2 (MW=424.49, from Schafer-N, Copenhagen) was dissolved in 20 mM MES buffer, pH=4.0 (1mg/ml). 1000 ul of the H-AAPPA-NH2 solution was incubated with 200 ul proline tolerant tripeptidyl peptidase (TRI083) solution (40ug/ml) (substrate/enzyme 100:0.8) at room temperature. At seven time points (0, 5, 15, 60, 180, 720 and 1440 min) 100 ul samples were withdrawn, diluted with 50ul 5% TFA, heat inactivated (10 min at 80°C) and kept at -20°C until LC-MS analysis.

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ±10 ppm relative to masses on the list.

The RAW files were accessed with the open source program Skyline 1.4.0.4421 which can use the MS1 intensities to build chromatograms. The precursor isotopic import filter was set to a count of three, (M, M+1, and M+2) at a resolution of 60,000 and the most intense charge state was used. Peptide sequences of the substrate as well as a cleavage product was typed into Skyline and intensities were calculated in each sample.

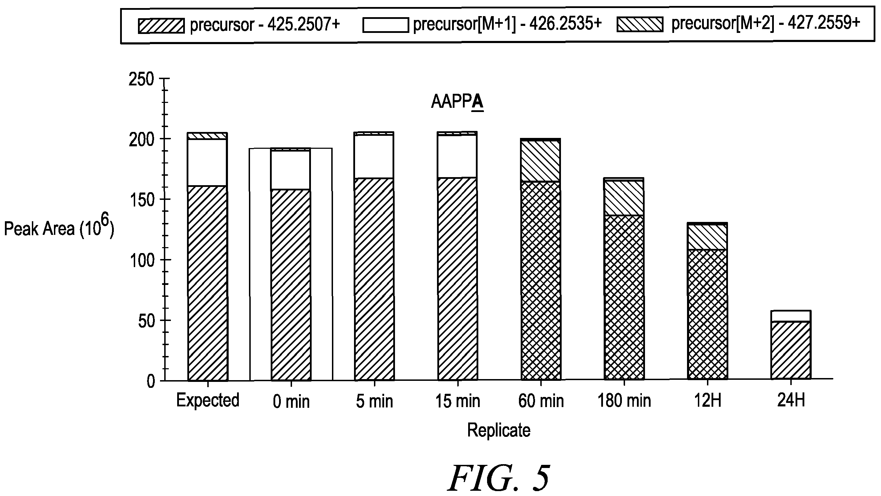

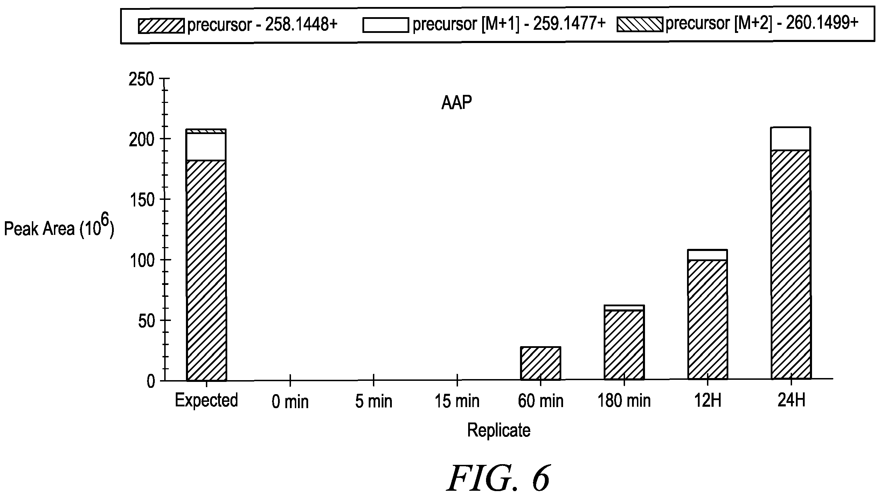

The analysis showed that proline tolerant tripeptidyl peptidase over time is able to degrade the peptide AAPPA (Figure 5) and generate the product AAP (Figure 6), indicating that the PP peptide bond in AAPPA is hydrolysed by the proline tolerant tripeptidyl peptidase.

### Use of proline tolerant tripeptidyl peptidase to reduce β-lactoglobulin allergenicity

The experiment was carried out using the kit: "Milk Protein ELISA Kit (β-lactoglobulin)", supplied by MIoBS (Morianga Institute of Biological Sciences, Inc., Yokohama, Japan. Catalog No. 171LG).

Reduction in potential allergenicity was investigated by treating a β-lactoglobulin solution with proline tolerant tripeptidyl peptidase, endoprotease and a combination of proline tolerant tripeptidyl peptidase and endoprotease.

Buffer: 50mM acetate, pH 4.5
β-Lactoglobulin solution: 100 µg/ml in buffer
Enzymes: proline tolerant tripeptidyl peptidase TRI083 (40000 nkat/ml in buffer) and Alphalase® AFP (100 SAPU/ml in buffer)
β-Lactoglobulin solution (1000µL) was placed in an Eppendorf tube and heated to 40°C. 20µL of buffer and 50µL of proline tolerant tripeptidyl peptidase and was added (t=0), and the solution mixed by pumping with the pipette. Immediately after addition of enzyme an aliquot of 50 µL was transferred to 950 µL ice cold sample extraction solution (supplied by the kit). Subsequent aliquots were taken and treated in the same way after 1h, 3h, 6h and 24h. All samples were stored on ice until further analysis. The experiment was repeated using 20 µl Alphalase® AFP + 50µl buffer; 20 µl Alphalase® AFP + 50 µl proline tolerant tripeptidyl peptidase or 70 µl buffer (no enzyme). All samples were analyzed using the protocol supplied in the kit (including incubating all samples in extraction solution at room temperature for 24h as the initial step of the procedure).

Using the Milk protein ELISA Kit (3-lactoglobulin) it was found that proline tolerant tripeptidyl peptidase in combination with endoprotease is able to reduce assay response and thereby the potential allergenicity significantly more than Alphalase® AFP does alone. Proline tolerant tripeptidyl peptidase alone only had a minor effect on assay response.

### EXAMPLE 3

### Materials and Methods

### 1. Enzyme treatment of cornsoy feed.

Feed flour was sifted to a particle size less than 212µm and suspended in water to 10% (w/w) slurry and pH adjusted to pH3.5. Then, 138µL of the 10% slurry was added to each well in 96 MTP well-plate using Agilent 0.5mL instruments with Biomeck NX robot. Wide bore tips were used. Then 20 µl of enzyme solution containing proteases to be tested in 20mM acetate pH 3.5 was added, after that 10µL (1.14U/µL) pepsin dissolved in water was added. The plate was incubated at 40°C for 45minutes in iEMS incubator/shaker at 1150 rpm. Then 34µL pancreatin 1.126mg/mL in 1M Na-bicarbonate was added and the plate was incubated at 40°C for 60minutes in iEMS at 1150 rpm. Afterwards, the plate was centrifuged at 5°C, 4000 rpm for 15 min, 10µL supernatant was transferred to new plates (corning plate #3641 nonbinding) containing 190µL water in each well to a 20x dilution. The obtained plates (master plates) were stored in the freezer -20°C.

### 2. Degree of Hydrolysis measurements.

The method of analysis of degree of hydrolysis (DH) of soluble protein is based on the reaction of primary amino groups with o-phthaldialdehyde (OPA - assay). Reference: P.M. Nielsen, D. Petersen and C. Dambmann. Improved Method for Determining Food Protein Degree of Hydrolysis. Journal of Food Science. 66 (2001) 642-646.

For OPA assay the following procedure was carried on. 10-25 µl feed sample treated by enzyme from master plate was transferred to the new plate, then 175 µl of OPA reagent containing sodium borate, dodecyl sulfate and dithiothreitol, were added to the plate. The end point measurements of optical density at 340 nm were performed right after 2 min and 5 second mixing.

### The effect of Alphalase® AFP (an acid protease) on corn soy feed in the presence of pepsin and pancreatin. In vitro studies.

The composition of cornsoy feed is presented in the Table below (Interactions of phytate and myo-inositol phosphate esters (IP₁₋₅) including IP₅ isomers with dietary protein and iron and inhibition of pepsin. S. Yu, A. Cowieson, C. Gilbert, P. Plumstead and S. Dalsgaard J. Anim. Sci. 90 (2012) 1824-1832. Supplementary Information).

| **Ingredient** | **Amount, %** |
|---|---|
| Corn | 60.01 |
| Soybean meal | 31.52 |
| Soy oil | 4.00 |
| Salt | 0.40 |
| DL-Methionine | 0.20 |
| Limestone | 1.16 |
| Dicalcium Phosphate | 1.46 |
| Vitamin and mineral mixture | 1.25 |

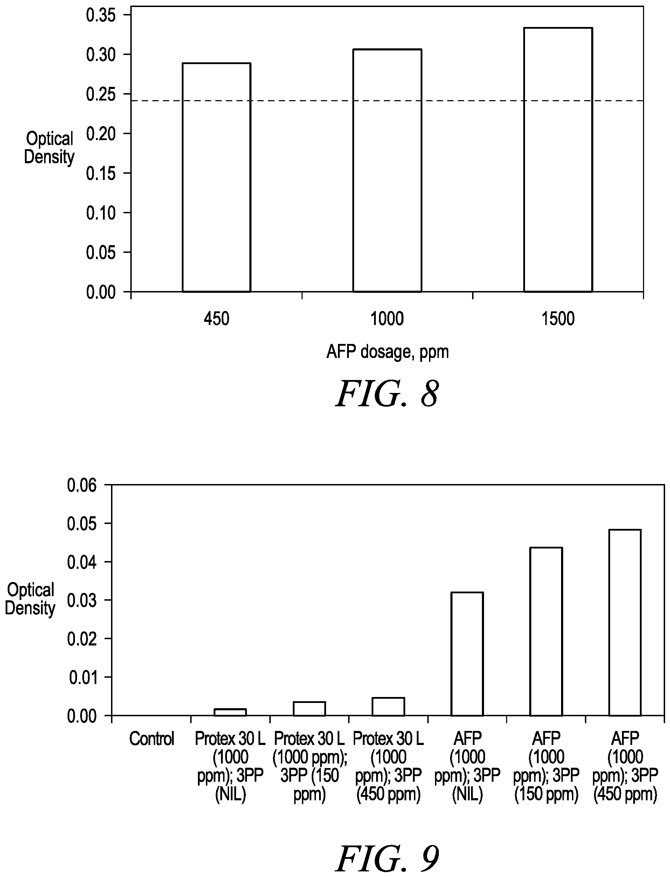

Cornsoy feed were treated with Alphalase® AFP (NSP24, available at Genencor Division, Food Enzymes) (herein referred to as "AFP") at different concentrations (450, 1000 and 1500 ppm in relation to the cornsoy feed) in the presence of pepsin and pancreatin (Figure 8). The results are presented below; the level of improvement of cornsoy feed DH is 4.5, 6.3 and 9.0 %, respectively.

### EXAMPLE 4

### The effect of a proline tolerant tripeptidyl peptidase on cornsoy feed in the presence of pepsin and pancreatin and in the presence and in the absence of Alphalase® AFP. In vitro studies.

In these studies, Alphalase® AFP and proline tolerant tripeptidyl peptidase were used only at the dosages of 1000 and 450 ppm, respectively. The results are presented in Table 3. In the control experiment only pepsin and pancreatin were used. Improvement of hydrolysis is the ratio between the treatment and control.

**Table 3. The effect of proline tolerant tripeptidyl peptidase and Alphalase® AFP on hydrolysis of cornsoy feed.**

| | **Control** | **3PP** | **AFP** | **AFP+3PP** |
|---|---|---|---|---|
| **Degree of hydrolysis, %** | 24.5±2.0 | 25.5±1.6 | 28.6±3.6 | 32.3±2.6 |
| **Improvement of hydrolysis, %.** | 100±8.2 | 104±6.5 | 117±14.7 | 132±10.7 |

As can be seen from Table 3 proline tolerant tripeptidyl peptidase on its own does not give sufficient benefit in cornsoy protein hydrolysis. The performance of Alphalase® AFP is similar to the results presented in the Example 3. However, the combination of proline tolerant tripeptidyl peptidase and Alphalase® AFP gives the maximal results and can be associated with the synergetic action of endo- and exo- proteases.

### EXAMPLE 5

### Validation of dose response of proline tolerant tripeptidyl peptidase in combination with endo-proteases on cornsoy feed hydrolysis in the absence of pepsin and pancreatin.

The aim of the work was to identify the origin of the enzymes performance and truly monitor possible additive or synergetic performance of endo- and exo-proteases. For this reason, the experiments were performed in the absence of pepsin and pancreatin and the results are summarized in Figure 9.

As can be seen from Figure 9, the degree of hydrolysis when proline tolerant tripeptidyl peptidase is used with endoproteases is very pronounced. It is clear that this phenomenon is synergetic, since the dose response depends on the level of proline tolerant tripeptidyl peptidase and the nature of endoprotease. In the case of Alphalase® AFP the effect is much more pronounced.

### EXAMPLE 6

### Validation of dose response of proline tolerant tripeptidyl peptidase in combination with different dosages of Alphalase® AFP endo-proteases on cornsoy feed hydrolysis in the presence of pepsin and pancreatin.

To estimate the level of synergism between Alphalase® AFP and proline tolerant tripeptidyl peptidase, the experiment with different dosages of the enzymes and in the presence of pepsin and pancreatin were performed.

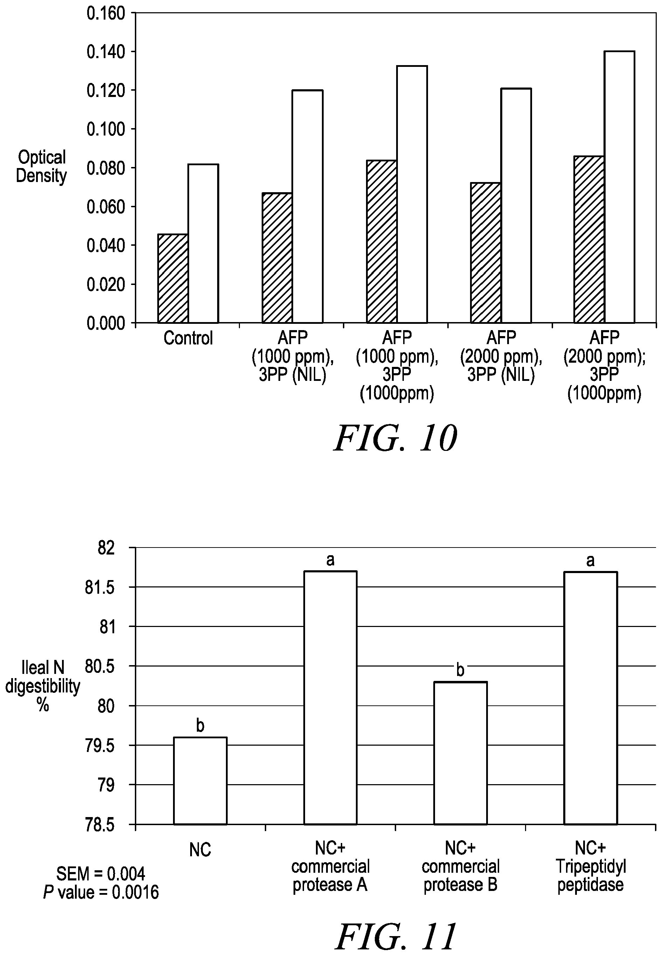

As can be seen from Figure 10 the level of degree of hydrolysis is not dependent on concentration of proline tolerant tripeptidyl peptidase and Alphalase® AFP in the composition. It likely that for both enzymes the concentrations 1000 ppm and 2000 ppm correspond to the saturation level. However, the degree of hydrolysis is dramatically increased when the treatment time increased from 100 min to 200 min.

### EXAMPLE 7

### Analysis of 20 tripeptidyl peptidases for their low pH stability at pH 2.5 40°C 60min

As requirements for utilization of the enzymes in feed for monogastric animals, the enzymes should be active at lower pH. For this reason the number of tripeptidyl peptidases has been tested with the reaction on synthetic substrate AAF (H-Ala-Ala-Phe-pNA . BACHEM, L-1095), at pH 2.5.

The tripeptide substrate AAF was prepared at 2.5mg/ml in DMSO. Tripeptidyl peptidases were used as broth. The reaction mixture containing 2.5µl enzyme and 90µL glycine acetic acid-Tris buffer (each buffer components at 50mM, pH 2.5) was prepared in 96 well incubation plates.

The incubation plate was mixed and incubated at 40°C for 60min. Then 50µl 0.2M Mes-NaOH pH 6.0 was added to each well, mixed at 600rpm for 2min. After that, 5ul of the incubation mixture were taken to 96 well assay plate already filled with 85µl 0.1 M acetic acid buffer, pH 4.0 and 5µl 2.5mg/ml AAF substrate solution.

The reaction mixture was stirred at 600rpm for 2min and read directly in a microplate reader (Molecular Devices, Denmark) at 410nm at 30°C every 30 second for 15min.

To measure initial enzyme activity, similar procedure was performed except the step of enzyme incubation at 40°C for 60 min.

The results of measuring initial and final activity with activity recovery are presented in Table 4.

**Table 4. Initial, final activity and its recovery for tripeptidyl peptidases.**

| **Protein name** | **OD₄₁₀nm (Initial)** | **OD₄₁₀nm (Final)** | **Activity recovery (%)** |
|---|---|---|---|
| TR1050.3 (SEQ ID No. 7/SEQ ID No. 34) | 0.603 | 0.501 | 83 |
| TR1053.1 (SEQ ID No. 10/SEQ ID No. 37) | 0.345 | 0.356 | 103 |
| 29.9 Induction control | | | |
| Morph 1.1 Background | | | |
| TRI050 (SEQ ID No. 7/SEQ ID No. 34) | 0.895 | 0.814 | 91 |
| TRI071 (pool1) 2% glu/soph (SEQ ID No. 12/SEQ ID No. 39) | 0.757 | 0.589 | 78 |
| TRI071 (pool1) 2% glu/soph (SEQ ID No. 12/SEQ ID No. 39) | 0.815 | 0.672 | 82 |
| TRI071 (pool1) 4% glu/soph (SEQ ID No. 12/SEQ ID No. 39) | 0.466 | 0.453 | 97 |
| TRI071 (pool1) 4% glu/soph (SEQ ID No. 12/SEQ ID No. 39) | 0.67 | 0.6 | 90 |

From Table 4 it can been seen that 11 out of 20 samples of tripeptidyl peptidases are considerably stable at pH 2.5 and 40°C for 60min since over 70% activities were retained. Keeping in mind that the final four samples are the same molecule (TRI071), and 2 samples are negative controls. From these results it can be concluded that proline tolerant tripeptidyl peptidases are stable at low pH.

### Conclusions

This work demonstrated the synergies of the serine exopeptidase proline tolerant tripeptidyl peptidase with the serine endoprotease FoodPro® 30L and also the aspartic acid protease Alphalase® AFP in the hydrolysis of corn soy based feed under conditions mimicking the monogastric digestion system. It was also shown that many tripeptidyl peptidases are stable at low pH and can be considered for feed applications.

### EXAMPLE 8

### Proline tolerant tripeptidyl peptidase in animal feed

A total of 288 one day old Ross 308 male broiler chicks were purchased form a commercial hatchery. At study initiation, 8 birds were randomly allocated to battery cages according to respective treatments by blocks. Only healthy birds were selected for the experiment, and no birds were replaced throughout the course of the study. The study consisted of the following treatments (Table 5):

**Table 5 - Experimental design**

| **Dietary treatment** | **Protease inclusion** | **Phytase level (FTU/kg)** |
|---|---|---|
| **1. Negative control (NC)** | - | 500 |
| **2. NC + commercial product A** (trypsin family protease 75000 PROT/g activity) | 0.2g/ kg | 500 |
| **3. NC + commercial product B** (subtilisin family protease 2750-3500 GSU/g activity) | 4000U/ kg | 500 |
| **4. NC + tripeptidyl peptidase** | 0.01 g/ kg | 500 |

Bird weights were recorded at study initiation (d0) on day 14, and at study termination (d21). The cage is the experimental unit. Diets were fed in mash form and were formulated to meet or exceed NRC standards, except for Ca and AvP (Table 5). All feed was mixed using a Davis S-20 mixer, the mixer was flushed between each treatment to prevent cross contamination between rations. Samples were collected from each treatment diet from the beginning, middle and end of each batch and were minced together for analysis of enzyme activity in feed.

All birds were fed a corn soy base ration until day 14; from day 14 the treatment rations were fed. Phytase was added to all treatment rations. At the feed change, feeders were removed from the cages, weighed back, emptied, and refilled with the appropriate treatment diet. On the final day of the study (d21), feed and birds were weighed, to determine feed intake (FI) and body weight gain (BWG) for the experimental period. Pens were checked daily for mortality. When a bird was culled or found dead, the date and removal weight (kg) were recorded. A gross necropsy was performed on all dead or culled birds to determine the possible cause of death. Feed conversion ratio (FCR) corrected for mortality was determined.

On d 21, all birds per cage were euthanised by intracardial injection of sodium pentobarbitone and contents of the lower ileum were expressed by gentle flushing with distilled water. Digesta from birds within a cage were pooled, resulting in eight samples per dietary treatment. The digesta samples were frozen immediately after collection, lyophilised and processed. Diets and digesta samples were analysed for the marker, nitrogen (N) and gross energy to enable calculation of digestibility co-efficients.

**Table 6 - Diet formulations**

| **Ingredient %** | **0-14 days** | **14-21 days** |
|---|---|---|
| Maize | 48.78 | 57.09 |
| Soybean Meal 48%CP | 40.06 | 34.7 |
| Canola meal | 4 | 4 |
| Soybean Oil | 3 | 1.35 |
| L-Lysine HCl | 0.13 | 0.07 |
| DL-methionine | 0.28 | 0.22 |
| L-threonine | 0.03 | 0 |
| Salt | 0.33 | 0.33 |
| Limestone | 1 | 0.98 |
| Dicalcium Phosphate | 2.09 | 0.97 |
| Poultry Vits/TE's | 0.3 | 0.3 |
| ***Calculated Analyses*** | | |
| PROTEIN % | 24.98 | 22.97 |
| MEP MJ/KG | 12.4 | 12.34 |
| CALCIUM % | 1.05 | 0.76 |
| AV PHOS % | 0.5 | 0.3 20 |
| ALYS % | 1.27 | 1.1 |
| AM+C % | 0.94 | 0.84 |
| ATHRE % | 0.83 | 0.73 |
| ATRYP % | 0.26 | 0.23 |

### Statistical Analysis

Data were analyzed using ANOVA, and means separation conducted to test differences between the different enzymes and enzyme dosages. Cage was used as the experimental unit.

### Results

**Table 7 - Performance and digestibility results**

| | **BWG (g)** | **FI (g)** | **FCR (kg/kg)** | **Ileal N Digestibility %** | **Ileal digestible energy (MJ/kg)** |
|---|---|---|---|---|---|
| NC | 386.3^{b} | 576.3^{a} | 1.496^{a} | 79.6^{b} | 12.45b |
| NC + commercial protease A (trypsin family protease 75000 PROT/g activity) | 414.1^{ab} | 582^{a} | 1.429^{b} | 81.7^{a} | 12.98a |
| NC + commercial protease B (subtilisin family protease 2750-3500 GSU/g activity) | 386.2^{b} | 572.2^{a} | 1.486^{a} | 80.3^{b} | 12.89a |
| NC + tripeptidyl peptidase | 420.8^{a} | 597.7^{a} | 1.422^{b} | 81.7^{a} | 13.12a |
| SEM | 11.1 | 9.4 | 0.018 | 0.4 | 0.112 |
| *Effect tests* | | | | | |
| Treatment | 0.0627 | 0.2563 | 0.0092 | 0.0016 | 0.0011 |

| | | | | | |
|---|---|---|---|---|---|
| Each value represents the mean of 9 replicates (8 birds per replicate). ^{ab} Means in a column not sharing a common superscript are different (P < 0.05). | | | | | |

Supplementation of the proline tolerant tripeptidyl peptidase resulted in a significant reduction in FCR compared to the negative control and commercial protease B and a numerical reduction in comparison to commercial protease A.

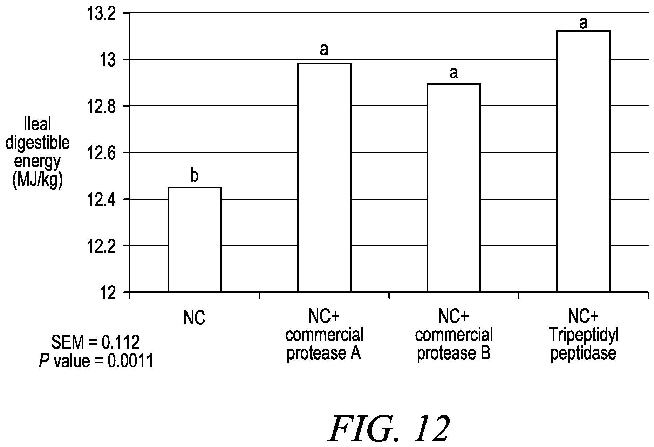

Proline tolerant tripeptidyl peptidase supplementation significantly increased BWG compared to the NC, this was not the case for either commercial protease A or commercial protease B. Proline tolerant tripeptidyl peptidase significantly increased ileal N digestibility % compared to the control and commercial protease A (Figure 11). Proline tolerant tripeptidyl peptidase also significantly increased the energy digested to a level significantly greater than the negative control and numerically greater than the two commercial proteases (Figure 12).

### Conclusions

In conclusion, supplementation of proline tolerant tripeptidyl peptidase resulted in significantly better bird performance than the NC and commercial protease B in terms of FCR and BWG. There was a numerical increase in BWG and reduction in FCR when proline tolerant tripeptidyl peptidase was supplemented compared to commercial protease A.

The improvements in bird performance we driven by improved energy and protein (N) digestibility compared to the commercial proteases.

### EXAMPLE 9

### Determination of proline tolerant tripeptidyl peptidase, aminopeptidase and endopeptidase activities

For proline tolerant tripeptidyl peptidase the chromogenic peptide H-Ala-Ala-Ala-pNA was used. The assay was conducted as follows, 27.5 µL of appropriately diluted enzyme was added to 200 µl Mcllvaine buffer (20 mM, pH 4.0). After incubation at 50°C for 5 min, 12.5 µL of the chromogenic peptide solution (5 mg/mL dissolved in dimethly sulfoxide (DMSO); Sigma-Aldrich, Denmark) were added to the reaction mixture. The reaction was terminated by adding 50 µL of 0.5 M trichloroacetic acid (TCA; Sigma-Aldrich, Denmark). After centrifugation (15,000 x *g,* 5 min), 240 µL of the solution were transferred to a microtiter plate (MTP; Kisker-biotech, Germany) and the absorption was measured in an MTP reader (Molecular Devices, Denmark) at 405 nm. One katal of proteolytic activity was defined as the amount of enzyme required to release 1 mol of *p*-nitroaniline per second.

Aminopeptidase activity was measured as described in Stressler, Eisele et al. 2013. Endopeptidase side-activity was measured with the azocasein as described in Example 2.

### EXAMPLE 10

### Whey Protein Hydrolysis employing an endopeptidase in combination with a proline tolerant tripeptidyl peptidase and a general aminopeptidase

A recombinantly in *E. coli* expressed and His-tag purified general aminopeptidase (PepN) from *Lactobacillus helveticus* ATCC 12046 was employed for whey protein isolate (WPI) hydrolyses (Stressler, Eisele et al. 2013). The applied PepN had biochemical parameters such as pH-optimum, temperature-range and sequence specificity comparable to other PepNs in the scientific literature (Table 8) and is therefore representative for general aminopeptidases from pro- and eukaryotic species.

**Table 8: Biochemical parameters and sequence specificity of general aminopeptidases (PepN) from different origin**

| Origin | pH-optimum] | temp.-[-range [C] | Substrate Specificity | Literature |
|---|---|---|---|---|
| *Streptomyces* sp. TH-4 | 7.0 | 30 - 55 | Gly, Ile, Val, Asp, Ala, Glu, His, Pro, Lys, Phe, Leu, Arg, Tyr | 1 |
| *Aspergillus oryzae* | 8.0 | 20 - 80 | Leu, Gly, Pro, Tyr, Lys, Ala, Phe, Glu, Arg, Met | 2 |
| *Geobacillus thermodenitrificans* NG80-2 | 8.0 | 20 - 90 | Leu, Met, Phe, Val, Gly, Asp | 3 |
| *Lactobacillus helveticus* ATCC 12046 | 6.5 | 10 - 50 | Ala, Lys, Arg, Leu, Phe, Val, Ile, Pro | 4 |

| | | | | |
|---|---|---|---|---|
| 1: Hatanaka, 2007; Appl Microbiol Biotechnol 74:347-356; 2: (Nakadai, Nasuno et al. 1973); **3:** (Wang, Guo et al. 2012); **4:** (Stressler, Eisele et al. 2013) | | | | |

The applied PepN in WPI hydrolysis was free of endopeptidase and X-prolyl-dipeptidyl (PepX) side-activity, which has been assayed by either the azocasein assay or with H-Ala-Pro-pNA as a substrate for PepX, respectively.

For whey protein hydrolysis, Lacprodan-9224 (Aria Food Ingredients, Denmark) was employed, a 15% (w/w) WPI suspension was prepared in H₂O_{d} and adjusted to pH 4.5 with acetic acid (Sigma-Aldrich, Denmark). To prevent microbial growth, 0.0285% (w/w) NaN₃ was added. Prior to the exopeptidase hydrolysis, 1% (w/w_{applied WPI}) Alphalase® FP2 was added to initiate the hydrolysis of the WPI in order to generate substrates for the exopeptidases (tripeptidyl peptidase and general aminopeptidase). A volume of 200 µl of the WPI suspension was transferred into each of the 96 wells of a microtiter plate (MTP; VWR, Denmark). Subsequently, 5 µl of consecutively diluted PepN (0 - 1572nkat_{H-Ala-*p*NA}/mL) and proline tolerant tripeptidyl peptidase (0 -70,000 nkat_{H-Ala-Ala-Ala-*p*NA}/mL) were added to each well. Then, the MTP was sealed and placed in an incubator (iEMS incubator/shaker HT, Thermo scientific, Denmark) which was incubated at 40°C. After 24 h of incubation at 400 rpm, the hydrolyses were stopped by addition of 20 µl of 2 M trichloroacetic acid (TCA; Sigma-aldrich, Denmark), except for the reference (0 h), whereas the TCA was added prior to endo- and exopeptidase addition. Unhydrolysed, precipitated WPI was removed by filtration (0.22 µm; Corning 3504 filter plate, Corning incorporated, USA). The filtered WPI hydrolysate was employed for o-phthaldehyde (OPA) derivatization (Nielsen, Petersen et al. 2001). The OPA derivatization was conducted according to Nielsen et al., (2001) with minor modifications. A sample volume of 25 µl was transferred to a well and 175 µl of OPA-reagent, containing trisodium phosphate-dodecahydrate, was added. The measured absorptions at 340 nm in a MTP reader (VersaMax, Molecular Devices, Denmark) were optionally transformed in the relative degree of hydrolysis (DH) in per cent, whereas a 15% (w/w) WPI suspension was prepared in 6 M HCI (Sigma-Aldrich, Denmark) and held at 100 °C for 24 h. The acid hydrolysate of the 15% (w/w) WPI served as a reference (100% DH) for the calculation of the degree of hydrolysis.

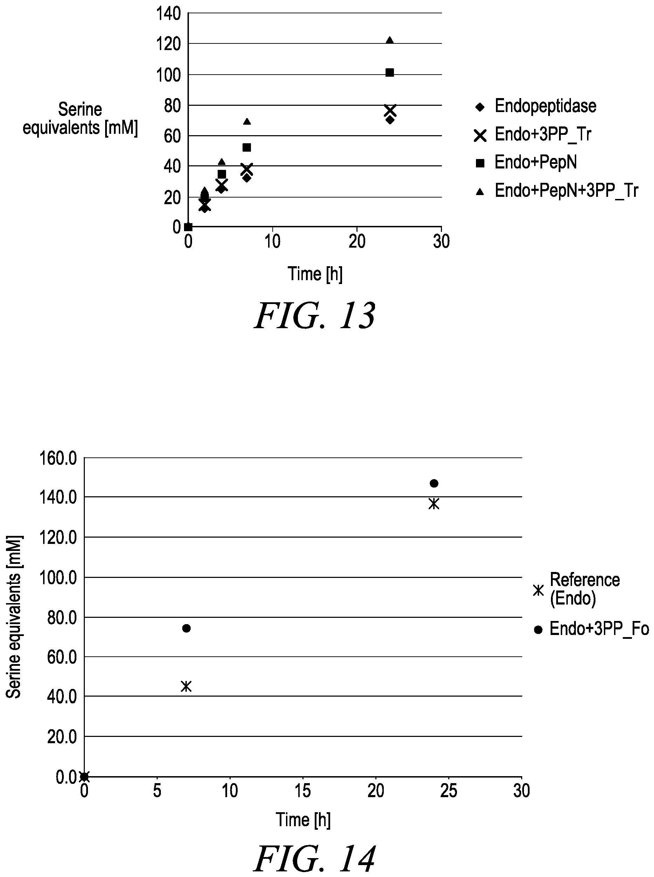

Figure 13 shows the increase of free alpha amino groups, expressed as serine equivalents (SE), over 24 h employing different endo- and exopeptidase combinations. The lowest increase in SE was determined with the sole addition of endopeptidases, followed by the combination of endopeptidase and proline tolerant tripeptidyl peptidase (TRI083), which releases tripeptides from the N-terminal end of peptides. Due to the fact that the proline tolerant tripeptidyl peptidase TRI083 is product inhibited by its liberated tripeptides, the increase in SE is not very high. The supplementation of the endopeptidase with a recombinatly expressed and His-tag purified general aminopeptidase (PepN) from *Lactobacillus helveticus* ATCC12046 resulted in a significant increase in SE due to the liberation of free amino acids (Figure 13). The combined application of endopeptidase, TRI083 and PepN resulted in a significantly higher release of free alpha amino groups compared to the addition of endopeptidase and TRI083 as well as endopeptidase and PepN of 43.5% (Figure 13).

A summary of the additional effect of the endopeptidase, the proline tolerant tripeptidyl peptidase and the general aminopeptidase (PepN) is shown in table 9.

**Table 9: Summary of the additional effect of an endopeptidase combined with a proline tolerant tripeptidyl peptidase (TRI083) and a general aminopeptidase (PepN) in a 15% WPI hydrolysis after 24 h.**

| | | | | | | | | | | | | |
|---|---|---|---|---|---|---|---|---|---|---|---|---|
| PepN [nkat/mL] | 0 | 197 | 393 | 0 | 0 | 0 | 197 | 197 | 197 | 393 | 393 | 393 |
| 3PP_Tr [nkat/ml] | 0 | 0 | 0 | 2,188 | 4,375 | 8,750 | 2,188 | 4,375 | 8,750 | 2,188 | 4,375 | 8,750 |
| Increase of DH [%] | | | | | | | **32.8** | **43.5** | **38.8** | **62.4** | **41.7** | **50.1** |

### EXAMPLE 11

### Casein hydrolysis employing an endopeptidase in combination with a proline tolerant tripeptidyl peptidase (TRI083) and a general aminopeptidase (PepN)

The hydrolysis of a 5% (w/w) sodium caseinate solution was conducted in the same set-up as described for the whey protein hydrolysis in Example 2, except that 5% (w/w) sodium caseinate (Aria Foods, Denmark) was employed. As shown in Table 10, independent of the applied activity of PepN or TRI083, the determined serine equivalents (SE) could be increased either by the addition of TRI083 to the endopeptidase or by addition of PepN to the endopeptidase.

In addition, a synergistic effect was observed for high PepN and high TRI083 dosages (Table 11). The doubling of the PepN activity from 786 to 1572 nkat/mL resulted in a rather small increase of serine equivalents of 10 mM, whereas the application of 17,500 nkat/mL TRI083 increased the SE by 18 mM. The application of both TRI083 and PepN at 17,500 and 1,572 nkat/mL, resulted in an increase of 97 mM SE. This corresponds to a 21% increased SE value compared to the sum of the sole additions of TRI083 and PepN on top of the endopeptidase.

**Table 10: Dose response of a 5% (w/w) sodium caseinate hydrolysis with an endopeptidase in combination with different activities of TRI083 and PepN after 24 h of hydrolysis, which is quantified in serine equivalents (SE)**

| TRI083 **[nkat/mL]** | | | | | |
|---|---|---|---|---|---|
| **PepN [nkat/mL]** | 0 | 1,094 | 2,188 | 4,375 | 8,750 |
| 0 | 0 | 4 | 4 | 11 | 11 |
| 25 | 8 | 16 | 15 | 16 | 17 |
| 49 | 15 | 26 | 17 | 27 | 35 |
| 98 | 27 | 33 | 34 | 44 | 39 |
| 197 | 36 | 41 | 37 | 45 | 47 |
| 393 | 49 | 58 | 51 | 56 | 62 |
| 786 | 52 | 59 | 62 | 67 | 67 |

### EXAMPLE 12

### Casein hydrolysis employing an endopeptidase in combination with a proline tolerant tripeptidyl peptidase and partly inactivated Flavourzyme 500

The sodium caseinate hydrolysis was conducted as describe in Example 10 except that partly inactivated Flavourzyme 500 (FZ500; Sigma-Aldrich, Denmark) instead of a purified PepN was applied. In order to inactivate the endopeptidases, FZ 500 was heat treated (55°C, 24 h). The endopeptidase activity was significantly reduced (96% inactivated, 4% residual activity). After heat treatment of the FZ 500, about 97% residual PepN activity was determined.

**Table 11: Dose response of a 5% (w/w) sodium caseinate hydrolysis with an endopeptidase in combination with a proline tolerant tripeptidyl peptidase (TRI083) and partly inactivated FZ 500 after 24 h of hydrolysis, which is quantified in serine equivalents (SE).**

| TRI083 [nkat/mL] | | | | | |
|---|---|---|---|---|---|
| **Flavourzyme 500** | 0 | 1,094 | 2,188 | 4,375 | 8,750 |
| Without FZ 500 addition | 0 | 8 | 10 | 17 | 21 |
| 64-fold diluted | 2 | 7 | 12 | 20 | 23 |
| 32-fold diluted | 0 | 18 | 13 | 27 | 39 |
| 16-fold diluted | 4 | 19 | 9 | 26 | 30 |
| 8-fold diluted | 17 | 25 | 25 | 26 | 35 |
| 4-fold diluted | 25 | 43 | 33 | 37 | 69 |
| 2-fold diluted | 54 | 65 | 56 | 67 | 86 |
| undiluted | 92 | 107 | 101 | 107 | 94 |

As shown in Table 11, the addition of TRI083 to either an endopeptidase, or endopeptidase which has been supplemented with several exopeptidases (Flavourzyme 500 (FZ500; Sigma-Aldrich, Denmark)) resulted for both in increased serine equivalents values, suggesting that the degree of hydrolysis of a hydrolysate produced with a complex mixture of exopeptidases, such as FZ500, can be increased by applying a tripeptidyl peptidase (e.g. a proline tolerant tripeptidyl peptidase).

### EXAMPLE 13

### Whey Protein Hydrolysis (WPI) employing an endopeptidase in combination with proline tolerant tripeptidyl peptidase homologues.

For WPI hydrolysis, Lacprodan-9224 (Aria Food Ingredients, Denmark) was employed, a 15% (w/w) WPI suspension was prepared in H₂O_{d} and adjusted to pH 4.5 with acetic acid (Sigma-Aldrich, Denmark). To prevent microbial growth, 0.0285% (w/w) NaN₃ was added. Subsequently, 1% (w/w) Alphalase® FP2 was added and a volume of 200 µl of the WPI suspension was transferred into each of the 96 wells of a microtiter plate (MTP; VWR, Denmark). Following this, 5 µl of each proline tolerant tripeptidyl peptidase homologue containing either 0 or 4375_{nkatH-Ala-Ala-Ala-*p*NA}/mL was added to the particular wells of the MTP. Then, the MTP was sealed and placed in an incubator at 40°C (iEMS incubator/shaker HT, Thermo Scientific, Denmark).

After 24 h of incubation and shaking at 400 rpm, the hydrolyses were stopped by addition of 20 µl of 2 M trichloroacetic acid (TCA; Sigma-Aldrich, Denmark), except for the reference (0 h), to which the TCA was added prior to endo- and exopeptidase addition. Unhydrolyzed, precipitated WPI was removed by filtration (0.22 µm; Corning 3504 filter plate, Corning Incorporated, USA). The filtered WPI hydrolysate was employed for o-phthaldehyde (OPA) derivatization (Nielsen, Petersen et al. 2001). The OPA derivatization was conducted according to Nielsen et al., (2001) with minor modifications. A sample volume of 25 µl was transferred to a well and 175 µl of OPA-reagent, dissolved in trisodium phosphate-dodecahydrate, was added subsequently. The measured absorptions at 340 nm in a MTP reader (VersaMax, Molecular Devices, Denmark) were transformed into serine equivalents employing a serine calibration curve (0 - 2 mM).

Proline tolerant tripeptidyl peptidase TRI071 (SEQ ID No. 12 or SEQ ID No. 39) increased the measured serine equivalents significantly over time compared to the reference (endopeptidase). The relative increase of SE was about 64% for TRI071 after 7 h of hydrolysis, respectively (Figure 14).

### EXAMPLE 14

### Product inhibition of a proline tolerant tripeptidyl peptidase (TRI083) with a native tripeptide (H-Ala-Ala-Ala-OH)

Inhibition experiments were conducted with TRI083 and Ala-Ala-Ala-pNA (0 - 2000 nM) as substrate in the assay described in Example 8. The assay was run without inhibitor, with 3.6 mM tripeptide (H-Ala-Ala-Ala-OH; Bachem, Switzerland), 18 mM dipeptide (H-Ala-Ala-OH; Bachem, Switzerland) or 18 mM alanine (Sigma-Aldrich, Denmark). Curve fitting and inhibition analysis was done by treating H-Ala-Ala-Ala-OH, H-Ala-Ala-OH and alanine as competitive inhibitors using GraphPad Prism version 6.02 for Windows, GraphPad Software, La Jolla California USA, www.graphpad.com.

For the tripeptide H-Ala-Ala-Ala-OH strong inhibition with a Ki of 0.34 mM was found, whereas the dipeptide H-Ala-Ala-OH only showed very weak inhibition with a Ki of 40 mM. Alanine showed no inhibition at all.

### EXAMPLE 15

### Decreased product inhibition of a proline tolerant tripeptidyl peptidase (TRI083) with a native tripeptide (H-Ala-Ala-Ala-OH) pre-hydrolysed with a general aminopeptidase (PepN)

The inhibition experiments were set up as described in Example 14. Furthermore, 1 mg/mL H-Ala-Ala-Ala-OH (Bachem, Switzerland) was subjected to general aminopeptidase (PepN) hydrolysis for 24 h at 40 °C in 12.5 times diluted Mcllvaine buffer (pH 4.0). For this, 1.2 mg/mL H-Ala-Ala-Ala-OH was dissolved in 8 mL 10 times diluted Mcllvaine buffer (pH 4.0) and filled up to 10 mL with purified PepN (1,572 nkat/mL [pH 6.5]). After 24 h of hydrolysis, the PepN hydrolysis was terminated by heat inactivation of the PepN (90 °C; 10 min). The degree of pre-hydrolysis of H-Ala-Ala-Ala-OH was determined by gas chromatography (Husek, 1991; FEBS-Letters 280, 354-356). About 0.18 mg/mL alanine was measured, which corresponds to about 54% hydrolyses of the initially applied H-Ala-Ala-Ala-OH. Subsequently, TRI083 was analyzed for product inhibition without inhibitor, with 1 mg/mL H-Ala-Ala-Ala-OH as well with the PepN pre-hydrolyzed H-Ala-Ala-Ala-OH (1 mg/mL).

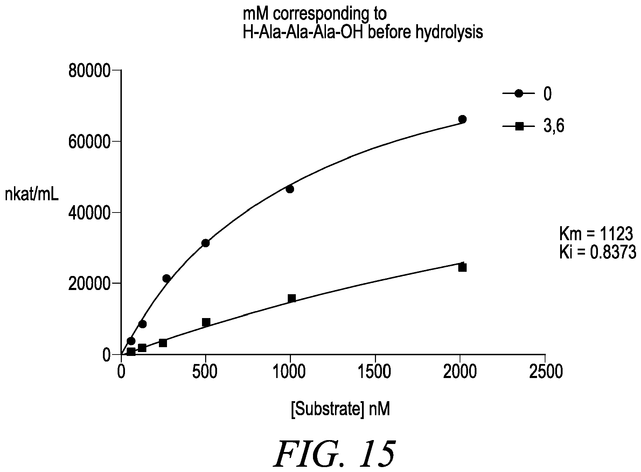

As shown in Figure 15 the pre-hydrolyzed H-Ala-Ala-Ala-OH was found to have Ki of 0.8 mM. This is in good agreement with the previously determined H-Ala-Ala-Ala-OH Ki of 0.34 mM, since slightly more than half of the H-Ala-Ala-Ala-OH had been pre-hydrolysed to H-Ala-Ala-OH and alanine. This indicates that a general aminopeptidase can reduce the product inhibition of a proline tolerant tripeptidyl peptidase by cleaving tripeptides into dipeptides and free amino acids.

### EXAMPLE 16

### Proline tolerant tripeptidyl peptidase in reduction of immunogenicity of β - lactoglobulin peptides in cow milk allergy (CMA)

### Description of hydrolysis procedure

β-lactoglobulin (1 mg/ml), in 10 mM Tris-HCl, pH 8.0, was hydrolysed at 25°Cfor 24 h with trypsin (10 ug/ml). After 24 h trypsin was inactivated by warming up to 80°Cfor 15 minutes.

The β-lactoglobulin used has the amino acid sequence:

The tryptic β-lactoglobulin hydrolysate (1mg/ml) in 10 mM TRIS HCL- pH 4.0 was diluted five-fold in 20 mM MES buffer pH 4.0. The hydrolysis was initiated by adding 100 ul of proline tolerant tripeptidyl peptidase TRI083 solution (80 ug/ml) in 20 mM MES buffer, pH 4.0, to 1 ml of the 5-fold diluted β-lactoglobulin solution (200 ug/ml) and incubated at RT.

The proteolysis by proline tolerant tripeptidyl peptidase was terminated after 1, 5, 15, 30, 60, 120, and 180 min as well as 24 h by addition of 5% TFA (50 ul) to 100 ul of the β-lactoglobulin solution. The proline tolerant tripeptidyl peptidase was heat inactivated at 80°C for 10 minutes. The solution was stored at -20°C until further LC-MS/MS analysis.

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ±10 ppm relative to masses on the list.

The RAW files were accessed with the open source program Skyline 1.4.0.4421 which can use the MS1 intensities to build chromatograms. The precursor isotopic import filter was set to a count of three, (M, M+1, and M+2) at a resolution of 60,000 and the most intense charge state was used. Peptide sequences of the substrate as well as a cleavage product was typed into Skyline and intensities were calculated in each sample.

### Results

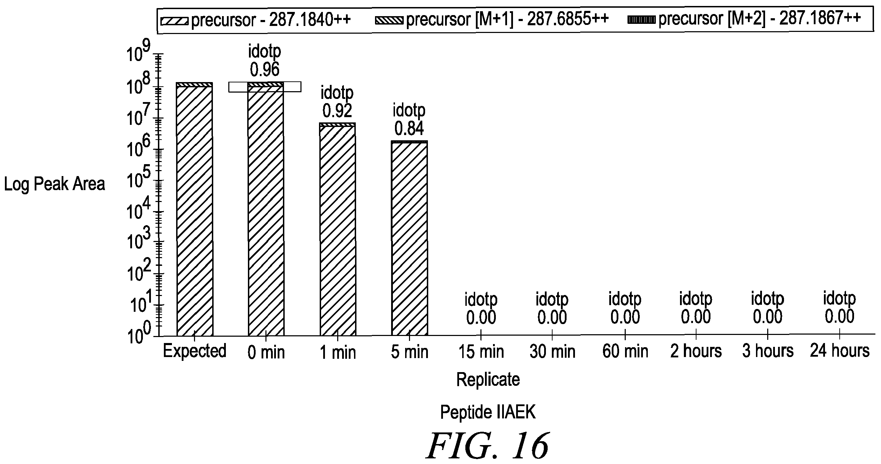

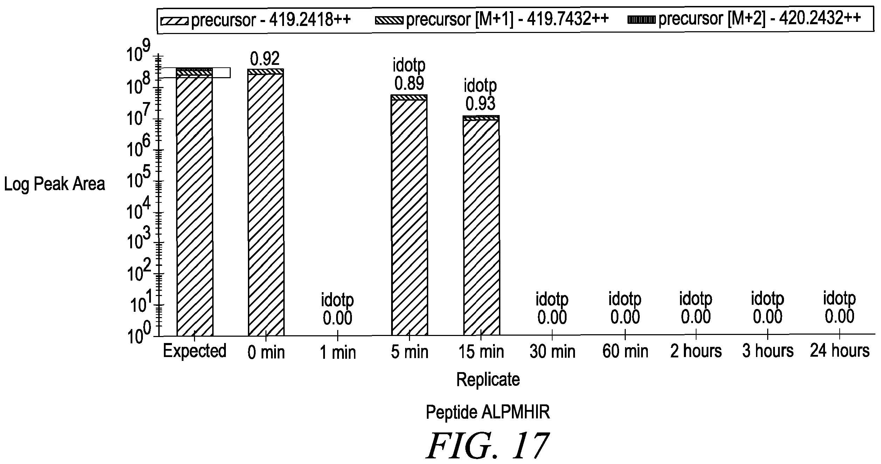

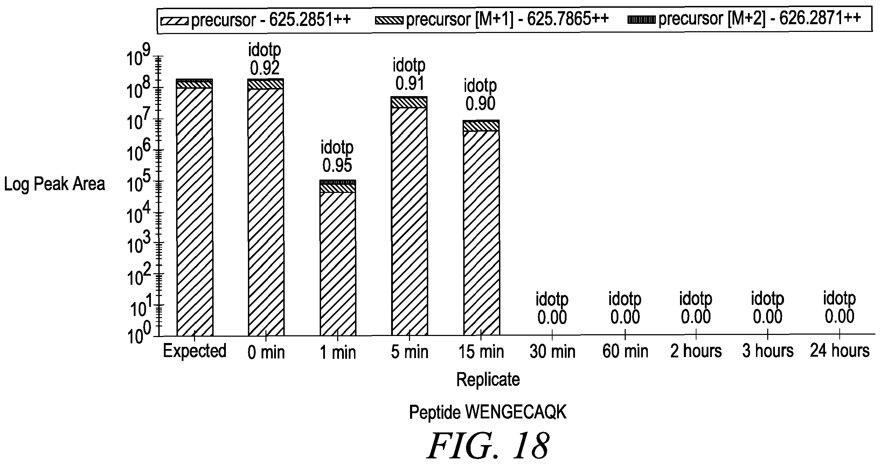

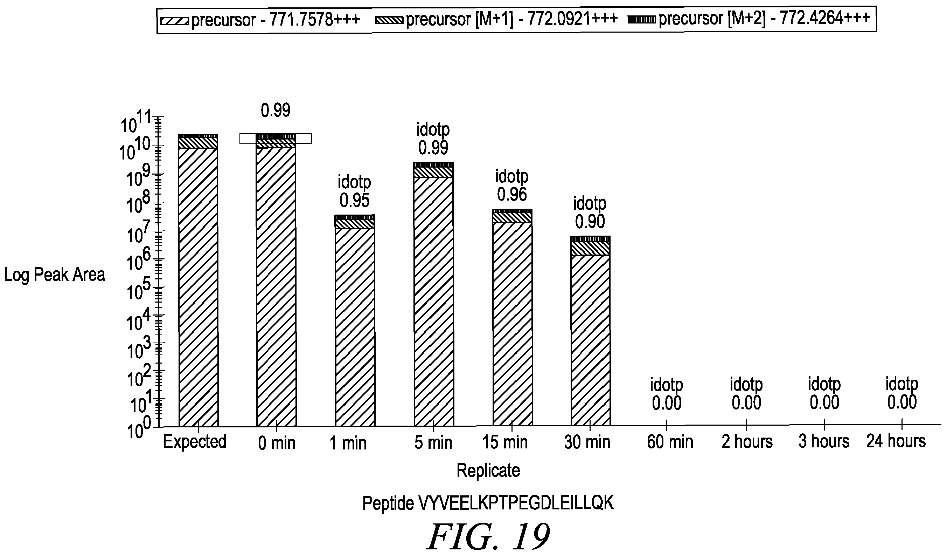

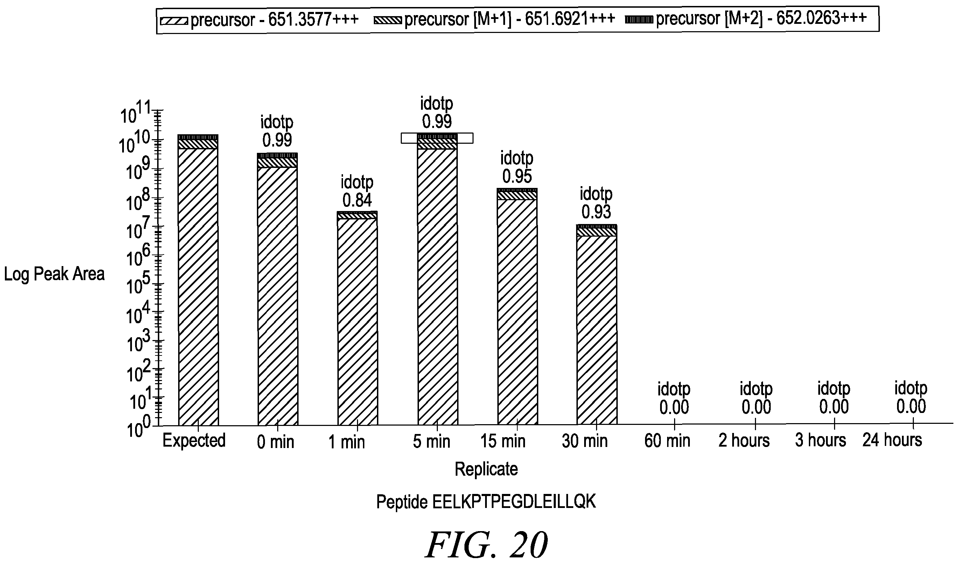

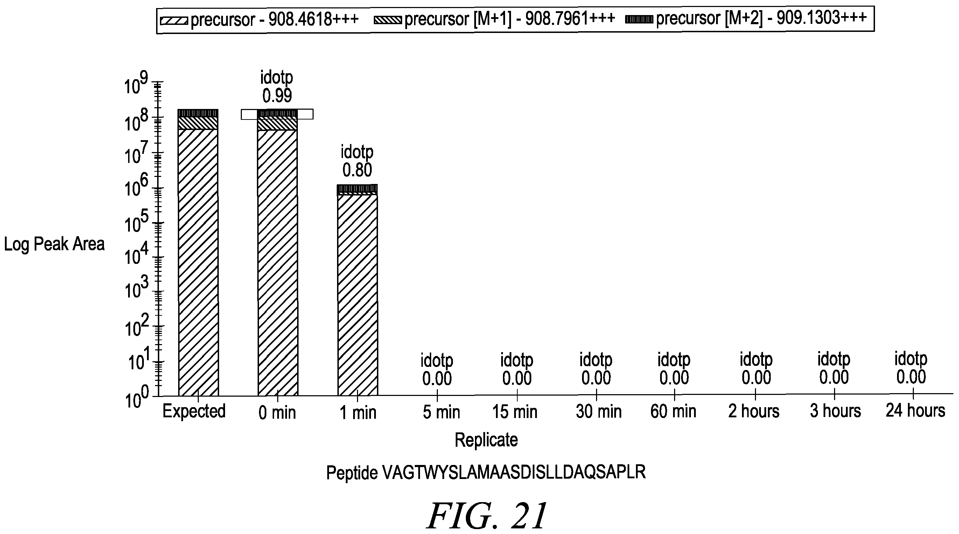

Trypsin treated β-lactoglobulin was efficiently hydrolysed by the proline tolerant tripeptidyl peptidase as shown in Figures 16-21. Notably, the proline tolerant tripeptidyl peptidase had a very broad substrate specificity and was able to cleave peptides having proline at P1 (Figure 17) and glycine at P1 (Figure 21).

### EXAMPLE 17

### Hydrolysis of beta-casein by a proline tolerant tripeptidyl peptidase

β-casein was first hydrolysed with trypsin before being subjected to cleavage with a proline tolerant tripeptidyl peptidase. β-casein (1 mg/ml) in 10 mM Tris-HCI pH=8.0, was hydrolysed at RT for 24 h with trypsin (10 ug /ml) and then inactivated by warming up to 80°C for 15 minutes.

The tryptic β-casein hydrolysate (1 mg/ml) in 10 mM TRIS HCL- pH 4.0 was diluted five-fold in 20 mM MES buffer pH 4.0. The hydrolysis was initiated by adding 100 ul of proline tolerant tripeptidyl peptidase TRI083 solution (80 ug/ml) in 20 mM MES buffer, pH 4.0, to 1 ml of the 5-fold diluted β-casein solution (200 ug/ml) and incubated at RT.

The proteolysis by proline tolerant tripeptidyl peptidase was terminated after 1, 5, 15, 30, 60, 120, and 180 min as well as 24 h by addition of 5% TFA (50 ul) to 100 ul of the β-casein solution. The proline tolerant tripeptidyl peptidase was heat inactivated at 80°C for 10 minutes. The solution was stored at -20°C until further LC-MS/MS analysis performed as described in Example 16.

The beta-casein used had the following amino acid sequence:

### Results

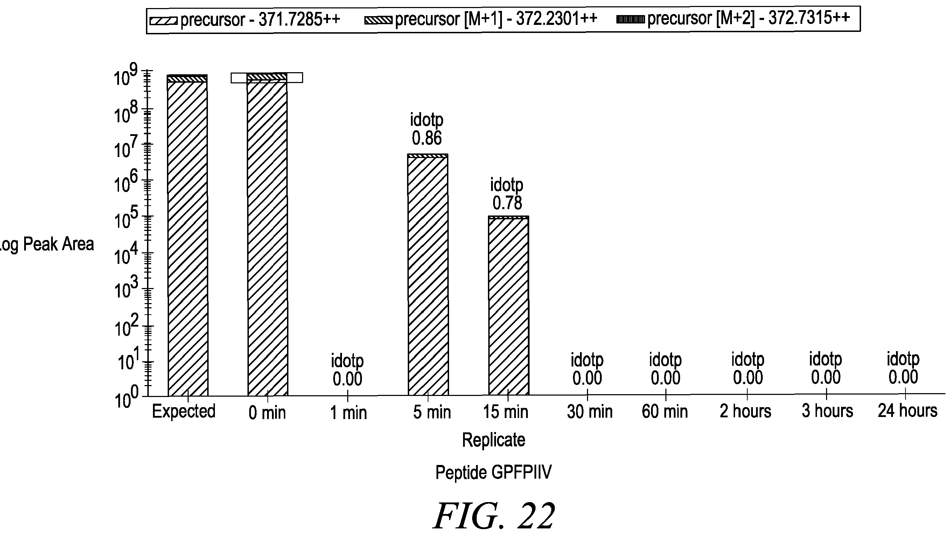

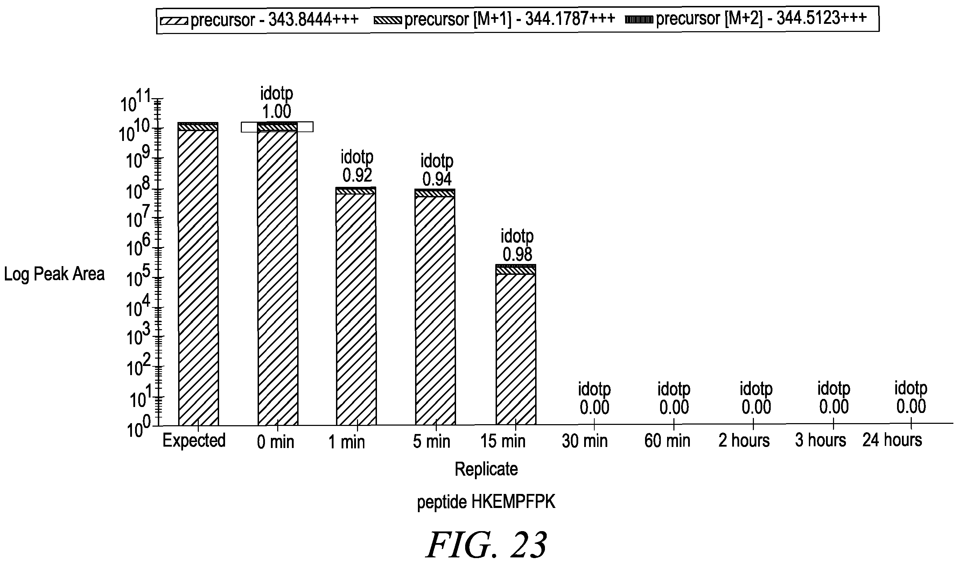

The peptide GPFPIIV resulting from a tryptic digest of β-casein is very hydrophobic and has a very bitter taste. Figure 22 shows that the proline tolerant tripeptidyl peptidase is able to efficiently digest this peptide with proline in the P1' position and in the P2 position. Figure 23 shows that the peptide HKEMPFPK is also efficiently digested by the proline tolerant tripeptidyl peptidase.

### EXAMPLE 18

### Degradation of a bradykinin peptide by a proline tolerant tripeptidyl peptidase

Bradykinin (RPPGFSPFR; MW 1660,21; B2259 from Sigma Aldrich) was dissolved at 1 mg/ml in 20 mM Mcllvaine buffer (pH 4.5). The proline tolerant tripeptidyl peptidase. TRI083 (10 ug) was added to the peptide solution. The hydrolysis was terminated after 1, 3, 5, 15, 30, and 60 min as well as 20 h by addition of 5% TFA (50 ul) to 100 ul of the solution. The proline tolerant tripeptidyl peptidase was heat inactivated at 80°C for 10 minutes. The solution was stored at -20°C until further LC-MS/MS analysis performed as described in Example 16.

### Results

The proline tolerant tripeptidyl peptidase efficiently digests the bradykinin peptide having the amino acid sequence RPPGFSPFR (see Figure 24). This shows that the proline tolerant tripeptidyl peptidase is capable of degrading substrates having proline in the P1 and P2 position, a characteristic which is highly surprising for tripeptidyl peptidases.

### EXAMPLE 19

### Hydrolysis of AAXF-NH₂ substrate with proline tolerant tripeptidyl peptidase TRI079

### Description of hydrolysis procedure and LC-MS analysis

An AAXF-NH2 substrate library (Synthesized by Schaefer, Copenhagen) with X representing all amino acids except Cys and Met, was solubilized in 20 mM Mcllvaine buffer, pH 4.5 (1 mg in 1 ml) The peptide solution was hydrolysed at 25°C for 1 or 2 h with TRI079 (SEQ ID NO: 57), 10 ul of 1 mg/ml produced as described in EXAMPLE 1. Subsequently, the hydrolysis was stopped by addition of 5% TFA (50 ul) to 50 ul of the peptide solution. The solution was stored at -20°C until further LC-MS/MS analysis.

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ±10 ppm relative to masses on the list.

The RAW files were accessed with the open source program Skyline 1.4.0.4421 which can use the MS1 intensities to build chromatograms. The precursor isotopic import filter was set to a count of three, (M, M+1, and M+2) at a resolution of 60,000 and the most intense charge state was used. Peptide sequences of the substrate as well as a cleavage product was typed into Skyline and intensities were calculated in each sample.

### Results

The LC-MS analysis showed that TRI079 could cleave all the AAXF-NH₂ peptides into tripeptides irrespective of which amino acid was in P1. With proline present in P1 the cleavage product AAP was observed to increase over time showing that TRI079 is tolerant to proline in the P1 position.

### EXAMPLE 20

### Whey Protein Hydrolysis (WPI) employing proline tolerant tripeptidyl peptidase TRI079 in combination with endopeptidases and general aminopeptidase PepN

For WPI hydrolysis, Lacprodan-9224 (Aria Food Ingredients, Denmark) was employed, a 15% (w/w) WPI suspension was prepared in H₂O_{d} and adjusted to pH 6 with sodium hydroxide. To prevent microbial growth, 0.0285% (w/w) NaN₃ was added. Subsequently, 0.5 % (w/w on protein substrate) Food Pro® Alkaline Protease and 0.5 % (w/w on protein substrate) Food Pro® PNL was added and a volume of 200 µl of the WPI suspension was transferred into each of the 96 wells of a microtiter plate (MTP; VWR, Denmark). Following this, 5 µl of the proline tolerant tripeptidyl peptidase TRI079 containing either 0 or 2188 nkat/mL and 5 ul of PepN (described in EXAMPLE 10) containing either 0, 197 or 393 nkat/mL were added to the particular wells of the MTP. Then, the MTP was sealed and placed in an incubator at 50°C (iEMS incubator/shaker HT, Thermo Scientific, Denmark). After 24 h of incubation and shaking at 400 rpm, the hydrolysis was stopped by addition of 20 µl of 2 M trichloroacetic acid (TCA; Sigma-Aldrich, Denmark), except for the reference (0 h) to which the TCA was added prior to endo- and exopeptidase addition. Unhydrolyzed, precipitated WPI was removed by filtration (0.22 µm; Corning 3504 filter plate, Corning Incorporated, USA). The filtered WPI hydrolysate was employed for o-phthaldehyde (OPA) derivatization (Nielsen, Petersen et al. 2001). The OPA derivatization was conducted according to Nielsen et al. (2001) with minor modifications. A sample volume of 25 µl was transferred to a well and 175 µl of OPA-reagent, dissolved in trisodium phosphate-dodecahydrate, was added subsequently. The measured absorptions at 340 nm in a MTP reader (VersaMax, Molecular Devices, Denmark) were transformed into serine equivalents employing a serine calibration curve (0 - 2 mM).

As shown in Table 12 the Tripeptidyl amino-peptidase TRI079 (SEQ ID No. 57) as well as PepN strongly increased the measured degree of hydrolysis compared to the reference with only endopeptidases. An additional increase in the degree of hydrolysis was obtained, when TRI079 and PepN were combined.

**Table 12. Analysis of DH (in %) of WPI hydrolysate prepared 24 h at 50°C, pH 6.0 with a combination of TRI079 and PepN**

| | **TRI 079 [nkat/mL]** | |
|---|---|---|
| **PepN [nkat/mL]** | 0 | 2188 |
| 0 | 4.2 | 11.0 |
| 197 | 18.2 | 21.7 |
| 393 | 18.8 | 25.1 |

### EXAMPLE 21

### Hydrolysis of AAFPA-NH₂ substrate with proline tolerant tripeptidyl peptidase TRI079

### Description of hydrolysis procedure and LC-MS analysis

AAFPA-NH2 substrate (synthesized by Schaefer, Copenhagen) was solubilized in 20 mM Mcllvaine buffer, pH 4.5 (1mg in 1 ml) and hydrolysed at 25°C for up to 8 h with TRI079 (10 ul of 1 mg/ml TRI079 solution) produced as described in EXAMPLE 1. Subsequently, the hydrolysis was stopped by addition of 5% TFA (50 ul) to 50 ul of the peptide solution. The solution was stored at -20°C until further LC-MS/MS analysis.

Nano LC-MS/MS analyses were performed using an Easy LC system (Thermo Scientific, Odense, DK) interfaced to a LTQ Orbitrap Classic hybrid mass spectrometer (Thermo Scientific, Bremen, Germany). Samples were loaded onto a custom-made 2 cm trap column (100 µm i.d., 375 µm o.d., packed with Reprosil C18, 5 µm reversed phase particles (Dr. Maisch GmbH, Ammerbuch-Entringen, Germany)) connected to a 10 cm analytical column (75 µm i.d., 375 µm o.d., packed with Reprosil C18, 3 µm reversed phase particles (Dr. Maisch GmbH, Ammerbuch-Entringen, Germany)) with a steel needle. Separation was performed at a flow rate of 300nL/min using a 10 min gradient of 0-34% Solvent B (H2O/CH3CN/TFE/HCOOH (100/800//100/1 v/v/v/v)) into the nanoelectrospray ion source (Thermo Scientific, Odense, DK). The LTQ Orbitrap Classic instrument was operated in a data-dependent MS/MS mode. The peptide masses were measured by the Orbitrap (MS scans were obtained with a resolution of 60 000 at m/z 400), and up to 2 of the most intense peptide m/z were selected and subjected to fragmentation using CID in the linear ion trap (LTQ). Dynamic exclusion was enabled with a list size of 500 masses, duration of 40 s, and an exclusion mass width of ±10 ppm relative to masses on the list.

The RAW files were accessed with the open source program Skyline 1.4.0.4421 which can use the MS1 intensities to build chromatograms. The precursor isotopic import filter was set to a count of three, (M, M+1, and M+2) at a resolution of 60,000 and the most intense charge state was used. Peptide sequences of the substrate as well as a cleavage product was typed into Skyline and intensities were calculated in each sample.

### Results

The LC-MS analysis showed a reduction of AAFPA-NH₂ and accumulation of AAF over time indicating that TRI079 could hydrolyze AAFPA-NH₂ and is tolerant to proline in the P1' position.

### EXAMPLE 22

### Stability of TRI045 (SEQ ID No. 99) tripeptidyl peptidase in the presence of pepsin

### Material and methods

Pepsin solution: Swine pepsin from Sigma (P7000, 674 Sigma units/mg, www.sigma.com) was used in this example and prepared in MilliQ water at 10000 Sigma unit/ml at 10000 Sigma unit/mL

Pre-incubation mixture contained: 2.5µl TRI045 sample, 95µl GAT buffer (50mM glycine-50mM acetic acid-50mM Tris, pH3.5), 5µl pepsin in milliQ water (10000 Sigma unit/ml) (final pepsin unit concentration: 500 Sigma unit/ml reaction mixture) in a half bottom area 96 well Corning MTP. For control, the pre-incubation mixture contained 5µl water instead of 5µl pepsin. The mixture was incubated at 40°C for 60min and then kept on ice. For assaying the residual activity, the assay mixture contained the pre-incubation mixture 5µl, 0.1M acetic acid-sodium acetate (pH4.0) 85µl, 5µl H-Ala-Ala-Phe-pNA (2.5mg/ml) from BACHEM.com (L-1095.0250). The 410nm reading was followed in a microplate reader every 0.5min at 30°C. N=2.

### Results

Under the assay conditions and pre-incubation at 40°C for 60min, the residual activity was found to be over 90% for TRI045 in the presence of 500 units pepsin/mL pre-incubation mixture.

### EXAMPLE 23

### TRI045 (SEQ ID No. 99) as feed enzyme to promote feed protein hydrolysis

Animals produce and secrete proteases into their digestive tracts for feed digestion. These proteases include endoproteases of pepsin, trypsin and chymotrypsin and the exopeptidases of Carboxypeptidase A and B etc. However, animals do not produce aminopeptidases as digestive enzymes or at least not in appreciable amount. As shown in this example, the addition of the tripeptidyl peptidase TRI045 (sedolisin) promotes protein digestion in an *in vitro* system for corn soy based feed. This example shows that TRI045 had high activity between pH 4 and 6.5

### Material and Methods

The tripeptide substrate H-Ala-Ala-Phe-pNA (AAF-pNA from BACHEM.com, L-1095.0250) for sedolisin was prepared at 2.5mg/ml in DMSO. The reaction was prepared by mixing 8.5µl AAF-pNA, 7 µl DMSO, 32µl water and 50 µl buffer with pH4.14-6.57, and 0.8 µl or 1.5ul TRI045. The reaction was started at 30°C by the addition of the enzyme TRI045. The initial reaction rate was recorded using a microplate reader at every 0.5min interval at 410nm in 96 well plate.

### Results

Table 13 shows that TRI045 had optimal activity around pH5.0, but still had around 50% activity at around pH4 and pH6.5, which is ideal for gastric feed digestion. Figure 22 furthers shows that the final reaction degree reached at pH5, pH5.5 and pH6 are very similar. The high pH optimum is ideal for animal feed as in such situation pancreatin would have time to make more oligopeptide substrates for TRI045.

**Table 13. Effect of pH on the TRI045 activity using H-Ala-Ala-Phe-pNA as substrate (values are the average one test with 0.8µl TRI045 (n=2) and one test with 1.5µl TRI045 dose (n=1).**

| Buffer | 0.1M HAC-NaAC | 0.1M HAC-NaAC | 0.1M HAC-NaAC | 0.1M HAC-NaAC | 0.1M Mes-NaOH | 0.1M Mes-NaOH |
|---|---|---|---|---|---|---|
| pH | 4.1 | 4.6 | 5.0 | 5.6 | 6.0 | 6.6 |
| Relative activity with pH 5.01 as 100% | 59.4 | 81.7 | 100.0 | 80.7 | 72.2 | 48.5 |

### EXAMPLE 24

### Effect of TRI045 (SEQ ID No. 99) plus pepsin and pancreatin on hydrolysis of corn soy feed substrate.

The *in vitro* reaction system contained 140µl 10% (w/v) corn soy feed slurry (14mg corn soy feed), 10µl TRI045 in 50m M MES-NaOH pH 6.0, 10µl swine pepsin (1,14U/µL in water). The reaction was incubated with shaking at 40°C for 45min and subsequently 34µl swine pancreatin (0.4636mg/mL in 1M Na-bicarbonate) were added and further incubated for additional 60min. After incubation, the 96-well plate was centrifuged and supernatants were used for residual TRI045 activity assay and for OPA and BCA assays (see below).

The degree of Hydrolysis measurements of soluble protein is based on the reaction of primary amino groups with o-phthaldialdehyde (OPA - assay). Reference: P.M. Nielsen, D. Petersen and C. Dambmann. Improved Method for Determining Food Protein Degree of Hydrolysis. Journal of Food Science. 66 (2001) 642-646.

For OPA assay the following procedure was carried out. 10-25 µl feed sample treated by enzyme from master plate was transferred to the new plate, then 175 µl of OPA reagent containing sodium borate, dodecyl sulfate and dithiothreitol, were added to the plate. The end point measurements of optical density at 340 nm were performed right after 2 min and 5 second mixing.

To quantify the protein concentrations of each protease samples, the Pierce BCA Protein Assay Reagent Kit (Thermo Scientific, cat no. 23228) was used. The TRI045 sample was not purified before quantification. The Pierce BCA Protein Assay Kit is a detergent-compatible formulation based on bicinchoninic acid (BCA) for colorimetric detection and quantification of total proteins. This method combines the well-known reduction of Cu²⁺ to Cu¹⁺ by protein in an alkaline medium with the highly sensitive and selective colorimetric detection of the cuprous cat ion (Cu⁺¹) using a unique reagent containing bicinchoninic acid. The purple-coloured reaction product of this assay is formed by the chelation of two molecules of BCA with one cuprous ion. This water soluble complex exhibits a strong absorbance at 562nm that is nearly linear with increasing protein concentration over a broad working range (20-2000µg/mL). The macromolecular structure of protein, the number of peptide bonds and the presence of four particular amino acids Cysteine, Cystine, Tryptophan and Tyrosine are reported to be responsible for colour formation with BCA.

For OPA (total amino group released) and BCA (total protein in the soluble fraction) determinations the supernatants were diluted 20 times and 10ul was used.

In the system containing TRI045 at 1000ppm, in the presence of swine pepsin and pancreatin, the release of free amino groups from corn soy feed (as a measure of protein hydrolysis (OPA value)) increased by 9% and the protein solubility increased by 5%.

In order to test the stability of TRI045 under the *in vitro* assay conditions described above (incubation in the presence of pepsin at pH3 for 45min at 40°C and subsequently in the presence of pancreatin for 60min at 40°C). The residual activity of TRI045 was subsequently assayed. The reaction mixture contained 50µl buffer 0.1M HAC-NaAC (pH5.0), 10µl the supernatant, and 5µl AAF-pNA (5mg/ml in DMSO). The reaction rate was followed at 410nm and 30°C every 30 seconds using a microplate reader. As a control, a commercial protease at the same concentration of 1000ppm was used.

### Conclusion for Examples 22-24

The reaction rate for the control (pepsin and pancreatin only) was 4.3mOD/min, for the commercial protease it was 11.0 mOD/min, and for TRI045 it was 19.3 mOD/min. After the subtraction of the control (4.3mOD/min), the residual activity of TRI045 on AAF-pNA substrate was 2.2 times higher than for the commercial protease. These results indicate that TRI045 is stable to pepsin and pancreatin at 40°C for at least 100 minutes.

In conclusion, this example demonstrates that TRI045 is stable to pepsin and pancreatin when incubated for 105min at 40°C in the presence of corn soy feed at a pH range of 3 to 7. It has also the additional effect of increasing protein solubilization and protein hydrolysis in the presence of pepsin and pancreatin when corn soy feed was used as the substrate under conditions mimicking the monogastric digestion system (BEDFORD, M.R., & CLASSEN, H.L. (1993) "An in vitro assay for prediction of broiler intestinal viscosity and growth when fed rye-based diets in the presence of exogenous enzymes". Poultry Science, 72: 137-143)., *i.e.,* the reaction was carried out at 40°C at pH3.0-3.3 in the presence of pepsin for 45min and then pH raised to pH6.5-7.0 and addition of pancreatin for additional 60min incubation,

### Literature

Nakadai, T., et al. (1973). "Purification and properties of leucine amino-peptidase I from Aspergillus oryzae." Agricultural and Biological Chemistry 37(4): 757-765.
Nielsen, P. M., et al. (2001). "Improved method for determining food protein degree of hydrolysis." Journal of Food Science 66(5): 642-646.
Stressler, T., et al. (2013). "Characterization of the Recombinant Exopeptidases PepX and PepN from Lactobacillus helveticus ATCC 12046 Important for Food Protein Hydrolysis." PLoS ONE 8(7) e70055.
Wang, F., et al. (2012). "Biochemical and conformational characterization of a leucine amino-peptidase from Geobacillus thermodenitrificans NG80-2." World Journal of Microbiology and Biotechnology 28(11): 3227-3237.

## Claims

1. A method for the production of a hydrolysate comprising:
(a) admixing at least one protein or a portion thereof with:
(A) at least one endoprotease; and
(B) at least one proline tolerant tripeptidyl peptidase having exopeptidase activity wherein said proline tolerant tripeptidyl peptidase is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1'; and
(b) recovering the hydrolysate,
wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

2. The method according to claim 1 wherein the at least one protein is: (a) selected from the group consisting of: a plant protein, preferably wherein the protein is one or more of a gliadin, an immunogenic fragment of a gliadin, a grain protein, gluten, a soy protein; and/or (b) selected from the group consisting of: a milk-based protein, preferably wherein the protein is one or more of a casein, e.g. beta-casein; a lactoglobulin, e.g. beta-lactoglobulin; or a whey protein; and/or
(c) is an egg protein.

3. A method according to claim 1 or claim 2, wherein: the proline tolerant tripeptidyl peptidase is further capable of cleaving tri-peptides from the N-terminus of a peptide having Proline at P1 and Proline at P1'; and/or
the method further comprises admixing the recovered hydrolysate with at least one feed or food ingredient.

4. A method according to any one of the preceding claims, wherein said at least one tripeptidyl peptidase is capable of cleaving tri-peptides from the N-terminus of peptides having one or more amino acids at P1 selected from the group consisting of: lysine, arginine and glycine.

5. A method according to claim 1, or 3-4, wherein step (a) and step (b) are carried out simultaneously.

6. A method according to any one of the preceding claims wherein: the at least one endoprotease and at least one proline tolerant tripeptidyl peptidase are active at a similar pH range; and/or
the endoprotease is an acid endoprotease; and/or
the at least one endoprotease is an alkaline endoprotease, preferably selected from one or more of: a trypsin or a chymotrypsin; and/or
the hydrolysate has a reduced immunogenicity in a subject predisposed to having an immune response to the at least one protein or portion thereof.

7. A method according to any one of claims 1 or 3-6 wherein the at least one protein is an animal protein or a plant protein, preferably wherein the protein is one or more of a gliadin, a beta-casein, a beta-lactoglobulin, an immunogenic fragment of a gliadin, whey protein, fish protein, meat protein, egg protein, soy protein, a hordein or grain protein.

8. Use of at least one endoprotease and at least one proline tolerant tripeptidyl peptidase or fermentate comprising a proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
in the manufacture of a hydrolysate for reducing the immunogenicity in a subject predisposed to having an immune reaction to an untreated hydrolysate or for reducing bitterness of the hydrolysate, wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

9. The method according to any one of claims 1 or 3-7 or a use according to claim 8 which further comprises the addition of one or more further protease(s) selected from the group consisting of: a carboxypeptidase and an aminopeptidase, preferably an aminopeptidase obtainable from *Lactobacillus,* more preferably *Lactobacillus helveticus.*

10. The method according to any one of claims 1-7 or 9 or a use according to claims 8-9 wherein the at least one protein or a portion thereof is admixed with the at least one endoprotease before adding the at least one proline tolerant tripeptidyl peptidase (and optionally the at least one further protease).

11. A hydrolysate comprising at least one endoprotease and a proline tolerant tripeptidyl peptidase predominantly having exopeptidase activity wherein said proline tolerant tripeptidyl peptidase is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1',
wherein the at least one proline tolerant tripeptidyl peptidase:
(a) comprises the amino acid sequenceSEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

12. The hydrolysate according to claim 11 wherein: the hydrolysate is substantially enriched in one or more tripeptides; and/or
the hydrolysate is a milk protein hydrolysate, a gliadin hydrolysate or a soy protein hydrolysate.

13. A composition comprising at least one endoprotease and at least one proline tolerant tripeptidyl peptidase predominantly having exopeptidase activity wherein said proline tolerant tripeptidyl peptidase is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
and one or more ingredients selected from the group consisting of: polyols, such as glycerol and/or sorbitol; sugars, such as glucose, fructose, sucrose, maltose, lactose and trehalose; salts, such as NaCl, KCl, CaCl₂, Na₂SO₄ or other food grade salts; a preservative, e.g. sodium benzoate and/or potassium sorbate; or combinations thereof,
wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

14. A method for producing a feedstuff or foodstuff comprising contacting a feed component or food component with a hydrolysate according to any one of claims 11 to 12 or a composition according to claim 13.

15. A food additive composition or feed additive composition comprising at least one endoprotease and at least one proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
or a hydrolysate according to any one of claims 12 to 13 optionally further comprising one or more ingredients selected from the group consisting of: polyols, such as glycerol and/or sorbitol; sugars, such as glucose, fructose, sucrose, maltose, lactose and trehalose; salts, such as NaCl, KCl, CaCl₂, Na₂SO₄ or other food grade salts; a preservative, e.g. sodium benzoate and/or potassium sorbate; or combinations thereof,
wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

16. A food additive or feed additive composition comprising a hydrolysate according to any one of claims 11 or 12.

17. A foodstuff or feedstuff comprising: at least one endoprotease and at least one proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
wherein the at least one proline tolerant tripeptidyl peptidase:
comprises an amino acid sequence selected from SEQ ID No. 3, SEQ ID No. 30 or a functional fragment thereof that retains said peptidase activity or an amino acid sequence having at least 80% identity thereto that retains said peptidase activity;
or a hydrolysate according to any one of claims 12 to 13 and optionally at least one food or feed ingredient;
preferably wherein the feed, feedstuff, foodstuff or food is a dairy product, (preferably a milk-based product), a whey-protein product, a bakery product (preferably a bread product), a fermentation product (preferably a soy-based fermentation product), a sports nutrition product, a performance food, a beverage, a baby food, a food for elderly, a food for people in medical care, a shake, or a casing (preferably, a casing for beer or dairy).

18. A kit comprising at least one proline tolerant tripeptidyl peptidase wherein said proline tolerant tripeptidyl peptidase predominantly has exopeptidase activity and is capable of cleaving tri-peptides from the N-terminus of peptides having:
Proline at P1'; and
an amino acid selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine, valine or synthetic amino acids at P1';
at least one endoprotease; and instructions for co-administering same, wherein the at least one proline tolerant tripeptidyl peptidase:
(a) comprises the amino acid sequence SEQ ID No. 3, SEQ ID No. 30 or a functional fragment thereof that retains said peptidase activity;
(b) comprises an amino acid sequence having at least 80% identity to SEQ ID No. 3, SEQ ID No. 30 or a functional fragment thereof that retains said peptidase activity;
(c) is encoded by a nucleotide sequence comprising the sequence SEQ ID No. 57;
(d) is encoded by a nucleotide sequence comprising at least 80% sequence identity to SEQ ID No. 57;
(e) is encoded by a nucleotide sequence which hybridises to SEQ ID No. 57 under high stringency conditions;
(f) is encoded by a nucleotide sequence which differs from SEQ ID No. 57 due to degeneracy of the genetic code; or
(g) is encoded by a nucleotide sequence comprising SEQ ID No. 57 or a nucleotide sequence having at least 90% identity thereto or a sequence which hybridises to SEQ ID No. 57 under high stringency conditions.

19. A nonfood product comprising the hydrolysate of according to any one of claims 11-12, wherein the nonfood product is a cosmetic, a lotion, or a cleanser for use on human skin.

## Patentansprüche

1. Verfahren zur Herstellung eines Hydrolysats, umfassend:
(a) Mischen mindestens eines Proteins oder eines Abschnitts davon mit:
(A) mindestens einer Endoprotease; und
(B) mindestens einer prolintoleranten Tripeptidylpeptidase mit Exopeptidase-Aktivität, wobei die prolintolerante Tripeptidylpeptidase in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1'; und
(b) Gewinnen des Hydrolysats,
wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

2. Verfahren nach Anspruch 1, wobei das mindestens eine Protein ist: (a) ausgewählt aus der Gruppe, bestehend aus: einem pflanzlichen Protein, vorzugsweise wobei das Protein aus einem oder mehreren von einem Gliadin, einem immunogenen Fragment eines Gliadins, einem Getreideprotein, Gluten, einem Sojaprotein besteht; und/oder (b) ausgewählt aus der Gruppe, bestehend aus: einem Protein auf Milchbasis, vorzugsweise wobei das Protein aus einem oder mehreren von einem Kasein, z. B. Beta-Kasein; einem Laktoglobulin, z. B. Beta-Laktoglobulin; oder einem Molkenprotein besteht; und/oder (c) ein Eiprotein.

3. Verfahren nach Anspruch 1 oder Anspruch 2, wobei: die prolintolerante Tripeptidylpeptidase weiterhin in der Lage ist, Tripeptide vom N-Terminus eines Peptids zu spalten, das Prolin an P1 und Prolin an P1' aufweist; und/oder
das Verfahren weiterhin Mischen des gewonnenen Hydrolysats mit mindestens einem Futter- oder Lebensmittelinhaltsstoff umfasst.

4. Verfahren nach einem der vorstehenden Ansprüche, wobei die mindestens eine Tripeptidylpeptidase in der Lage ist, Tripeptide vom N-Terminus von Peptiden zu spalten, die eine oder mehrere Aminosäuren an P1 aufweisen, die aus der Gruppe ausgewählt sind, die besteht aus: Lysin, Arginin und Glycin.

5. Verfahren nach Anspruch 1, oder 3-4, wobei Schritt (a) und Schritt (b) gleichzeitig ausgeführt werden.

6. Verfahren nach einem der vorstehenden Ansprüche, wobei: die mindestens eine Endoprotease und mindestens eine prolintolerante Tripeptidylpeptidase bei einem ähnlichen pH-Wert-Bereich aktiv sind; und/oder
die Endoprotease eine saure Endoprotease ist; und/oder
die mindestens eine Endoprotease eine alkalische Endoprotease ist, vorzugsweise ausgewählt aus einem oder mehreren von: einem Trypsin oder einem Chymotrypsin; und/oder
das Hydrolysat eine reduzierte Immunogenität bei einem Individuum aufweist, das dafür prädisponiert ist, eine Immunantwort auf das mindestens eine Protein oder einen Abschnitt davon zu haben.

7. Verfahren nach einem der Ansprüche 1 oder 3-6, wobei das mindestens eine Protein ein tierisches Protein oder ein pflanzliches Protein ist, vorzugsweise wobei das Protein aus einem oder mehreren von einem Gliadin, einem Beta-Kasein, einem Beta-Laktoglobulin, einem immunogen Fragment eines Gliadins, Molkenprotein, Fischprotein, Fleischprotein, Eiprotein, Sojaprotein, einem Hordein oder Getreideprotein besteht.

8. Verwendung von mindestens einer Endoprotease und mindestens einer prolintoleranten Tripeptidylpeptidase oder einem eine prolintolerante Tripeptidylpeptidase umfassenden Fermentat, wobei die prolintolerante Tripeptidylpeptidase vorrangig Exopeptidase-Aktivität aufweist und in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
in der Herstellung eines Hydrolysats zur Reduzierung der Immunogenität bei einem Individuum, das dafür prädisponiert ist, eine Immunantwort auf ein unbehandeltes Hydrolysat zu haben, oder zur Reduzierung der Bitterkeit des Hydrolysats, wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

9. Verfahren nach einem der Ansprüche 1 oder 3-7 oder Verwendung nach Anspruch 8, das bzw. die weiterhin die Zugabe einer oder mehrerer weiterer Proteasen umfasst, die aus der Gruppe ausgewählt sind, die besteht aus: einer Carboxypeptidase und einer Aminopeptidase, bevorzugt einer Aminopeptidase, erhaltbar von *Lactobacillus,* stärker bevorzugt *Lactobacillus helveticus.*

10. Verfahren nach einem der Ansprüche 1-7 oder 9 oder Verwendung nach Ansprüchen 8-9, wobei das mindestens eine Protein oder ein Abschnitt davon mit der mindestens einen Endoprotease vermischt wird, bevor die mindestens eine prolintolerante Tripeptidylpeptidase (und wahlweise die mindestens eine weitere Protease) zugegeben wird.

11. Hydrolysat, umfassend mindestens eine Endoprotease und eine prolintolerante Tripeptidylpeptidase, die vorrangig Exopeptidase-Aktivität aufweist, wobei die prolintolerante Tripeptidylpeptidase in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

12. Hydrolysat nach Anspruch 11, wobei: das Hydrolysat mit einem oder mehreren Tripeptiden wesentlich angereichert ist; und/oder
das Hydrolysat ein Milchprotein-Hydrolysat, ein Gliadin-Hydrolysat oder ein Sojaprotein-Hydrolysat ist.

13. Zusammensetzung, umfassend mindestens eine Endoprotease und mindestens eine prolintolerante Tripeptidylpeptidase, die vorrangig Exopeptidase-Aktivität aufweist, wobei die prolintolerante Tripeptidylpeptidase in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
und einen oder mehrere Inhaltsstoffe, ausgewählt aus der Gruppe, bestehend aus: Polyolen, wie Glycerin und/oder Sorbit; Zuckern, wie Glukose, Fruktose, Saccharose, Maltose, Laktose und Trehalose; Salzen, wie NaCl, KCl, CaCl₂, Na₂SO₄ oder sonstigen Salzen von Lebensmittelgüte; einem Konservierungsstoff, z. B. Natriumbenzoat und/oder Kaliumsorbat; oder Kombinationen davon,
wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

14. Verfahren zur Herstellung eines Futtermittels oder Nahrungsmittels, umfassend In-Kontakt-Bringen einer Futterkomponente oder Nahrungskomponente mit einem Hydrolysat nach einem der Ansprüche 11 bis 12 oder einer Zusammensetzung nach Anspruch 13.

15. Lebensmitteladditivzusammensetzung oder Futteradditivzusammensetzung, umfassend mindestens eine Endoprotease und mindestens eine prolintolerante Tripeptidylpeptidase, wobei die prolintolerante Tripeptidylpeptidase vorrangig Exopeptidase-Aktivität aufweist und in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
oder ein Hydrolysat nach einem der Ansprüche 12 bis 13, wahlweise weiterhin umfassend einen oder mehrere Inhaltsstoffe, ausgewählt aus der Gruppe, bestehend aus: Polyolen, wie Glycerin und/oder Sorbit; Zuckern, wie Glukose, Fruktose, Saccharose, Maltose, Laktose und Trehalose; Salzen, wie NaCl, KCl, CaCl₂, Na₂SO₄ oder sonstigen Salzen von Lebensmittelgüte; einem Konservierungsstoff, z. B. Natriumbenzoat und/oder Kaliumsorbat; oder Kombinationen davon,
wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

16. Lebensmitteladditiv- oder Futteradditivzusammensetzung, umfassend ein Hydrolysat nach einem der Ansprüche 11 oder 12.

17. Nahrungsmittel oder Futtermittel, umfassend: mindestens eine Endoprotease und mindestens eine prolintolerante Tripeptidylpeptidase, wobei die prolintolerante Tripeptidylpeptidase Exopeptidase-Aktivität aufweist und in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
wobei die mindestens eine prolintolerante Tripeptidylpeptidase: eine Aminosäuresequenz, ausgewählt aus SEQ ID Nr. 3, SEQ ID Nr. 30, oder ein funktionelles Fragment davon, das die Peptidase-Aktivität behält, oder eine Aminosäuresequenz mit mindestens 80% Identität damit, das die Peptidase-Aktivität behält, umfasst;
oder ein Hydrolysat nach einem der Ansprüche 12 bis 13 und wahlweise mindestens einen Lebensmittel- oder Futterinhaltsstoff;
vorzugsweise wobei das Futter, Futtermittel, Nahrungsmittel oder Lebensmittel ein Molkereiprodukt (bevorzugt ein Produkt auf Milchbasis), ein Molkenproteinprodukt, ein Bäckereiprodukt (bevorzugt ein Brotprodukt), ein Fermentationsprodukt (bevorzugt ein Fermentationsprodukt auf Sojabasis), ein Sporternährungsprodukt, ein leistungssteigerndes Lebensmittel, ein Getränk, eine Säuglingsnahrung, ein Lebensmittel für Senioren, ein Lebensmittel für Menschen in medizinischer Betreuung, ein Shake oder eine Umhüllung (bevorzugt eine Umhüllung für Bier oder Molkereiprodukte) ist.

18. Kit, umfassend mindestens eine prolintolerante Tripeptidylpeptidase, wobei die prolintolerante Tripeptidylpeptidase vorrangig Exopeptidase-Aktivität aufweist und in der Lage ist, Tripeptide vom N-Terminus von Peptiden zu spalten, die aufweisen:
Prolin an P1'; und
eine Aminosäure, ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin oder synthetischen Aminosäuren, an P1';
mindestens eine Endoprotease; und Anweisungen für die Koadministration derselben, wobei die mindestens eine prolintolerante Tripeptidylpeptidase:
(a) die Aminosäuresequenz SEQ ID Nr. 3, SEQ ID Nr. 30 oder ein funktionelles Fragment davon umfasst, das die Peptidase-Aktivität behält;
(b) eine Aminosäure umfasst, die mindestens 80% Identität mit SEQ ID Nr. 3, SEQ ID Nr. 30 oder einem funktionellen Fragment davon aufweist, das die Peptidase-Aktivität behält;
(c) durch eine Nukleotidsequenz codiert ist, die die Sequenz SEQ ID Nr. 57 umfasst;
(d) durch eine Nukleotidsequenz codiert ist, die mindestens 80% Sequenzidentität mit SEQ ID Nr. 57 umfasst;
(e) durch eine Nukleotidsequenz codiert ist, die an SEQ ID Nr. 57 unter Bedingungen hoher Stringenz hybridisiert;
(f) durch eine Nukleotidsequenz codiert ist, die sich von SEQ ID Nr. 57 aufgrund der Degeneration des genetischen Codes unterscheidet; oder
(g) durch eine Nukleotidsequenz codiert ist, die SEQ ID Nr. 57 umfasst, oder eine Nukleotidsequenz, die mindestens 90% Identität damit aufweist, oder eine Sequenz, die an SEQ ID Nr. 57 unter Bedingungen hoher Stringenz hybridisiert.

19. Nichtlebensmittelprodukt, umfassend das Hydrolysat nach einem der Ansprüche 11-12, wobei das Nichtlebensmittelprodukt ein Kosmetikum, eine Lotion oder ein Reinigungsmittel zur Verwendung auf der menschlichen Haut ist.

## Revendications

1. Procédé de production d'un hydrolysat, comprenant:
(a) ajouter à/mélanger au moins une protéine ou une partie de celle-ci avec:
(A) au moins une endoprotéase; et
(B) au moins une tripeptidyl-peptidase tolérant la proline ayant une activité d'exopeptidase où ladite tripeptidyl-peptidase tolérant la proline est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1'; et
(b) recueillir l'hydrolysat,
où la au moins une tripeptidyl-peptidase tolérant la proline:
(a) comprend la séquence d'acides aminés SEQ ID no 3, SEQ ID no 30 ou un fragment fonctionnel de celle-ci qui conserve son activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

2. Procédé selon la revendication 1, dans lequel la au moins une protéine est: (a) sélectionnée parmi le groupe consistant en: une protéine végétale, de préférence où la protéine est l'un ou plusieurs d'entre une gliadine, un fragment immunogène d'une gliadine, une protéine de graine, du gluten, une protéine de soja; et/ou (b) est sélectionnée parmi le groupe consistant en: une protéine à base de lait, de préférence où la protéine est l'une ou plusieurs d'entre une caséine, par ex. une bêta-caséine; une lactoglobuline, par ex. une bêta-lactoglobuline; ou une protéine de lactosérum; et/ou (c) est une protéine d'œuf.

3. Procédé selon la revendication 1 ou la revendication 2, dans lequel: la tripeptidyl-peptidase tolérant la proline est en outre capable de cliver des tripeptides de la terminaison N d'un peptide ayant de la proline à P1 et de la proline à P1'; et/ou
le procédé comprend en outre ajouter à/mélanger l'hydrolysat recueilli avec au moins un ingrédient d'alimentation pour animaux ou d'alimentation humaine.

4. Procédé selon l'une quelconque des revendications précédentes, dans lequel ladite au moins une tripeptidyl-peptidase est capable de cliver des tripeptides de la terminaison N de peptides ayant un ou plusieurs acides aminés à P1 sélectionnés parmi le groupe consistant en: lysine, arginine et glycine.

5. Procédé selon la revendication 1, ou 3-4, dans lequel l'étape (a) et l'étape (b) sont effectuées simultanément.

6. Procédé selon l'une quelconque des revendications précédentes dans lequel: la au moins une endoprotéase et au moins une tripeptidyl-peptidase tolérant la proline sont actives à une plage de pH semblable; et/ou
l'endoprotéase est une endoprotéase acide; et/ou
la au moins une endoprotéase est une endoprotéase alcaline, sélectionnée de préférence parmi l'une ou plusieurs d'entre: une trypsine ou une chymotrypsine; et/ou
l'hydrolysat a une immunogénicité réduite chez un sujet prédisposé à présenter une réponse immune à la au moins une protéine ou partie de celle-ci.

7. Procédé selon l'une quelconque des revendications 1 ou 3-6, dans lequel la au moins une protéine est une protéine animale ou une protéine végétale, de préférence où la protéine est l'un ou plusieurs d'entre une gliadine, une bêta-caséine, une bêta-lactoglobuline, un fragment immunogène d'une gliadine, une protéine de lactosérum, une protéine de poisson, une protéine de viande, une protéine d'œuf, une protéine de soja, une hordéine ou une protéine de graine.

8. Utilisation d'au moins une endoprotéase et d'au moins une tripeptidyl-peptidase tolérant la proline ou d'un produit de fermentation comprenant une tripeptidyl-peptidase tolérant la proline où ladite tripeptidyl-peptidase tolérant la proline a de manière prédominante une activité d'exopeptidase et est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1';
dans la fabrication d'un hydrolysat pour réduire l'immogénicité chez un sujet prédisposé à présenter une réaction immune à un hydrolysat non traité ou pour réduire l'amertume de l'hydrolysat, où la au moins une tripeptidyl-peptidase tolérant la proline:
(a) comprend la séquence d'acides aminés de la SEQ ID no 3, SEQ ID no 30 ou un fragment fonctionnel de celle-ci qui conserve son activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

9. Procédé selon l'une quelconque des revendications 1 ou 3-7, ou utilisation selon la revendication 8, qui comprend en outre l'addition d'une ou de plusieurs protéases supplémentaires sélectionnées parmi le groupe consistant en: une carboxypeptidase et une aminopeptidase, de préférence une aminopeptidase pouvant être obtenue de *Lactobacillus,* plus préférablement de *Lactobacillus helveticus.*

10. Procédé selon l'une quelconque des revendications 1-7 ou 9, ou utilisation selon les revendications 8-9, où la au moins une protéine ou une partie de celle-ci est mélangée avec la au moins une endoprotéase avant d'ajouter la au moins une tripeptidyl-peptidase tolérant la proline (et optionnellement la au moins une protéase supplémentaire).

11. Hydrolysat comprenant au moins une endoprotéase et une tripeptidyl-peptidase tolérant la proline ayant de manière prédominante une activité d'exopeptidase où ladite tripeptidyl-peptidase tolérant la proline est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1';
dans lequel la au moins une tripeptidyl-peptidase tolérant la proline:
(a) comprend la séquence d'acides aminés de la SEQ ID no 3, SEQ ID no 30 ou un fragment fonctionnel de celle-ci qui conserve son activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

12. Hydrolysat selon la revendication 11, où: l'hydrolysat est sensiblement enrichi d'un ou de plusieurs tripeptides; et/ou
l'hydrolysat est un hydrolysat de protéine de lait, un hydrolysat de gliadine ou un hydrolysat de protéine de soja.

13. Composition comprenant au moins une endoprotéase et au moins une tripeptidyl-peptidase tolérant la proline ayant de manière prédominante une activité d'exopeptidase où ladite tripeptidyl-peptidase tolérant la proline est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, de la tryptophane, de la tyrosine, de la valine ou des acides aminés synthétiques à P1';
et un ou plusieurs ingrédients sélectionnés parmi le groupe consistant en: polyols, tels que glycérol et/ou sorbitol; sucres, tels que glucose, fructose, saccharose, maltose, lactose et tréhalose; sels, tels que NaCl, KCl, CaCl₂, Na₂SO₄ ou d'autres sels de qualité alimentaire; un conservateur, par ex. du benzoate de sodium et/ou du sorbate de potassium; ou des combinaisons de ceux-ci,
dans laquelle la au moins une tripeptidyl-peptidase tolérant la proline:
(a) comprend la séquence d'acides aminés de la SEQ ID no 3, SEQ ID no 30 ou un fragment fonctionnel de celle-ci qui conserve son activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

14. Procédé de production d'un produit d'alimentation pour animaux ou d'un produit d'alimentation humaine comprenant mettre un composant d'alimentation pour animaux ou un composant d'alimentation humaine en contact avec un hydrolysat selon l'une quelconque des revendications 11 à 12 ou une composition selon la revendication 13.

15. Composition d'additif alimentaire ou composition d'additif alimentaire pour animaux comprenant au moins une endoprotéase et au moins une tripeptidyl-peptidase tolérant la proline, où ladite tripeptidyl-peptidase tolérant la proline ayant de manière prédominante une activité d'exopeptidase est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1';
ou un hydrolysat selon l'une quelconque des revendications 12 à 13, comprenant optionnellement en outre un ou plusieurs ingrédients sélectionnés parmi le groupe consistant en: polyols, tels que glycérol et/ou sorbitol; sucres, tels que glucose, fructose, saccharose, maltose, lactose et tréhalose; sels, tels que NaCl, KCl, CaCl₂, Na₂SO₄ ou d'autres sels de qualité alimentaire; un conservateur, par ex. du benzoate de sodium et/ou du sorbate de potassium; ou des combinaisons de ceux-ci,
où la au moins une tripeptidyl-peptidase tolérant la proline:
(a) comprend la séquence d'acides aminés SEQ ID no 3, SEQ ID no 30 ou un fragment fonctionnel de celle-ci qui conserve son activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

16. Composition d'additif d'alimentation humaine ou d'additif d'alimentation pour animaux comprenant un hydrolysat selon l'une quelconque des revendications 11 ou 12.

17. Produit d'alimentation humaine ou produit d'alimentation pour animaux comprenant: au moins une endoprotéase et au moins une tripeptidyl-peptidase tolérant la proline, où ladite tripeptidyl-peptidase tolérant la proline a une activité d'exopeptidase et est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1';
où la au moins une tripeptidyl-peptidase tolérant la proline: comprend une séquence d'acides aminés sélectionnée parmi la SEQ ID no 3, la SEQ ID no 30 ou un fragment fonctionnel de celle-ci qui conserve ladite activité de peptidase ou une séquence d'acides aminés ayant au moins 80 % d'identité avec celle-ci et conserve ladite activité de peptidase;
ou un hydrolysat selon l'une quelconque des revendications 12 à 13 et optionnellement au moins un ingrédient d'alimentation humaine ou d'alimentation pour animaux;
de préférence où l'aliment pour animaux, le produit d'alimentation pour animaux, le produit d'alimentation humaine ou l'aliment est un produit laitier, (de préférence un produit à base de lait), un produit de protéine de lactosérum, un produit de boulangerie (de préférence un produit de pain), un produit de fermentation (de préférence un produit de fermentation à base de soja), un produit nutritif de sport, un aliment de performance, une boisson, un aliment pour bébés, un aliment pour personnes âgées, un aliment pour personnes sous soins médicaux, un shake ou un conditionnement (de préférence un conditionnement pour bière ou produit laitier).

18. Kit comprenant au moins une tripeptidyl-peptidase tolérant la proline où ladite tripeptidyl-peptidase tolérant la proline a de manière prédominante une activté d'exopeptidase et est capable de cliver des tripeptides de la terminaison N de peptides ayant:
de la proline à P1'; et
un acide aminé sélectionné parmi de l'alanine, de l'arginine, de l'asparagine, de l'acide aspartique, de la cystéine, de la glutamine, de l'acide glutamique, de la glycine, de l'histidine, de l'isoleucine, de la leucine, de la lysine, de la méthionine, de la phénylalanine, de la sérine, de la thréonine, du tryptophane, de la tyrosine, de la valine ou des acides aminés synthétiques à P1';
au moins une endoprotéase; et des instructions pour la co-administration des mêmes, où la au moins une tripeptidyl-peptidase tolérant la proline:
(a) comprend la séquence d'acides aminés SEQ ID no 3, SEQ ID no 30 ou un fragment fonctionnel de celle-ci qui conserve ladite activité de peptidase;
(b) comprend un acide aminé ayant au moins 80 % d'identité avec la SEQ ID no 3, SEQ ID no 30 ou un fragment fonctionnel de celui-ci qui conserve son activité de peptidase;
(c) est codée par une séquence de nucléotides comprenant la séquence SEQ ID no 57;
(d) est codée par une séquence de nucléotides comprenant au moins 80 % d'identité de séquence avec la SEQ ID no 57;
(e) est codée par une séquence de nucléotides qui s'hybride à la SEQ ID no 57 dans des conditions de stringence élevée;
(f) est codée par une séquence de nucléotides qui diffère de la SEQ ID no 57 du fait de la dégénérescence du code génétique; ou bien
(g) est codée par une séquence de nucléotides comprenant la SEQ ID no 57 ou une séquence de nucléotides ayant au moins 90 % d'identité avec celle-ci ou une séquence qui s'hybride à la SEQ ID no 57 dans des conditions de stringence élevée.

19. Produit non alimentaire comprenant l'hydrolysat selon l'une quelconque des revendications 11-12, où le produit non alimentaire est un cosmétique, une lotion ou un démaquillant pour la peau humaine.
